# EUROPEAN PATENT SPECIFICATION

(11) **EP 1 742 908 B1**
(45) Date of publication and mention of the grant of the patent: **11.11.2009**
(21) Application number: 05742851.8
(22) Date of filing: 15.04.2005
(51) Int. Cl.: C07C 235/38, A61K 31/167, A61P 31/18, C07C 235/78, C07C 255/54, C07C 255/57, C07C 311/08, C07C 311/46, C07C 311/51, C07C 317/32, C07C 317/40, C07C 317/44, C07C 323/49, C07C 323/62, C07D 207/32

(54) **NON-NUCLEOSIDE REVERSE TRANSCRIPTASE INHIBITORS**
INHIBITOREN DER NICHT-NUKLEOSID-INHIBITOREN DER REVERSEN TRANSKRIPTASE
INHIBITEURS DE LA TRANSCRIPTASE INVERSE NON NUCLEOSIDIQUES

(30) Priority: 23.04.2004 US 565116 P; 23.04.2004 US 565117 P
(43) Date of publication of application: 17.01.2007
(73) Proprietor: F.HOFFMANN-LA ROCHE AG, 4070 Basel (CH)
(72) Inventor: DUNN, James, Patrick, Los Altos, CA 94022 (US); HIRSCHFELD, Donald, Roy, Mountain View, CA 94041 (US); SILVA, Tania, Sunnyvale, CA 94086 (US); VORA, Harit, Fort Collins, CO 80521-3615 (US)
(74) Representative: Rauber, Beat
(86) International application number: PCT/EP2005/004048
(87) International publication number: WO 2005/102989

(56) References cited:
- WO-A-92/00952
- WO-A-03/075907
- J. MILTON, ET AL.: "Biaryl acids: novel non-nucleoside inhibitors of HIV reverse transcriptase types 1 and 2" BIOORGANIC & MEDICINAL CHEMISTRY LETTERS, vol. 8, no. 19, 6 October 1998 (1998-10-06), pages 2623-2628, XP004139590 ELSEVIER SCIENCE PUBLISHERS, OXFORD, GB ISSN: 0960-894X

## Description

The invention relates to the field of antiviral therapy and, in particular, to non-nucleoside compounds that inhibit HIV reverse transcriptase and are useful for treating Human Immunodeficiency Virus (HIV) mediated diseases. The invention provides novel N-phenyl phenylacetamide compounds according to formula I, for treatment or prophylaxis of HIV mediated diseases, AIDS or ARC, employing said compounds in monotherapy or in combination therapy.

The human immunodeficiency virus HIV is the causative agent of acquired immunodeficiency syndrome (AIDS), a disease characterized by the destruction of the immune system, particularly of the CD4⁺ T-cell, with attendant susceptibility to opportunistic infections. HIV infection is also associated with a precursor AIDs-related complex (ARC), a syndrome characterized by symptoms such as persistent generalized lymphadenopathy, fever and weight loss.

In common with other retroviruses, the HIV genome encodes protein precursors known as gag and gag-pol which are processed by the viral protease to afford the protease, reverse transcriptase (RT), endonuclease/integrase and mature structural proteins of the virus core. Interruption of this processing prevents the production of normally infectious virus. Considerable efforts have been directed towards the control of HIV by inhibition of virally encoded enzymes.

Currently available chemotherapy targets two crucial viral enzymes: HIV protease and HIV reverse transcriptase. (J. S. G. Montaner et al. Antiretroviral therapy: 'the state of the art', Biomed & Pharmacother. 1999 53:63- 72; R. W. Shafer and D. A. Vuitton, Highly active retroviral therapy (HAART) for the treatment of infection with human immunodeficiency virus type, Biomed. & Pharmacother. 1999 53 :73-86; E. De Clercq, New Developments in Anti-HIV Chemotherap. Curr. Med. Chem. 2001 8:1543-1572). Two general classes of RTI inhibitors have been identified: nucleoside reverse transcriptase inhibitors (NRTI) and non-nucleoside reverse transcriptase inhibitors.

NRTIs typically are 2',3'-dideoxynucleoside (ddN) analogs which must be phosphorylated prior to interacting with viral RT. The corresponding triphosphates function as competitive inhibitors or alternative substrates for viral RT. After incorporation into nucleic acids the nucleoside analogs terminate the chain elongation process. HIV reverse transcriptase has DNA editing capabilities which enable resistant strains to overcome the blockade by cleaving the nucleoside analog and continuing the elongation. Currently clinically used NRTIs include zidovudine (AZT), didanosine (ddI), zalcitabine (ddC), stavudine (d4T), lamivudine (3TC) and tenofovir (PMPA).

NNRTIs were first discovered in 1989. NNRTI are allosteric inhibitors which bind reversibly at a nonsubstrate-binding site on the HIV reverse transcriptase thereby altering the shape of the active site or blocking polymerase activity (R. W. Buckheit, Jr., Non-nucleoside reverse transcriptase inhibitors: perspectives for novel therapeutic compounds and strategies for treatment of HIV infection, Expert Opin. Investig. Drugs 2001 10(8):1423-1442; E. De Clercq The role of non-nucleoside reverse transcriptase inhibitors (NNRTIs) in the therapy of HIV infection, Antiviral Res. 1998 38:153-179; E. De Clercq New Developments in Anti-HIV Chemotherapy, Current Medicinal Chem. 2001 8(13):1543-1572; G. Moyle, The Emerging Roles of Non-Nucleoside Reverse Transcriptase Inhibitors in Antiviral Therapy, Drugs 2001 61 (1):19-26). Although over thirty structural classes of NNRTIs have been identified in the laboratory, only three compounds have been approved for HIV therapy: efavirenz, nevirapine and delavirdine.

Initially viewed as a promising class of compounds, *in vitro* and *in vivo* studies quickly revealed the NNRTIs presented a low barrier to the emergence of drug resistant HIV strains and class-specific toxicity. Drug resistance frequently develops with only a single point mutation in the RT. While combination therapy with NRTIs, PIs and NNRTIs has, in many cases, dramatically lowered viral loads and slowed disease progression, significant therapeutic problems remain. (R M. Gulick, Eur. Soc. Clin. Microbiol. and Inf. Dis. 2003 9(3):186-193) The cocktails are not effective in all patients, potentially severe adverse reactions often occur and the rapidly reproducing HIV virus has proven adroit at creating mutant drug-resistant variants of wild type protease and reverse transcriptase. There remains a need for safer drugs with activity against wild type and commonly occurring resistant strains of HIV.

Certain N-phenyl phenylacetamide compounds have been found to have a variety of pharmacological properties.

US 20030187068 (H. Miyachi et al.) discloses N-phenyl phenylacetamide compounds which are peroxisome proliferators-activated receptor (PPARα) ligands.

US 20030220241 (D. Defoe-Jones et al.) disclose N-phenyl phenylacetamide compounds use to prepare protein conjugates with a prenyl protein transferase which are cleaved by prostate-specific antigen and are useful for treating cancer. WO9917777 (J. S. Desolms et al.) teach prenyl protein transferase compounds which include N-phenyl phenylacetamides.

N-(substituted)phenyl 3-phenoxy-phenylacetamide compounds have been disclosed in WO01/21596 (A. A. Mortlock et al.) as inhibitors of aurora 2 kinase which are potentially useful in the treatment of proliferative diseases.

N-phenyl 3-(substituted)phenoxy-phenylacetamide compounds have be disclosed in WO2000059930 as inhibitors of prenyl protein transferase.

N-(substituted)phenyl3-phenoxy-phenylacetamide compounds have been disclosed in US2003/0114435 (K. Tani et al.) as EP4 receptor antagonists which are potentially useful in the suppression of TNF-α production and induction of IL-10 production.

Benzanilide compounds have been disclosed in WO9965874 (Y. Ohtake et al.) as vasopressin antagonists.

N-phenyl phenylacetamide compounds 1 wherein R¹ can be substituted aryl, X can be O, n can be 0, R⁴ and R⁵ can be hydrogen have been disclosed in WO9315043 (T. Oe et al.) as acetyl CoA cholesterol O-acyltransferase inhibitors useful for reducing blood lipid levels and for treating arteriosclerosis.

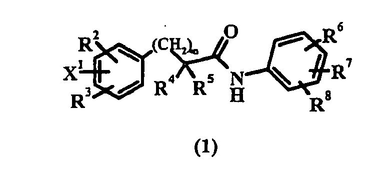

N-Phenyl phenylacetamides have also been used as synthetic intermediates for the preparation of pharmacologically active compounds. N-(2-carboalkoxy-5-chloro-phenyl) phenylacetamides (A. Kreimeyer et al., J. Med. Chem. 1999 42:4394-4404; J. J. Kulagowski et al., J. Med. Chem. 1994 37:1402-1405 K. Ackermann et al., WO 97/26244), N-(2-cyano-5-chloro-phenyl) phenylacetamides (M. Rowley et al., J. Med. Chem. 1997 40:4053-4068; R. W. Carling et al., J. Med. Chem., 1997 40:754-765 and N-(2-nitrophenyl) phenylacetamides (J. F. W. Keana et al., WO 96/22990) have been disclosed and utilized as intermediates for the synthesis of ligands for the glycine site on the N-methyl-D-aspartate (NMDA) receptor. NMDA ligands have been investigated for treating CNS disorders thought to be related neuronal death caused by over-stimulation of the post synaptic receptor sensitive to N-methyl-D-aspartic acid. Such disorders include Alzheimer's disease, epilepsy and cerebral ischemia. These compounds and indications are unrelated to the present invention.

2-Benzoyl phenyl-N-[phenyl]-acetamide compounds **2a** and **2b** have been shown to inhibit HIV-1 reverse transcriptase (P. G. Wyatt et al., J. Med. Chem. 1995 38(10):1657-1665). Further screening identified related compounds, *e.g*. 2-benzoyl phenyloxy-N-[phenyl]-acetamide, **3a**, and a sulfonamide derivative **3b** which also inhibited reverse transcriptase (J. H. Chan et al., J. Med Chem. 2004 47(5):1175-1182; C. L. Webster et al., WO01/17982).

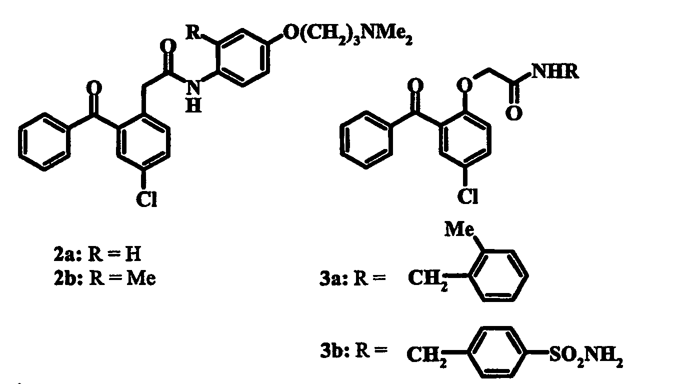

α-Anilinophenylacetamide and α-anilinophenylthioacetamide derivatives and their use in the treatment of HIV, AIDS and AIDS-related complex (ARC) are disclosed in the international patent application no. WO92/00952.

One object of present invention is: (i) Use of compounds of formula I 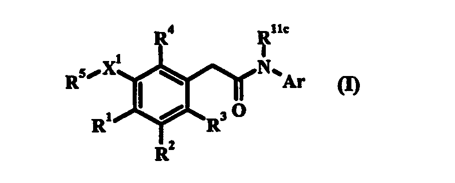 wherein
- X¹: is selected from the group consisting of -O-, -S -, -CH₂-, -C(O)-;
- R¹ and R²: are each independently selected from the group consisting of hydrogen, C₁₋₆ alkyl, C₁₋₆ haloalkyl, C₃₋₈ cycloalkyl, C₁₋₆ alkoxy, C₁₋₆ alkylthio, C₁₋₆ alkylsulfinyl, C₁₋₆ sulfonyl, C₁₋₆ haloalkoxy, C₁₋₆ haloalkylthio, halogen, amino, alkylamino, dialkylamino, acylamino, nitro and cyano; or together R¹ and R² are -O-CH=CH- or -O-CH₂CH₂-;
- R³ and R⁴: are independently selected from the group consisting of hydrogen, C₁₋₆ alkyl, C₁₋₆ haloalkyl, C₁₋₆ alkoxy, C₁₋₆ haloalkoxy C₁₋₆ alkylthio, C₁₋₆ haloalkylthio, halogen, amino, nitro and cyano;
- R⁵: is selected from the group consisting of C₁₋₆ alkyl, C₁₋₆ haloalkyl, C₃₋₈ cycloalkyl, aryl or heteroaryl selected from the group consisting of pyridinyl, N-hydroxypyridine, pyrimidinyl, indole, pyrazinyl and pyrrolyl; wherein, said aryl and said heteroaryl are optionally substituted with one to three substituents independently selected from the group consisting of C₁₋₆ alkyl, C₂₋₆ alkenyl, C₂₋₆ alkynyl, C₁₋₆ haloalkyl, C₃₋₈ cycloalkyl, C₁₋₆ alkoxy, C₁₋₆ alkylthio, C₁₋₆ alkylsulfinyl, C₁₋₆ sulfonyl, C₁₋₆ haloalkoxy, C₁₋₆ haloalkylthio, hydroxy, halogen, amino, C₁₋₆ alkylamino, C₁₋₆ dialkylamino, acylamino, acyl, C₁₋₆ alkoxycarbonyl, carbamoyl, C₁₋₆ N-alkylcarbamoyl, C₁₋₆ N,N-dialkylcarbamoyl, alkynol, -C≡CCH₂OH, -C≡CCH₂N(CH₃)₂, nitro and cyano, said alkyl and said cycloalkyl are optionally substituted with one or two substituents independently selected from the group consisting of alkyl, hydroxy, alkoxy, thiol, alkylthio, halogen, amino, alkylamino, amino alkyl, alkylaminoalkyl, and dialkylamino;
- Ar: is phenyl optionally substituted with 1 to 3 substituents independently selected in each incidence is from the group consisting of C₁₋₆ alkyl, C₃₋₈ cycloalkyl, C₁₋₃ haloalkyl, C₁₋₆ alkoxy, C₁₋₆ haloalkoxy, halogen, cyano, C₁₋₆ alkylthio, C₁₋₆ alkylsulfinyl, C₁₋₆ sulfonyl, amino C₁₋₆ alkylsulfonyl, C₁₋₆ haloalkoxy, C₁₋₆ haloalkylthio, C₁₋₆ acyl, nitro, C₁₋₆ heteroalkyl, C₁₋₆ heteroalkoxy, hydroxyl,-X²(CH₂)ₚS(O)ₙNR⁸R⁹; -(CH₂)ₚCOOR¹¹; -X²(CH₂)ₚNHC(O)NHR⁸R⁹, X²(CH₂)ₚCONR⁸R⁹,-SO₂R¹³, -NR^{8a}R^{9a}, X²(CH₂)ₚNR¹¹S(O)₂NR⁸R⁹,-X²(CH₂)pNHCOOR¹⁰, -X⁶(CH₂)ₚCOOR¹⁰, -X²(CH₂)ₚCN, -OR¹⁵ and C(=O)CH₂N[(CH₂)₂]₂X⁴; or,
heteroaryl ring selected from the group consisting of pyridinyl, pyrazolyl and triazolyl said heteroaryl ring optionally substituted with one to three substituents selected from the group consisting of C₁₋₆ alkyl, C₁₋₆ alkoxy, C₁₋₆ alkylthio, halogen, C₁₋₆ aminoacyl cyano and NR^{8b}R^{9b};
- R⁸ and R⁹: taken independently, one of R⁸ and R⁹ is hydrogen or C₁₋₆ alkyl and the other of R⁸ and R⁹ is selected from the group consisting of hydrogen, -C(=O)R¹⁴,-C(=O)CHR¹²NH₂, -(CH₂)₂N[(CH₂)₂]₂O, COCO₂Me, C₃₋₈ cycloalkyl said cycloalkyl optionally substituted with one or two hydroxyl substituents, pyranyl, C₁₋₆ alkyl and aryl said alkyl and said aryl groups optionally substituted with one or two substituents independently selected from the group consisting of hydroxy, C₁₋₆ alkoxy, thiol, C₁₋₆ alkylthio, C₁₋₆ alkylsulfinyl, C₁₋₆ sulfonyl, and halogen; or, (ii) R⁸ and R⁹ taken together are (CH₂)₂-X⁵-(CH₂)₂ or -(CH₂)ₒ- optionally substituted with one or two substituents selected from the group consisting of halogen, hydroxyl and NR^{11a}R^{11b};
- R^{8a} and R^{9a}: taken independently are selected from the group consisting of hydrogen, C₁₋₆ alkyl, C(=O)CO₂R¹¹ and SO₂R¹⁰, or (ii) taken together are (CH₂)ᵣSO₂, (CH₂)₂S(O)ₚ(CH₂)₂;
- R¹⁰: is C₁₋₆ alkyl;
- R¹¹: is hydrogen or C₁₋₆ alkyl;
- R^{11a} R^{11b} and R^{11c}: are independently R¹¹;
- R¹²: is the side chain of a naturally occurring α-amino acid;
- R¹³: is C₁₋₆ alkyl; -(CH₂)ₛCO₂R¹¹, -(CH₂)₂CN, -(CH₂)₂NH₂, -(CH₂)ᵤOH;
- R¹⁴: is C₁₋₆ alkyl, -(CH₂)ₛNHR^{11a}R^{11b}, (CH₂)ₛOR1¹¹, -CH₂CH(OH)CH₃, CH₂N[(CH₂)₂]₂O, -(CH₂)₂CO₂R¹¹, optionally substituted phenyl or pyridinyl;
- R¹⁵: is C₁₋₆ alkyl optionally substituted with one to three hydroxyl groups;
- X²: is -O- or a bond;
- X⁴: is -O- or -NMe-;
- X⁵: is -O-, -S(O)ₙ- or NR¹¹;
- X⁶: is O- or -S(O)ₙ-;
- n: is an integer from 0 to 2;
- o: is an integer from 4 to 6;
- p: is an integer from 0 to 6;
- r: is an integer from 3 to 4
- s: is an integer from 1 to 2;
- u: is an integer from 2 to 3; and,
hydrates, solvates and salts thereof;
for the manufacture of a medicament for treating an HIV infection, or preventing an HIV infection, or treating AIDS or ARC.

Further objects of the present invention are: (ii) Use of compounds of formula I according to (i) wherein
- X¹: is selected from the group consisting of -O-, -S -, -CH₂-, -C(O)-;
- R¹ and R²: are each independently selected from the group consisting of hydrogen, C₁₋₆ alkyl, C₁₋₆ haloalkyl, C₃₋₈ cycloalkyl, C₁₋₆ alkoxy, C₁₋₆ alkylthio, C₁₋₆ alkylsulfinyl, C₁₋₆ sulfonyl, C₁₋₆ haloalkoxy, C₁₋₆ haloalkylthio, halogen, amino, alkylamino, dialkylamino, acylamino, nitro and cyano; or together R¹ and R² are -O-CH=CH- or -0-CH₂CH₂-;
- R³ and R⁴: are independently selected from the group consisting of hydrogen, C₁₋₆ alkyl, C₁₋₆ haloalkyl, C₁₋₆ alkoxy, C₁₋₆ haloalkoxy, C₁₋₆ alkylthio, C₁₋₆ haloalkylthio, halogen, amino, nitro and cyano;
- R⁵: is selected from the group consisting of C₁₋₆ alkyl, C₁₋₆ haloalkyl, C₃₋₈ cycloalkyl, aryl or heteroaryl selected from the group consisting of pyridinyl, N-hydroxypyridine, pyrimidinyl, indole, pyrazinyl and pyrrolyl; wherein, said aryl and said heteroaryl are optionally substituted with one to three substituents independently selected from the group consisting of C₁₋₆ alkyl, C₂₋₆ alkenyl, C₂₋₆ alkynyl, C₁₋₆ haloalkyl, C₃₋₈ cycloalkyl, C₁₋₆ alkoxy, C₁₋₆ alkylthio, C₁₋₆ alkylsulfinyl, C₁₋₆ sulfonyl, C₁₋₆ haloalkoxy, C₁₋₆ haloalkylthio, hydroxy, halogen, amino, C₁₋₆ alkylamino, C₁₋₆ dialkylamino, acylamino, acyl, C₁₋₆ alkoxycarbonyl, carbamoyl, C₁₋₆ N-alkylcarbamoyl, C₁₋₆ N,N-dialkylcarbamoyl, nitro and cyano, said alkyl and said cycloalkyl are optionally substituted with one or two substituents independently selected from the group consisting of alkyl, hydroxy, alkoxy, thiol, alkylthio, halogen, amino, alkylamino, amino alkyl, akylaminoalkyl, and dialkylamino;
- Ar: is phenyl optionally substituted with 1 to 3 substituents independently selected in each incidence is from the group consisting of C₁₋₆ alkyl, C₃₋₈ cycloalkyl, C₁₋₃ haloalkyl, C₁₋₆ alkoxy, C₁₋₆ haloalkoxy, halogen, cyano, C₁₋₆ alkylthio, C₁₋₆ alkylsulfinyl, C₁₋₆ sulfonyl, amino C₁₋₆ alkylsulfonyl, C₁₋₆ haloalkoxy, C₁₋₆ haloalkylthio, C₁₋₆ acyl, nitro, C₁₋₆ heteroalkyl, C₁₋₆ heteroalkoxy, hydroxyl,-X²(CH₂)ₚS(O)ₙNR⁸R⁹; -(CH₂)ₚCOOR¹¹ ;-X₂(CH₂)ₚNHC(O)NHR⁸R⁹, X²(CH₂)ₚCONR⁸R⁹,-SO₂R¹³, -NR^{8a}R^{9a}, X²(CH₂)ₚNR¹¹S(O)₂NR⁸R⁹,-X²(CH₂)ₚNHCOOR¹⁰, -X⁶(CH₂)pCOOR¹⁰, -X²(CH₂)ₚCN, -OR¹⁵ and C(=O)CH₂N[(CH₂)₂]₂X⁴; or,
heteroaryl ring selected from the group consisting of pyridinyl, pyrazolyl and triazolyl said heteroaryl ring optionally substituted with one to three substituents selected from the group consisting of C₁₋₆ alkyl, C₁₋₆ alkoxy, C₁₋₆ alkylthio, halogen, C₁₋₆ aminoacyl and NR^{8b}R^{9b};
- R⁸ and R⁹: taken independently, one of R⁸ and R⁹ is hydrogen or C₁₋₆ alkyl and the other of R⁸ and R⁹ is selected from the group consisting of hydrogen, -C(=O)R¹⁴,-C(=O)CHR¹²NH₂, -(CH₂)₂N[(CH₂)₂]₂O, COCO₂Me, C₃₋₈ cycloalkyl said cycloalkyl optionally substituted with one or two hydroxyl substituents, pyranyl, C₁₋₆ alkyl and aryl said alkyl and said aryl groups optionally substituted with one or two substituents independently selected from the group consisting of hydroxy, C₁₋₆ alkoxy, thiol, C₁₋₆ alkylthio, C₁₋₆ alkylsulfinyl, C₁₋₆ sulfonyl, and halogen; or, (ii) R⁸ and R⁹ taken together are (CH₂)₂-X⁵-(CH₂)₂ or -(CH₂)ₒ- optionally substituted with one or two substituents selected from the group consisting of halogen, hydroxyl and NR^{11a}R^{11b};
- R^{8a} and R^{9a}: taken independently are selected from the group consisting of hydrogen, C₁₋₆ alkyl, C(=O)CO₂R¹¹ and SO₂R¹⁰, or (ii) taken together are (CH₂)ᵣSO₂, (CH₂)₂S(O)ₚ(CH₂)₂;
- R¹⁰: is C₁₋₆ alkyl;
- R¹¹: is hydrogen or C₁₋₆ alkyl;
- R^{11a}, R^{11b} and R^{11c}: are independently R¹¹;
- R¹²: is the side chain of a naturally occurring α-amino acid;
- R¹³: is C₁₋₆ alkyl; -(CH₂)ₛC0O₂R¹¹, -(CH₂)₂CN, -(CH₂)₂NH₂, -(CH₂)ᵤOH;
- R¹⁴: is C₁₋₆ alkyl, -(CH₂)ₛNHR^{11a}R^{11b}, (CH₂)ₛOR¹¹, -CH₂CH(OH)CH₃, CH₂N[(CH₂)₂]₂O, -(CH₂)₂CO₂R¹¹, optionally substituted phenyl or pyridinyl;
- R¹⁵: is C₁₋₆ alkyl optionally substituted with one to three hydroxyl groups;
- X²: is -O- or a bond;
- X⁴: is -O- or -NMe-;
- x⁵: is -O-, -S(O)ₙ- or NR¹¹;
- X⁶: is O- or -S(O)ₙ-;
- n: is an integer from 0 to 2;
- o: is an integer from 4 to 6;
- p: is an integer from 0 to 6;
- r: is an integer from 3 to 4
- s: is an integer from 1 to 2;
- u: is an integer from 2 to 3; and,
hydrates, solvates and salts thereof;
for the manufacture of a medicament for treating an HIV infection, or preventing an HIV infection, or treating AIDS or ARC.

(iii) Use of compounds of formula I according to (i) or (ii), wherein
- X¹: is selected from the group consisting of -O-, -S -, -CH₂-, -C(O)-;
- R¹ and R²: are each independently selected from the group consisting of hydrogen, C₁₋₆ alkyl, C₁₋₆ alkoxy and halogen; or together R¹ and R² are -O-CH=CH- or -O-CH₂CH₂-;
- R³ and R⁴: are independently selected from the group consisting of hydrogen and halogen;
- R⁵: is aryl or heteroaryl selected from the group consisting of pyridinyl, N-hydroxypyridine, pyrimidinyl, indole, pyrazinyl and pyrrolyl; wherein, said aryl and said heteroaryl are unsubstituted or substituted with one to three substituents independently selected from the group consisting of C₁₋₆ alkyl, C₁₋₆ haloalkyl, C₁₋₆ haloalkoxy, C₁₋₆ haloalkoxy, halogen, -C≡CCH₂OH, -C≡CCH₂N(CH₃)₂ and cyano.
- Ar: is phenyl optionally substituted with 1 to 3 substituents independently selected from the group consisting of C₁₋₆ alkyl, C₃₋₈ cycloalkyl, C₁₋₃ haloalkyl, C₁₋₆ alkoxy, halogen, cyano, C₁₋₆ alkylsulfinyl, C₁₋₆ sulfonyl, amino C₁₋₆ alkylsulfonyl, C₁₋₆ haloalkoxy, C₁₋₆ haloalkylthio, C₁₋₆ acyl, nitro, C₁₋₆ heteroalkyl, C₁₋₆ heteroalkoxy, hydroxyl, -X²(CH₂)ₚS(O)ₙNR⁸R⁹; -(CH₂)ₚCOOR¹¹, -X²(CH₂)ₚNHC(O)NHR⁸R⁹, X²(CH₂)pCONR⁸R⁹,-SO₂R¹³, -NR^{8a}R^{9a}, X²(CH₂)ₚNR¹¹S(O)₂NR⁸R⁹, -X²(CH₂)ₚNHCOOR¹⁰, -X⁶(CH₂)ₚCOOR¹⁰, -X²(CH₂)ₚCN, -OR¹⁵ and C(=O)CH₂N[(CH₂)₂]₂X⁴; or,
heteroaryl ring selected from the group consisting of pyridinyl, pyrazolyl and triazolyl said heteroaryl ring optionally substituted with one to three substituents selected from the group consisting of C₁₋₆ alkyl, C₁₋₆ alkoxy, C₁₋₆ alkylthio, halogen, C₁₋₆ aminoacyl, cyano and NR^{8b}R^{9b};
- R⁸ and R⁹: taken independently, one of R⁸ and R⁹ is hydrogen or C₁₋₆ alkyl and the other of R⁸ and R⁹ is selected from the group consisting of hydrogen, -C(=O)R¹⁴, -C(=O)CHR¹²NH₂, -(CH₂)₂N[(CH₂)₂]₂O, COCO₂Me, C₃₋₈ cycloalkyl said cycloalkyl optionally substituted with one or two hydroxyl substituents, pyranyl, C₁₋₆ alkyl and aryl said alkyl and said aryl groups optionally substituted with one or two substituents independently selected from the group consisting of hydroxy, C₁₋₆ alkoxy, thiol, C₁₋₆ alkylthio, C₁₋₆ alkylsulfinyl, C₁₋₆ sulfonyl, and halogen; or, (ii) R⁸ and R⁹ taken together are (CH₂)₂-X⁵-(CH₂)₂ or -(CH₂)ₒ- optionally substituted with one or two substituents selected from the group consisting of halogen, hydroxyl and NR^{11a}R^{11b};
- R^{8a} and R^{9a}: taken independently are selected from the group consisting of hydrogen, C₁₋₆ alkyl, C(=O)CO₂R¹¹ and SO₂R¹⁰, or (ii) taken together are (CH₂)ᵣSO₂, (CH₂)₂S(O)ₚ(CH₂)₂;
- R¹⁰: is C₁₋₆ alkyl;
- R¹¹: is hydrogen or C₁₋₆ alkyl;
- R^{11a}, R^{11b} and R^{11c}: are independently R¹¹;
- R¹²: is the side chain of a naturally occurring α-amino acid;
- R¹³: is C₁₋₆ alkyl; -(CH₂)ₛCO₂R¹¹, -(CH₂)₂CN, -(CH₂)₂NH₂, -(CH₂)ᵤOH;
- R¹⁴: is C₁₋₆alkyl, -(CH₂)ₛNHR^{11a}R^{11b}, (CH₂)ₛOR¹¹, -CH₂CH(OH)CH₃, CH₂N[(CH₂)₂]₂O, -(CH₂)₂CO₂R¹¹, optionally substituted phenyl or pyridinyl;
- R¹⁵: is C₁₋₆ alkyl optionally substituted with one to three hydroxyl groups;
- X²: is -O- or a bond;
- X⁴: is -O- or -NMe-;
- X⁵: is -O-, -S(O)ₙ or NR¹¹;
- X⁶: is O- or -S(O)ₙ-;
- n: is an integer from 0 to 2;
- o: is an integer from 4 to 6;
- p: is an integer from 0 to 6;
- r: is an integer from 3 to 4
- u: is an integer from 2 to 3; and,
hydrates, solvates and salts thereof.

(iv) Use of compounds of formula I according to (iii), wherein
- X¹: is selected from the group consisting of -O-, -S -, -CH₂-, -C(O)-;
- R¹ and R²: are each independently selected from the group consisting of hydrogen, methyl, ethyl, i-propyl, methoxy, chloro and bromo; or together R¹ and R² are -O-CH=CH- or -O-CH₂CH₂-;
- R³ and R⁴: are independently selected from the group consisting of hydrogen and fluoro;
- R⁵: is phenyl, wherein said phenyl is unsubstituted or substituted with one to three substituents independently selected from the group consisting of methyl, ethyl, CHF₂, CF₃, -CF₂CH₃, -OCHF₂, chloro, bromo, fluoro, -C≡CCH₂OH, -C≡CCH₂N(CH₃)₂ and cyano.
or pyridinyl, wherein said pyridinyl is unsubstituted or substituted with one to three substituents independently selected from the group consisting of methyl and cyano.
- Ar: is phenyl, wherein said phenyl is unsubstituted or substituted with 1 to 3 substituents selected from the group consisting of methyl, ethyl, i-propyl, 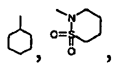 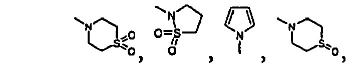 -CF₃, methoxy, chloro, fluoro, cyano, hydroxyl, -SCH₃, -SO₂NH₂, -SO₂NHCH₃, -SO₂NCH₃CH₂CH₂OH, -SO₂NHCH₂CHCH₃OH, -SO₂NHCH₂CH₂CH₂OH, -SO₂NHCH₂CH₂ 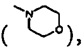 -SO₂ 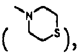 -SO₂N(CH₃)₂, -SO₂ 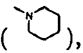 -SO₂ 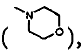 -SO₂ 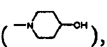 -SO₂NH 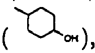 -SO₂ 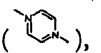 -SO₂ 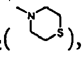 - SO₂NHCOCH₂CH₃, -SO₂NHCOCH₂CH₂CH₃, -SO₂NHCOCH(NH₂)CH(CH₃)₂, -SO₂NHCOCH₂ 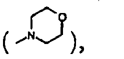 -SO₂NCH₃COCH₂CH₂CH₃, -SO₂NHCOCH₂CH₂CH₂CH₃, -SO₂NHCOCH₂CH(CH₃)₂, -SO₂NHCOCH₂CH(CH₂CH₃)₂, -SO₂NHCOCH₂CH₂CH(CH₃)₂, -SO₂NHCOCH₂CH₂NH₂, -SO₂NCH₃COCH₂CH₂NH₂, -SO₂NHCOCH₂OCH₃, -SO₂NHCO 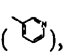 -SO₂NH 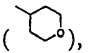 -SO₂NHCOCH(i-propyl)NH₂, -SO₂NHCH₂CH₂SCH₃, -SO₂NCH₃COCH₂CH₃, -SO₂NCH₃COCH₂OCH₃, -SO₂NCH₃COCH₂CH₂OCH₃, -SO₂NCH₃COCH₂CH₃, -SO₂NCH₃COCH₂OCH₃, -SO₂NCH₃COCH₂CH₂OCH₃, -SO₂NHCOCH₂OCH₃, -SO₂NHCOCH₂OH, -SO₂NHCOCH₂CH₂COOH, -SO₂CH₂CH₂NH₂, -SO₂CH₂COOH, -SO₂CH₂CH₂COOH, -SO₂CH₃, -SO₂CH₂CH₂CN, -SO₂CH₂CH₂CH₂OH,
-O(CH₂)₃SO₂NH₂, -OCH₂COOH, -OCH₂COOCH₃, -OCH₂CN, -OCH₂CHOHCH₂OH, -OCH₂CH₂CHOHCH₂OH, -SCH₂COOCH₃, -COOCH₃, -COOH, -COCH₂ 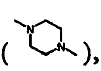 -COCH₂ 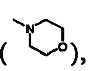 -CH₂COOCH₃, -CH₂COOH, -CH₂CH₂COOCH₃, CH₂CH₂CH₂COOCH₃, -N(CH₃)₂, -NHCOCOOCH₂CH₃, -NHSO₂CH₃ and -NCH₃SO₂CH₃; or
heteroaryl ring selected from the group consisting of pyridinyl, pyrazolyl and triazolyl said heteroaryl is unsubstituted or substituted with one or two substituents selected from the group consisting of methyl, methoxy, -SCH₃, and -NHCOOCH₃;
- R^{11c}: is hydrogen or methyl; and
hydrates, solvates and salts thereof.

(v) Use of compound of formula I according to (i), which compound is:
2-[4-Chloro-3-(3-chloro-5-cyano-phenoxy)-2-fluoro-phenyl]-N-(2-chloro-4-sulfamoyl-phenyl)-acetamide;
2-[4-chloro-3-(3-chloro-5-cyano-phenoxy)-2-fluoro-phenyl]-N-(2-chloro-4-propionylsulfamoyl-phenyl)-acetamide; sodium salt;
2-[3-(3-chloro-5-cyano-phenoxy)-2-fluoro-4-methyl-phenyl]-N-(2-chloro-4-sulfamoyl-phenyl)-acetamide;
2-[4-bromo-3-(3-chloro-5-cyano-phenoxy)-2-fluoro-phenyl]-N-(2-chloro-4-sulfamoyl-phenyl)-acetamide;
2-[4-bromo-3-(3-chloro-5-cyano-phenoxy)-2-fluoro-phenyl]-N-(2-chloro-4-propionylsulfamoyl-phenyl)-acetamide; sodium salt;
N-(4-butyrylsulfamoyl-2-chloro-phenyl)-2-[4-chloro-3-(3-chloro-5-cyano-phenoxy)-2-fluoro-phenyl]-acetamide; sodium salt;
N-[4-((S)-2-amino-3-methyl-butyrylsulfamoyl)-2-chloro-phenyl]-2-[4-chloro-3-(3-chloro-5-cyano-phenoxy)-2-fluoro-phenyl]-acetamide; sodium salt;
N-(2-chloro-4-sulfamoyl-phenyl)-2-[3-(3-cyano-5-difluoromethyl-phenoxy)-4-ethyl-2-fluoro-phenyl] -acetamide;
2-[3-(3-cyano-5-dinuoromethyl-phenoxy)-4-ethyl-2-fluoro-phenyl]-N-(2-methyl-4-sulfamoyl-phenyl)-acetamide;
2-[4-bromo-3-(3-chloro-5-cyano-phenoxy)-2-fluoro-phenyl] -N-(2-methyl-4-sulfamoyl-phenyl)-acetamide;
2-[4-bromo-3-(3-cyano-5-difluoromethyl-phenoxy)-2-fluoro-phenyl]-N-(2-methyl-4-sulfamoyl-phenyl)-acetamide;
2-[4-bromo-3-(3-cyano-5-difluoromethyl-phenoxy)-2-fluoro-phenyl]-N-(2-chloro-4-sulfamoyl-phenyl)-acetamide;
2-[4-chloro-3-(3-cyano-5-difluoromethyl-phenoxy)-2-fluoro-phenyl]-N-(2-methyl-4-sulfamoyl-phenyl)-acetamide;
2-[3-(3-chloro-5-cyano-phenoxy)-2-fluoro-4-methoxy-phenyl]-N-(2-chloro-4-sulfamoyl-phenyl)-acetamide;
2-[3-(3-chloro-5-cyano-phenoxy)-2-fluoro-4-methoxy-phenyl]-N-(2-methyl-4-sulfamoyl-phenyl)-acetamide; and,
2-[3-(3-chloro-5-cyano-phenoxy)-2-fluoro-4-methyl-phenyl]-N-(2-chloro-4-propionylsulfamoyl-phenyl)-acetamide; sodium salt;
wherein in each case the compound can either be a salt or a neutral form of the above.

(vi) A compound according to formula Ia 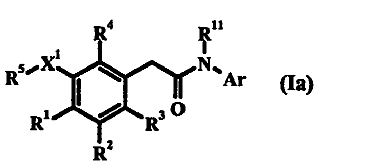 wherein
- X¹: is -O-;
- R¹ and R²: are each independently selected from the group consisting of hydrogen, C₁₋₆ alkyl, C₁₋₆ haloalkyl, C₃₋₈ cycloalkyl, C₁₋₆ alkoxy, C₁₋₆ alkylthio, C₁₋₆ alkylsulfinyl, C₁₋₆ sulfonyl, C₁₋₆ haloalkoxy, C₁₋₆ haloalkylthio, halogen, amino, alkylamino, dialkylamino, aminoacyl, nitro and cyano; or, (ii) together R¹ and R² are -O-CH=CH- or -O-CH₂CH₂- provided that R¹ is not hydrogen;
- R³ and R⁴: are independently selected from the group consisting of hydrogen, C₁₋₆ alkyl, C₁₋₆ haloalkyl, C₁₋₆ alkoxy, C₁₋₆ haloalkoxy C₁₋₆ alkylthio, C₁₋₆ haloalkylthio, halogen, amino, nitro and cyano;
- R⁵: is aryl substituted with one to three substituents independently selected from the group consisting of C₁₋₆ alkyl, C₂₋₆ alkenyl, C₁₋₆ alkynyl, -C≡CCH₂OH, -C≡CCH₂NMe₂, C₁₋₆ haloalkyl, C₃₋₈ cycloalkyl, C₁₋₆ alkoxy, C₁₋₆ alkylthio, C₁₋₆ alkylsulfinyl, C₁₋₆ sulfonyl, C₁₋₆ haloalkoxy, C₁₋₆ haloalkylthio, hydroxy, halogen, , nitro and cyano, said alkyl and said cycloalkyl are optionally substituted with one or two substituents independently selected from the group consisting of alkyl, hydroxy, alkoxy, thiol, alkylthio, halogen, amino, alkylamino, amino alkyl, alkylaminoalkyl, and dialkylamino;
- Ar: is a substituted phenyl ring according to formula IIa with the proviso that R^{7a} and R^{7c} are not both hydrogen or if R^{7c} is hydrogen, then R^{7a} is chlorine: 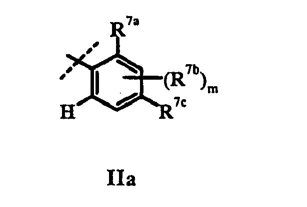
- R^{7a}: is selected from the group consisting of hydrogen, C₁₋₆ alkyl, C₁₋₃ haloalkyl, C₁₋₆ alkoxy, C₁₋₆ haloalkoxy, halogen and cyano;
- R^{7b}: is independently selected from the group consisting of C₁₋₆ haloalkyl, C₁₋₆ alkoxy, C₁₋₆ alkylthio, C₁₋₆ alkylsulfinyl, C₁₋₆ sulfonyl, amino C₁₋₆ alkylsulfonyl, SO₂NR^{11a}R^{11b}, C₁₋₆ haloalkoxy, C₁₋₆ haloalkylthio, hydroxy, amino, C₁₋₆ alkylamino, C₁₋₆ dialkylamino, aminoacyl, acyl, CONR⁸R⁹, nitro, cyano, C₁₋₆ heteroalkoxy, -X²(CH₂)ₚS(O)₂NR⁸R⁹, -X²(CH₂)ₚNHC(O)NHR⁸R⁹, -X²(CH₂)ₚNHS(O)₂NR⁸R⁹ and -X²(CH₂)ₚNHCOOR¹⁰;
- R^{7c}: is selected from the group consisting of C₁₋₆ heteroalkoxy, -S(O)₂NR⁸R⁹, -X²CH₂(CH₂)ₚS(O)₂NR⁸R⁹; -X²(CH₂)ₚNHC(O)NHR⁸R⁹, X⁶(CH₂)ᵥCONR⁸R⁹, -SO₂R¹³, -NR⁸R⁹, X²(CH₂)ₚNR¹¹(O)₂NR⁸R⁹, -X²(CH₂)pNHCOOR¹⁰, -X⁶(CH₂)ᵥCOOR¹⁰, -X⁶(CH₂)ᵥCN, -OR¹⁵ and C(=O)CH₂N(CH₂)₂]₂X⁴
- R⁸ and R⁹: taken independently, one of R⁸ and R⁹ is hydrogen or C₁₋₆ alkyl and the other of R⁸ and R⁹ is selected from the group consisting of hydrogen, -C(=O)R¹⁴, -C(=O)CR¹²NH₂, -(CH₂)₂N[(CH₂)₂]₂O, COCO₂Me, C₃₋₈ cycloalkyl said cycloalkyl optionally substituted with one or two hydroxyl substituents, pyranyl, C₁₋₆ alkyl and aryl said alkyl and said aryl groups optionally substituted with one or two substituents independently selected from the group consisting of hydroxy, C₁₋₆ alkoxy, thiol, C₁₋₆ alkylthio, C₁₋₆ alkylsulfinyl, C₁₋₆ sulfonyl, and halogen; or,
taken together are (CH²)₂-X⁵-(CH₂)₂, -(CH₂)ₒ- or (CH₂)ᵣS(O)ₙ optionally substituted with one or two substituents selected from the group consisting of halogen, hydroxyl and NR^{11a}R^{11b};
- R¹⁰: is C₁₋₆ alkyl;
- R¹¹: is hydrogen or C₁₋₆ alkyl;
- R^{11a} and R^{11b}: are independently R¹¹;
- R¹²: is the sidechain of a naturally occurring α-amino acid;
- R¹³: is C₁₋₆ alkyl; -(CH₂)ₛCO₂R¹¹, -(CH₂)₂CN, -(CH₂)₂NH₂, -(CH₂)ᵤOH;
- R¹⁴: is C₁₋₁₀ alkyl, -(CH₂)ₛNHR^{11a}R^{11b}, -(CH₂)ₛOR¹¹, -CH₂CH(OH)CH₃, -CH₂N[(CH₂)₂]₂O, -(CH₂)₂CO₂R¹¹, optionally substituted phenyl or pyridinyl;
- R¹⁵: is C₁₋₆ alkyl substituted with one to three hydroxyl groups;
- X²: is -O- or a bond;
- X⁴: is -O- or -NMe-;
- X⁵: is -O-, -S(O)ₙ- or NR¹¹;
- X⁶: is -O- or -S(O)ₙ-;
- m: is an integer from 0 to 2;
- n: is an integer from 0 to 2;
- o: is an integer from 4 to 6;
- p: is an integer from 0 to 6;
- r: is an integer from 3 to 4
- s: is an integer from 1 to 2;
- u: is an integer from 2 to 3;
- v: is an integer from 2 to 6; and,
hydrates, solvates, and acid addition salts or salts of the conjugate base thereof.

(vii) Compound of formula Ia according to (vi), wherein
- X¹: is -O-;
- R¹ and R²: are each independently selected from the group consisting of hydrogen, C₁₋₆ alkyl, C₁₋₆ alkoxy and halogen; or together R¹ and R² are -O-CH=CH-, provided that R¹ is not hydrogen;
- R³ and R⁴: are independently selected from the group consisting of hydrogen, and halogen;
- R⁵: is aryl substituted with one to three substituents independently selected from the group consisting of C₁₋₆ alkyl, -C≡CCH₂OH, -C≡CCH₂NMe₂, C₁₋₆ haloalkyl, C₁₋₆ haloalkoxy, cyano and halogen;
- Ar: is a substituted phenyl ring according to formula IIa with the proviso that R^{7a} and R^{7c} are not both hydrogen or if R^{7c} is hydrogen, then R^{7a} is chlorine: 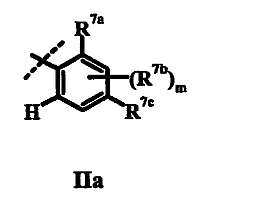
- R^{7a}: is selected from the group consisting of hydrogen, C₁₋₆ alkyl, C₁₋₃ haloalkyl, C₁₋₆ alkoxy, C₁₋₆ haloalkoxy, halogen and cyano;
- R^{7b}: is independently selected from the group consisting of C₁₋₆ haloalkyl, C₁₋₆ alkoxy, C₁₋₆ alkylthio, C₁₋₆ alkylsulfinyl, C₁₋₆ sulfonyl, amino C₁₋₆ alkylsulfonyl, SO₂NR^{11a}R^{11b}, C₁₋₆ haloalkoxy, C₁₋₆ haloalkylthio, hydroxy, amino, C₁₋₆ alkylamino, C₁₋₆ dialkylamino, aminoacyl, acyl, CONR⁸R⁹, nitro, cyano, C₁₋₆ heteroalkoxy,-X²(CH₂)ₚS(O)₂NR⁸R⁹, -X²(CH₂)ₚNHC(O)NHR⁸R⁹, -X²(CH₂)ₚNHS(O)₂NR⁸R⁹ and -X²(CH₂)ₚNHCOOR¹⁰;
- R^{7c}: is selected from the group consisting of C₁₋₆ heteroalkoxy, -S(O)₂NR⁸R⁹, -X²CH₂(CH₂)ₚS(O)₂NR⁸R⁹; -X²(CH₂)ₚNHC(O)NHR⁸R⁹, X⁶(CH₂)ᵥCONR⁸R⁹, -SO₂R¹³, -NR⁸R⁹, X²(CH₂)ₚNR¹¹S(O)₂NR⁸R⁹, -X²(CH₂)ₚNHCOOR¹⁰, -X⁶(CH₂)ᵥCOOR¹⁰, -X⁶(CH₂)ᵥCN, -OR¹⁵ and C(=O)CH₂N[(CH₂)₂]₂X⁴;
- R⁸ and R⁹: taken independently, one of R⁸ and R⁹ is hydrogen or C₁₋₆ alkyl and the other of R⁸ and R⁹ is selected from the group consisting of hydrogen, -C(=O)R¹⁴, -C(=O)CR¹²NH₂, -(CH₂)₂N[(CH₂)₂]₂O, COCO₂Me, C₃₋₈ cycloalkyl said cycloalkyl optionally substituted with one or two hydroxyl substituents, pyranyl, C₁₋₆ alkyl and aryl said alkyl and said aryl groups optionally substituted with one or two substituents independently selected from the group consisting of hydroxy, C₁₋₆ alkoxy, thiol, C₁₋₆ alkylthio, C₁₋₆ alkylsulfinyl, C₁₋₆ sulfonyl, and halogen; or,
taken together are (CH₂)₂-X⁵-(CH₂)₂, -(CH₂)ₒ- or (CH₂)ᵣS(O)ₙ optionally substituted with one or two substituents selected from the group consisting of halogen, hydroxyl and NR^{11a}R^{11b};
- R¹⁰: is C₁₋₆ alkyl;
- R¹¹: is hydrogen or C₁₋₆ alkyl;
- R^{11a} and R^{11b}: are independently R¹¹;
- R¹²: is the sidechain of a naturally occurring α-amino acid;
- R¹³: is C₁₋₆ alkyl; -(CH₂)ₛCO₂R¹¹, -(CH₂)₂CN, -(CH₂)₂NH₂, -(CH₂)ᵤOH;
- R¹⁴: is C₁₋₁₀ alkyl, -(CH₂)ₛNHR^{11a}R^{11b}, -(CH₂)ₛOR¹¹, -CH₂CH(OH)CH₃, -CH₂N[(CH₂)₂]₂O, -(CH₂)₂CO₂R¹¹, optionally substituted phenyl or pyridinyl;
- R¹⁵: is C₁₋₆ alkyl substituted with one to three hydroxyl groups;
- X²: is -O- or a bond;
- X⁴: is -O- or -NMe-;
- X⁵: is -O-, -S(O)ₙ- or NR¹¹;
- X⁶: is O- or -S(O)ₙ-;
- m: is an integer from 0 to 2;
- n: is an integer from 0 to 2;
- o: is an integer from 4 to 6;
- p: is an integer from 0 to 6;
- r: is an integer from 3 to 4
- s: is an integer from 1 to 2;
- u: is an integer from 2 to 3;
- v: is an integer from 2 to 6; and,
hydrates, solvates, and acid addition salts or salts of the conjugate base thereof.

(viii) Compound of formula Ia according to (vi), wherein
- X¹: is -O-;
- R¹ and R²: are each independently selected from the group consisting of hydrogen, methyl, ethyl, i-propyl, methoxy, chloro and bromo; or together R¹ and R² are -O-CH=CH-;
- R³ and R⁴: are independently selected from the group consisting of hydrogen and fluoro;
- R⁵: is phenyl, wherein said phenyl is unsubstituted or substituted with one to three substituents independently selected from the group consisting of methyl, ethyl, -CHF₂, -CF₃, -CF₂CH₃, -OCHF₂, chloro, bromo, fluoro, -C≡CCH₂OH, -C≡CCH₂N(CH₃)₂ and cyano.
- Ar: is phenyl wherein said phenyl is unsubstituted or substituted with 1 to 3 substituents selected from the group consisting of methyl, 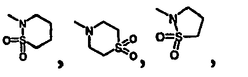 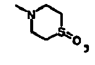 -SO₂NH₂, -SO₂NHCH₃, -SO₂NCH₃CH₂CH₂OH, -SO₂NHCH₂CHCH₃OH, -SO₂NHCH₂CH₂ 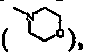 -SO₂NHCH₂CH₂CH₂OH, -SO₂ 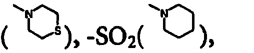 -SO₂ 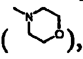 -SO₂ 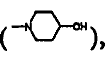 -SO₂NH 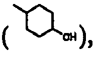 -SO₂ 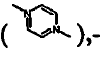 -SO₂NHCOCH₂CH₃, -SO₂NHCOCH₂CH₂CH₃, -SO₂NHCOCH(NH₂)CH(CH₃)₂, -SO₂NCH₃COCH₂CH₂CH₃, -SO₂NHCOCH₂CH₂CH₂CH₃, -SO₂NHCOCH₂CH(CH₃)₂, -SO₂NHCOCH₂CH(CH₂CH₃)₂, -SO₂NHCOCH₂CH₂CH(CH₃)₂, -SO₂NHCOCH₂CH₂NH₂, -SO₂NCH₃COCH₂CH₂NH₂, -SO₂NHCOCH₂OCH₃, -SO₂NHCO 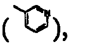 -SO₂NH 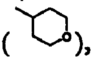 -SO₂NHCOCH(i-propyl)NH₂, -SO₂NHCH₂CH₂SCH₃, -SO₂NCH₃COCH₂CH₃, -SO₂NCH₃COCH₂OCH₃, -SO₂NCH₃COCH₂CH₂OCH₃, -SO₂NHCOCH₂OH, -SO₂NHCOCH₂CH₂COOH, -SO₂CH₂COOH, -SO₂CH₂CH₂COOH, -SO₂CH₃, -SO₂CH₂CH₂NH₂, -SO₂CH₂CH₂CN, -SO₂CH₂CH₂CH₂OH, -O(CH₂)₃SO₂NH₂, -OCH₂CN, -OCH₂CHOHCH₂OH, -OCH₂CH₂CHOHCH₂OH, -COOCH₃, -COOH, -COCH₂ 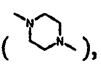 -COCH₂ 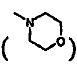 -N(CH₃)₂, and -NHCOCOOCH₂CH₃;
- R¹¹: is hydrogen or methyl; and
hydrates, solvates, and acid addition salts or salts of the conjugate base thereof.

(xi) Compound of formula Ia according to (vi), which is
2-[4-Chloro-3-(3-chloro-5-cyano-phenoxy)-2-fluoro-phenyl]-N-(2-chloro-4-sulfamoyl-phenyl)-acetamide;
2-[4-Chloro-3-(3-chloro-5-cyano-phenoxy)-2-fluoro-phenyl]-N-(2-chloro-4-propionylsulfamoyl-phenyl)-acetamide; sodium salt;
2-[3-(3-Chloro-5-cyano-phenoxy)-2-fluoro-4-methyl-phenyl]-N-(2-chloro-4-sulfamoyl-phenyl)-acetamide;
2-[4-Bromo-3-(3-chloro-5-cyano-phenoxy)-2-fluoro-phenyl]-N-(2-chloro-4-sulfamoyl-phenyl)-acetamide;
2-[4-Bromo-3-(3-chloro-5-cyano-phenoxy)-2-fluoro-phenyl]-N-(2-chloro-4-propionylsulfamoyl-phenyl)-acetamide; sodium salt;
N-(4-Butyrylsulfamoyl-2-chloro-phenyl)-2-[4-chloro-3-(3-chloro-5-cyano-phenoxy)-2-fluoro-phenyl]-acetamide; sodium salt;
N-[4-((S)-2-Amino-3-methyl-butyrylsulfamoyl)-2-chloro-phenyl]-2-[4-chloro-3-(3-chloro-5-cyano-phenoxy)-2-fluoro-phenyl]-acetamide; sodium salt;
N-(2-Chloro-4-sulfamoyl-phenyl)-2-[3-(3-cyano-5-difluoromethyl-phenoxy)-4-ethyl-2-fluoro-phenyl]-acetamide;
2-[3-(3-Cyano-5-difluoromethyl-phenoxy)-4-ethyl-2-fluoro-phenyl]-N-(2-methyl-4-sulfamoyl-phenyl)-acetamide;
2-[4-Bromo-3-(3-chloro-5-cyano-phenoxy)-2-fluoro-phenyl]-N-(2-methyl-4-sulfamoyl-phenyl)-acetamide;
2- [4-Bromo-3-(3-cyano-5-difluoromethyl-phenoxy)-2-fluoro-phenyl] -N-(2-methyl-4-sulfamoyl-phenyl)-acetamide;
2-[4-Bromo-3-(3-cyano-5-difluoromethyl-phenoxy)-2-fluoro-phenyl]-N-(2-chloro-4-sulfamoyl-phenyl)-acetamide;
2-[4-Chloro-3-(3-cyano-5-difluoromethyl-phenoxy)-2-fluoro-phenyl]-*N*-(2-methyl-4-sulfamoyl-phenyl)-acetamide;
2-[3-(3-Chloro-5-cyano-phenoxy)-2-fluoro-4-methoxy-phenyl]-*N*-(2-chloro-4-sulfamoyl-phenyl)-acetamide;
2-[3-(3-Chloro-5-cyano-phenoxy)-2-fluoro-4-methoxy-phenyl]-N-(2-methyl-4-sulfamoyl-phenyl)-acetamide; or,
2-[3-(3-Chloro-5-cyano-phenoxy)-2-fluoro-4-methyl-phenyl]-N-(2-chloro-4-propionylsulfamoyl-phenyl)-acetamide; sodium salt.

(x) Compound of formula Ia according to any one of claims (vi) to (ix) for use as medicament.

(xi) Use of the compound of formula Ia according to any one of (vi) to (ix) for the manufacture of a medicament for the treatment of diseases mediated by the human immunodeficiency virus (HIV)

(xii) A pharmaceutical composition comprising a pharmaceutically effective amount of compound or a pharmaceutically acceptable salt thereof as defined in any one of (i) to (xi) and a pharmaceutical inert carrier.

(xiiii) A pharmaceutical composition according to (xii) for the use in the treatment of diseases mediated by the human immunodeficiency virus (HIV).

The phrase "a" or "an" entity as used herein refers to one or more of that entity; for example, a compound refers to one or more compounds or at least one compound. As such, the terms "a" (or "an"), "one or more", and "at least one" can be used interchangeably herein.

The phrase "as defined hereinabove" refers to the first definition provided in the Summary of the Invention.

The term "optional" or "optionally" as used herein means that a subsequently described event or circumstance may, but need not, occur, and that the description includes instances where the event or circumstance occurs and instances in which it does not. For example, "optionally substituted" means that the moiety may be hydrogen or a substituent.

The term "C₁₋₆ alkyl" as used herein denotes an unbranched or branched chain, saturated, monovalent hydrocarbon residue containing 1 to 6 carbon atoms. Examples of alkyl groups include, but are not limited to, lower alkyl groups include methyl, ethyl, propyl, *i*-propyl, n-butyl, *i*-butyl, *t*-butyl or pentyl, isopentyl, neopentyl, hexyl.

The term "haloalkyl" as used herein denotes an unbranched or branched chain alkyl group as defined above wherein 1, 2, 3 or more hydrogen atoms are substituted by a halogen. Examples are 1-fluoromethyl, 1-chloromethyl, 1-bromomethyl, 1-iodomethyl, trifluoromethyl, trichloromethyl, tribromomethyl, triiodomethyl, 1-fluoroethyl, 1-chloroethyl,1-bromoethyl,1-iodoethyl, 2-fluoroethyl, 2-chloroethyl, 2-bromoethyl, 2-iodoethyl, 2,2-dichloroethyl, 3-bromopropyl or 2,2,2-trifluoroethyl.

The term "C₃₋₈ cycloalkyl" as used herein denotes a saturated carbocyclic ring containing 3 to 8 carbon atoms, i.e. cyclopropyl, cyclobutyl, cyclopentyl, cyclohexyl, cycloheptyl or cyclooctyl.

The term "aryl" as used herein means a monocyclic or polycyclic-aromatic group comprising carbon and hydrogen atoms. Examples of suitable aryl groups include, but are not limited to, phenyl, tolyl, indenyl, and 1- or 2-naphthyl, as well as benzo-fused carbocyclic moieties such as 5,6,7,8-tetrahydronaphthyl. An aryl group can be unsubstituted or substituted with one or more suitable substituents which substituents include C₁₋₆ alkyl, C₁₋₆ haloalkyl, C₃₋₈ cycloalkyl, C₁₋₆ alkoxy, C₁₋₆ alkylthio, C₁₋₆ alkylsulfinyl, C₁₋₆ sulfonyl, C₁₋₆ haloalkoxy, C₁₋₆ haloalkylthio, halogen, amino, alkylamino, dialkylamino, aminoacyl, acyl, alkoxycarbonyl, carbamoyl, N-alkylcarbamoyl, N,N-dialkylcarbamoyl, nitro and cyano.

A "heteroaryl group" or "heteroaromatic"as used herein means a monocyclic- or polycyclic aromatic ring comprising up to 15 carbon atoms, hydrogen atoms, and one or more heteroatoms, preferably, 1 to 3 heteroatoms, independently selected from nitrogen, oxygen, and sulfur. As well known to those skilled in the art, heteroaryl rings have less aromatic character than their all-carbon counter parts. Thus, for the purposes of the invention, a heteroaryl group need only have some degree of aromatic character. Illustrative examples of heteroaryl groups include, but are not limited to, pyridinyl, pyridine N-oxide, pyridazinyl, pyrimidyl, pyrazyl, triazinyl, pyrrolyl, pyrazolyl, imidazolyl, (1,2,3,)- and (1,2,4)-triazolyl, pyrazinyl, pyrimidinyl, tetrazolyl, furyl, thienyl, isoxazolyl, thiazolyl, thienyl, isoxazolyl, indole, indole N-oxide, quinoline, quinoline N-oxide and oxazolyl. A heteroaryl group can be unsubstituted or substituted with one or more suitable substituents selected from hydroxy, oxo, cyano, alkyl, alkoxy, haloalkoxy, alkylthio, halo, haloalkyl, nitro, alkoxycarbonyl, amino, alkylamino, dialkylamino, aminoacyl, alkylsulfonyl, arylsulfinyl, alkoxycarbonyl, carbamoyl, N-alkylcarbamoyl, N,N-dialkylcarbamoyl, acyl unless otherwise indicated.

The term "heterocyclyl" means the monovalent saturated cyclic radical, consisting of one or more rings, preferably one to two rings, of three to eight atoms per ring, incorporating one or more ring heteroatoms (chosen from N,O or S(O)₀₋₂), and which can optionally be substituted with one or more, preferably one to three substituents selected from hydroxy, oxo, cyano, alkyl, alkoxy, haloalkoxy, alkylthio, halo, haloalkyl, nitro, alkoxycarbonyl, amino, alkylamino, dialkylamino, aminoacyl, alkylsulfonyl, arylsulfinyl, alkoxycarbonyl, carbamoyl, N-alkylcarbamoyl, N,N-diallcylcarbamoyl, acyl unless otherwise indicated. Examples of heterocyclic radicals include, but are not limited to, furanyl, tetrahydropyranyl, tetrahydrothiophenyl and the like. A nitrogen atom in the heteroaryl ring can optionally be an N-oxide.

The term "alkoxy group" as used herein means an -O-alkyl group, wherein alkyl is as defined above such as methoxy, ethoxy, n-propyloxy, *i*-propyloxy, n-butyloxy, *i-*butyloxy, *t*-butyloxy, pentyloxy, hexyloxy, heptyloxy including their isomers.

The term "alkylthio group" as used herein means an -S-alkyl group, wherein alkyl is as defined above such as methylthio, ethylthio, n-propylthio, *i*-propylthio, *n*-butylthio, *i-*butylthio, *t*-butylthio, pentylthio including their isomers.

The term "haloalkoxy group" as used herein means an -O-haloalkyl group, wherein haloalkyl is as defined above. Examples of haloalkoxy groups include, but are not limited to, 2,2,2-trifluoroethoxy, difluoromethoxy and 1,1,1,3,3,3-hexafluoro-iso-propoxy.

The term "haloalkthio group" as used herein means an -S-haloalkyl group, wherein haloalkyl is as defined above. An example of haloalkthio group includes, but are not limited to, 2,2,2-trifluoroeththanthiol.

The term "aryloxy group" as used herein means an O-aryl group wherein aryl is as defined above. An aryloxy group can be unsubstituted or substituted with one or more suitable substituents. Preferably, the aryl ring of an aryloxy group is a monocyclic ring,
wherein the ring comprises 6 carbon atoms, referred to herein as "(C₆) aryloxy". The term "optionally substituted aryloxy" means the aryl or group may be substituted with one to three groups selected from the group consisting of C₁₋₆ alkyl, C₁₋₆ haloalkyl, C₃₋₈ cycloalkyl, C₁₋₆ alkoxy, C₁₋₆ alkylthio, C₁₋₆ alkylsulfinyl, C₁₋₆ sulfonyl, C₁₋₆ haloalkoxy, C₁₋₆ haloalkylthio, halogen, amino, alkylamino, dialkylamino, aminoacyl, acyl, alkoxycarbonyl, carbamoyl, N-alkylcarbamoyl, N,N-diakylcarbamoyl, nitro and cyano.

The term "heteroaryloxy group" as used herein means an O-heteroaryl group, wherein heteroaryl is as defined above. The heteroaryl ring of a heteroaryloxy group can be unsubstituted or substituted with one or more suitable substituents. Examples of heteroaryl groups include, but are not limited to, 2-pyridyloxy, 3-pyrrolyloxy, 3-pyrazolyloxy, 2-imidazolyloxy, 3-pyrazinyloxy, and 4-pyrimidyloxy.

The term "acyl" or "alkylcarbonyl" as used herein denotes a radical of formula C(=O)R wherein R is hydrogen, unbranched or branched alkyl containing 1 to 6 carbon atoms or a phenyl group.

The term "alkoxycarbonyl" as used herein denotes a radical of formula C(=O)OR wherein R is, unbranched or branched alkyl as described above.

The term "acylamino" as used herein denotes a radical of formula -NH-(acyl) where acyl is as defined herein.

The term "arylboronic acid" as used herein denotes a radical of formula ArB(OH)₂ wherein Ar is an optionally substituted aryl group as described above.

The term "alkylene" as used herein denotes a divalent linear or branched saturated hydrocarbon radical, having from one to six carbons inclusive, unless otherwise indicated. Examples of alkylene radicals include, but are not limited to, methylene, ethylene, propylene, 2-methyl-propylene, butylene, 2-ethylbutylene.

The term "arylalkyl" or "aralkyl" as used herein denotes the radical R'R"-, wherein R' is an aryl radical as defined herein, and R" is an alkylene radical as defined herein and the arylalkyl group is attached through the alkylene radical. Examples of arylalkyl radicals include, but are not limited to, benzyl, phenylethyl, 3-phenylpropyl.

The term "halogen" as used herein means fluorine, chlorine, bromine, or iodine. Correspondingly, the meaning of the term "halo" encompasses fluoro, chloro, bromo, and iodo. The term "hydrohalic acid" refers to an acid comprised of hydrogen and a halogen.

The term "alkylsulfinyl" as used herein means the radical -S(O)R', wherein R' is alkyl as defined herein. Examples of alkylaminosulfonyl include, but are not limited to methylsulfinyl and *iso*-propylsulfinyl.

The term "alkylsulfonyl" as used herein means the radical -S(O)₂R', wherein R' is alkyl as defined herein. Examples of alkylaminosulfonyl include, but are not limited to methylsulfonyl and *iso*-propylsulfonyl.

The terms "amino", "alkylamino" and "dialkylamino" as used herein refer to -NH₂, - NHR and -NR₂ respectively and R is alkyl as defined above. The two alkyl groups attached to a nitrogen in a dialkyl moiety can be the same or different. The terms "aminoalkyl", "alkylaminoalkyl" and "dialkylaminoalkyl" as used herein refer to NH₂(CH₂)n-, RHN(CH₂)n-, and R₂N(CH₂)n- respectively wherein n is 1 to 6 and R is alkyl as defined above

The prefix "carbamoyl" as used herein means the radical -CONH₂. The prefix "N-alkylcabamoyl" and "N,N-dialkylcarbamoyl" means the radical CONHR' or CONR'R" respectively wherein the R' and R" groups are independently alkyl as defined herein.

The term "amino acid" as used herein refers to naturally occurring amino acids, as well as to optical isomers (enantiomers and diastereomers), synthetic analogs and derivatives thereof. α-Amino acids comprise a carbon atom bonded to a carboxyl group, an amino group, a hydrogen atom and a unique "side chain" group. The term "naturally occurring amino acids" means the L-isomers of the naturally occurring amino acids. The naturally occurring amino acids are glycine, alanine, valine, leucine, isoleucine, serine, methionine, threonine, phenylalanine, tyrosine, tryptophan, cysteine, proline, histidine, aspartic acid, asparagine, glutamic acid, glutamine, α-carboxyglutamic acid, arginine, ornithine and lysine. The side chains of naturally occurring amino acids include: hydrogen, methyl, *iso*-propyl, *iso*-butyl, *sec*-butyl, -CH₂OH**,** -CH(OH)CH₃, -CH₂SH, -CH₂CH₂SMe, -(CH₂)ₚCOR wherein R is -OH or -NH₂ and p is 1 or 2, -(CH₂)_{q}-NH₂ where q is 3 or 4, -(CH₂)₃-NHC(=NH)NH₂, -CH₂C₆H₅, -CH₂-*p*-C₆H₄-OH, (3-indolinyl)methylene, (4-imidazolyl)methylene. The term "conjugate base" as used herein means the chemical species produced when a proton is abstracted from an acid (including here a carbon acid).

Compounds of formula I exhibit tautomerism. Tautomeric compounds can exist as two or more interconvertable species. Prototropic tautomers result from the migration of a covalently bonded hydrogen atom between two atoms. Tautomers generally exist in equilibrium and attempts to isolate an individual tautomers usually produce a mixture whose chemical and physical properties are consistent with a mixture of compounds. The position of the equilibrium is dependent on chemical features within the molecule. For example, in many aliphatic aldehydes and ketones, such as acetaldehyde, the keto form predominates while; in phenols, the enol form predominates. Common prototropic tautomers include keto/enol (-C(=O)-CH- ↔ -C(-OH)=CH-), amide/imidic acid (-C(=O)-NH- ↔ -C(-OH)=N-) and amidine (-C(=NR)-NH- ↔-C(-NHR)=N-) tautomers. The latter two are particularly common in heteroaryl and heterocyclic rings and the present invention encompasses all tautomeric forms of the compounds.

Compounds of formula I which are basic can form pharmaceutically acceptable acid addition salts with inorganic acids such as hydrohalic acids (e.g. hydrochloric acid and hydrobromic acid), sulphuric acid, nitric acid and phosphoric acid, and the like, and with organic acids (e.g. with acetic acid, tartaric acid, succinic acid, fumaric acid, maleic acid, malic acid, salicylic acid, citric acid, methanesulphonic acid and p-toluenesulfonic acid, and the like).

A "prodrug" of a compound of formula (I) herein refers to any compound which releases an active drug according to Formula I *in vivo* when such prodrug is administered to a mammalian subject. Prodrugs of a compound of Formula I are prepared by modifying one or more functional group(s) present in the compound of Formula I in such a way that the modification(s) may be cleaved *in vivo* to release the compound of Formula I. Prodrugs include compounds of Formula I wherein a hydroxy, amino, or sulfhydryl group in a compound of Formula I is bonded to any group that may be cleaved *in vivo* to regenerate the free hydroxyl, amino, or sulfhydryl group, respectively. Examples of produgs include N-acyl-benzenesulfonamide described.

The term "solvate" as used herein means a compound of the invention or a salt, thereof, that further includes a stoichiometric or non-stoichiometric amount of a solvent bound by non-covalent intermolecular forces. Preferred solvents are volatile, non-toxic, and/or acceptable for administration to humans in trace amounts.

The term "hydrate" as used herein means a compound of the invention or a salt thereof, that further includes a stoichiometric or non-stoichiometric amount of water bound by non-covalent intermolecular forces.

The term "clathrate" as used herein means a compound of the invention or a salt thereof in the form of a crystal lattice that contains spaces (e. g., channels) that have a guest molecule (e. g., a solvent or water) trapped within.

The term "wild type" as used herein refers to the HIV virus strain which possesses the dominant genotype which naturally occurs in the normal population which has not been exposed to reverse transcriptase inhibitors. The term "wild type reverse transcriptase" used herein has refers to the reverse transcriptase expressed by the wild type strain which has been sequenced and deposited in the SwissProt database with an accession number P03366.

The term "reduced susceptibility" as used herein refers to about a 10 fold, or greater, change in sensitivity of a particular viral isolate compared to the sensitivity exhibited by the wild type virus in the same experimental system.

The term "nucleoside and nucleotide reverse transcriptase inhibitors" ("NRTI"s) as used herein means nucleosides and nucleotides and analogues thereof that inhibit the activity of HIV-1 reverse transcriptase, the enzyme which catalyzes the conversion of viral genomic HIV-1 RNA into proviral HIV-1 DNA.

Typical suitable NRTIs include zidovudine (AZT) available under the RETROVIR tradename; didanosine (ddl) available under the VIDEX tradename.; zalcitabine (ddC) available under the HIVID tradename; stavudine (d4T) available under the ZERIT trademark.; lamivudine (3TC) available under the EPIVIR tradename; abacavir (1592U89) disclosed in WO96/30025 and available under the ZIAGEN trademark; adefovir dipivoxil [bis(POM)-PMEA] available under the PREVON tradename; lobucavir (BMS-180194), a nucleoside reverse transcriptase inhibitor disclosed in EP-0358154 and EP-0736533 and under development by Bristol-Myers Squibb; BCH-10652, a reverse transcriptase inhibitor (in the form of a racemic mixture of BCH-10618 and BCH-10619) under development by Biochem Pharma; emitricitabine [(-)-FTC] licensed from Emory University under U.S. Pat. No. 5,814,639 and under development by Triangle Pharmaceuticals; beta-L-FD4 (also called beta-L-D4C and named beta-L-2', 3'-dicleoxy-5-fluoro-cytidene) licensed by Yale University to Vion Pharmaceuticals; DAPD, the purine nucleoside, (-)-beta-D-2,6,-diamino-purine dioxolane disclosed in EP-0656778 and licensed to Triangle Pharmaceuticals; and lodenosine (FddA), 9-(2,3-dideoxy 2-fluoro-b-D-threo-pentofuranosyl)adenine, an acid stable purine-based reverse transcriptase inhibitor discovered by the NIH and under development by U.S. Bioscience Inc.

The term "non-nucleoside reverse transcriptase inhibitors" ("NNRTI"s) as used herein means non-nucleosides that inhibit the activity of HN-1 reverse transcriptase.

Typical suitable NNRTIs include nevirapine (BI-RG-587) available under the VIRAMUNE tradename; delaviradine (BHAP, U-90152) available under the RESCRIPTOR tradename; efavirenz (DMP-266) a benzoxazin-2-one disclosed in WO94/03440 and available under the SUSTIVA tradename; PNU-142721, a furopyridine-thio-pyrimide; AG-1549 (formerly Shionogi # S-1153); 5-(3,5-dichlorophenyl)-thio-4-isopropyl-1-(4-pyridyl)methyl-1H-imidazol-2- ylmethyl carbonate disclosed in WO 96/10019; MKC-442 (1-(ethoxy-methyl)-5-(1-methylethyl)-6-(phenylmethyl)-(2,4(1H,3H)-pyrimidinedione); and (+)-calanolide A (NSC-675451) and B, coumarin derivatives disclosed in U.S. Pat. No. 5,489,697.

The term "protease inhibitor" ("PI") as used herein means inhibitors of the HIV-1 protease, an enzyme required for the proteolytic cleavage of viral polyprotein precursors (e.g., viral GAG and GAG Pol polyproteins), into the individual functional proteins found in infectious HIV-1. HIV protease inhibitors include compounds having a peptidomimetic structure, high molecular weight (7600 daltons) and substantial peptide character, e.g. CRIXIVAN as well as nonpeptide protease inhibitors e.g., VIRACEPT.

Typical suitable PIs include saquinavir available in hard gel capsules under the INVIRASE tradename and as soft gel capsules under the FORTOVASE tradename; ritonavir (ABT-538) available under the NORVIR tradename; indinavir (MK-639) available under the CRIXIVAN tradename; nelfnavir (AG-1343) available under the VIRACEPT; amprenavir (141W94), tradename AGENERASE, a non-peptide protease inhibitor; lasinavir (BMS-234475; originally discovered by Novartis, Basel, Switzerland (CGP-61755); DMP-450, a cyclic urea discovered by Dupont; BMS-2322623, an azapeptide under development by Bristol-Myers Squibb, as a 2nd-generation HIV-1 PI; ABT-378; AG-1549 an orally active imidazole carbamate.

Other antiviral agents include hydroxyurea, ribavirin, IL-2, IL-12, pentafuside and Yissum Project No. 11607. Hydroxyurea (Droxia), a ribonucleoside triphosphate reductase inhibitor, the enzyme involved in the activation of T-cells. Hydroxyurea was shown to have a synergistic effect on the activity of didanosine and has been studied with stavudine. IL-2 is disclosed in Ajinomoto EP-0142268, Takeda EP-0176299, and Chiron U.S. Pat. Nos. RE 33,653, 4,530,787, 4,569,790, 4,604,377, 4,748,234, 4,752,585, and 4,949,314, and is available under the PROLEUKIN (aldesleukin) tradename as a lyophilized powder for IV infusion or sc administration upon reconstitution and dilution with water; a dose of about 1 to about 20 million 1U/day, sc is preferred; a dose of about 15 million 1 U/day, sc is more preferred. IL-12 is disclosed in WO96/25171 and is available as a dose of about 0.5 microgram/kg/day to about 10 microgram/kg/day, sc is preferred. Pentafuside (DP-178, T-20) a 36-amino acid synthetic peptide, disclosed in U.S. Pat. No. 5,464,933 and available under the FUZEON tradename; pentafuside acts by inhibiting fusion of HIV-1 to target membranes. Pentafuside (3-100 mg/day) is given as a continuous sc infusion or injection together with efavirenz and 2 PI's to HIV-1 positive patients refractory to a triple combination therapy; use of 100 mg/day is preferred. Yissum Project No. 11607, a synthetic protein based on the HIV-1 Vif protein. Ribavirin, 1-.beta.-D-ribofuranosyl-1H-1,2,4-triazole-3-carboxamide, is described in U.S. Pat. No. 4,211,771. The term "anti-HIV-1 therapy" as used herein means any anti-HIV-1 drug found useful for treating HIV-1 infections in man alone, or as part of multidrug combination therapies, especially the HAART triple and quadruple combination therapies. Typical suitable known anti-HIV-1 therapies include, but are not limited to multidrug combination therapies such as (i) at least three anti-HIV-1 drugs selected from two NRTIs, one PI, a second PI, and one NNRTI; and (ii) at least two anti-HIV-1 drugs selected from NNRTIs and PIs. Typical suitable HAART--multidrug combination therapies include: (a) triple combination therapies such as two NRTIs and one PI; or (b) two NRTIs and one NNRTI; and (c) quadruple combination therapies such as two NRTIs, one PI and a second PI or one NNRTI. In treatment of naive patients, it is preferred to start anti-HIV-1 treatment with the triple combination therapy; the use of two NRTIs and one PI is preferred unless there is intolerance to PIs. Drug compliance is essential. The CD4⁺ and HIV-1-RNA plasma levels should be monitored every 3-6 months. Should viral load plateau, a fourth drug, e.g., one PI or one NNRTI could be added.

Abbreviations used in this application include: acetyl (Ac), acetic acid (HOAc), azo-*bis*-isobutyrylnitrile (AIBN), 1-N-hydroxybenzotriazole (HOBT), atmospheres (Atm), high pressure liquid chromatography (HPLC), 9-borabicyclo[3.3.1]nonane (9-BBN or BBN), methyl (Me), *tert*-butoxycarbonyl (Boc), acetonitrile (MeCN), di-*tert*-butyl pyrocarbonate or boc anhydride (BOC₂O), 1-(3-dimethylaminopropyl)-3-ethylcarbodiimide hydrochloride (EDCI), benzyl (Bn), *m*-chloroperbenzoic acid (MCPBA), butyl (Bu), methanol (MeOH), benzyloxycarbonyl (cbz or Z), melting point (mp), carbonyl diimidazole (CDI), MeSO₂- (mesyl or Ms), 1,4-diazabicyclo[2.2.2]octane (DABCO), mass spectrum (ms) diethylaminosulfur trifluoride (DAST), methyl *t*-butyl ether (MTBE), dibenzylideneacetone (Dba), N-carboxyanhydride (NCA), 1,5-diazabicyclo[4.3.0]non-5-ene (DBN), N-bromosuccinimide (NBS), 1,8-diazabicyclo[5.4.0]undec-7-ene (DBU), N-methylpyrrolidone (NMP), 1,2-dichloroethane (DCE), pyridinium chlorochromate (PCC), N,N'-dicyclohexylcarbodiimide (DCC), pyridinium dichromate (PDC), dichloromethane (DCM), propyl (Pr), diethyl azodicarboxylate (DEAD), phenyl (Ph), di-*iso-*propylazodicarboxylate, DIAD, pounds per square inch (psi), diethyl iso-propylamine (DEIPA), pyridine (pyr), di-*iso*-butylaluminumhydride , DIBAL-H, room temperature, rt or RT, N,N-dimethyl acetamide (DMA), *tert*-butyldimethylsilyl or t-BuMe₂Si, (TBDMS), 4-N,N-dimethylaminopyridine (DMAP), triethylamine (Et₃N or TEA), N,N-dimethylformamide (DMF), triflate or CF₃SO₂- (Tf), dimethyl sulfoxide (DMSO), trifluoroacetic acid (TFA), 1,1'-*bis*-(diphenylphosphino)ethane (dppe), 2,2,6,6-tetramethylheptane-2,6-dione (TMHD), 1,1'-*bis*-(diphenylphosphino)ferrocene (dppf), thin layer chromatography (TLC), ethyl acetate (EtOAc), tetrahydrofuran (THF), diethyl ether (Et₂O), trimethylsilyl or Me₃Si (TMS), ethyl (Et), *p*-toluenesulfonic acid monohydrate (TsOH or pTsOH), lithium hexamethyl disilazane (LiHMDS), 4-Me-C₄H₄SO₂- or tosyl (Ts), *iso*-propyl (*i*-Pr), N-urethane-N-carboxyanhydride (UNCA), ethanol (EtOH). Conventional nomenclature including the prefixes *normal (n), iso* (*i-*), *secondary* (*sec*-)*, tertiary* (*tert*-) and *neo* have their customary meaning when used with an alkyl moiety. (J. Rigaudy and D. P. Klesney, Nomenclature in Organic Chemistry, IUPAC 1979 Pergamon Press, Oxford.).

### COMPOUNDS AND PREPARATION

Examples of representative compounds encompassed by the present invention and within the scope of the invention are contained in the Table 1. The compounds in Table 1 and the preparative examples which follow are provided to enable those skilled in the art to more clearly understand and to practice the present invention. They should not be considered as limiting the scope of the invention, but merely as being illustrative and representative thereof.

In general, the nomenclature used in this Application is based on AUTONOM^{™} v.4.0, a Beilstein Institute computerized system for the generation of IUPAC systematic nomenclature. If there is a discrepancy between a depicted structure and a name given that structure, the depicted structure is to be accorded more weight. In addition, if the stereochemistry of a structure or a portion of a structure is not indicated with, for example, bold or dashed lines, the structure or portion of the structure is to be interpreted as encompassing all stereoisomers of it.

| Table 1 | | | |
|---|---|---|---|
| Cpd # | Name | MS | MP |
| **I-1** | 2-[4-Chloro-3-(3-chloro-5-cyano-phenoxy)-phenyl]-*N*-(4-sulfamoyl-phenyl)-acetamide | | 210.5-214.0 |
| **I-2** | 2-[4-Chloro-3-(3,5-dicyano-phenoxy)-phenyl]-*N*-(4-sulfamoyl-phenyl)-acetamide | | 218-219 |
| **I-3** | 2-[3-(2-Bromo-5-chioro-phenoxy)-4-chloro-phenyl]-*N*-[2-methyl-4-(3-sulfamoyl-propoxy)-phenyl]-acetamide | | 190.1-192.2 |
| **I-4** | 2-[3-(2-Bromo-5-chloro-phenoxy)-4-chloro-phenyl phenyl]-*N*-(4-sulfamoyl-phenyl)-acetamide | | 260.1-261.9 |
| **I-5** | 2-[4-Chloro-3-(3,5-dicyano-phenoxy)- | 422 (M+H)⁺ | |
| **I-6** | 2-[4-Chloro-3-(3,5-dicyano-phenoxy)-phenyl]-*N*-(4-dimethylamino-phenyl)-acetamide | 431 (M+H)⁺ | |
| **I-7** | 2-[3-(2-Chloro-5-cyano-phenoxy)-4-ethyl-2-fluoro-phenyl]-*N*-(4-sulfamoyl-phenyl)-acetamide | | |
| **I-8** | 2-[4-Chloro-3-(2,5-dicyano-phenoxy)-2-fluoro-phenyl]-*N*-(4-sulfamoyl-phenyl)-acetamide | 483 (M-H)⁻ | |
| **I-9** | 2-[4-Chloro-3-(3,5-dicyano-phenoxy)-2-fluoro-phenyl]-*N*-(4-sulfamoyl-phenyl)-acetamide | 483 (M-H)⁻ | 135.7-138.1 |
| **I-10** | 2-[4-Chloro-2-fluoro-3-(2,3,5-trichloro-phenoxy)-phenyl]-*N*-(4-sulfamoyl-phenyl)-acetamide | 537 (M-H)⁻ | 247.0-249.9 |
| **I-11** | 2-[3-(3-Cyano-5-fluoro-phenoxy)-4-methyl-phenyl]-*N*-(4-sulfamoyl-phenyl)-acetamide | 440 (M+H)⁺ | 165.8-168.1 |
| **I-12** | *N*-(2-Chloro-phenyl)-2-[3-(3-cyano-5-fluoro-phenoxy)-4-methyl-phenyl]-acetamide | 395 (M+H)⁺ | 131.1-132.2 |
| **I-13** | 2-[4-Chloro-3-(3-cyano-5-difluoromethyl-phenoxy)-2-fluoro-phenyl]-*N*-(4-sulfamoyl-phenyl)-acetamide | | 214-218 |
| **I-14** | 2-[4-Chloro-3-(3-cyano-5-difluoromethyl-phenoxy)-2-fluoro-phenyl]-*N*-(2-chloro-phenyl)-acetamide | | 169.5-171.0 |
| **I-15** | 2-[3-(2-Bromo-5-chloro-phenoxy)-4-ethyl-phenyl]-*N*-(4-sulfamoyl-phenyl)-acetamide | | 193.7-195.9 |
| **I-16** | 2-[3-(2-Bromo-5-chloro-phenoxy)-4-ethyl-phenyl]-*N*-(2-chloro-phenyl)-acetamide | | 154-155.5 |
| **I-17** | 2-[3-(5-Chloro-2-cyano-phenoxy)-4-ethyl-phenyl]-*N*-(4-sulfamoyl-phenyl)-acetamide | | 198.1-202.6 |
| **I-18** | 2-[3-(5-Chloro-2-cyano-phenoxy)-4-ethyl-phenyl]-*N*-(2-chloro-phenyl)-acetamide | | 168-169.9 |
| **I-19** | 2-[3-(2,5-Dichloro-phenoxy)-4-ethyl-phenyl]-*N*-(4-sulfamoyl-phenyl)-acetamide | | 190-193.4 |
| **I-20** | 2-[3-(2,6-Dichloro-phenoxy)-4-ethyl-phenyl]-*N*-(4-sulfamoyl-phenyl)-acetamide | | 222.5-225 |
| **I-21** | *N*-(2-Chloro-phenyl)-2-[3-(2,5-dichloro-phenoxy)-4-ethyl-phenyl]-acetamide | | 146.3-147.1 |
| **I-22** | 2-[3-(3-Bromo-5-chloro-phenoxy)-4-ethyl-phenyl]-*N*-(4-sulfamoyl-phenyl)-acetamide | | 216.1-221.3 |
| **I-23** | *N*-(2-Chloro-phenyl)-2-[3-(2,6-dichloro-phenoxy)-4-ethyl-phenyl] -acetamide | | 107.0-110.5 |
| **I-24** | 2-[3-(3-Bromo-5-chloro-phenoxy)-4-ethyl-phenyl]-*N*-(2-chloro-phenyl)-acetamide | | 119.9-122.2 |
| **I-25** | *N*-(2-Chloro-phenyl)-2-[3-(3,5-dicyano-phenoxy)-4-ethyl-phenyl]-acetamide | 416 (M+H)⁺ | |
| **I-26** | 2- [3-(2-Bromo-5-chloro-phenoxy)-4-isopropyl-phenyl]-*N*-(4-sulfamoyl-phenyl)-acetamide | | 204.1-206.7 |
| **I-27** | 2-[3-(2-Bromo-5-chloro-phenoxy)-4-isopropyl-phenyl]-*N*-(2-chloro-phenyl)-acetamide | | 142.9-143.6 |
| **I-28** | 2- [4-Chloro-3-(3,5-dicyano-phenoxy)-2-fluoro-phenyl]-*N*-(2-chloro-phenyl)-acetamide | | 182.6-196.5 |
| **I-29** | 2-[4-Chloro-3-(3,5-dicyano-phenoxy)-phenyl]-*N*-[2-methyl-4-(3-sulfamoyl-propoxy)-phenyl]-acetamide | 539 (M)⁺ | |
| **I-30** | 2- [3-(2-Bromo-5-chloro-phenoxy)-4-methyl-phenyl]-*N*-(4-sulfamoyl-phenyl)-acetamide | | 236.9-239.3 |
| **I-31** | 2-[3-(2-Bromo-5-chloro-phenoxy)-4-methyl-phenyl]-*N*-(2-methyl-4-sulfamoyl-phenyl)-acetamide | | 200.5-203.5 |
| **I-32** | 2-[4-Chloro-3-(3,5-dicyano-phenoxy)-2-fluoro-phenyl]-*N*-(2-methyl-4-sulfamoyl-phenyl)-acetamide | 499 (M+H)⁺ | 248.4-250.8 |
| **I-33** | 2-[3-(2-Bromo-5-chloro-phenoxy)-4-methyl-phenyl]-*N*-(2-chloro-phenyl)-acetamide | | 133.9-135.9 |
| **I-34** | *N*-(2-Chloro-phenyl)-2-[3-(3,5-dicyano-phenoxy)-4-methyl-phenyl]-acetamide | | 175.1-177.5 |
| **I-35** | 2-[3-(3-Bromo-2,5-dichloro-phenoxy)-4-ethyl-phenyl]-*N*-(4-sulfamoyl-phenyl)-acetamide | | 206.9-209.9 |
| **I-36** | 2-[3-(3-Bromo-2,5-dichloro-phenoxy)-4-ethyl-phenyl]-*N*-(2-methyl-4-sulfamoyl-phenyl)-acetamide | | 213.2-219.6 |
| **I-37** | 2-[3-(3-Bromo-2,5-dichloro-phenoxy)-4-ethyl-phenyl]-*N*-(2-chloro-phenyl)-acetamide | | 146.5-147.6 |
| **I-38** | *N*-(2-Chloro-phenyl)-2-[3-(3,5-dibromo-2-chloro-phenoxy)-4-ethyl-phenyl]-acetamide | | 155.5-155.8 |
| **I-39** | 2-[3-(5-Bromo-2-chloro-3-fluoro-phenoxy)-4-ethyl-phenyl]-*N*-(4-sulfamoyl-phenyl)-acetamide | | 209.9-211.1 |
| **I-40** | 2-[3-(5-Bromo-2-chloro-3-fluoro-phenoxy)-4-ethyl-phenyl]-*N*-(2-methyl-4-sulfamoyl-phenyl)-acetamide | | 207.0-209.9 |
| **I-41** | 2-[3-(5-Bromo-2-chloro-3-fluoro-phenoxy)-4-ethyl-phenyl]-*N*-(2-chloro-phenyl)-acetamide | | 146.6-147.2 |
| **I-42** | 2-[4-Chloro-3-(3-chloro-5-cyano-phenoxy)-2-fluoro-phenyl]-*N*-(2-methyl-4-sulfamoyl-phenyl)-acetamide | 509 (M+H)⁺ | 254.7-260.0 |
| **I-43** | 2-[3-(3,5-Dicyano-phenoxy)-4-methyl-phenyl]-*N*-(2-methyl-4-sulfamoyl-phenyl)-acetamide | 459 (M-H)- | 228.6-230.9 |
| **I-44** | *N*-(2-Chloro-4-sulfamoyl-phenyl)-2-[3-(3,5-dicyano-phenoxy)-4-methyl-phenyl]-acetamide | 479 (M-H)⁻ | 209.9-211.5 |
| **I-45** | 2-[3-(3-Chloro-5-cyano-phenoxy)-4-ethyl- phenyl]-*N*-(4-sulfamoyl-phenyl)-acetamide | 468 (M-H)⁻ | 170.7-172.4- |
| **I-46** | 2-[3-(3-Chloro-5-cyano-phenoxy)-4-ethyl-phenyl]-*N-*(2-methyl-4-sulfamoyl-phenyl)-acetamide | 482 (M-H)⁻ | 189.1-189.4 |
| **I-47** | 2-[3-(3-Chloro-5-cyano-phenoxy)-4-ethyl-phenyl]-*N-*(2-chloro-4-sulfamoyl-phenyl)-acetamide | 502 (M-H)- | 181.1-183.9 |
| **I-48** | 2-[3-(3-Chloro-5-cyano-phenoxy)-4-ethyl-phenyl]-*N-*(4-methylsulfamoyl-phenyl)-acetamide | 482 (M-H)⁻ | 149.1-151.3 |
| **I-49** | 2- [3-(2-Chloro-S-cyano-3-fluoro-phenoxy)-4-ethyl-phenyl]-*N*-(4-sulfamoyl-phenyl)-acetamide | 486 (M-H)⁻ | 167.9-170.5 |
| **I-50** | 2-[3-(2-Chloro-5-cyano-3-fluoro-phenoxy)-4-ethyl-phenyl]-*N*-(2-methyl-4-sulfamoyl-phenyl)-acetamide | 500 (M-H)⁻ | 217.6-220.4 |
| **I-51** | 2-[4-Chloro-3-(3-chloro-5-cyano-phenoxy)-2-fluoro-phenyl]-*N*-(2-methyl-4-propionylsulfamoyl-phenyl)-acetamide | 563 (M-H)⁻ | 163.3-205.3 |
| **I-52** | 2-[3-(2-Chloro-3,5-dicyano-phenoxy)-4-ethyl-phenyl]-*N*-(4-sulfamoyl-phenyl)-acetamide | 493 (M-H)⁻ | 119.7-123.3 |
| **I-53** | 2-[3-(2-Chloro-3,5-dicyano-phenoxy)-4-ethyl-phenyl]-*N*-(2-methyl-4-sulfamoyl-phenyl)-acetamide | 507 (M-H)⁻ | 199.4-205.3 |
| **I-54** | 2-[3-(2-Chloro-3,5-dicyano-phenoxy)-4-ethyl-phenyl]-*N*-(2-chloro-4-sulfamoyl-phenyl)-acetamide | 527 (M-H)⁻ | 180.3-183.1 |
| **I-55** | 2-[4-Chloro-3-(4-cyano-2,6-dimethyl-phenoxy)-phenyl]-*N-*(4-sulfamoyl-phenyl)-acetamide | 468 (M-H)⁻ | 227.3-231.4 |
| **I-56** | 2-[4-Chloro-3-(4-cyano-2,6-dimethyl-phenoxy)-phenyl]-*N*-(2-methyl-4-sulfamoyl-phenyl)-acetamide | 482 (M-H)⁻ | 232.1-234.6 |
| **I-57** | 2-[3-(3-Chloro-5-cyano-phenoxy)-4-methoxy-phenyl]-*N*-(4-sulfamoyl-phenyl)-acetamide | 472 (M+H)⁺ | 218.3-221.4 |
| **I-58** | 2-[3-(3-Chloro-5-cyano-phenoxy)-4-ethyl-phenyl]-*N*-(2-chloro-phenyl)-acetamide | 425 (M+H)⁺ | |
| **I-59** | 2-[3-(2-Chloro-5-cyano-3-fluoro-phenoxy)-4-ethyl-phenyl]-*N*-(2-chloro-phenyl)-acetamide | 443 (M+H)⁺ | 142.1-142.8 |
| **I-60** | 2-[3-(2-Chloro-3,5-dicyano-phenoxy)-4-ethyl-phenyl]-*N*-(2-chloro-phenyl)-acetamide | 450 (M+H)⁺ | 110.0-111.5 |
| **I-61** | 2-[3-(3-Chloro-5-cyano-phenoxy)-4-methoxy-phenyl]-*N-*(2-chloro-phenyl)-acetamide | 427 (M+H)⁺ | 175.7-178.3 |
| **I-62** | 2-[3-(2-Chloro-5-cyano-phenoxy)-4-methoxy-phenyl]-*N-*(2-chloro-phenyl)-acetamide | 427 (M+H)⁺ | 194.0-197.8 |
| **I-63** | 2-[4-Bromo-3-(3-chloro-5-cyano-phenoxy)-2-fluoro-phenyl]-*N*-(2-chloro-4-propionylsulfamoyl-phenyl)-acetamide; sodium salt | 630 (M+H)⁺ | 164.8-166.2 |
| **I-64** | 2-[3-(3-Chloro-5-cyano-phenoxy)-4-methoxy-phenyl]-*N*-(2-chloro-4-sulfamoyl-phenyl)-acetamide | 505 (M)⁺ | 186.3-189.7 |
| **I-65** | 2- [3-(2-Chloro-5-cyano-phenoxy)-4-methoxy-phenyl]-*N*-(4-sulfamoyl-phenyl)-acetamide | 471 (M)⁺ | 252.3-254.3 |
| **I-66** | 2-[3-(2-Chloro-5-cyano-phenoxy)-4-methoxy-phenyl]-*N*-(2-methyl-4-sulfamoyl-phenyl)-acetamide | 485 (M)⁺ | 232.9-236.9 |
| **I-67** | 2-[3-(2-Chloro-5-cyano-phenoxy)-4-methoxy-phenyl]-*N*-(2-chloro-4-sulfamoyl-phenyl)-acetamide | 505 (M)⁺ | 214.6-216.4 |
| **I-68** | 2-[4-Chloro-3-(4-cyano-2,6-dimethyl-phenoxy)-phenyl]-*N*-(2-chloro-phenyl)-acetamide | 425 (M+H)⁺ | 183.9-185.1 |
| **I-69** | 2-[4-Chloro-3-(3,5-dicyano-phenoxy)-phenyl]-*N*-(4-methylsulfamoyl-phenyl)-acetamide | | |
| **I-70** | 2-[4-Chloro-3-(3,5-dicyano-phenoxy)-phenyl]-*N*-(4-dimethylsulfamoyl-phenyl)-acetamide | | |
| **I-71** | 2-[4-Chloro-3-(3,5-dicyano-phenoxy)-phenyl]-*N*-[4-(piperidine-1-sulfonyl)-phenyl] -acetamide | | |
| **I-72** | 2-[4-Chloro-3-(3,5-diryano-phenoxy)-phenyl]-*N-*[4-(morpholine-4-sulfonyl)-phenyl] -acetamide | | |
| **I-73** | 2-[3-(3-Chloro-5-cyano-phenoxy)-4-methoxy-phenyl]-*N*-(2-methyl-4-sulfamoyl-phenyl)-acetamide | 484 (M-H)⁻ | 222.3-224.3 |
| **I-74** | 2-[3-(3-Chloro-5-cyano-phenoxy)-2-fluoro-4-methyl-phenyl]-*N-*(2-methyl-4-sulfamoyl-phenyl)-acetamide | 486 (M-H)⁻ | 230.0-232.2 |
| **I-75** | 2-[4-Chloro-3-(3-cyano-5-difluoromethyl-phenoxy)-2-fluoro-phenyl]-*N*-(2-methyl-4-sulfamoyl-phenyl)-acetamide | 524 (M+H)⁺ | x |
| **I-76** | 2-{4-Chloro-3-(3,5-dicyano-phenoxy)-phenyl]-*N*-(2-chloro-4-methanesulfonyl-phenyl)-acetamide | | |
| **I-77** | *N*-[4-((S)-2-Amino-3-methyl-butyrylsulfamoyl)-2-methyl-phenyl]-2-[4-chloro-3-(3-chloro-5-cyano-phenoxy)-2-fluoro-phenyl]-acetamide; compound with hydrochloric acid | 607 (M+H)⁺ | 169.0-180.3 |
| **I-78** | 2-[4-Chloro-3-(3,5-dicyano-phenoxy)-phenyl]-*N*-(4-cyanomethoxy-phenyl)-acetamide | | |
| **I-79** | 2-[4-Chloro-3-(3,5-dicyano-phenoxy)-phenyl]-*N*-(4-methanesulfonyl-phenyl)-acetamide | | |
| **I-80** | 3-Chloro-4-{2-[4-chloro-3-(3,5-dicyano-phenoxy)-phenyl]-acetylamino}-benzoic acid methyl ester | | |
| **I-81** | 2-[4-Chloro-3-(3,5-dicyano-phenoxy)-phenyl]-*N*-{4-[(2-hydroxy-ethyl)-methyl-sulfamoyl]-phenyl}-acetamide | | |
| **I-82** | 2- [4-Chloro-3-(3,5-dicyano-phenoxy)-phenyl]-*N*-[4-(4-hydroxy-piperidine-1-sulfonyl)-phenyl]-acetamide | | |
| **I-83** | 2-[3-(3-Chloro-5-cyano-phenoxy)-2-fluoro-4-methyl-phenyl]-*N*-(2-chloro-4-sulfamoyl-phenyl)-acetamide | 506 (M-H)⁻ | 228.9-230.6 |
| **I-84** | *N*-(2-Chloro-4-sulfamoyl-phenyl)-2-[3-(3-cyano-5-difluoromethyl-phenoxy)-2-fluoro-4-methyl-phenyl] -acetamide | 522 (M-H)⁻ | 218.0-218.7 |
| **I-85** | 2-[3-(3-Cyano-5-difluoromethyl-phenoxy)-2-fluoro-4-methyl-phenyl]-*N-*(2-methyl-4-sulfamoyl-phenyl)-acetamide | 504 (M+H)⁺ | 218.0-220.4 |
| **I-86** | *N*-(2-Chloro-phenyl)-2-[3-(3-cyano-5-difluoromethyl-phenoxy)-2-fluoro-4-methyl-phenyl]-acetamide | 445 (M+H)⁺ | 163.3-164.4 |
| **I-87** | 2-[4-Chloro-3-(3-chloro-5-cyano-phenoxy)-2-fluoro-phenyl]-*N*-{2-methyl-4-[(pyridine-3-carbonyl)-sulfamoyl]-phenyl}-acetamide; compound with hydrochloric acid | 613 (M+H)⁺ | 261.8-263.6 |
| **I-88** | 2-[4-Chloro-3-(3-cyano-5-trifluoromethyl-phenoxy)-phenyl]-*N*-(4-sulfamoyl-phenyl)-acetamide | 508 (M-H)⁻ | 219-221.4 |
| **I-89** | 2-[4-Chloro-3-(3-cyano-5-trifluoromethyl-phenoxy)-phenyl]-*N*-(2-methyl-4-sulfamoyl-phenyl)-acetamide | 522 (M-H)⁻ | 211.6-214.6 |
| **I-90** | 2-[4-Chloro-3-(3-cyano-5-trifluoromethyl-phenoxy)-phenyl]-*N*-(2-chloro-4-sulfamoyl-phenyl)-acetamide | 542 (M-H)⁻ | 198.0-202.0 |
| **I-91** | 2-[3-(3-Cyano-5-difluoromethyl-phenoxy)-4-ethyl-phenyl]-*N*-(4-sulfamoyl-phenyl)-acetamide | 486 (M+H)⁺ | 188.0-198.0 |
| **I-92** | 2-[3-(3-Cyano-5-difluoromethyl-phenoxy)-4-methoxy-phenyl]-*N-*(4-sulfamoyl-phenyl)-acetamide | 488 (M+H)⁺ | 213.9-214.2 |
| **I-93** | 2-[3-(3-Cyano-5-difluoromethyl-phenoxy)-4-methoxy-phenyl]-*N-*(2-methyl-4-sulfamoyl-phenyl)-acetamide | 502 (M+H)⁺ | 175.0-177.2 |
| **I**-**94** | *N*-(2-Chloro-4-sulfamoyl-phenyl)-2-[3-(3-cyano-5-difluoromethyl-phenoxy)-4-methoxy-phenyl]-acetamide | 520 (M-H)⁻ | 185.0-187.9 |
| **I-95** | 2-{4-Chloro-3-[3-cyano-5-(1,1-difluoro-ethyl)-phenoxy]-2-fluoro-phenyl}-*N*-(4-sulfamoyl-phenyl)-acetamide | 522 (M-H)⁻ | 207.0-209.8 |
| **I-96** | 2-(4-Chloro-3-[3-cyano-5-(1,1-difluoro-ethyl)-phenoxy]-2-fluoro-phenyl}-*N*-(2-methyl-4-sulfamoyl-phenyl)-acetamide | 538 (M+H)⁺ | 198.5-201.0 |
| **I-97** | 2-{4-Chloro-3-[3-cyano-5-(1,1-difluoro-ethyl)-phenoxy]-2-fluoro-phenyl}-*N*-(2-chloro-4-sulfamoyl-phenyl)-acetamide | 556 (M-H)⁻ | 192.0-197.6 |
| **I-98** | 2-[4-Chloro-3-(3-chloro-5-cyano-phenoxy)-2-fluoro-phenyl]-*N*-(2-chloro-phenyl)-acetamide | 448 (M-H)⁻ | 181.3-184.0 |
| **I-99** | 2-[4-Chloro-3-(3-chloro-5-cyano-phenoxy)-2-fluoro-phenyl]-*N*-[2-methyl-4-(methyl-propionyl-sulfamoyl)-phenyl]-acetamide | 578 (M+H)⁺ | 212.0-216.9 |
| **I-100** | 2-[4-Chloro-3-(3-chloro-5-cyano-phenoxy)-2-fluoro-phenyl]-*N*-(2-chloro-4-sulfamoyl-phenyl)-acetamide | | 234.4-234.9 |
| **I**-**101** | 2-[3-(3-Cyano-5-difluoromethyl-phenoxy)-4-ethyl-phenyl]-*N*-(2-methyl-4-sulfamoyl-phenyl)-acetamide | 500 (M+H)⁺ | |
| **I-102** | 2-[3-(2-Chloro-5-cyano-phenoxy)-5-methoxy-phenyl]-*N*-(4-sulfamoyl-phenyl)-acetamide | 472 (M+H)⁺ | 180.5-181.3 |
| **I-103** | 2-[3-(2-Chloro-5-cyano-phenoxy)-5-methoxy-phenyl]-*N*-(2-methyl-4-sulfamoyl-phenyl)-acetamide | 486 (M+H)⁺ | 179.8-184.0 |
| **I**-**104** | 2-[4,5-Dibromo-3-(3-cyano-5-difluoromethyl-phenoxy)-2-fluoro-phenyl]-*N*-(2-methyl-4-sulfamoyl-phenyl)-acetamide | 646 (M+H)⁺ | |
| **I-105** | 2-[3-(2-Chloro-5-cyano-phenoxy)-5-ethyl-phenyl]-*N*-(4-sulfamoyl-phenyl)-acetamide | 470 (M+H)⁺ | |
| **I-106** | 2-[3-(2-Chloro-5-cyano-phenoxy)-5-ethyl-phenyl]-*N*-(2-methyl-4-sulfamoyl-phenyl)-acetamide | 484 (M+H)⁺ | 231.6-233.1 |
| **I-107** | 2-[3-(2-Chloro-5-cyano-phenoxy)-5-ethyl-phenyl]-*N*-(2-chloro-4-sulfamoyl-phenyl)-acetamide | 503 (M)⁺ | 204.0-207.0 |
| **I-108** | 2-[4-Chloro-3-(3-chloro-5-cyano-phenoxy)-2-fluoro-phenyl]-*N*-(2-methyl-4-methylsulfamoyl-phenyl)-acetamide | 522 (M+H)⁺ | 204.4-207.3 |
| **I-109** | 2-[4-Bromo-3-(3-chloro-5-cyano-phenoxy)-2-fluoro-phenyl]-*N*-(2-chloro-4-sulfamoyl-phenyl)-acetamide | 572 (M-H)⁻ | 165.0-166.6 |
| **I-110** | 3-Chloro-4-{2-[3-(3-cyano-5-difluoromethyl-phenoxy)-2-fluoro-4-methyl-phenyl]-acetylamino}-benzoic acid | 487 (M-H)⁻ | 216.9-218.3 |
| **I-111** | 2-[4-Chloro-3-(3-chloro-5-cyano-phenoxy)-2-fluoro-phenyl]-*N*-(2-chloro-phenyl)-*N*-methyl-acetamide | 463 (M+H)⁺ | 134.0-137.1 |
| **I-112** | *N*-(2-Chloro-4-methanesulfonyl-phenyl)-2-[3-(3-cyano-5-difluoromethyl-phenoxy)-2-fluoro-4-methyl-phenyl]-acetamide | 521 (M-H)⁻ | 179.9-182.0 |
| **I-113** | 2- [3-(2-Chloro-5-cyano-phenoxy)-5-methoxy-phenyl]-*N*-(2-chloro-4-sulfamoyl-phenyl)-acetamide | 506 (M+H)⁺ | |
| **I-114** | 2-[4,5-Dibromo-3-(3-cyano-5-difluoromethyl-phenoxy)-2-fluoro-phenyl]-*N*-(4-sulfamoyl-phenyl)-acetamide | 631 (M)⁺ | 144.0-147.0 |
| **I-115** | 2-[3-(3-Chloro-5-cyano-phenoxy)-4-methyl-phenyl]-*N*-[2-methyl-4-(morpholine-4-sulfonyl)-phenyl]-acetamide | 540 (M+H)⁺ | |
| **I-116** | 2-[3-(3-Chloro-5-cyano-phenoxy)-4-methyl-phenyl]-*N*-[2-methyl-4-(4-methyl-piperazine-1-sulfonyl)-phenyl]-acetamide | 553 (M+H)⁺ | |
| **I-117** | 2-[3-(3-Chloro-5-cyano-phenoxy)-4-methyl-phenyl]-*N*-[2-methyl-4-(thiomorpholine-4-sulfonyl)-phenyl]-acetamide | 554 (M-H)⁻ | 151.9-155.3 |
| **I-118** | 2-[3-(3-Chloro-5-cyano-phenoxy)-4-methyl-phenyl]-*N*-[2-methyl-4-(2-morpholin-4-yl-ethylsulfamoyl)-phenyl] -acetamide | 581 (M-H)⁻ | 177.0-181.0 |
| **I-119** | 2-[3-(3-Chloro-5-cyano-phenoxy)-4-methyl-phenyl]-*N*-[4-(3-hydroxy-propylsulfamoyl)-2-methyl-phenyl]-acetamide | 528 (M+H)⁺ | |
| **I-120** | 2-[4-Chloro-3-(3-cyano-5-trifluoromethyl-phenoxy)-2-fluoro-phenyl]-*N*-(2-methyl-4-sulfamoyl-phenyl)-acetamide | 540 (M-H)⁻ | 212.0-216.1 |
| **I-121** | 2-[3-(3-Chloro-5-cyano-phenoxy)-4-methyl-phenyl]-*N*-[4-(4-hydroxy-cyclohexylsulfamoyl)-2-methyl-phenyl]-acetamide | 568 (M+H)⁺ | 195.1-197.7 |
| **I-122** | 2-[3-(3-Chloro-5-cyano-phenoxy)-4-methyl-phenyl]-*N*-(2-methyl-4-sulfamoyl-phenyl)-acetamide | 468 (M-H)⁻ | 192.8-194.1 |
| **I-123** | 2-[3-(3-Chloro-5-cyano-phenoxy)-4-methyl-phenyl]-*N*-(2-chloro-4-sulfamoyl-phenyl)-acetamide | 488 (M-H)- | 194.0-194.9 |
| **I**-**124** | 2-[4-Chloro-3-(4-cyano-6-methyl-pyridin-2-yloxy)-2-fluoro-phenyl]-*N*-(2-methyl-4-sulfamoyl-phenyl)-acetamide | 489 (M+H)⁺ | 230.1-230.7 |
| **I-125** | 3-(4-{2-[3-(3-Chloro-5-cyano-phenoxy)-4-methyl-phenyl]-acetylamino}-3-methyl-benzenesulfonyl)-propionic acid | 525 (M-H)⁻ | 74.3-76.5 |
| **I-126** | 2-[4-Chloro-3-(3-chloro-5-cyano-phenoxy)-2-fluoro-phenyl]-*N*-(2-chloro-4-propionylsulfamoyl-phenyl)-acetamide; sodium salt | | 155.4-157.5 |
| **I-127** | *N*-[4-((S)-2-Amino-3-methyl-butyrylsulfamoyl)-2-chloro-phenyl)]-2-[4-chloro-3-(3-chloro-5-cyano-phenoxy)-2-fluoro-phenyl]-acetamide; sodium salt | 627 (M+H)⁺ | 219.4-220.2 |
| **I-128** | 2-[3-(3-Chloro-5-cyano-phenoxy)-4-methyl-phenyl]-*N*-[2-methyl-4-(tetrahydro-pyran-4-ylsulfamoyl)-phenyl]-acetamide | 554 (M+H)⁺ | 171.7-172.6 |
| **I-129** | 2-[3-(3-Chloro-5-cyano-phenoxy)-4-methyl-phenyl]-*N*-[4-(2-hydroxy-propylsulfamoyl)-2-methyl-phenyl]-acetamide | 528 (M+H)⁺ | 172.2-173.4 |
| **I-130** | 2-[3-(3-Chloro-5-cyano-phenoxy)-4-methyl-phenyl]-*N*-[2-methyl-4-(2-methylsulfanyl-ethylsulfamoyl)-phenyl] -acetamide | 544 (M+H)⁺ | 155.6-156.6 |
| **I-131** | 2-[3-(3-Chloro-5-cyano-phenoxy)-4-methyl-phenyl]-*N*-[2-methyl-4-(3-methylsulfanyl-propylsulfamoyl)-phenyl]-acetamide | 558 (M+H)⁺ | 159.9-162.8 |
| **I-132** | 2-[3-(3-Chloro-5-cyano-phenoxy)-5-methoxy-phenyl]-*N*-(4-sulfamoyl-phenyl)-acetamide | 472 (M+H)⁺ | 184.1-185.5 |
| **I-133** | 2-[3-(3-Chloro-5-cyano-phenoxy)-5-methoxy-phenyl]-*N*-(2-methyl-4-sulfamoyl-phenyl)-acetamide | 486 (M+H)⁺ | 169.9-171.1 |
| **I-134** | *N*-(2-Chloro-4-sulfamoyl-phenyl)-2-[4,5-dibromo-3-(3-cyano-5-difluoromethyl-phenoxy)-2-fluoro-phenyl] -acetamide | 664 (M-H)⁻ | |
| **I-135** | 2-[4-Chloro-3-(4-cyano-2,6-dimethyl-phenoxy)-phenyl]-*N*-(2-chloro-4-sulfamoyl-phenyl)-acetamide | 502 (M-H)⁻ | |
| **I-136** | 2-[3-(3-Chloro-5-cyano-phenoxy)-5-methoxy-phenyl]-*N*-(2-chloro-4-sulfamoyl-phenyl)-acetamide | 505 (M)⁺ | 159.0-161.1 |
| **I-137** | 2-[3-(3-Chloro-5-cyano-phenoxy)-4-methyl-phenyl]-*N*-(4-(2-cyano-ethanesulfonyl)-2-methyl-phenyl]-acetamide | 508 (M+H)⁺ | 186.1-188.0 |
| **I-138** | *N*-(4-Butyrylsulfamoyl-2-chloro-phenyl)-2-[4-chloro-3-(3-chloro-5-cyano-phenoxy)-2-fluoro-phenyl]-acetamide; sodium salt | 598 (M+H)⁺ | 157.2-157.9 |
| **I-139** | *N*-[4-(Butyryl-methyl-sulfamoyl)-2-chloro-phenyl]-2-[4-chloro-3-(3-chloro-5-cyano-phenoxy)-2-fluoro-phenyl]-acetamide | 612 (M+H)⁺ | 186.9-187.6 |
| **I-140** | *N*-(2-Chloro-4-sulfamoyl-phenyl)-2-[3-(3-cyano-5-difluoromethyl-phenoxy)-4-ethyl-phenyl]-acetamide | 518 (M-H)⁻ | 191.8-192.5 |
| **I-141** | 2-[3-(3-Chloro-5-cyano-phenoxy)-5-methyl-phenyl]-*N*-(4-sulfamoyl-phenyl)-acetamide | 454 (M-H)⁻ | 183.9-185.2 |
| **I-142** | 2-[3-(3-Chloro-5-cyano-phenoxy)-5-methyl-phenyl]-*N*-(2-methyl-4-sulfamoyl-phenyl)-acetamide | 468 (M-H)⁻ | 184.8-186.0 |
| **I-143** | 2-[3-(3-Chloro-5-cyano-phenoxy)-5-methyl-phenyl]-*N*-(2-chloro-4-sulfamoyl-phenyl)-acetamide | 488 (M-H)⁻ | 194.1-195.9 |
| **I-144** | 2-[7-(3-Cyano-phenoxy)-benzofuran-5-yl]-*N*-(4-sulfamoyl-phenyl)-acetamide | 446 (M-H)⁻ | 196.4-198.7 |
| **I-145** | 2-[7-(3-Cyano-phenoxy)-benzofuran-5-yl]- *N*-(2-methyl-4-sulfamoyl-phenyl)-acetamide | 460 (M-H)⁻ | 218.9-220.0 |
| **I-146** | 2-[3-(2-Chloro-5-cyano-phenoxy)-5-methyl-phenyl]-*N*-(4-sulfamoyl-phenyl)-acetamide | 454 (M-H)⁻ | 235.9-236.7 |
| **I-147** | 2-[3-(2-Chloro-5-cyano-phenoxy)-5-methyl-phenyl]-*N*-(2-methyl-4-sulfamoyl-phenyl)-acetamide | 468 (M-H)⁻ | 200.3-201.1 |
| **I**-**148** | 2-[3-(2-Chloro-5-cyano-phenoxy)-5-methyl-phenyl]-*N*-(2-chloro-4-sulfamoyl-phenyl)-acetamide | 488 (M-H)⁻ | 212.9-214.4 |
| **I-149** | *N*-(2-Chloro-4-sulfamoyl-phenyl)-2-[7-(3,5-dichloro-phenoxy)-benzofuran-5-yl]-acetamide | 523 (M-H)⁻ | 215.1-216.6 |
| **I-150** | 2-[3-(3-Chloro-5-cyano-phenoxy)-4-methyl-phenyl]-*N*-[4-((S)-2,3-dihydroxy-propoxy)-2-methyl-phenyl]-acetamide | 481 (M+H)⁺ | 137.5-138.4 |
| **I**-**151** | 2-[3-(3-Chloro-5-cyano-phenoxy)-4-methyl-phenyl]-*N*-[4-((R)-3,4-dihydroxy-butoxy)-2-methyl-phenyl]-acetamide | 495 (M+H)⁺ | 104.9-106.4 |
| **I-152** | (4-{2-[3-(3-Chloro-5-cyano-phenoxy)-4-methyl-phenyl]-acetylamino}-3-methyl-benzenesulfonyl)-acetic acid | 513 (M+H)⁺ | 180.5-181.4 |
| **I-153** | *N*-[4-(3-Amino-propionylsulfamoyl)-2-chloro-phenyl] -2- [4-chloro-3-(3-chloro-5-cyano-phenoxy)-2-fluoro-phenyl]-acetamide; compound with hydrochloric acid | 599 (M+H)⁺ | 199.3-199.6 |
| **I-154** | *N*-[4-(2-Amino-ethanesulfonyl)-2-methyl-phenyl]-2-[3-(3-chloro-5-cyano-phenoxy)-4-methyl-phenyl]-acetamide | 498 (M+H)⁺ | 92.4-95.5 |
| **I-155** | 2-[4-Chloro-3-(3-chloro-5-cyano-phenoxy)-2-fluoro-phenyl]-*N*-[2-chloro-4-(2-methoxy-acetylsulfamoyl)-phenyl]-acetamide; sodium salt | 598 (M-H)⁻ | 151.7-152.3 |
| **I-156** | 2-[4-Chloro-3-(3-chloro-5-cyano-phenoxy)-2-fluoro-phenyl]-*N*-[2-chloro-4-(2-hydroxy-acetylsulfamoyl)-phenyl]-acetamide; sodium salt | 584 (M-H)⁻ | 178.0-179.5 |
| **I-157** | 2-[3-(3-Chloro-5-cyano-phenoxy)-4-methyl-phenyl]-*N*-[4-(3-hydroxy-propane-1-sulfonyl)-2-methyl-phenyl] -acetamide | 513 (M+H)⁺ | 78.9-79.5 |
| **I-158** | 2-[3-(3-Chloro-5-cyano-phenoxy)-4-methyl-phenyl]-*N*-[4-(1,1-dioxo-1λ⁶-[1,2]thiazinan-2-yl)-2-methyl-phenyl] -acetamide | 522 (M-H)⁻ | 84.3-85.6 |
| **I-159** | 2-(3-(3-Chloro-5-cyano-phenoxy)-4-methyl-phenyl]-*N*-{2-methyl-4-[2-(4-methyl-piperazin-1-yl)-acetyl]-phenyl}-acetamide | 531 (M+H)⁺ | 62.3-64.1 |
| **I-160** | 2-[3-(3-Chloro-5-cyano-phenoxy)-4-methyl-phenyl]-*N*-[2-methyl-4-(1-oxo-1λ⁴-thiomorpholin-4-yl)-phenyl]-acetamide | 508 (M+H)⁺ | 181.3-182.1 |
| **I-161** | 2-[3-(3-Chloro-5-ryano-phenoxy)-4-methyl-phenyl]-*N*-[4-(1,1-dioxo-1λ⁶-thiomorpholin-4-yl)-2-methyl-phenyl] -acetamide | 524 (M+H)⁺ | 64.8-65.4 |
| **I-162** | 2-[4-Chloro-3-(3-chloro-5-cyano-phenoxy)-2-fluoro-phenyl]-*N*-[2-chloro-4-(methyl-propionyl-sulfamoyl)-phenyl]-acetamide | 598 (M+H)⁺ | 198.2-200.6 |
| **I-163** | 2-[3-(3-Chloro-5-cyano-phenoxy)-4-methyl-phenyl]-*N*-[2-methyl-4-(2-morpholin-4-yl-acetyl)-phenyl]-acetamide; compound with trifluoro-acetic acid | 518 (M+H)⁺ | 110.3-110.5 |
| **I-164** | 2-[4-Chloro-3-(3-chloro-5-cyano-phenoxy)-2-fluoro-phenyl]-*N*-[2-chloro-4-(3-methoxy-propionylsulfamoyl)-phenyl]- acetamide; sodium salt | 614 (M+H)⁺ | 133.1-140.7 |
| **I-165** | 2-[4-Bromo-3-(3-cyano-5-difluoromethyl-phenoxy)-2-fluoro-phenyl]-*N*-(2-chloro-4-sulfamoyl-phenyl)-acetamide | 586 (M-H)⁻ | 205.4-207.7 |
| **I-166** | 2-[4-Chloro-3-(3-cyano-5-difluoromethoxy-phenoxy)-2-fluoro-phenyl]-*N*-(4-sulfamoyl-phenyl)-acetamide | 524 (M-H)⁻ | |
| **I-167** | 2-[4-Chloro-3-(3-cyano-5-difluoromethoxy-phenoxy)-2-fluoro-phenyl]-*N*-(2-chloro-4-sulfamoyl-phenyl)-acetamide | 558 (M-H)⁻ | |
| **I**-**168** | 2-[4-Chloro-3-(3-chloro-5-cyano-phenoxy)-2-fluoro-phenyl]-*N*-(2-chloro-4-methylsulfamoyl-phenyl)-acetamide | 540 (M-H)⁻ | 199.1-200.0 |
| **I-169** | 2-[4-Bromo-3-(3-cyano-5-difluoromethyl-phenoxy)-2-fluoro-phenyl]-*N*-(2-methyl-4-sulfamoyl-phenyl)-acetamide | 566 (M-H)⁻ | 223.9-225.0 |
| **I-170** | 2-[3-(3-Chloro-5-cyano-phenoxy)-4-methyl-phenyl]-*N*-[4-(1,1-dioxo-1λ⁶-isothiazolidin-2-yl)-2-methyl-phenyl] -acetamide | 510 (M+H)⁺ | 182.5-184.6 |
| **I-171** | 2-[4-Chloro-3-(3-chloro-5-cyano-phenoxy)-2-fluoro-phenyl]-*N*-{2-chloro-4-[(2-methoxy-acetyl)-methyl-sulfamoyl]-phenyl}-acetamide | 614 (M+H)⁺ | 183.2-185.5 |
| **I-172** | 2-[4-Chloro-3-(3-chloro-5-cyano-phenoxy)-2-fluoro-phenyl]-*N-*{2-chloro-4-[(3-methoxy-propionyl)-methyl-sulfamoyl] -phenyl}-acetamide | 626 (M-H)⁻ | 199.3-203 |
| **I-173** | 2-[4-Chloro-3-(3-chloro-5-cyano-phenoxy)-2-fluoro-phenyl]-*N-*(2-chloro-4-pentanoylsulfamoyl-phenyl)-acetamide; sodium salt | 612 (M+H)⁺ | 157.6-159.2 |
| **I-174** | 2-[4-Chloro-3-(3-chloro-5-cyano-phenoxy)-2-fluoro-phenyl]-*N*-[2-chloro-4-(3-methyl-butyrylsulfamoyl)-phenyl] -acetamide; sodium salt | 610 (M-H)⁻ | 159.3-163.4 |
| **I-175** | 2-[4-Chloro-3-(3-chloro-5-cyano-phenoxy)-2-fluoro-phenyl]-*N*-[2-chloro-4-(2-diethylamino-acetylsulfamoyl)-phenyl]-acetamide; compound with hydrochloric acid | 641 (M+H)⁺ | 195.3-197.2 |
| **I-176** | 2-[4-Chloro-3-(3-chloro-5-cyano-phenoxy)-2-fluoro-phenyl]-*N*-[2-chloro-4-(4-methyl-pentanoylsulfamoyl)-phenyl]-acetamide; sodium salt | 626 (M+H)⁺ | 195.6-197.0 |
| **I-177** | 2-[4-Chloro-3-(3-chloro-5-cyano-phenoxy)-2-fluoro-phenyl]-*N*-[2-chloro-4-(2-morpholin-4-yl-acetylsulfamoyl)-phenyl]-acetamide; compound with hydrochloric acid | 655 (M+H)⁺ | 223.3-225.0 |
| **I**-**178** | *N*-(4-{2-[3-(3-Chloro-5-cyano-phenoxy)-4-methyl-phenyl]-acetylamino}-3-methyl-phenyl)-oxalamic acid ethyl ester | 506(M+H)⁺ | 182.5-183.9 |
| **I-179** | *N*-(2-Chloro-4-sulfamoyl-phenyl)-2-[3-(3-cyano-5-difluoromethyl-phenoxy)-4-ethyl-2-fluoro-phenyl]-acetamide | | 239.6-240.7 |
| **I-180** | 2-[3-(3-Cyano-5-difluoromethyl-phenoxy)-4-ethyl-2-fluoro-phenyl]-*N*-(2-methyl-4-sulfamoyl-phenyl)-acetamide | | 237.6-238.4 |
| **I-181** | *N*-{4-[(3-Amino-propionyl)-methyl-sulfamoyl] -2-chloro-phenyl}-2- [4-chloro-3-(3-chloro-5-cyano-phenoxy)-2-fluoro-phenyl]-acetamide; compound with hydrochloric acid | 650 (M+H)⁺ | >300 |
| **I-182** | 2-[3-(3-Chloro-5-cyano-phenoxy)-4,5-dimethyl-phenyl]-*N*-(4-sulfamoyl-phenyl)-acetamide | 468 (M-H)⁻ | 229.7-231.3 |
| **I-183** | 2-[3-(3-Chloro-5-cyano-phenoxy)-4,5-dimethyl-phenyl]-*N*-(2-methyl-4-sulfamoyl-phenyl)-acetamide | 482 (M-H)⁻ | 230.8-233.4 |
| **I**-**184** | 2-[3-(3-Chloro-5-cyano-phenoxy)-4,5-dimethyl-phenyl]-*N*-(2-chloro-4-sulfamoyl-phenyl)-acetamide | 502 (M-H)⁻ | 224.3-225.7 |
| **I-185** | 2-[3-(3-Bromo-5-cyano-phenoxy)-4-chloro-2-fluoro-phenyl]-*N*-(2-methyl-4-sulfamoyl-phenyl)-acetamide | 552 (M-H)⁻ | 246.0-250.6 |
| **I-186** | 2-(4-Chloro-3-[3-cyano-5-(3-hydroxy-prop-1-ynyl)-phenoxy]-2-fluoro-phenyl}-*N*-(2-methyl-4-sulfamoyl-phenyl)-acetamide | 526 (M-H)⁻ | 170.1-173.2 |
| **I-187** | 2-{4-Chloro-3-[3-cyano-5-(3-dimethylamino-prop-1-ynyl)-phenoxy]-2-fluoro-phenyl}-*N*-(2-methyl-4-sulfamoyl-phenyl)-acetamide | 555 (M+H)⁺ | 156.2-158.2 |
| **I-188** | 4-(3-Chloro-4-{2-[4-chloro-3-(3-chloro-5-cyano-phenoxy)-2-fluoro-phenyl]-acetylamino}-benzenesulfonylamino)-4-oxo-butyric acid | | 143.4-144.0 |
| I**-189** | 2-[4-Bromo-3-(3-chloro-5-cyano-phenoxy)-2-fluoro-phenyl]-*N*-(2-methyl-4-sulfamoyl-phenyl)-acetamide | 551 (M-H)⁻ | 244-245.1 |
| **I-190** | 2-[3-(3-Chloro-5-cyano-phenoxy)-2-fluoro-4-methoxy-phenyl]-*N*-(2-chloro-4-sulfamoyl-phenyl)-acetamide | 522(100%), 534(60%) (M-H)⁻ | 212.0-212.8 |
| **I-191** | 2-[3-(3-Chloro-5-cyano-phenoxy)-2-fluoro-4-methoxy-phenyl]-*N*-(2-methyl-4-sulfamoyl-phenyl)-acetamide | 502, 504(35%) (M-H)⁻ | 239.9-240.8 |
| **I-192** | 2-[4-Chloro-3-(3-chloro-phenoxy)-phenyl]- *N*-phenyl-acetamide | 372 (M)⁺ | 126.8-127.7 |
| **I-193** | 2-[3-(2-Bromo-5-chloro-phenoxy)-4-chloro-phenyl]-*N*-(4-sulfamoyl-phenyl)-acetamide | | 260.1-261.9 |
| **I-194** | 2-[4-Chloro-3-(3,5-dicyano-phenoxy)-phenyl]-*N*-(2-pyrrol-1-yl-phenyl)-acetamide | 453 (M+H)⁺ | |
| **I-195** | 2-[4-Chloro-3-(3,5-dicyano-phenoxy)-phenyl]-*N*-(2-fluoro-phenyl)-acetamide | 406 (M+H)⁺ | |
| **I-196** | 2-[4-Chloro-3-(3,5-dicyano-phenoxy)-phenyl]-*N*-(2-methoxy-phenyl)-acetamide | 418 (M+H)⁺ | |
| **I-197** | 2-[4-Chloro-3-(3,5-dicyano-phenoxy)-phenyl]-*N*-(2-isopropyl-phenyl)-acetamide | 430 (M+H)⁺ | |
| **I-198** | 2-[4-Chloro-3-(3,5-dicyano-phenoxy)-phenyl]-*N*-*o*-tolyl-acetamide | 402 (M+H)⁺ | |
| **I-199** | 2-(4-Chloro-3-(3,5-dicyano-phenoxy)-phenyl]-*N*-(2-ethyl-phenyl)-acetamide | 416 (M+H)⁺ | |
| **I-200** | 2-[4-Chloro-3-(3,5-dicyano-phenoxy)-phenyl]-*N*-(2,3-dimethyl-phenyl)-acetamide | 416 (M+H)⁺ | |
| **I-201** | 2-[4-Chloro-3-(3,5-dicyano-phenoxy)-phenyl]-*N*-(4-methoxy-2-methyl-phenyl)-acetamide | 432 (M+H)⁺ | |
| **I-202** | 2-[4-Chloro-3-(3,5-dicyano-phenoxy)-phenyl]-*N*-(4-cyclohexyl-phenyl)-acetamide | 471 (M+H)⁺ | |
| **I-203** | 2-[4-Chloro-3-(3,5-dicyano-phenoxy)-phenyl]-*N*-(2-cyano-phenyl)-acetamide | 413(M+H)⁺ | |
| **I-204** | 2-[4-Chloro-3-(3,5-dicyano-phenoxy)-phenyl]-*N*-(2-trifluoromethyl-phenyl)-acetamide | 456 (M+H)⁺ | |
| **I-205** | 2-{2-[4-Chloro-3-(3,5-dicyano-phenoxy)-phenyl]-acetylamino}-4,5-dimethoxy benzoic acid methyl ester | 506 (M+H)⁺ | |
| **I-207** | 2-[3-(3-Chloro-5-cyano-phenoxy)-4-ethyl-phenyl]-*N*-phenyl-acetamide | 390 (M+H)⁺ | |
| **I-208** | 2-[3-(2-Chloro-3,5-dicyano-phenoxy)-4-ethyl-phenyl]-*N*-phenyl-acetamide | 416 (M+H)⁺ | 189.3-192.3 |
| **I-209** | 2- [4-Chloro-3-(2,5-dichloro-benzyl)-phenyl)-*N*-(2-chloro-4-sulfamoyl-phenyl)-acetamide | | 219.3-221.5 |
| **I-210** | 2-[3-(3-Chloro-5-cyano-phenoxy)-4-methoxy-phenyl]-*N*-phenyl-acetamide | 393 (M+H)⁺ | 171.3-171.6 |
| **I-211** | 2-[4-Chloro-3-(3,5-dicyano-phenoxy)-2-[4-Chloro-3-(3,5-dicyano-phenoxy)-phenyl)-*N*-phenyl-acetamide | 386 (M-H)⁻ | 199.0-201.9 |
| **I-212** | 2-[4-Chloro-3-(3,5-dicyano-phenoxy)-phenyl]-*N*-(4-methyl-3-sulfamoyl-phenyl)-acetamide | | |
| **I-213** | 2-(4-Chloro-3-(3,5-dicyano-phenoxy)-phenyl]-*N*-(3-methanesulfonyl-phenyl)-acetamide | | |
| **I-214** | (4-{2-[4-Chloro-3-(3,5-dicyano-phenoxy)-phenyl]-acetylamino}-phenoxy)-acetic acid methyl ester | | |
| **I-215** | 3-(4-{2-[4-Chloro-3-(3,5-dicyano-phenoxy)-phenyl]-acetylamino}-phenyl)-propionic acid methyl ester | | |
| **I-216** | (4-{2-[4-Chloro-3-(3,5-dicyano-phenoxy)-phenyl]-acetylamino}-phenylsulfanyl)-acetic acid methyl ester | | |
| **I-217** | 4-(4-{2-[4-Chloro-3-(3,5-dicyano-phenoxy)-phenyl]-acetylamino}-phenyl)-butyric acid methyl ester | | |
| **I-218** | 2-[4-Chloro-3-(3,5-dicyano-phenoxy)-phenyl]-*N*-(2-fluoro-5-methanesulfonyl-phenyl)-acetamide | | |
| **I-219** | (4-{2-[4-Chloro-3-(3,5-dicyano-phenoxy)-phenyl]-acetylamino}-phenyl)-acetic acid methyl ester | | |
| **I-220** | 2-[4-Chloro-3-(3,5-dicyano-phenoxy)-phenyl]-*N*-(4-cyano-phenyl)-acetamide | | |
| **I-221** | 4-{2-[4-Chloro-3-(3,5-dicyano-phenoxy)-phenyl]-acetylamino}-benzoic acid methyl ester | | |
| **I-222** | 2-[4-Chloro-3-(3,5-dicyano-phenoxy)-phenyl]-*N*-(4-hydroxy-phenyl)-acetamide | | |
| **I-224** | 2-[3-(3-Chloro-5-cyano-phenoxy)-4-methyl-phenyl]-*N*-(4-methyl-pyridin-3-yl)-acetamide | 392 (M+H)⁺ | 158.6-159.1 |
| **I-225** | 2-[3-(3-Chloro-5-cyano-phenoxy)-4-methyl-phenyl]-*N*-(3-methyl-pyridin-2-yl)-acetamide | 392 (M+H)⁺ | 137.9-138.8 |
| I**-226** | 2-[3-(3-Chloro-5-cyano-phenoxy)-4-methyl-phenyl]-*N*-(2,5-dimethyl-2*H*-pyrazol-3-yl)-acetamide | 395 (M+H)⁺ | 155.6-159.0 |
| **I-227** | 2-[3-(3-Chloro-5-cyano-phenoxy)-4-methyl-phenyl]-*N*-(2-methyl-2*H*-pyrazol-3-yl)-acetamide | 381 (M+H)⁺ | 129.5-132.8 |
| **I-228** | *N*-(6-Acetylamino-4-methyl-pyridin-3-yl)-2-[3-(3-chloro-5-cyano-phenoxy)-4-methyl-phenyl]-acetamide | 449 (M+H)⁺ | 222.5-224.0 |
| **I-229** | 2-[3-(3-Chloro-5-cyano-phenoxy)-4-methyl-phenyl]-*N*-(6-methoxy-2-methyl-pyridin-3-yl)-acetamide | 422 (M+H)⁺ | 168.3-170.4 |
| **I-230** | 2-[3-(3-Chloro-5-cyano-phenoxy)-4-methyl-phenyl]-*N*-(2-methyl-5-methylsulfanyl-2*H*-[1,2,4]triazol-3-yl)-acetamide | 428 (M+H)⁺ | 173-174 |
| **I-231** | *N*-(2-Chloro-phenyl)-2-[3-(3,5-dichloro-benzoyl)-5-methyl-phenyl]-acetamide | 431 (M)⁺ | 142.0-146.2 |
| **I-232** | 2-(3-(3,5-Dichloro-benzoyl)-5-methyl-phenyl]-*N*-(2-methyl-4-sulfamoyl-phenyl)-acetamide | 490 (M+H)⁺ | 247.1-249.0 |
| **I-233** | 2-4-Chloro-3-(4-cyano-6-methyl-pyridin-2-yloxy)-2-fluoro-phenyl]-*N*-(2-methyl-4-sulfamoyl-phenyl)-acetamide | 489 (M+H)⁺ | 230.1-230.7 |
| **I-234** | (4-{2-[3-(3-Chloro-5-cyano-phenoxy)-4-methyl-phenyl]-acetylamino}-3-methyl-phenoxy)-acetic acid | 465 (M+H)⁺ | 174.8-176.4 |
| **I-235** | (3-Chloro-4-{2-[3-(3-chloro-5-cyano-phenoxy)-4-methyl-phenyl]-acetylamino}-phenyl)-acetic acid methyl ester | 483 (M+H)⁺ | 116.9-118.0 |
| **I-236** | (3-Chloro-4-(2-[3-(3-chloro-5-cyano-phenoxy)-4-methyl-phenyl]-acetylamino}-phenyl)-acetic acid | 468 (M)⁺ | 159.6-160.0 |
| **I-237** | 2-[3-(3-Chloro-5-cyano-phenoxy)-4-methyl-phenyl]-*N*-(4-methanesulfonylamino-2-methyl-phenyl)-acetamide | 484 (M+H)⁺ | 180.0-181.4 |
| **I-238** | 2-[3-(3-Chloro-5-cyano-phenoxy)-4-methyl-phenyl]-*N*-[4-(methanesulfonyl-methyl-amino)-2-methyl-phenyl] -acetamide | 498 (M+H)⁺ | 184.0-184.8 |
| **I-239** | 4-(2-[4-Chloro-3-(3-chloro-5-cyano-phenoxy)-2-fluoro-phenyl]-acetylamino}-3-methyl-benzamide | 477 (M+H)⁺ | 255.9-257.1 |
| **I-240** | 2-[7-(4-Chloro-benzoyl)-2,3-dihydro-benzofuran-5-yl]-*N*-(2-chloro-phenyl)-acetamide | | 141.1-144.9 |
| **I-241** | 2-[7-(4-Chloro-benzoyl)-2,3-dihydro-benzofaran-5-yl]-*N*-(4-sulfamoyl-phenyl)-acetamide | | 259.0-261.0 |
| **I-242** | 2-[4-Chloro-3-(3,5-dicyano-phenylsulfanyl)-phenyl]-*N*-(4-sulfamoyl-phenyl)-acetamide | | 247.9-250.9 |
| **I-243** | 2-[3-(2-Bromo-5-chloro-phenylsulfanyl)-4-chloro-phenyl]-*N*-(4-sulfamoyl-phenyl)-acetamide | | 220-222.3 |
| **I-244** | 2-[3-(2-Bromo-5-chloro-phenylsulfanyl)-4-chloro-phenyl]-*N*-(2-chloro-phenyl)-acetamide | | 172.2-172.8 |
| **I-247** | 2-[4-Chloro-3-(3,5-dichloro-benzoyl)-phenyl]-*N*-(2-chloro-phenyl)-acetamide | 451 (M)⁺ | 197.2-198.1 |
| **I-248** | 2-[4-Chloro-3-(2,5-dichloro-benzoyl)-phenyl]-*N*-(2-chloro-phenyl)-acetamide | 451 (M)⁺ | 121.0-123.5 |
| **I-249** | 2-[4-Chloro-3-(2,5-dichloro-benzoyl)-phenyl]-*N*-(2-methyl-4-sulfamoyl-phenyl)-acetamide | 510 (M)⁺ | 214.9-218.8 |
| **I-250** | 2-[4-Chloro-3-(2,5-dichloro-benzoyl)-phenyl]-*N*-(4-sulfamoyl-phenyl)-acetamide | 496 (M)⁺ | 213.9-216.1 |
| **I-251** | 2-[4-Chloro-3-(2,5-dichloro-benzoyl)-phenyl]-*N*-phenyl-acetamide | 417 (M)⁺ | 167.1-168.5 |
| **I-252** | 2-(4-Chloro-3-(3,5-dichloro-benzoyl)-phenyl]-*N*-(2-methyl-4-sulfamoyl-phenyl)-acetamide | 510 (M)⁺ | 263-264.4 |
| **I-253** | 2-[4-Chloro-3-(2,5-dichloro-benzoyl)-phenyl]-*N*-(2-chloro-4-sulfamoyl-phenyl)-acetamide | 530 (M)⁺ | 197.0-199.6 |
| **I-254** | 2-[3-(4-Chloro-benzoyl)-5-methyl-phenyl]-*N*-(4-sulfamoyl-phenyl)-acetamide | 442 (M)⁺ | 260.9-263.3 |
| **I-255** | 2-[3-(4-Chloro-benzoyl)-5-methyl-phenyl]-*N*-(2-methyl-4-sulfamoyl-phenyl)-acetamide | 465(M)⁺ | 205.8-208.0 |
| **I-256** | 2-[3-(2-Chloro-benzoyl)-5-methyl-phenyl]-*N*-(2-methyl-4-sulfamoyl-phenyl)-acetamide | 456 (M)⁺ | 207.7-209.3 |
| **I-257** | 2-[3-(4-Chloro-benzoyl)-5-methyl-phenyl]-*N*-(2-chloro-4-sulfamoyl-phenyl)-acetamide | 476 (M+H)⁺ | 218.9-220.7 |
| **I-258** | 2-[3-(4-Chloro-benzoyl)-5-methyl-phenyl]-*N*-(2-chloro-phenyl)-acetamide | 397 (M)⁺ | 134.7-135.5 |
| **I-259** | 2-[3-(2-Chloro-benzoyl)-5-methyl-phenyl]-*N*-(2-chloro-phenyl)-acetamide | 397 (M)⁺ | 125.1-127.0 |
| **I-260** | 2-[3-(3-Chloro-benzoyl)-5-methyl-phenyl]-*N*-(2-methyl-4-sulfamoyl-phenyl)-acetamide | 456 (M)⁺ | 210.3-211.6 |
| **I-261** | 2-[3-(3-Chloro-benzoyl)-5-methyl-phenyl]-*N*-(4-sulfamoyl-phenyl)-acetamide | 442 (M)⁺ | 211-213.1 |
| **I-262** | 2-[3-(3-Chloro-benzoyl)-5-methyl-phenyl]-*N*-(2-chloro-phenyl)-acetamide | 397 (M)⁺ | 113.0-113.8 |
| **I-263** | 2-[3-(2-Chloro-benzoyl)-5-methyl-phenyl]-*N*-(4-sulfamoyl-phenyl)-acetamide | 442 (M)⁺ | 165-167 |
| **I-264** | 2-[3-(5-Bromo-2-chloro-benzoyl)-4-methoxy-phenyl]-*N*-(2-methyl-4-sulfamoyl-phenyl)-acetamide | 551 (M+H)+ | 177.0-177.9 |
| **I**-**265** | 2-[3-(5-Cyano-2-methyl-benzoyl)-4-methyl-phenyl]-*N*-(2-methyl-4-sulfamoyl-phenyl)-acetamide | 462 (M+H)+ | 224.3-228.1 |
| **I-266** | 2-[3-(5-Cyano-2-methyl-benzoyl)-4-methyl-phenyl]-*N*-(4-sulfamoyl-phenyl)-acetamide | 448 (M+H)+ | 229.6-231.1 |
| **I-267** | *N*-(2-Chloro-phenyl)-2-[3-(5-cyano-2-methyl-benzoyl)-4-methyl-phenyl]-acetamide | 403 (M+H)+ | 129.4-131.6 |
| **I-268** | 2-[3-(5-Cyano-2-ethyl-benzoyl)-4-ethyl-phenyl]-*N*-(2-methyl-4-sulfamoyl-phenyl)-acetamide | 490 (M+H)+ | |
| **I-269** | 2-[3-(3,5-Dichloro-benzoyl)-5-methyl-phenyl]-*N*-(4-sulfamoyl-phenyl)-acetamide | 476 (M)⁺ | 226.0-227.9 |
| **I-270** | 2-[3-(3-Chloro-5-cyano-phenoxy)-2-fluoro-4-methyl-phenyl]-*N*-(2-chloro-4-propionylsulfamoyl-phenyl)-acetamide; sodium salt | 564 (M+H)+ | 158.8-162 |

Compounds of the present invention can be made by a variety of methods depicted in the illustrative synthetic reaction schemes shown and described below. The starting materials and reagents used in preparing these compounds generally are either available from commercial suppliers, such as Aldrich Chemical Co., or are prepared by methods known to those skilled in the art following procedures set forth in references such as Fieser and Fieser's Reagents for Organic Synthesis; Wiley & Sons: New York, Volumes 1-21; R C. LaRock, Comprehensive Organic Transformations, 2nd edition Wiley-VCH, New York 1999; Comprehensive Organic Synthesis, B. Trost and I. Fleming (Eds.) vol. 1-9 Pergamon, Oxford, 1991; Comprehensive Heterocyclic Chemistry, A. R. Katritzky and C. W. Rees (Eds) Pergamon, Oxford 1984, vol. 1-9; Comprehensive Heterocyclic Chemistry II, A. R. Katritzky and C. W. Rees (Eds) Pergamon, Oxford 1996, vol. 1-11; and Organic Reactions, Wiley & Sons: New York, 1991, Volumes 1-40. The following synthetic reaction schemes are merely illustrative of some methods by which the compounds of the present invention can be synthesized, and various modifications to these synthetic reaction schemes can be made and will be suggested to one skilled in the art having referred to the disclosure contained in this Application.

The starting materials and the intermediates of the synthetic reaction schemes can be isolated and purified if desired using conventional techniques, including but not limited to, filtration, distillation, crystallization, chromatography, and the like. Such materials can be characterized using conventional means, including physical constants and spectral data.

Unless specified to the contrary, the reactions described herein preferably are conducted under an inert atmosphere at atmospheric pressure at a reaction temperature range of from about -78°C to about 150 °C, more preferably from about 0°C to about 125 °C, and most preferably and conveniently at about room (or ambient) temperature, e.g., about 20 °C.

Some compounds in following schemes are depicted with generalized substituents; however, one skilled in the art will immediately appreciate that the nature of the R groups can varied to afford the various compounds contemplated in this invention. Moreover, the reaction conditions are exemplary and alternative conditions are well known. The reaction sequences in the following examples are not meant to limit the scope of the invention as set forth in the claims.

N-Phenyl 3-phenoxy-phenylacetamide compounds are prepared from phenyl acetic acid derivatives 4 (X¹ = O, R⁵ = optionally substituted phenyl) as depicted in Scheme 1. The preparation of phenylacetic acids related to the present compounds have been described in US Serial Nos. 10/807,993 and 10/807,766 entitled *Non-nucleoside Reverse Transcriptase Inhibitors* filed March 23, 2004 These applications are hereby incorporated by reference in their entirety.

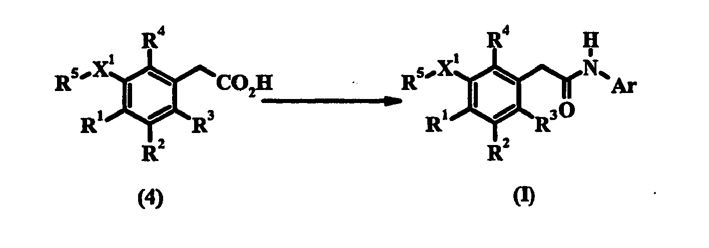

4-Alkyl-3-aryloxy-phenylacetic acid compounds are useful precursors to some embodiments of the present invention and can be prepared from 4-hydroxy-3-methoxyphenyl acetate by Pd catalyzed coupling of a dialkyl zinc species and ethyl 3-methoxy-4-trifluorosulfonyloxy-phenylacetate **(4b)** to produce the corresponding 4-alkyl compound (SCHEME 1). The Negishi coupling of organozinc halides or dialkylzinc with haloarenes and aryl triflates is an effective means for attachment of an alkyl group to an arene (E.-I. Negishi, Acc. Chem. Res. 1982 15:340-348). The reaction is catalyzed by palladium Pd(0) and palladium is preferably ligated to a bidentate ligand including Pd(dppf)Cl₂ and Pd(dppe)Cl₂. (J. M. Herbert Tetrahedron Lett. 2004 45:817-819) Typically the reaction is run in an inert aprotic solvent and common ethereal solvents include dioxane, DME and THF are suitable. The reaction is commonly run at elevated temperature.

Alternatively Friedel-Crafts acylation of ethyl 3-methoxy-phenylacetate affords 4-acetyl-3-methoxy-phenylacetate **8.** Reduction of the ketone to afford the 4-ethyl derivative **12a** can be accomplished under a variety of conditions including triethylsilylhydride/TFA, catalytic hydrogenation and hydrogenolysis, Clemmenson and Wolf-Kischner procedures. Alternatively the side chain can be further elaborated by subjecting the ketone to a Wittig condensation and reduction of the resulting olefin. In the present invention triphenylphosphonium methylide is condensed with **8** affording ethyl 4-isopropenyl-3-methoxy-phenylacetate **(14)** which was reduced to the corresponding ethyl 4-*iso*-propyl-3-methoxy-phenylacetate **(16).** One skilled in the art will recognize many closely related alternative procedures are available that afford other 4-alkyl substituents. Demethylation of the methyl ether affords the corresponding ethyl 4-alkyl-3-hydroxyphenylacetate **18a** which are useful synthetic intermediates for introduction of the 3-aryloxy moiety.

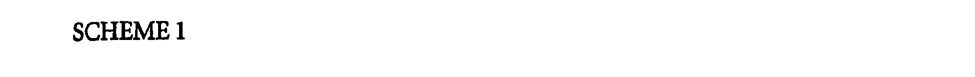

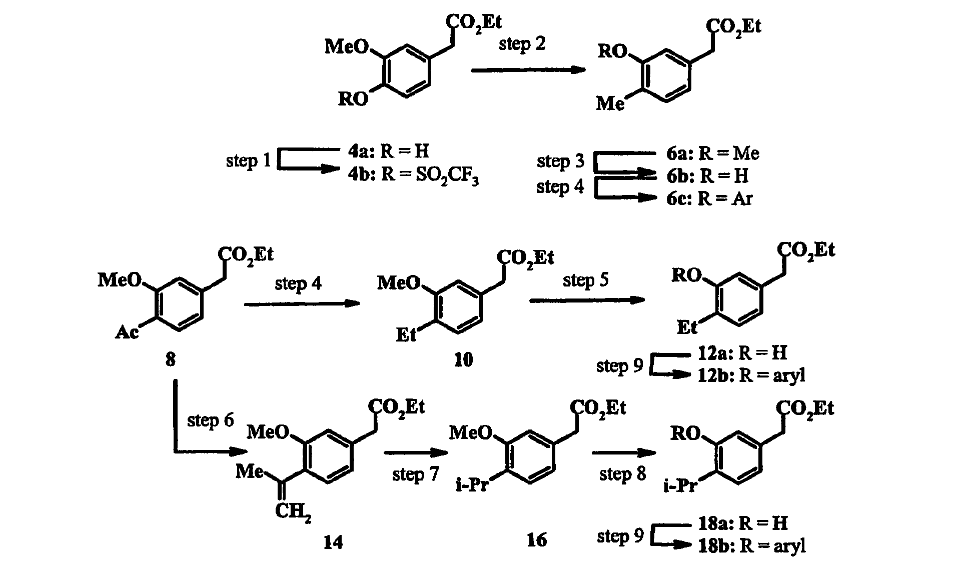

3,4-Dimethyl-5-aryloxy-phenylacetic acid compounds were also prepared using a dialkylzinc mediated coupling; however, prior to that coupling the activating effect of the hydroxyl was exploited to formylate the aromatic ring and afford **22a.** Reduction of the formyl substituent provides the methyl compound **22c.** (SCHEME 2) The formyl moiety provides a versatile intermediate that can be further modified to incorporate a variety of substitution into the 3-position. Demethylation of the methyl ether affords the phenol **24c** which can be used to introduce the biaryl ether linkage.

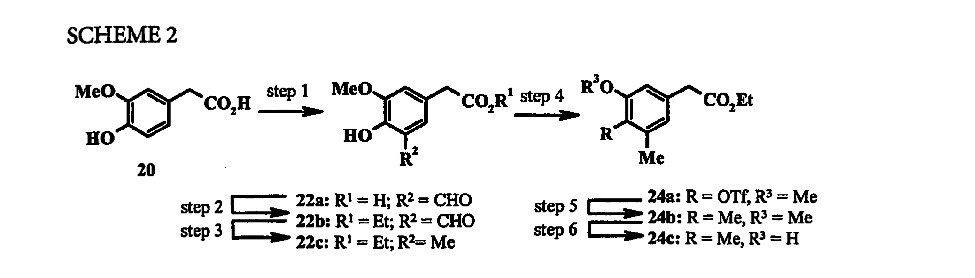

The preparation of diaryl ethers has been reviewed (J. S. Sawyer, Recent Advances in Diaryl Ether Synthesis, Tetrahedron 2000 56:5045-5065). Introduction of the (hetero)aryloxy ether can often be accomplished by direct S_{N}Ar displacement reaction on a aromatic ring bearing a leaving group and electronegative substituents. Fluoroaromatic compounds with electronegative substituents are known to be sensitive to nucleophilic attack by soft nucleophiles. Fluorine substituents are generally significantly more labile than other halogen substituents. While hard nucleophiles like water and hydroxide fail to displace fluoride, soft nucleophiles like phenols, imidazoles, amines, thiols and some amides undergo facile displacement reactions even at room temperature (D. Boger et al., Biorg. Med. Chem. Lett. 2000 10:1471-75; F. Terrier Nucleophilic Aromatic Displacement: The Influence of the Nitro Group VCH Publishers, New York, NY 1991**).** Phenols typified by **6b** and **12a** can be treated with appropriately substituted aryl fluorine compounds to produce diaryl ethers(*infra*).

Aryl ethers also can be efficiently prepared by Cu(OAc)₂ catalyzed condensation of substituted benzene boronic acids and phenols (D. A. Evans et al., Tetrahedron Lett., 1998 39:2937-2940 and D. M. T. Chan et al., Tetrahedron Lett. 1998 39:2933-2936). This protocol can also be adapted to phenols such as **6b** and **12a**. Substituted benzene boronic acids with a variety of other substituents are widely available.

Alternatively, variations of the Ullmann diaryl ether synthesis with Cu(I) salts (J.-F. Marcoux et al., J. Am. Chem. Soc. 1997 119:10539-540; E. Buck et al, Org. Lett. 2002 4(9):1623-1626) or palladium-catalyzed coupling procedures also has been reported (G. Mann et al, J. Am. Chem. Soc., 1999 121:3224-3225) have been described. One skilled in the art will appreciate that optimal procedure will vary depending on the nature and position of substituents on the aryl rings to be coupled and useful conditions for the coupling can by identified without undue experimentation.

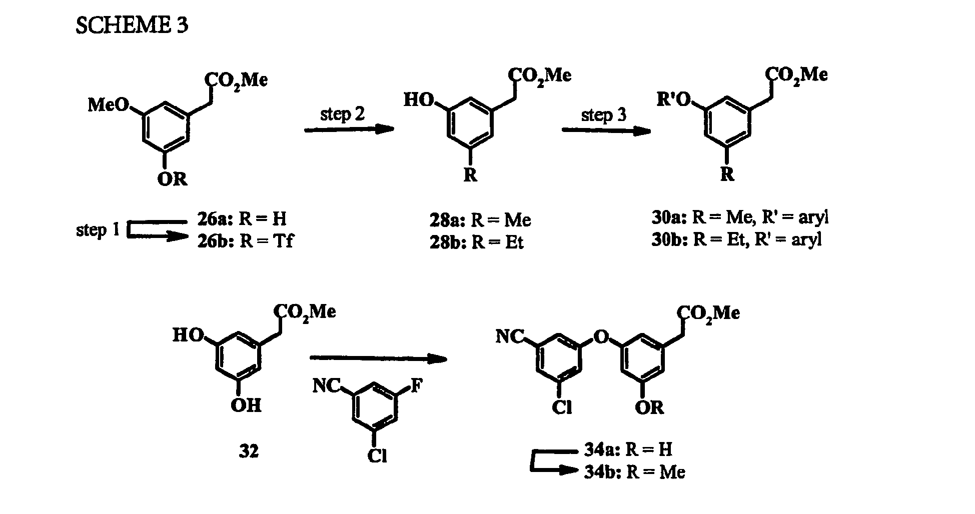

5-Methyl- and 5-ethyl-3-hydroxy-phenylacetic derivatives were prepared by monomethylation of 3,5-dihydroxy-phenylacetic acid to afford 26a followed sequentially by treatment with triflic anhydride and dialkylzinc/Pd[P(Ph)₃]₄ mediated coupling to afford 28a and 28b. While the reactions are illustrated with methyl and ethyl groups, one skilled in the art will appreciate that a variety of substitutents can be introduced depending on the reagent selected. Demethylation of the methyl ether was accomplished as previously described to afford phenols that can be used to introduce the diaryl ethers. 3-Aryloxy-5-methoxy-phenylacetic acids compounds can be prepared by mono-arylation of alkyl 3,5-dihydroxy-phenylacetates and subsequent methylation to afford **34b** (SCHEME 3). Other alkoxy compounds are readily prepared by replacing methyl iodide with other alkylating agents.

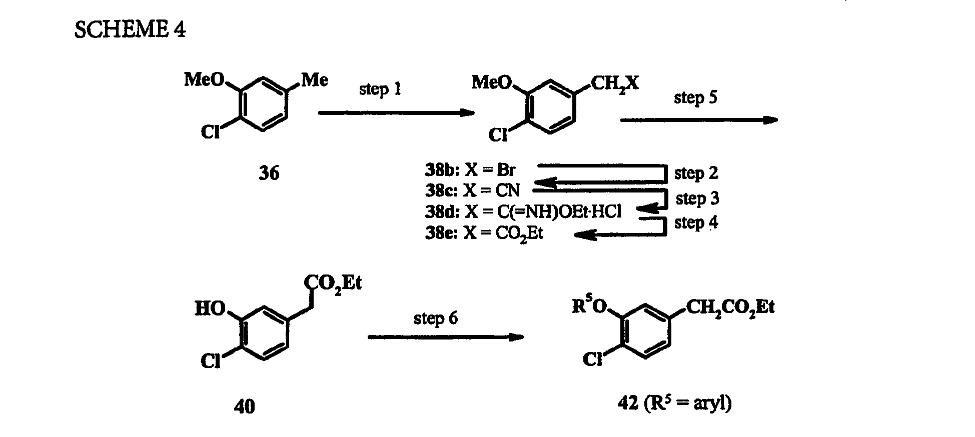

While useful substituted phenylacetic acid precursors are sometimes available, an alternative route utilizing methyl substituents to elaborate the acetic acid side chain has been used. Ethyl 4-chloro-3-hydroxy-phenylacetate **(40)** was prepared from 1-chloro-2-methoxy-4-methyl-benzene by benzylic bromination (step 1) and displacement of the bromine atom with sodium cyanide (step 2). Hydrolysis of the nitrile (steps 3 and 4) and demethylation of the ether under standard conditions afforded **40**. Demethylations with BBr₃ or LiI/*syn* collidine are effective techniques for conversion of methyl ethers to the corresponding phenols. Incorporation of the aryl or heteroaryl ether is achieved by one of the methods described previously.

4-Chloro-2-fluoro-3-phenoxy-phenylacetic acid compounds (SCHEME 5) can prepared by starting from 1-chloro-3-fluoro-2-methoxy-4-methylbenzene which is subjected to benzylic bromination with NBS and AIBN, cyanide displacement, hydrolysis of the nitrile and esterification of the carboxylic acid utilizing a sequence analogous to that described in SCHEME 4

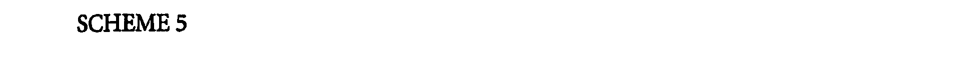

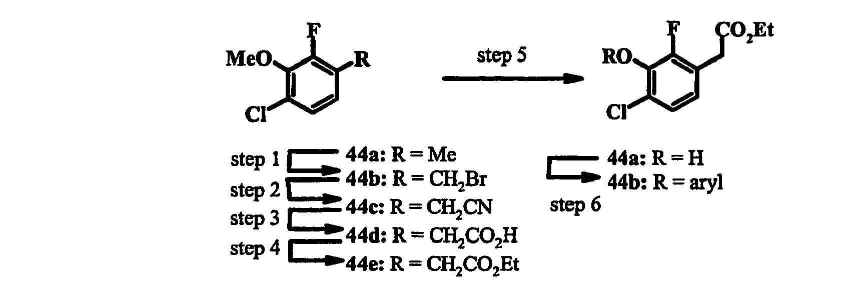

Alternatively, the synthesis of 2-fluoro substituted compounds can be accomplished by exploiting the facile displacement of fluoroaromatic compounds. Thus, treatment of 1,2,3-triflouro-4-nitro-benzene (**46**) with an alkali metal phenolate resulted in displacement of the 3-fluoro group with good regioselectivity to afford **48a** (SCHEME 6). Treatment of **48a** with carbanion formed by deprotonation of *tert*-butyl ethyl malonate results in the regioselective introduction of a malonic ester (**48b**) which is subjected to acid-catalyzed hydrolysis of the tert-butyl ester and decarboxylation to afford **48c**. After introduction of the phenoxy and acetic acid moieties, the nitro group is readily converted to other substituents at the 4-position. Reduction of the nitro substituent to the corresponding amine **50a** which can be subjected to Sandmeyer reactions. In the present case, the Sandmeyer reaction was used to introduce a bromo **50a** or chloro **50e** substituent. The bromo substituent could be further reacted with a dialkyl zinc (the Negishi Reaction) to afford 4-alkyl-3-aryloxy-2-fluoro-phenylacetic acid compounds exemplified by **50c** and **50d.**

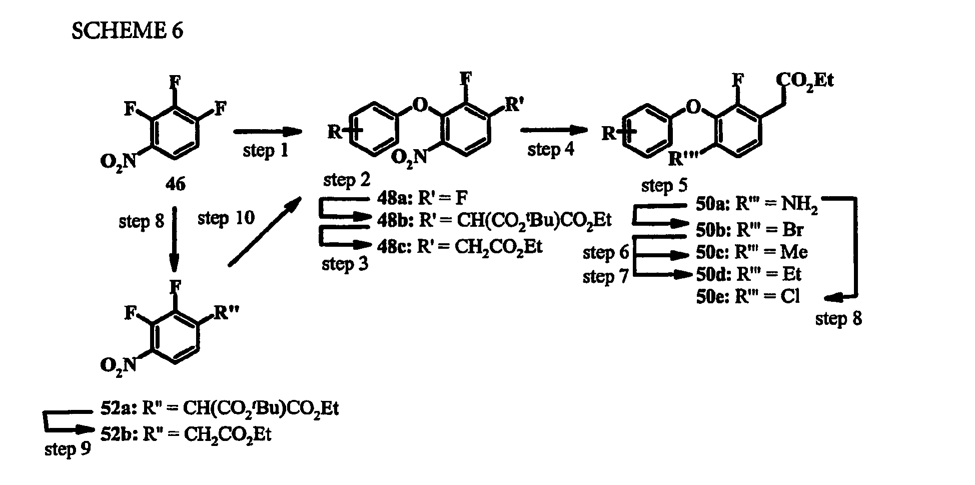

Alternatively, the reaction of the *tert*-butyl ethyl ester of malonic acid affords a 2:1 regioisomeric mixture of adducts in which displacement of the fluorine at the 1-position **52a** predominates. Hydrolysis and decarboxylation of **52a** affords the phenylacetic acid **52b** which also is an effective substrate for introduction of an aryl ether and Sandmeyer-type chemistry

4-Alkoxy-2-fluoro-3-phenoxyphenylacetic acid compounds are prepared by a alternate route. A mixture *ortho*-difluorobenzene (**54a**) and trimethylsilylchloride was treated with butyl lithium which afforded 2,3-difluoro-1,4-*bis*-trimethylsilanyl-benzene (**54b**) which was brominated to afford **54c**. Selective monometallation of **54c** with *iso*propylmagnesium chloride-lithium chloride complex and quenching the organomagnesium compound with DMF afforded **54d**. Reaction of **54d** with a phenol in the presence of K₂CO₃ resulted in displacement of the fluorine atom adjacent to the aldehyde to afford **56a**. The aldehyde was subjected to a Baeyer-Villiger oxidation with trifluoroperacetic acid which underwent concomitant hydrolysis to the phenol **56b** which was alkylated with Cs₂CO₃ and methyl iodide to afford the methoxy substituted analog **56c**. Metallation of the remaining bromine substituent with *iso*-PrMgCl/LiI/THF and alkylation of the resulting Grignard reagent afforded **58a** which was oxidatively cleaved with NaIO₄/Ru(III)Cl₃ to produce the phenylacetic acid **58b** and converted to the corresponding amides by procedures described previously.

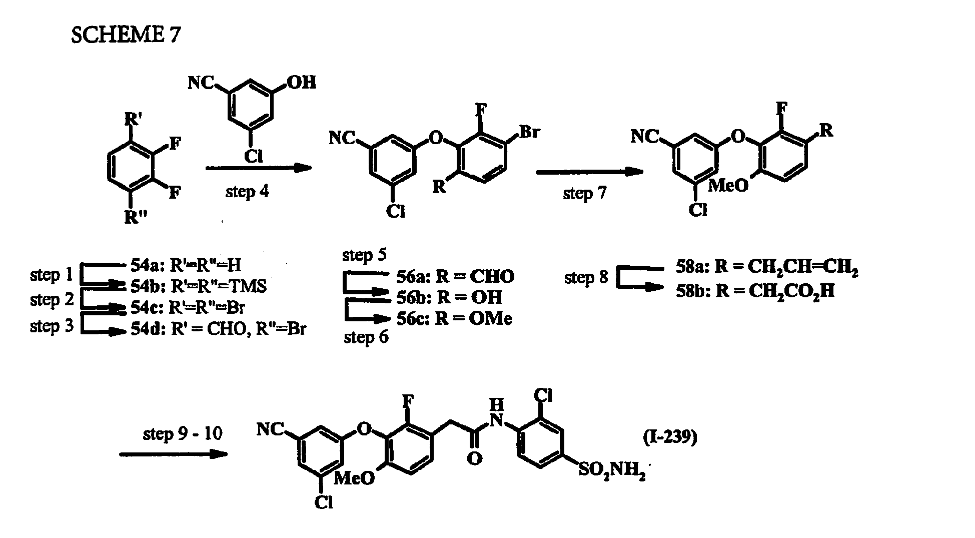

The substituted phenylacetic acid esters thus obtained were converted to the corresponding amides through a three-step sequence including hydrolysis of the ester, conversion of the resulting acid to an acid chloride and condensation of the acid chloride with an aryl amine or heteroarylamine.

Fluorine-substituted aromatic compounds which are useful intermediates for formation of the biaryl ether are commercially available or readily prepared from commercially available precursors. 3-Chloro-5-fluoro-benzonitrile (**59a**), 1-bromo-3-chloro-5-fluorobenzene,1-bromo-2-fluoro-4-chloro-benzene, 4-chloro-3-fluorobenzonitrile, 3-fluoro-5-trifluoromethyl-benzonitrile (**59b**), 3,5-dibromo-fluoro-benzene, 1,3-dichloro-2-fluorobenzene (**59c**), 1,4-dichloro-2-fluoro-6-bromo-benzene (**59d**), 1-chloro-2-fluoro-4,6-dibromo-benzene (**59e**) and 1-chloro-2,6-difluoro-4-bromo-benzene (**59f**) were purchased. Cyanide substituents can be introduced into a aromatic ring by Zn(CN)₂/palladium-catalyzed displacement of a halogen by cyanide which can be carried out either after after formation of the biaryl ether or on a halogenated precursor (SCHEME 8 - reaction B).

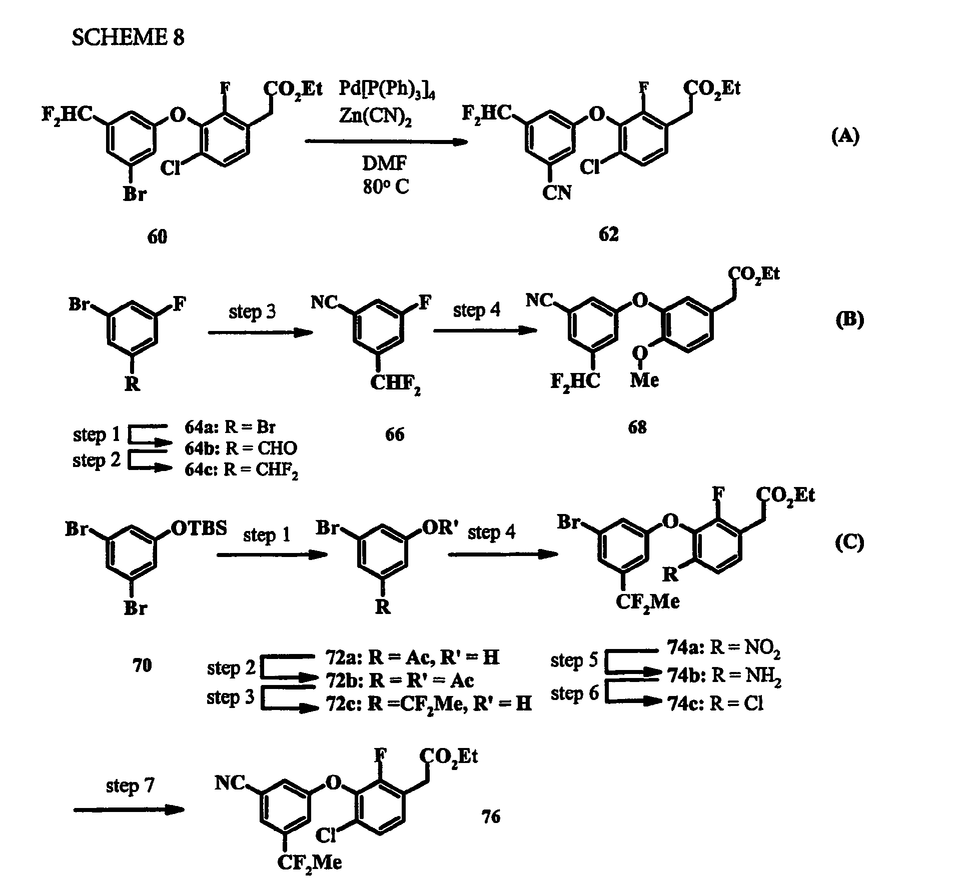

Fluoroalkyl substituted compounds are useful embodiments of the invention and 66 and **72c** were prepared by fluorination of a carbonyl compound with a fluorinating agent such as (diethylamino)sulfur trifluoride (DAST) or [*bis*(2-methoxyethylamino)sulfur difluoride (Deoxo-Fluor^{®}). Thus 1,3-dibromo-5-fluorobenzene (**64a**) was mono-metallated and formylated to afford **64b**. Fluorination of **64b** with DAST afforded **64c** which was optionally treated with Zn(CN)₂ and Pd[P(Ph)₃]₄ to introduce a nitrile prior to formation of the *bis*-aryl ether **68** by condensation of **66** and **52b.** The 1,1,-difluoroethyl substituent was introduced utilizing an analogous strategy whereby an acetyl derivative was fluorinated with Deoxo-Fluor^{®}. The acetyl compound **72b** was obtained by mono-metallation of **70** and quenching with N-methyl-N-(methoxy)acetamide. The fully elaborated phenylacetic acid **74** was obtained by condensation of **72c** and **52b** followed Sandmeyer reaction in the presence of Cu(I)Cl and HCl. Optional introduction of a cyanide into the aryl ring (step 7) was carried out as described previously.

Di-cyano substitution is another feature in some embodiments of the present invention and dicyano aromatic compounds, *e.g.,* **80** are readily available by *bis*-cyanation of a dihalo precursor **78.** One skilled in the art will appreciate this transformation can be carried out on a variety of dihalogenated 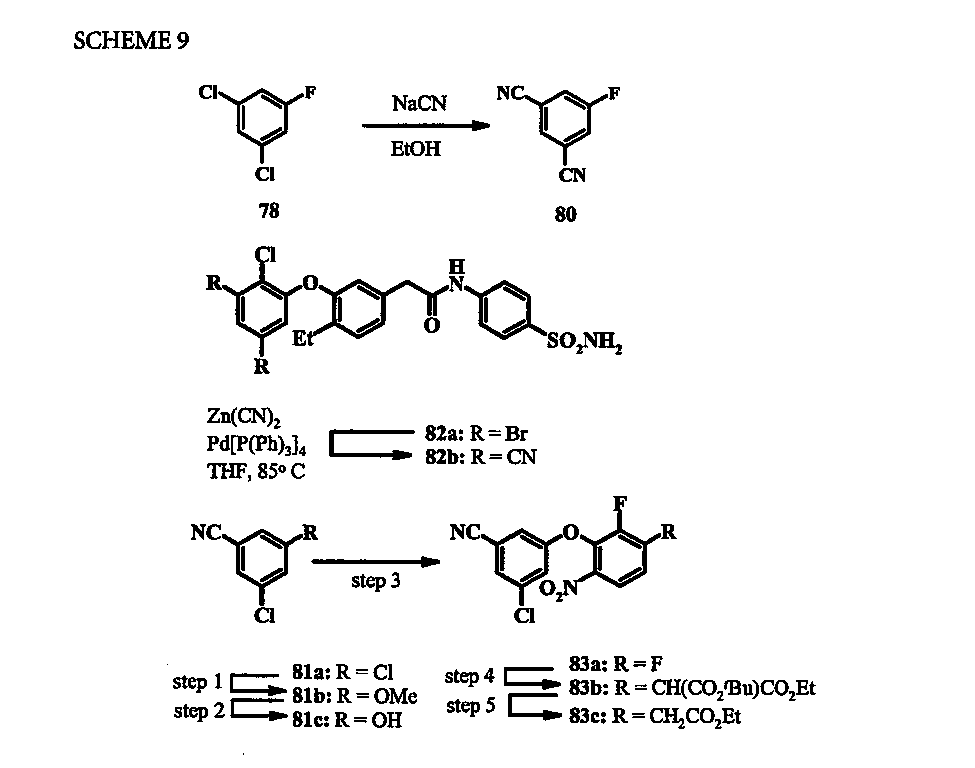 substrates. Considerable flexibility in the sequence of the transformations is possible and *bis*-cyanation can be carried out either prior to or after formation of the biaryl ether, *e.g.,* transformation of **82a** to **82b**.

In some examples of the present invention the biaryl ether is introduced by reacting a suitably substituted phenol and a fluoro-substituted phenylacetic acid or precursor thereof. (see SCHEME 6) Many useful phenols are commercially available. 3-Chloro-5-hydroxy-benzonitrile (**81c**) was a useful intermediate in the synthesis of some embodiments of the present invention. 81c was conveniently prepared from 3,5-dichlorobenzonitrile (**81a**) by displacement of one chlorine substituent with sodium methoxide and LiI/collidine mediated demethylation of the resulting arylmethyl ether **81b**. The phenol was reacted with **46** to afford **83a** which was further transformed to **83c** as described previously.

Introduction of haloalkoxy substituents were readily accomplished by addition of a dihalocarbene to a phenol. 3,5-Dihydroxy-benzonitrile (**84a**) is transformed into the base-stable mono SEM-ether **84c** which is reacted with difluorocarbene generated by decarboxylative elimination of 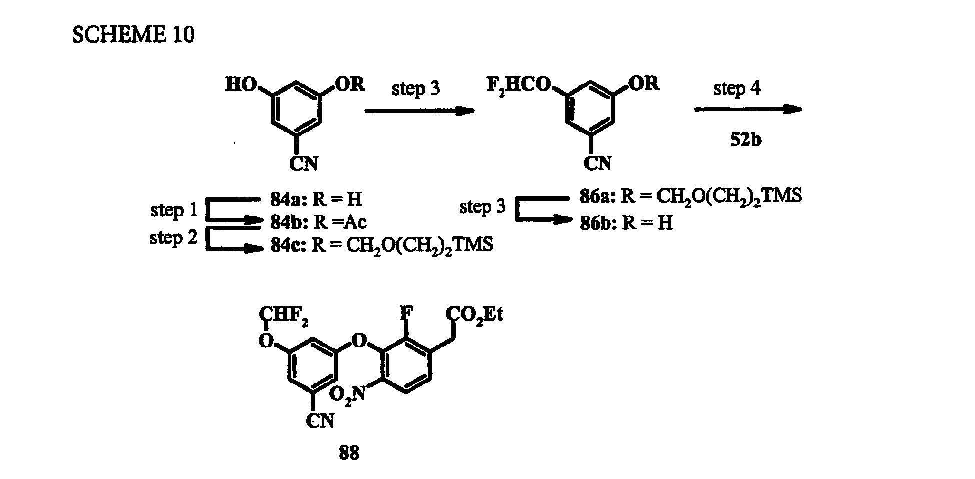 difluorochloroacetic acid (SCHEME 10). Deprotection and condensation of the resulting phenol **86b** with ethyl 2,3-difluoro-4-nitro-phenylacetate **52b** affords **88** which can be further converted to compounds of the present invention.

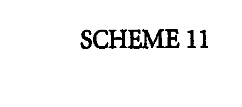

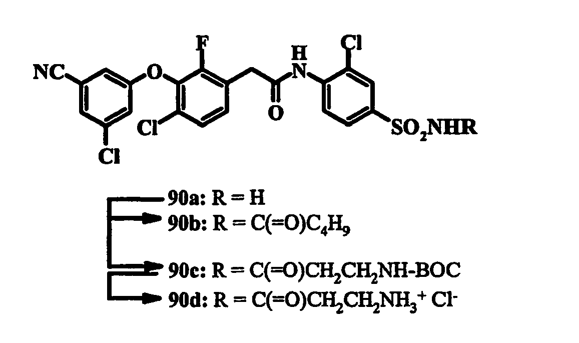

Other embodiments of the invention include N-acylsulfonamides (SCHEME 11) which can be prepared by acylation of the corresponding sulfonamide. The acylation can be achieved by reacting the sulfonamide with acyl halides or anhydrides, *e.g*, propionyl anhydride, or, in the case of acylation with N-protected amino acids, by transient activation of the carboxylic acid followed by condensation with **90a** and deprotection. Protocols for activation, coupling and deprotection of amino acids are well known in the art. Although the acyl sulfonamides in SCHEME 11 are depicted as neutral molecules, the acidic proton on the acyl sulfonamide nitrogen can be readily deprotonated to form salts. Acyl sulfonamides formed from amino acids can be deprotected by well established procedures and the free amine group can be converted into an acid addition salt.

Other N-substituted sulfonamides were prepared by amidation with a 4-amino-benzensulfonyl fluoride (**95**) to afford an intermediate sulfonyl fluoride **114** which is further reacted with a (di)alkylamine to afford the desired sulfonamide derivatives.

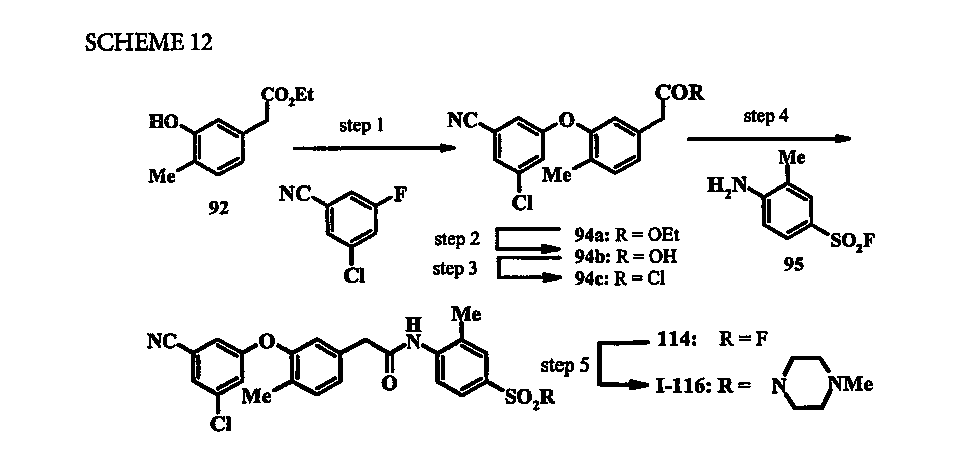

4-Amino-benzene-sulfones are useful intermediates for the synthesis of some embodiments of the present invention (SCHEME 13). The requisite anilines were prepared from 4-fluoro-2-methylnitrobenzene. Displacement of the fluoride with sodium sulfide and alkylation of the resulting thiol with ethyl bromoacetamide and oxidation of the thiol to the corresponding sulfone with MCPBA affords **98d.** Reduction of the nitro substituent and condensation of the resulting amine **100** with an aroyl chloride affords **122.** Subsequent modification of the function groups, *e.g.,* hydrolysis of the carboxylic acid ester is possible using previously described methodology. Other alkylating agents useful to prepare compounds of the present invention include haloalkanols, e.g. bromoethanol and brompropanol, N-protected halo amines, *e.g.,* (2-bromo-ethyl)-carbamic acid *tert*-butyl ester and bromoacetonitrile. The corresponding ether analogs were available by alkylation of the analogous phenols. (see, *e.g.,* P. G. Wyatt et al. J. Med. Chem. 1995 38(10):1657-1665).

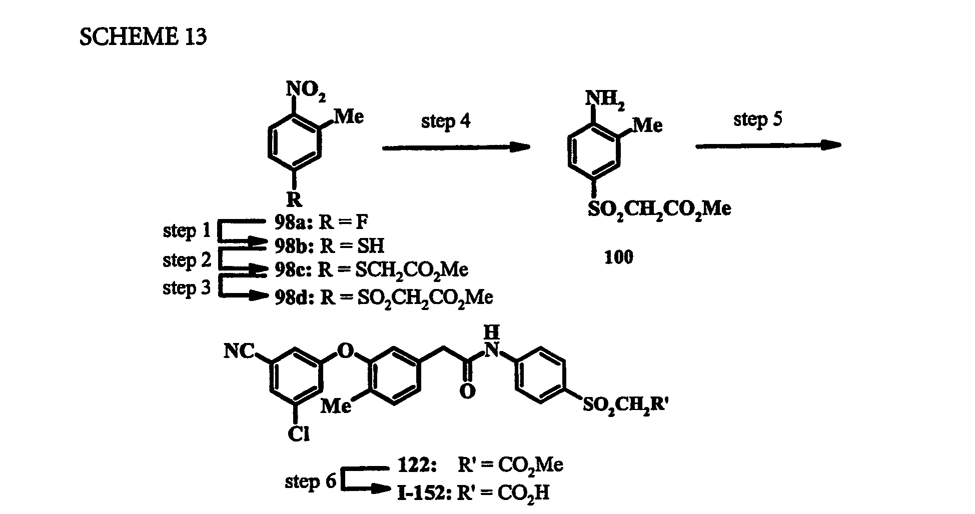

(7-Aryloxy-benzofuran-5-yl)-acetic acid derivatives were prepared from (2,3-dihydro-benzofuran-5-yl)-acetic acid ethyl ester (**101a**). Introduction of the oxygen substituent was achieved by Friedel-Craft acetylation and subsequent Baeyer-Villiger rearrangement and hydrolysis of the acetate to afford **101d.** Introduction of the aryloxy moiety is accomplished by S_{N}Ar displacement of an aryl halide or by Cu(OAc)₂ catalyzed condensation of substituted benzene boronic acids and phenols as described previously. Oxidation of the dihydrofuran by allylic bromination which spontaneously underwent dehydrobromination afforded **105a.**

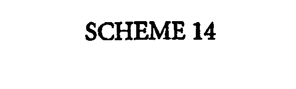

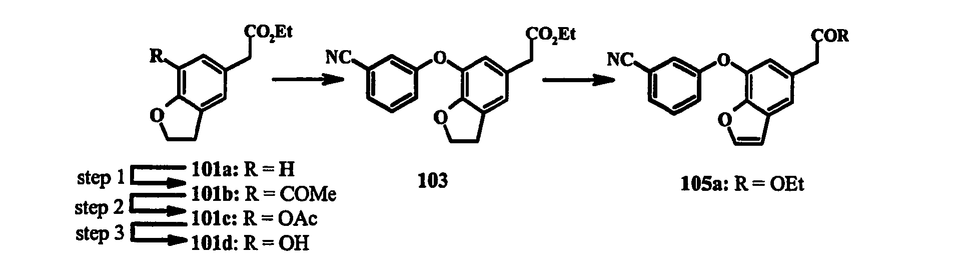

### DOSAGE AND ADMINISTRATION

The compounds of the present invention may be formulated in a wide variety of oral administration dosage forms and carriers. Oral administration can be in the form of tablets, coated tablets, dragées, hard and soft gelatine capsules, solutions, emulsions, syrups, or suspensions. Compounds of the present invention are efficacious when administered by other routes of administration including continuous (intravenous drip) topical parenteral, intramuscular, intravenous, subcutaneous, transdermal (which may include a penetration enhancement agent), buccal, nasal, inhalation and suppository administration, among other routes of administration. The preferred manner of administration is generally oral using a convenient daily dosing regimen which can be adjusted according to the degree of affliction and the patient's response to the active ingredient.

A compound or compounds of the present invention, as well as their pharmaceutically useable salts, together with one or more conventional excipients, carriers, or diluents, may be placed into the form of pharmaceutical compositions and unit dosages. The pharmaceutical compositions and unit dosage forms may be comprised of conventional ingredients in conventional proportions, with or without additional active compounds or principles, and the unit dosage forms may contain any suitable effective amount of the active ingredient commensurate with the intended daily dosage range to be employed. The pharmaceutical compositions may be employed as solids, such as tablets or filled capsules, semisolids, powders, sustained release formulations, or liquids such as solutions, suspensions, emulsions, elixirs, or filled capsules for oral use; or in the form of suppositories for rectal or vaginal administration; or in the form of sterile injectable solutions for parenteral use. A typical preparation will contain from about 5% to about 95% active compound or compounds (w/w). The term "preparation" or "dosage form"is intended to include both solid and liquid formulations of the active compound and one skilled in the art will appreciate that an active ingredient can exist in different preparations depending on the target organ or tissue and on the desired dose and pharmacokinetic parameters.

The term "excipient" as used herein refers to a compound that is useful in preparing a pharmaceutical composition, generally safe, non-toxic and neither biologically nor otherwise undesirable, and includes excipients that are acceptable for veterinary use as well as human pharmaceutical use. The term "excipient" as used herein includes both one and more than one such excipient.

The phrase "pharmaceutically acceptable salt" of a compound means a salt that is pharmaceutically acceptable and that possesses the desired pharmacological activity of the parent compound. Such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbiryclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, *e.g.,* an alkali metal ion, an alkaline earth ion, or an aluminum ion; or coordinates with an organic base such as ethanolamine, diethanolamine, triethanolamine, tromethamine, N-methylglucamine, and the like. N-acylsulfonamides have an acidic proton which can be abstracted to form a salt with an organic or inorganic cation. Other ocompounds of the invention have a basic nitrogen which can from acid addition salts.

The preferred pharmaceutically acceptable salts are the salts formed from acetic acid, hydrochloric acid, sulphuric acid, methanesulfonic acid, maleic acid, phosphoric acid, tartaric acid, citric acid, sodium, potassium, calcium, zinc, and magnesium. It should be understood that all references to pharmaceutically acceptable salts include solvent addition forms (solvates) or crystal forms (polymorphs) as defined herein, of the same acid addition salt.

Solid form preparations include powders, tablets, pills, capsules, cachets, suppositories, and dispersible granules. A solid carrier may be one or more substances which may also act as diluents, flavoring agents, solubilizers, lubricants, suspending agents, binders, preservatives, tablet disintegrating agents, or an encapsulating material. In powders, the carrier generally is a finely divided solid which is a mixture with the finely divided active component. In tablets, the active component generally is mixed with the carrier having the necessary binding capacity in suitable proportions and compacted in the shape and size desired. Suitable carriers include but are not limited to magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. Solid form preparations may contain, in addition to the active component, colorants, flavors, stabilizers, buffers, artificial and natural sweeteners, dispersants, thickeners, solubilizing agents, and the like.

Liquid formulations also are suitable for oral administration include liquid formulation including emulsions, syrups, elixirs, aqueous solutions, aqueous suspensions. These include solid form preparations which are intended to be converted to liquid form preparations shortly before use. Emulsions may be prepared in solutions, for example, in aqueous propylene glycol solutions or may contain emulsifying agents such as lecithin, sorbitan monooleate, or acacia. Aqueous solutions can be prepared by dissolving the active component in water and adding suitable colorants, flavors, stabilizing, and thickening agents. Aqueous suspensions can be prepared by dispersing the finely divided active component in water with viscous material, such as natural or synthetic gums, resins, methylcellulose, sodium carboxymethylcellulose, and other well known suspending agents.

The compounds of the present invention may be formulated for parenteral administration (*e.g*., by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, for example solutions in aqueous polyethylene glycol. Examples of oily or nonaqueous carriers, diluents, solvents or vehicles include propylene glycol, polyethylene glycol, vegetable oils (*e.g.,* olive oil), and injectable organic esters (*e.g*., ethyl oleate), and may contain formulatory agents such as preserving, wetting, emulsifying or suspending, stabilizing and/or dispersing agents. Alternatively, the active ingredient may be in powder form, obtained by aseptic isolation of sterile solid or by lyophilisation from solution for constitution before use with a suitable vehicle, *e.g*., sterile, pyrogen-free water.

The compounds of the present invention may be formulated for topical administration to the epidermis as ointments, creams or lotions, or as a transdermal patch. Ointments and creams may, for example, be formulated with an aqueous or oily base with the addition of suitable thickening and/or gelling agents. Lotions may be formulated with an aqueous or oily base and will in general also containing one or more emulsifying agents, stabilizing agents, dispersing agents, suspending agents, thickening agents, or coloring agents. Formulations suitable for topical administration in the mouth include lozenges comprising active agents in a flavored base, usually sucrose and acacia or tragacanth; pastilles comprising the active ingredient in an inert base such as gelatin and glycerin or sucrose and acacia; and mouthwashes comprising the active ingredient in a suitable liquid carrier.

The compounds of the present invention may be formulated for administration as suppositories. A low melting wax, such as a mixture of fatty acid glycerides or cocoa butter is first melted and the active component is dispersed homogeneously, for example, by stirring. The molten homogeneous mixture is then poured into convenient sized molds, allowed to cool, and to solidify.

The compounds of the present invention may be formulated for vaginal administration. Pessaries, tampons, creams, gels, pastes, foams or sprays containing in addition to the active ingredient such carriers as are known in the art to be appropriate.

The compounds of the present invention may be formulated for nasal administration. The solutions or suspensions are applied directly to the nasal cavity by conventional means, for example, with a dropper, pipette or spray. The formulations may be provided in a single or multidose form. In the latter case of a dropper or pipette, this may be achieved by the patient administering an appropriate, predetermined volume of the solution or suspension. In the case of a spray, this may be achieved for example by means of a metering atomizing spray pump.

The compounds of the present invention may be formulated for aerosol administration, particularly to the respiratory tract and including intranasal administration. The compound will generally have a small particle size for example of the order of five (5) microns or less. Such a particle size may be obtained by means known in the art, for example by micronization. The active ingredient is provided in a pressurized pack with a suitable propellant such as a chlorofluorocarbon (CFC), for example, dichlorodifluoromethane, trichlorofluoromethane, or dichlorotetrafluoroethane, or carbon dioxide or other suitable gas. The aerosol may conveniently also contain a surfactant such as lecithin. The dose of drug may be controlled by a metered valve. Alternatively the active ingredients may be provided in a form of a dry powder, for example a powder mix of the compound in a suitable powder base such as lactose, starch, starch derivatives such as hydroxypropylmethyl cellulose and polyvinylpyrrolidine (PVP). The powder carrier will form a gel in the nasal cavity. The powder composition may be presented in unit dose form for example in capsules or cartridges of *e.g*., gelatin or blister packs from which the powder may be administered by means of an inhaler.

When desired, formulations can be prepared with enteric coatings adapted for sustained or controlled release administration of the active ingredient. For example, the compounds of the present invention can be formulated in transdermal or subcutaneous drug delivery devices. These delivery systems are advantageous when sustained release of the compound is necessary and when patient compliance with a treatment regimen is crucial. Compounds in transdermal delivery systems are frequently attached to a skin-adhesive solid support. The compound of interest can also be combined with a penetration enhancer, e.g., Azone (1-dodecylaza-cycloheptan-2-one). Sustained release delivery systems are inserted subcutaneously into to the subdermal layer by surgery or injection. The subdermal implants encapsulate the compound in a lipid soluble membrane, e.g., silicone rubber, or a biodegradable polymer, e.g., polyactic acid.

Suitable formulations along with pharmaceutical carriers, diluents and expcipients are described in Remington: The Science and Practice of Pharmacy 1995, edited by E. W. Martin, Mack Publishing Company, 19the edition, Easton, Pennsylvania. A skilled formulation scientist may modify the formulations within the teachings of the specification to provide numerous formulations for a particular route of administration without rendering the compositions of the present invention unstable or compromising their therapeutic activity.

The modification of the present compounds to render them more soluble in water or other vehicle, for example, may be easily accomplished by minor modifications (salt formulation, esterification, etc.), which are well within the ordinary skill in the art. It is also well within the ordinary skill of the art to modify the route of administration and dosage regimen of a particular compound in order to manage the pharmacokinetics of the present compounds for maximum beneficial effect in patients.

The term "therapeutically effective amount" as used herein means an amount required to reduce symptoms of the disease in an individual. The dose will be adjusted to the individual requirements in each particular case. That dosage can vary within wide limits depending upon numerous factors such as the severity of the disease to be treated, the age and general health condition of the patient, other medicaments with which the patient is being treated, the route and form of administration and the preferences and experience of the medical practitioner involved. For oral administration, a daily dosage of between about 0.01 and about 100 mg/kg body weight per day should be appropriate in monotherapy and/or in combination therapy. A preferred daily dosage is between about 0.1 and about 500 mg/kg body weight, more preferred 0.1 and about 100 mg/kg body weight and most preferred 1.0 and about 10 mg/kg body weight per day. Thus, for administration to a 70 kg person, the dosage range would be about 7 mg to 0.7 g per day. The daily dosage can be administered as a single dosage or in divided dosages, typically between 1 and 5 dosages per day. Generally, treatment is initiated with smaller dosages which are less than the optimum dose of the compound. Thereafter, the dosage is increased by small increments until the optimum effect for the individual patient is reached. One of ordinary skill in treating diseases described herein will be able, without undue experimentation and in reliance on personal knowledge, experience and the disclosures of this application, to ascertain a therapeutically effective amount of the compounds of the present invention for a given disease and patient.

In embodiments of the invention, the active compound or a salt can be administered in combination with another antiviral agent, such as a nucleoside reverse transcriptase inhibitor, another nonnucleoside reverse transcriptase inhibitor or HIV protease inhibitor. When the active compound or its derivative or salt are administered in combination with another antiviral agent the activity may be increased over the parent compound. When the treatment is combination therapy, such administration may be concurrent or sequential with respect to that of the nucleoside derivatives. "Concurrent administration" as used herein thus includes administration of the agents at the same time or at different times. Administration of two or more agents at the same time can be achieved by a single formulation containing two or more active ingredients or by substantially simultaneous administration of two or more dosage forms with a single active agent.

It will be understood that references herein to treatment extend to prophylaxis as well as to the treatment of existing conditions, and that the treatment of animals includes the treatment of humans as well as other animals. Furthermore, treatment of a HIV infection, as used herein, also includes treatment or prophylaxis of a disease or a condition associated with or mediated by HIV infection, or the clinical symptoms thereof.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

### Example 1

### 2-[4-Chloro-3-(3-chloro-5-cyano-phenoxy)-phenyl]-N-(4-sulfamoyl-phenyl)-acetamide (I-1)

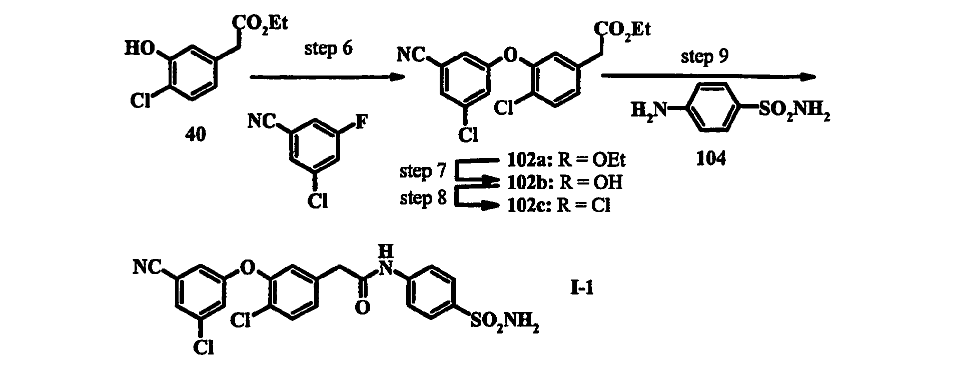

step 1- A solution of 4-chloro-3-methoxytoluene (**36**; 0.5 g; 3.2 mmol), NBS (0.57 g; 3.2 mmol) and benzoyl peroxide (0.031 g; 0.13 mmol) and 32 mL of DCE were heated at reflux for 3 h. The reaction mixture was cooled, diluted with CH₂Cl₂ and washed with water and brine. The organic extract was dried, filtered and evaporated to yield the bromomethyl compound **38b** which was used without further purification.

step 2 - The 28 g (0.166 mmol) of **38b** from the previous step, NaCN (28 g; 0.58 mmol; 3.5 equiv.) and 500 mL of 90% aqueous EtOH were stirred at room temperature overnight. The crude residue was partitioned between EtOAc/H₂O (359 mL of each), washed with brine, dried, filtered and evaporated. The crude product was purified by SiO₂ chromatography eluting with a EtOAc/hexane gradient (100 % hexane to 90%hexane) to afford 21 g of **38c.**

step 3 - Gaseous HCl was slowly bubbled into a cooled solution of 4-chloro-3-methoxyacetonitrile (**38c**) in toluene (10mL), ether (10 mL) and EtOH (1 mL) for about 10 min. The reaction was stoppered and stored at -30°C for one week. TLC failed to detect any remaining starting material. The solvent was evaporated and the yellow solid was stirred with Et₂O, filtered and washed with Et₂O and dried in a vacuum oven to yield 0.57 g (90%) of ethyl 4-chloro-3-methoxyphenylmethylimidate (**38d**).

step 4 - A solution of 0.57 g of 38d and 10 mL of H₂O was heated at 40°C for 3 h. The reaction was cooled to RT and extracted with EtOAc. The reaction was dried (MgS04), filtered and evaporated and the resulting product **38e** was used without further purification.

step 5 - A solution of ethyl 4-chloro-3-methoxyphenylacetate (**38e**; 36 g; 157 mmol) and DCM (2 L) was cooled to -78°C and a solution of BBr₃ (74 mL; 785 mmol; 1.0 M in CH₂Cl₂) was added over 30 min. After 1 h at -78 °C the reaction was allowed to warm to RT. When starting material was consumed the reaction was cooled in an ice-water bath and the reaction quenched with 200 mL of water. The aqueous phase was extracted with CH₂Cl₂:EtOAc (4:1 v/v). The combined extracts were washed with water and brine, dried (Na₂SO₄), filtered and evaporated to afford 30 g (90%) of **40**.

step 6 - A solution of **40** (1.07 g, 5 mmol), 3,5-dichloro-benzonitrile (1.3 g, 7.56 mmol), K₂CO₃ (2.07 g, 15.0 mmol) and NMP (10 mL) was stirred and heated to 110°C for 6 h. The reaction mixture was cooled to RT and diluted with H₂O (50 mL) and twice extracted with EtOAc. The combined organic extracts were washed sequentially with water and brine, dried (Na₂SO₄), filtered and concentrated *in vacuo.* The crude product was purified by SiO₂ chromatography eluting with EtOAc/hexane (10:90) to afford 0.328 g of 102a.

step 7 - To a solution of **102a** (0.80 g; 0.1.98 mmol) and LiOH (142.5 mg, 5.94 mmol) dissolved in EtOH (8 mL) and water (2 mL) and stirred for at RT. The mixture was concentrated *in vacuo* and the residue acidified with 1 N HCl and twice extracted with EtOAc. The combined extracts were washed sequentially with water and brine, dried (Na₂SO₄), filtered and evaporated to afford 102b which was used without further purification in step 8.

step 8 - To a solution of 102b (0.75 g,1.994 mmol) and DCM (8 mL) was added DMF (2 drops) and 0.05 mL of and oxalyl chloride (0.348 mL, 0.505 g; 3.99 mmol). The reaction mixture was stirred overnight at RT. The volatile reactants were removed *in vacuo* and the crude acid chloride **102c** was used directly in the next step.

step 9 - The acid chloride **102c** (0.092 g)was dissolved in 3 mL of acetone and purged with nitrogen. A separate flask was charged with 4-amino-benzenesulfonamide (0.046 g; 0.270 mmol) and water (1 mL) was added to dissolve the sulfonamide. NaHCO₃ (0.050 g) was added and the acetone solution slowly added and allowed to stir overnight. The reaction mixture was extracted with EtOAc and washed with 10% HCl and brine. The EtOAc solution was dried (Na₂SO₄) filtered and evaporated and the crude product purified by SiO₂ chromatography eluting with EtOAc:hexanes (60:40) to afford 1-1: mp 211-213; ms = 476.

Compound **I-3** was prepared in a by a similar procedure except in step 9, 4-amino-benzene was replaced by 3-(4-amino-3-methyl-phenoxy)-propane-1-sulfonic acid amide (J. H. Chan et al. J. Med. Chem. 2004 47(5):1175-1182)

### Example 2

### 2-[3-(2-Bromo-5-chloro-phenoxy)-4-chloro-phenyl]-N-(4-sulfamoyl-phenyl)-acetamide (I-4)

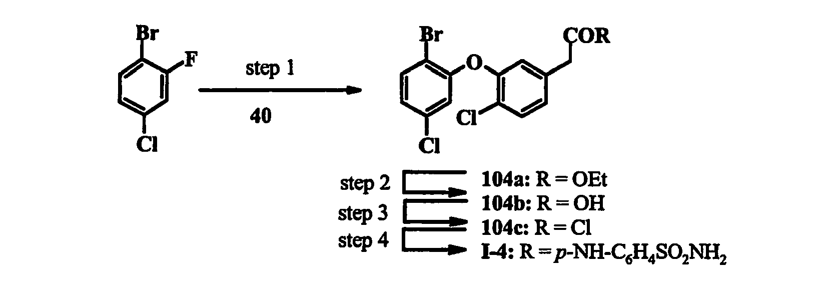

step 1- A solution of phenol 59c (2.0 g, 9.32 mmol),1-bromo-2-fluoro-4-chlorobenzene (1.28 mL, 2.15 g,10.25 mmol), K₂CO₃ (3.84 g, 30 mmol) and NMP (20 mL) was stirred and heated to 130°C for 8 h. The reaction mixture was cooled to RT and diluted with H₂O (50 mL) and twice extracted with EtOAc. The combined organic extracts were washed sequentially with water (6 times) and brine, dried (Na₂SO₄), filtered and concentrated *in vacuo.* The crude product was purified by SiO₂ column chromatography eluting with (10% EtOAc/hexane) to afford 0.328 g (% theory) of **I-4.**

Steps 2-4 were carried out as described in steps 7-9 of Example 1 which afforded I-4.

Compound **I-3** was prepared in the same manner except in step 4, 4-amino-benzenesulfonamide was replaced by 3-(4-amino-3-methyl-phenoxy)-propane-1-sulfonic acid amide (J. H. Chan et al. J. Med. Chem. 2004 47(5):1175-1182).

### Example 3

### 2-[4-Chloro-3-(4-cyano-2,6-dimethyl-phenoxy)-phenyl]-N-(4-sulfamoyl-phenyl)-acetamide (I-55)

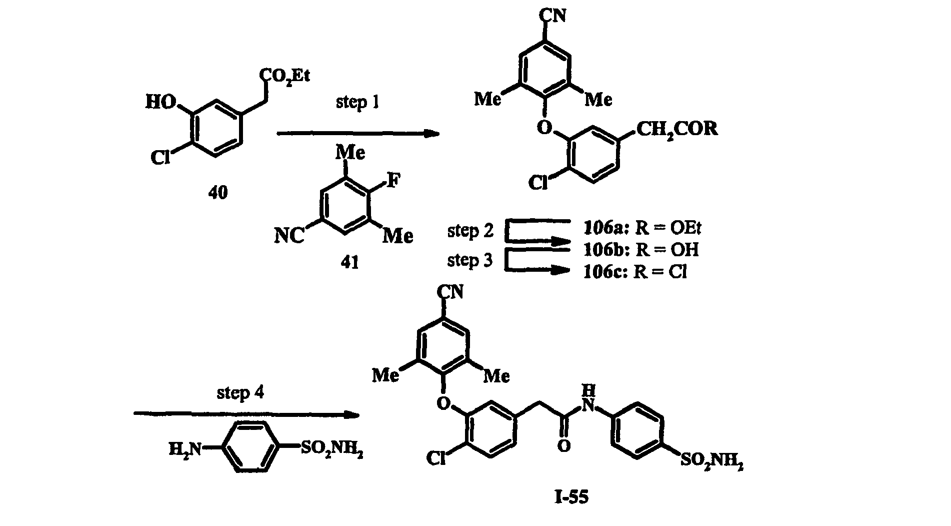

step 1 - A solution of **40** (0.600 g, 2.79 mmol), 3,5-dimethyl-4-fluorobenzonitrile (41, 0.459 g, 3.07 mmol), K₂CO₃ (1.157 g, 8.37 mmol) and NMP (6 mL) was stirred and heated to 120° C for 6 h. When the starting material was consumed the solution was cooled and acidified with 10% HCl and twice extracted with EtOAc. The combined extracts were washed sequentially with water and brine, dried (Na₂SO₄), filtered and evaporated *in vacuo.* The crude product was purified by SiO₂ chromatography and eluted with an EtOAc/hexane gradient (100% hexane to 50% hexane) to afford **106a.**

Steps 2-4 were carried out as described in steps 7-9 of Example 1 which afforded I-55.

Compound **I-56** was prepared using a similar procedure except in step 4, 4-amino-benzenesulfonamide was replaced with 4-amino-2-methyl-benzenesulfonamide.

Compound **I-68** was prepared using a similar procedure except in step 4, 4-amino-benzenesulfonamide was replaced with 2-chloroaniline.

Compound **I-135** was prepared using a similar procedure except in step 4, 4-amino-benzenesulfonamide was replaced with 4-amino-2-chloro-benzenesulfonamide.

### Example 4

### 2-[4-Chloro-3-(3,5-dicyano-phenoxy)-phenyl]-N-(4-sulfamoyl-phenyl)-acetamide (I-2)

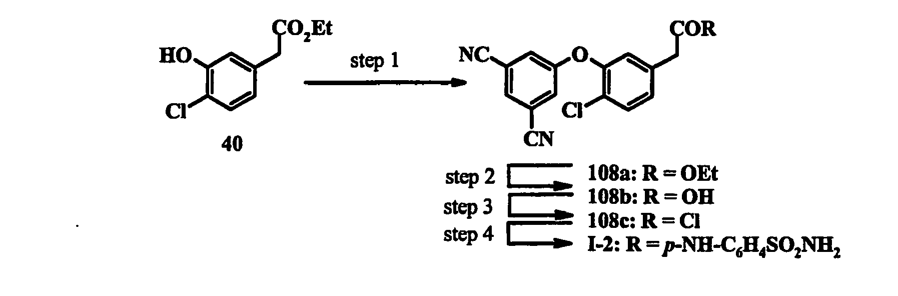

step 1 - A solution of **40** (1.335 g, 6.22 mmol), 5-fluoro-isophthalonitrile (1.0 g, 6.84 mmol), K₂CO₃ (2.579 g, 18.66 mmol) and NMP (10 mL) was stirred and heated to 100°C for 12 h. The reaction mixture was cooled to RT and diluted with H₂O (50 mL) and twice extracted with EtOAc. The combined organic extracts were washed sequentially with water and brine, dried (Na₂SO₄), filtered and concentrated *in vacuo* to afford **108a.**

Steps **2-4** were carried out as described in steps 7-9 of Example 1 which afforded I-3.

Compound **I-5** was prepared using a similar procedure except in step 4 of Example 3, 4-amino-benzenesulfonamide was replaced with 2-chloro-phenylamine.

Compound **I-6** was prepared using a similar procedure except in step 4, 4-amino-benzenesulfonamide was replaced with N,N-dimethyl-benzene-1,4-diamine.

Compound **I-194** was prepared using a similar procedure except in step 4 of Example 3, 4-amino-benzenesulfonamide was replaced with 2-pyrrol-1-yl-phenylamine.

Compound **I-195** was prepared using a similar procedure except in step 4 of Example 3, 4-amino-benzenesulfonamide was replaced with 2-fluoro-phenylamine.

Compound **I-196** was prepared using a similar procedure except in step 4 of Example 3, 4-amino-benzenesulfonamide was replaced with 2-methoxy-phenylamine.

Compound **I-197** was prepared using a similar procedure except in step 4 of Example 3, 4-amino-benzenesulfonamide was replaced with 2-iso-propyl-phenylamine.

Compound **I-198** was prepared using a similar procedure except in step 4 of Example 3, 4-amino-benzenesulfonamide was replaced with 2-methyl-phenylamine.

Compound **I-199** was prepared using a similar procedure except in step 4 of Example 3, 4-amino-benzenesulfonamide was replaced with 2-ethyl-phenylamine.

Compound **I-200** was prepared using a similar procedure except in step 4 of

Example 3, 4-amino-benzenesulfonamide was replaced with 2,3-dimethyl-phenylamine.

Compound **I-201** was prepared using a similar procedure except in step 4 of Example 3, 4-amino-benzenesulfonamide was replaced with 2-methyl-4-methoxy-phenylamine.

Compound **I-202** was prepared using a similar procedure except in step 4 of Example 3, 4-amino-benzenesulfonamide was replaced with 4-cyclohexyl-phenylamine.

Compound **I-203** was prepared using a similar procedure except in step 4 of Example 3, 4-amino-benzenesulfonamide was replaced with 2-amino-benzonitrile.

Compound **I-204** was prepared using a similar procedure except in step 4, 4-amino-benzenesulfonamide was replaced with 2-trifluoromethyl-phenylamine.

Compound **I-205** was prepared using a similar procedure except in step 4 of Example 3, 4-amino-benzenesulfonamide was replaced with 2-amino-4,5-dimethoxybenzoic acid methyl ester.

Compound **I-208** was prepared in the same manner except in step 4, 4-amino-benzenesulfonamide was replaced with phenylamine.

### Example 5

### 2-[4-Chloro-3-(3-cyano-5-trifluoromethyl-phenoxy)-phenyl]-N-(4-sulfamoyl-phenyl)-acetamide (I-88)

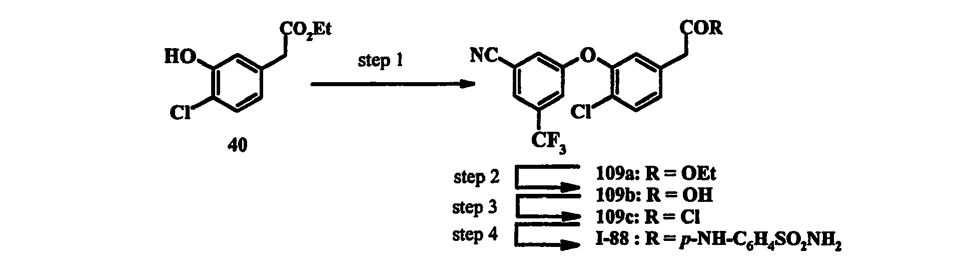

[4-Chloro-3-(3-cyano-5-trifluoromethyl-phenoxy)-phenyl]-acetic acid ethyl ester3-bromo-5-trifluoromethylphenol (**109a**) was prepared by condensing **40** and 3-fluoro-5-trifluoromethyl-benzontrile (**59b**) using the procedure described in step 6 of example 1. Steps 2 to 4 were carried out by the procedure described in steps 7 to 9 of example 1 which afford **I-88.**

Compound **I-89** was in the same procedure as **I-88** except in step 4, 4-amino-benzenesulfonamide was replaced with 4-amino-2-methyl-benzenesulfonamide was replaced with

Compound **I-90** was in the same procedure as **I-88** except in step 4, 4-amino-benzenesulfonamide was replaced with 4-amino-2-chloro-benzenesulfonamide was replaced with

### Example 6

### 2- [3-(3-Chloro-5-cyano-phenoxy)-4-methyl-phenyl]-N-[2-methyl-4-(4-methyl-piperazine-1-sulfonyl)-phenyl]-acetamide (I-116)

Steps 1-3 are depicted in SCHEME 1 and Steps **4-8** are depicted in SCHEME 12

step 1 - To a cooled solution of ethyl 4-hydroxy-3-methoxyphenylacetate (**4a**; 13.7 g; 65.2 mmol) and 260 mL of CH₂Cl₂ under N₂ atmosphere was added dropwise triflic anhydride (16 mL; 97.9 mmol) followed by dropwise addition of pyridine (8.9 mL; 8.8 mmol). The reaction was stirred in an ice-water bath for 3 h. The solution was transferred to a separatory funnel and washed with water and brine, dried (Na₂SO₄), filtered and evaporated to yield 21 g (90%) of 4b.

step 2 - To a solution of ethyl 3-methoxy-4-trifluorosulfonyloxyphenylacetate (**4b**) in 4 mL of THF cooled in an ice-water bath was added slowly a solution of Pd(dppf)Cl₂ (0.024 g; 0.029 mmol) and DIBAL-H (6 mL; 0.058 mmol; 1.0M in PhMe)and a small quantity of THF followed by dimethylzinc (0.29 mL; 0.58 mmol; 2.0 M in PhMe). After addition was completed the ice bath was removed and the reaction allowed to warm to rt and then heated to reflux for 1 h. The reaction was carefully quenched with a small quantity of water, filtered through a pad of CELITE® and the solids washed thoroughly with EtOAc. The combined organic extracts were washed with water and brine, dried (MgSO₄) and the solvent evaporated to afford 0.240 g (85%) of ethyl 3-methoxy-4-methylphenylacetate (**4c**).

step 3 - To a solution of **4c** (2.2 g; 8.0 mmol) and 250 mL CH₂Cl₂ cooled to -78 °C was added dropwise *via* syringe BBr₃ (9.8 mL; 0.104 mol). After 1 h at -78°C the reaction was stirred for 4 h in an ice-water bath. The reaction mixture was recooled to -78 °C and the reaction quenched aqueous NaHCO₃ then warmed to rt and the organic phase washed with water, saturated NaHCO₃ and brine. The organic phase was dried (MgSO₄) and the solvent evaporated to afford 1.4 g of ethyl 3-hydroxy-4-methylphenylacetate (**6**).

step 4 - A solution of 3-chloro-5-fluorobenzonitrile (**59a**, 9.2 g, 59.14 mmol), **6** (10.44 g, 53.76 mmol), K₂CO₃ (22.29 g, 161.3 mmol) and NMP (100 mL) was stirred and heated to 120° C for 6 h. The reaction mixture was cooled to 0° C and diluted with saturated NaHSO₃ (100 mL) and twice extracted with EtOAc. The combined organic extracts were washed sequentially with water (6 times) and brine, dried (Na₂SO₄), filtered and concentrated *in vacuo.* The crude product was purified by SiO₂ column chromatography eluting with a EtOAc/hexane gradient (0 to 10% EtOAc) to afford 9.28 g (48% theory) of **112a.**

Steps 5-7 were carried out by the procedure described in steps 7-9 of Example 1 except in step 7, 4-amino-benzenesulfonamide was replaced with 4-amino-3-methyl-benzenesulfonyl fluoride which afforded **114.**

step 8 - A mixture of **114** (0.150 g, 0.317 mmol)and N-methyl-piperazine (0.176 □L, 0.159 g,1.59 mmol) was heated to 120° C without any solvent. The reaction mixture was cooled to RT and partitioned between EtOAc (25 mL) and 10% HCl (mL). The aqueous phase was extracted with EtOAc (25 mL). The aqueous phase was made basic with 10% NaOH and extracted twice with EtOAc and the combined organic extracts were washed sequentially with water and brine, dried (Na₂SO₄), filtered and evaporated *in vacuo.* The crude product was purified by column chromatography on SiO₂ eluting with DCM:MeOH (95:5) to afford **I-116.**

Compound **I-115** was prepared using a similar procedure except in step 8 N-methyl-piperazine was replaced with morpholine.

Compound **I-117** was prepared using a similar procedure except in step 8, N-methyl-piperazine was replaced with thiomorpholine.

Compound **I-118** was prepared using a similar procedure except in step 8, N-methyl-piperazine was replaced with 2-morpholin-4-yl-ethylamine.

Compound **I-119** was prepared using a similar procedure except in step 8, N-methyl-piperazine was replaced with 3-amino-propan-1-ol.

Compound **I-121** was prepared using a similar procedure except in step 8, N-methyl-piperazine was replaced with trans-4-amino-cyclohexanol.

Compound **I-122** was prepared using a similar procedure except in step 7, 4-amino-3-methyl-benzenesulfonyl fluoride was replaced with 4-amino-3-methyl-benzenesulfonamide and step 8 was omitted

Compound **I-123** was prepared using a similar procedure except in step 7,4-amino-3-methyl-benzenesulfonyl fluoride was replaced with 4-amino-3-chloro-benzenesulfonamide and step 8 was omitted

Compound **I-128** was prepared using a similar procedure except in step 8, N-methyl-piperazine was replaced with tetrahydro-pyran-4-ylamine.

Compound **I-129** was prepared using a similar procedure except in step 8, N-methyl-piperazine was replaced with 1-amino-propan-2-ol.

Compound **I-130** was prepared using a similar procedure except in step 8, N-methyl-piperazine was replaced with 2-methylsulfanyl-ethylamine.

Compound **I-131** was prepared using a similar procedure except in step 8, N-methyl-piperazine was replaced with 3-methylsulfanyl-propylamine.

Compound **I-224** was prepared using a similar procedure except in step 7, 4-amino-3-methyl-benzenesulfonyl fluoride was replaced with 3-amino-4-methyl-pyridine and step 8 was omitted.

Compound **I-225** was prepared using a similar procedure except in step 7, 4-amino-3-methyl-benzenesulfonyl fluoride was replaced with 2-amino-3-methyl-pyridine and step 8 was omitted.

Compound **I-226** was prepared using a similar procedure except in step 7, 4-amino-3-methyl-benzenesulfonyl fluoride was replaced with 2,5-dimethyl-2H-pyrazol-3-ylamine and step 8 was omitted.

Compound **I-227** was prepared using a similar procedure except in step 7, 4-amino-3-methyl-benzenesulfonyl fluoride was replaced with 2-methyl-2H-pyrazol-3-ylamine and step 8 was omitted.

Compound **I-228** was prepared using a similar procedure except in step 7, 4-amino-3-methyl-benzenesulfonyl fluoride was replaced with N-(5-amino-4-methyl-pyridin-2-yl)-acetamide and step 8 was omitted.

Compound **I-229** was prepared using a similar procedure except in step 7, 4-amino-3-methyl-benzenesulfonyl fluoride was replaced with 3-amino-6-methoxy-2-methyl-pyridine and step 8 was omitted.

Compound **I-230** was prepared using a similar procedure except in step 7, 4-amino-3-methyl-benzenesulfonyl fluoride was replaced with 2-methyl-5-methylsulfanyl-2H-[1,2,4]triazol-3-ylamineand step 8 was omitted.

Compound **I-235** was prepared using a similar procedure except in step 7,4-amino-3-methyl-benzenesulfonyl fluoride was replaced with (4-amino-3-methyl-phenyl)-acetic acid methyl ester and step 8 was omitted.

Compound **I-236** was prepared base hydrolysis 1-235 with LiOH in EtOH/H₂O.

Compound **I-237** was prepared using a similar procedure except in step 7, 4-amino-3-methyl-benzenesulfonyl fluoride was replaced with N-(4-amino-3-methyl-phenyl)-methanesulfonamide and step 8 was omitted. N-(4-Amino-3-methyl-phenyl)-methanesulfonamide was prepared by mesylation of 3-methyl-4-nitro-phenylamine followed by catalytic hydrogenation of the nitro group.

Compound **I-238** was prepare as described for 1-237 except N-(3-methyl-4-nitrophenyl)-methanesulfonamide (**113a**) was N-methylated to produce N-methyl-N-(3-methyl-4-nitro-phenyl)-methanesulfonamide (**113b**) prior to reduction and acylation.

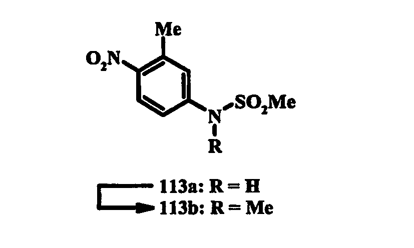

NaH (0.057 g of a 60% dispersion in mineral oil, 1.1 equiv) was added to a solution of **113a** (0.30 g,1.3 mmol) in DMF (6 mL) cooled to 0°C. The solution was stirred for 10 min, and iodomethane (0.12 mL, 1.5 equiv) was added dropwise. The solution was stirred for 16 h and poured into a saturated NH₄Cl solution. The solution was extracted with EtOAc, and the combined organic layers were washed with water, brine, and dried (MgSO₄). Evaporation of the volatile materials **113b** as a yellow oil (0.30 g, 94%).

### Example 7

### 2-[3-(3,5-Dicyano-phenoxy)-4-methyl-phenyl]-N-(2-methyl-4-sulfamoyl-phenyl)-acetamide (I-43)

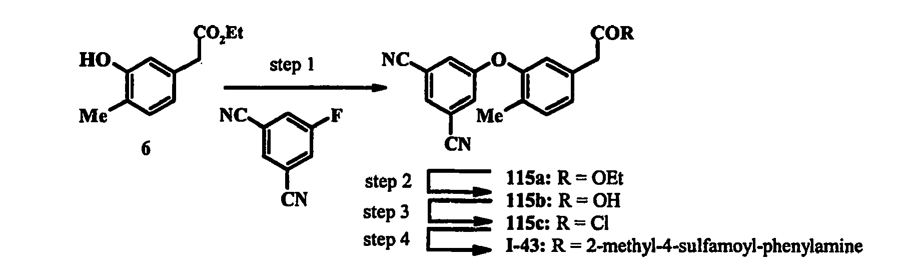

5-Fluoro-isophthlaonitrile was prepared from 3,5 dibromo-fluoro-benzene (**59b**) with Zn(CN)2/ Pd[P(Ph)3]4 mediated coupling.

step 1- A solution of 5-fluoro-isophthalonitrile (0.3742 g), **6** (0.450 g, 2.55 mmol), K₂CO₃ (0.962 g, 6.96 mmol) and NMP (5mL) was stirred and heated to 120°C for 6 h. The reaction mixture was cooled to RT and diluted with H₂O (25 mL) and twice extracted with EtOAc. The combined organic extracts were washed sequentially with water (6 times) and brine, dried (Na₂SO₄), filtered and concentrated *in vacuo.* The crude product was purified by SiO₂ column chromatography eluting with a EtOAc/hexane gradient (100% to 75% hexane) to afford **115a.**

Steps 2-4 were carried out by the procedure described in steps 7-9 of Example 1 except in step 4 4-amino-3-methyl-benzenesulfonamide replaced 4-amino-3-benzenesulfonamide which afforded **I-43**

Compound 1-34 was prepared using a similar procedure except in step 4, 2-chloro-phenylamine replaced 4-amino-3-methyl-benzenesulfonamide.

Compound **I-44** was prepared using a similar procedure except in step 4, 4-amino-3-chloro-benzenesulfonamide replaced 4-amino-3-methyl-benzenesulfonamide.

### Example 8

### 2-[3-(2-Bromo-5-chloro-phenoxy)-4-methyl-phenyl]-N-(4-sulfamoyl-phenyl)-acetamide (I-30)

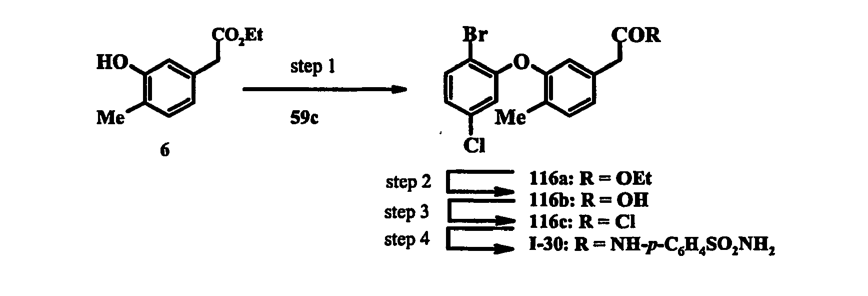

step 1- A solution of 1-bromo-4-chloro-2-fluoro-benzene (59c, 0.415 g, 1.98 mmol), **6** (0.350 g, 1.8 mmol), K₂CO₃ (0.746 g, 5.4 mmol) and NMP (4 mL) was stirred and heated to 120° C for 6 h. The reaction mixture was cooled to RT and diluted with H₂O (25 mL) and twice extracted with EtOAc. The combined organic extracts were washed sequentially with water (6 times) and brine, dried (Na₂SO₄), filtered and concentrated *in vacuo.* The crude product was purified by SiO₂ column chromatography eluting with a EtOAc/hexane (100% to 75% hexane) to afford **116a**

Steps 2-4 were carried out by the procedure described in steps 7-9 of Example 1 which afforded **I-30.**

Compound **I-31** was prepared using a similar procedure except in step 4, 4-amino-sulfonamide was replaced by 4-amino-3-methyl-sulfonamide.

Compound **I-33** was prepared using a similar procedure except in step 4, 4-amino-sulfonamide was replaced by 2-chloro-phenylamine.

### Example 9

### (4-{2-[3-(3-Chloro-5-cyano-phenoxy)-4-methyl-phenyl]-acetylamino}-3-methyl-benzenesulfonyl)-acetic acid (I-152)

Reactions depicted in SCHEME 13

step 1 - To a solution of **98a** (7.76 g, 50 mmol) and EtOH (15 mL) was added portion wise a hot solution of Na₂S·9H₂O (9.6 g, 40 mmol), powdered sulfur (1.28 g, 40 mmol) and EtOH (50 mL). The solution was heated at reflux for 10 min after the addition was completed. To the solution was added portion wise NaOH (2 g, 50 mmol) over 5 min and the solution was stirred for an additional 10 min. The reaction mixture was cooled to RT and poured into ice water (200 mL), stirred, filtered and the solid washed with water. The filtrate was acidified with aqueous HCl to pH 2 and the resulting orange precipitate was washed with water and air dried. The crude product was chromatographed on SiO₂ and eluted with 20% EtOAc/hexane to afford 3 g of **98b** as an orange powder.

step 2 - To a solution of **98b** (0.508 g, 3 mmol) and MeCN (10 mL) was added TEA (0.83 ml, 6 mmol). To the dark red solution was added methyl bromoacetate (0.31 mL, 3.3 mmol) and the reaction mixture was stirred overnight at RT. The reaction mixture was diluted with saturated NH₄Cl and twice extracted with EtOAc, dried (MgSO₄) and concentrated *in vacuo.* The crude product was purified by SiO₂ chromatography eluting with a gradient of hexane/EtOAc (20% to 30% EtOAc) which afford 0.5 g of **98c** as a yellow oil.

step 3 - To a solution of **98c** (0.484 g, 2 mmol) in DCM (20 mL) was added MCPBA (1.44 g, 5 mmol, 60% assay) and the reaction stirred at RT. Stirring was continued until the reaction was complete. m-Chlorobenzoic acid precipitated from the reaction. The precipitated solid was dissolved by addition of DCM until the precipitate dissolved and the DCM solution then was washed sequentially with saturated sodium bisulfite and 5% NaHCO₃, dried (MgSO₄) and evaporated to afford 0.538 g of **98d** as an oil which solidified upon standing.

step 4 - A mixture of **98d** (0.537 g, 1.96 mmol), iron powder (0.55 g, 9.8 mmol, Fischer electrolyte iron powder), NH₄Cl (0.53 g, 9.8 mmol) and EtOH/water (1:1, 40 mL) was stirred at 85° C for 2 h. The red solution was cooled to RT and filtered through a pad of CELITE® and the pad washed with EtOAc. The organic solvents were removed in *vacuo* and the residue suspended in water and twice extracted with EtOAc. The combined extracts were dried (MgSO₄), filtered and evaporated to afford 0.449 g of 100 as a brown oil which solidified upon standing.

step 5 - To a solution of **94b** (0.140 g) dissolved in DCM (10 mL) and 1 drop of DMF was added oxalyl chloride (1 mL). The reaction was stirred at RT for 1 h and the volatile solvents removed *in vacuo* to afford [3-(3-chloro-5-cyano-phenoxy)-4-methyl-phenyl]-acetyl chloride. To a solution of 100 (0.113 g, 0.465 mmol), TEA (0.13 mL, 0.93 mmol) and EtOAc (6 mL) was added dropwise a solution of the acid chloride and EtOAc (4 mL) over a 2 h period. The reaction was stirred at RT for 2 h. The reaction mixture was diluted with water, twice extracted with EtOAc and the combined extracts washed sequentially with diluted HCl, saturated NaHCO₃, water and brine. The organic phase was dried (MgSO₄), filtered and concentrated *in vacuo.* The crude product was redissolved in a minimum volume of warm EtOAc and hexane was added until slightly cloudy. The white powder which precipitated was filtered and washed with 20% EtOAc/hexane to afford 0.161 g of **122.**

step 6 - To a solution of 122 (0.161 g,0.036 mmol), THF (12 mL) and water (6 mL) was added LiOH.H₂O (0.0384 g, 0.916 mmol) and the reaction stirred at RT for 2 h. The reaction mixture was evaporated, 1N HCl was added and the resulting mixture twice extracted with EtOAc. The combined extracts were washed with brine, dried (MgSO₄), filtered and concentrated *in vacuo.* The crude product was purified by SiO₂ chromatography eluting with MeOH/DCM/HOAc (5:94.5:0.5) which afforded 0.255g of **I-152** as a white foam.

Compounds **I-137, I-154** and **I-157** were prepared similarly except in step 3 methyl bromoacetate was replaced with bromoacetonitrile, (2-bromo-ethyl)-carbamic acid *tert-*butyl ester and 3-bromoporpano respectively. After condensation of [2-(4-amino-3-methyl-benzenesulfonyl)-ethyl]-carbamic acid *tert*-butyl ester with **112c**, the Boc group was removed by treatment with TFA/DCM.

### Example 10

### 2-[3-(3-Chloro-5-cyano-phenoxy)-4-ethyl-phenyl]-N-(4-sulfamoyl-phenyl)-acetamide (I-45)

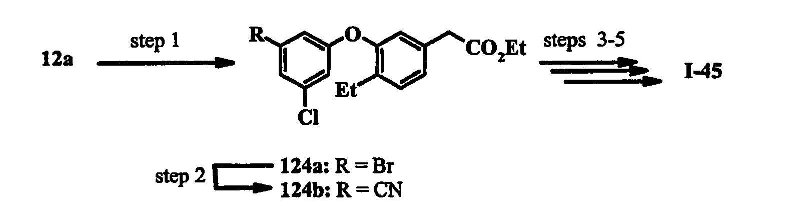

(4-Ethyl-3-hydroxy-phenyl)-acetic acid ethyl ester (**12a**) was prepared as depicted in SCHEME 1. Alternatively, **12a** can be prepared by the procedures in steps 1-3 of example 6 except diethylzinc was used in place of dimethylzinc in step 2.

To a stirred solution of ethyl 3-methoxyphenylacetate (16.0 g; 82.38 mmol) in CH₂Cl₂ (200 mL) at rt was added dropwise AcCl (9.88 mL; 138.9 mmol) followed by stannic chloride (16.9 mL;169 mmol; 1.0 M solution in CH₂Cl₂). The reaction mixture was stirred at rt for 6 h and poured into an ice-water mixture. The aqueous phase was extracted with CH₂Cl₂ and the combined extracts were washed with water, dried (Na₂SO₄) and the solvent removed *in vacuo.* The crude product 8 was purified by chromatography on silica gel and eluted with CH₂Cl₂:EtOAc (20:1) to yield 13.96 g (69.5%) of a white solid.

To a solution of 8 (19g; 80.42 mmol) and 200 mL of TFA cooled to 0°C was added an excess of Et₃SiH and the reaction allowed to warm to rt for 3 h. Excess TFA was removed in *vacuo* and the residue partitioned between water and CH₂Cl₂. The crude product was purified by chromatography on silica gel and eluted with CH₂Cl₂:hexane (3:1) to yield 3.0 g (16%) of 10.

A solution of ethyl 4-ethyl-3-methoxyphenylacetate (10; 3.0 g;13.50 mmol) and CH₂Cl₂ (80 mL) was cooled to -78°C and a solution of BBr₃ (5.10 mL; 53.94 mmol; 1.0 M in CH₂Cl₂) over 30 min. After 1 h at -78°C the reaction was allowed to warm to rt and stirred for 12 h. The reaction was cooled in an ice-water bath and the reaction quenched with 20 mL of water. The aqueous phase was extracted with CH₂Cl₂:EtOAc (4:1 v/v), dried (Na₂SO₄), filtered and evaporated. The crude product was purified by silica gel chromatography and eluted with a CH₂Cl₂:EtOAc gradient (100:1 to 100:4) to yield **12a** (2.0 g; 71%): ms 209.2 (M+H)⁺.

step 1 - To a solution of ethyl 4-ethyl-3-hydroxyphenylacetate (**12a**, 0.700 g; 3.36 mmol) and NMP (8 mL) was added K₂CO₃ (1.393 g; 10.08 mmol) and 1-bromo-3-chloro-5-fluorobenzene (**59b**, 0.774 g; 3.7 mmol). The reaction was heated to 120° C and monitored by TLC. After 8 h the reaction was cooled to RT and 10% HCl was added. The mixture was extracted with EtOAc and the combined extracts were washed with H₂O and brine. The extracts were dried (Na₂SO₄) filtered and evaporated. The crude product was chromatographed with silica gel and eluted with a gradient of hexane/EtOAc (100 to 60% hexane) to afford **124a**.

step 2 - To a solution of the **124a** (0.300 g; 0.754mmol) in DMF (5mL) was added Zn(CN)₂ (0.354 g; 3.017 mmol) and Pd(PPh₃)₄ (0.131 g; 0.113 mmol). The solution was purged with N₂ and heated at 85° C overnight. H₂O and EtOAc were added and the mixture stirred for 30 min. The suspension was filtered and the combined filtrate extracted with EtOAc. The organic phase was washed with H₂O and brine, dried (Na₂SO₄) and evaporated. The crude product was chromatographed with silica gel and eluted with a gradient of hexane/EtOAc (100 to 70% hexane) to afford **124b**

step 3 - A dried round bottom purged with nitrogen was charged with **124b** (0.380 g; 1.1 mmol) and THF (15 mL) and stirred under a stream of nitrogen. To the reaction vessel was added LiOH (0.079 g; 3.32 mmol) was added followed by deionized water (4 mL). The reaction was allowed to stir for an additional hour under nitrogen. The homogeneous mixture was cooled to 0° C and quenched with 10% aqueous HCl. The reaction mixture was stirred for an additional 15 min. The crude mixture was extracted with DCM and washed with water and brine. The organic layers were dried (Na₂SO₄) and filtered. Solvent removed *in vacuo* to yield a crude oil which was used without any further purification.

step 4 - A round bottom flask was charged with carboxyl acid from step 3 (1.1 mmol) and DCM (5 mL) and the solution stirred under nitrogen at RT. To the solution was added SOCl₂ (0.192 mL; 2.2 mmol) dropwise followed by a single drop of DMF. The reaction was stirred for 1 h at RT. Excess solvent and oxalyl chloride were removed *in vacuo,* to yield acid chloride as a crude yellow oil which was used without any further purification.

step 5 - The acid chloride (0.15 mmol) from the previous step dissolved in acetone (1 mL) and purged with nitrogen. NaHCO₃ (0.025 g; 0.3 mmol) was added followed by 4-amino-benzenesulfonamide (0.026 g; 0.15 mmol) and water (2 mL). The mixture was sonicated for 5 min and allowed to stir for 12 h at RT. The reaction mixture was filtered and the crude product was washed sequentially with water and diethyl ether to afford **I-**45.

Compound **I-46** was prepared in the same manner except 4-amino-benzenesulfonamide was replaced with 4-amino-3-methyl-benzenesulfonamide.

Compound **I-47** was prepared in the same manner except 4-amino-benzenesulfonamide was replaced with 4-amino-3-chloro-benzenesulfonamide.

Compound **I-207** was prepared in the same manner except 4-amino-benzenesulfonamide was replaced with phenylamine.

Compound **I-48** was prepared in the same manner except 4-amino-benzenesulfonamide was replaced with 4-amino-3,N-dimethyl-benzenesulfonamide.

Compound **I-73** was prepared in the same manner except 4-amino-benzenesulfonamide was replaced with 2-chloro-phenylamine.

### Example 11

### 2-[3-(3-Cyano-5-fluoro-phenoxy)-4-methyl-phenyl]-N-(4-sulfamoyl-phenyl)-acetamide (I-11)

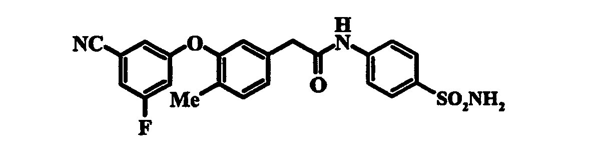

Compound **I-11** was prepared by condensation of 6b with 1,3-dibromo-5-fluorobenzene as described in step 1 of Example 10 which affords [3-(3-bromo-5-fluoro-phenoxy)-4-methyl-phenyl]-acetic acid ethyl ester. Displacement of the bromide with Zn(CN)₂ as described in step 2 of Example 10 affords [3-(3-cyano-5-fluoro-phenoxy)-4-methyl-phenyl]-acetic acid ethyl ester which was converted to I-11 as described in step 7 to 9 of Example 1.

Compound **I-12** was prepared in the same manner except 4-amino-benzenesulfonamide was replaced with 2-chloro-phenylamine.

### Example 12

### 2-[3-(3-Chloro-5-cyano-phenoxy)-4-methyl-phenyl]-N-[4-((S)-2,3-dihydroxy-propoxy)-2-methyl-phenyl]-acetamide (I-150)

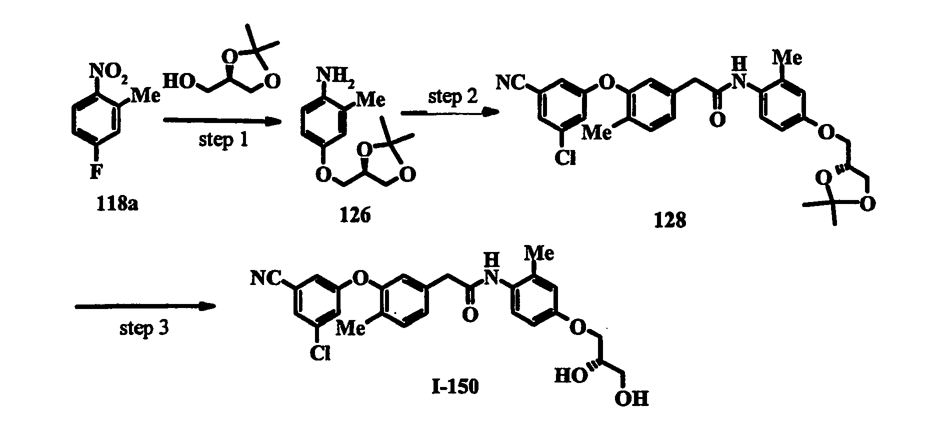

step 1- The (R)-dioxolane (1.04g, 7.86 mmol) was added to a suspension of NaH (0.35 g of a 60% dispersion in mineral oil, 1.1 equiv) in DMF (30 mL) under N₂ atmosphere at 0° C. The solution was stirred for 10 min, and **118a** (0.96 mL, 1 equiv) was added dropwise. The resulting red solution was stirred overnight, poured into a saturated solution of NH₄Cl, and extracted with a 1:1 EtOAc/hexane solution. The organic solution was washed with water, brine, and dried (MgSO₄). The solvent was evaporated and the crude product purified by SiO₂ chromatography eluting with a EtOAc/hexane gradient (5:95 to 25:75) to afford 1.10 g (52%) of the nitro compound. The oil was dissolved in EtOH (40 mL), and 10% Pd/C (0.09 g) was added. The solution was shaken under 40 psi of hydrogen for 3 h, filtered, and evaporated to afford 0.95 g (97%)of **126.**

step 2 - Condensation of 94c and 126 was carried out as described in step 9 of example 1 which afforded **128.**

step 3 - The amide **128** (0.23 g, 0.44 mmol) was dissolved in a mixture of 2M HCl (2 mL) and dioxane (2 mL). The solution was stirred at RT for 3 h. DCM was added, and the organic extracts were dried (MgSO₄) and concentrated *in vacuo.* The crude product was purified by SiO₂ chromatography eluting with a MeOH/DCM gradient (0% to 5% MeOH) which afforded 0.12 g (55%) of I-150.

Compound **I-151** was prepared as described above except the acetonide of 1,2,4-butanetriol was used in step 1.

### Example 13

### 2-[3-(3-Chloro-5-ryano-phenoxy)-4-methyl-phenyl]-N-[2-methyl-4-(2-morpholin-4-yl-acetyl)-phenyl]-acetamide; compound with trifluoro-acetic acid (I-163)

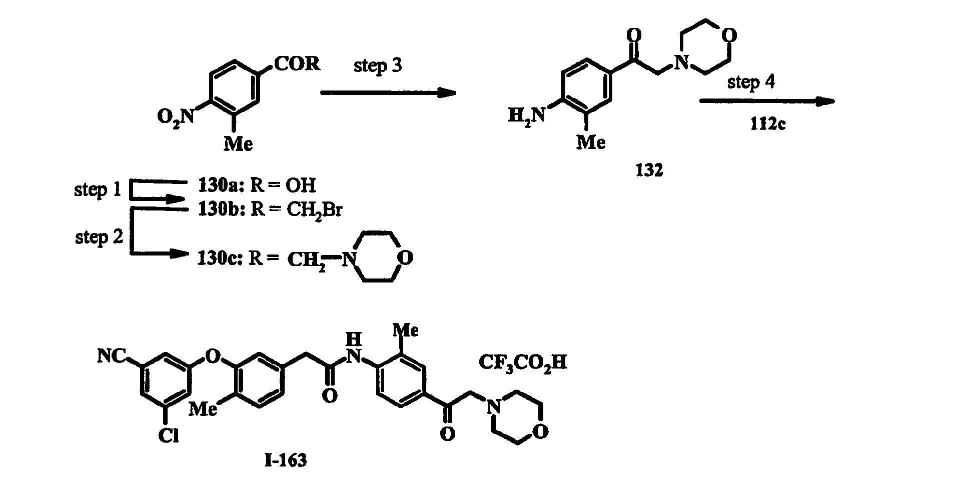

Step 1 - Thionyl chloride (7.55 g, 1.1 equiv) was added to a suspension of 130a (10.45 g, 57.7 mmol) in anhydrous toluene (50 mL) under N₂ atmosphere. DMF (0.44 mL, 0.1 equiv) was added, and the reaction was heated to reflux. The solution became homogenous, and was heated 4 h, cooled to RT and stirred for 2 d. The volatile materials were removed. Dry benzene (40 mL) was added and the volatile materials were again removed. This was repeated two more times. NMR of the resulting oil (C₆D₆) showed formation of the acid chloride. The resulting oil (11.3 g) was divided into 3 portions for conversion into the α-bromoketone. To a solution of the acid chloride in dry Et₂O (60 mL) that had been cooled to -78° C was quickly added an excess of a fresh ethereal solution of diazomethane. The solution was warmed to 0° C and bubbling was observed. The reaction was monitored by TLC, and after 20 min at 0° C a solution of 48% HBr (25 mL) was added. A precipitate formed, and the reaction mixture was diluted with an additional 100 mL of ether. The three reaction solutions were combined, the aqueous layer was separated and extracted with ether. The organic layers were combined, washed sequentially with water, NaHCO₃ and brine, and dried (MgSO₄). The solvent was removed, and the material was crystallized from EtOAc/hexanes which afforded 130b. NMR of the resulting solid (shows a 8:1 ratio of bromomethyl product to chlororomethyl impurity and was used in the subsequent reactions. step 2 - Morpholine (1.35 mL, 5 equiv) was added dropwise to a 0° C solution of

130b (0.80 g, 3.1 mmol) in dry THF (10 mL) under N₂ atmosphere. The solution was warmed to 10° C. After 30 min, the solution was added dropwise to a mixture of EtOAc and saturated NaHCO₃ solution. The mixture was extracted with EtOAc, the volatile materials were removed, and the residue was purified by SiO₂ chromatography eluting with a EtOAc/hexane gradient (25% to 75% EtOAc) to afford 0.51 grams (62%) of **130c.**

step 3 - The nitro compound **130c** (0.50 g,1.9 mmol) was dissolved in EtOH (10 mL) and PtO₂ (21 mg, 0.05 equiv) was added. The solution was degassed and placed under a H₂ atmosphere (balloon pressure). After 1 h, a grey solid precipitated. The solution was stirred for 2 h and filtered, and the precicitate washed with EtOAc and MeCN. The solvent was removed, and the residue was purified by SiO₂ chromatography eluting with a MeOH/DCM gradient (2% to 5% MeOH) to afford 0.21 g (47%) of 132.

step 4 - Condensation of **94c** and 132 was carried out as described in step 9 of example 1 which afforded **I-163.**

Compound **I-159** was prepared in a similar manner except N-methyl piperazine was used in place of morpholine. **I-161** and **I-163** were prepared in a similar manner except thiomorpholine was used in place of morpholine. The sulfur atom was oxidized to the corresponding sulfoxide and sulfone with HIO₄ and MCPBA.

### Example 14

### 2-[3-(3-Chloro-5-cyano-phenoxy)-4-methyl-phenyl]-N-[4-(1,1-dioxo-1λ6-isothiazolidin-2-yl)-2-methyl-phenyl]-acetamide (I-170)

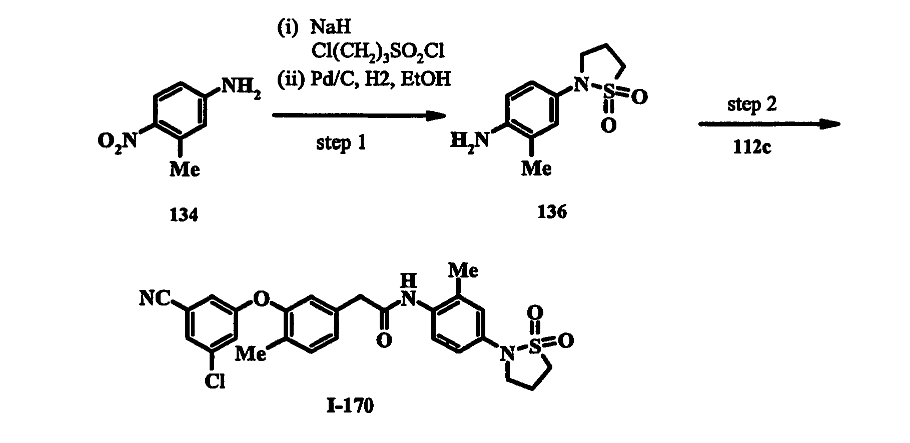

step 1 - NaH (0.40 g of a 60% dispersion, 3 equiv) was added to a solution of the **134** (0.50 g, 3.29 mmol) in DMF (18 mL) at 0° C. The solution was warmed to RT. After 30 min, a DMF (4 mL) solution of 3-chloro-propane-1-sulfonyl chloride (0.81 g,1.4 equiv) was added. The solution was stirred at RT for 45 min, and then heated to reflux for 4 h. The reaction mixture was cooled to RT and quenched with ice water. The aqueous layer was extracted with EtOAc, and the organic layers were dried (MgSO₄). Filtration and evaporation of the volatile materials afforded an oil that was purified by SiO₂ chromatography eluting with an EtOAc/hexane gradient (50% to 70% EtOAc) to afford the sulfonamide as a yellow solid (0.49 g, 59%). This sulfonamide was dissolved in EtOH (20 mL). 10% Pd/C (0.05 g) was added, the solution was placed under a H₂ atmosphere (50 psi), and the solution was shaken for 3 h. Filtration of the reaction mixture and evaporation of the volatile materials afforded 0.44g of **136.**

step 2 - Condensation of 94c and 136 was carried out as described in step 9 of example 1 which afforded 1-170.

Compound I-158 was made by a similar procedure except in 3-chloro-propane-1-sulfonyl chloride was replaced with 3-chloro-butane-1-sulfonyl chloride

### Example 15

### N-(4-{2-[3-(3-Chloro-5-cyano-phenoxy)-4-methyl-phenyl]-acetylamino}-3-methyl-phenyl)-oxalamic acid ethyl ester (I-178)

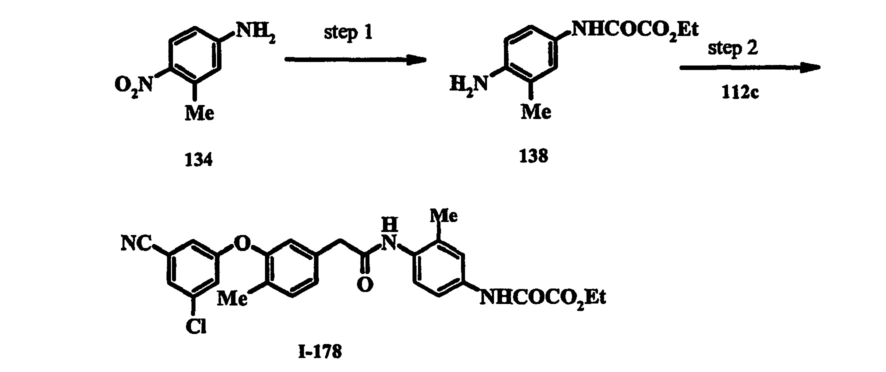

step 1- TEA (1 mL, 1.1 equiv) and chloro-oxo-acetic acid ethyl ester (0.9 mL, 1.2 equiv) were added consecutively to a 0° C solution of 134 (1.0 g, 6.57 mmol) in THF (60 mL). The reaction was stirred overnight at RT, evaporated to dryness, and dissolved in DCM. The organic layer was washed with water, dried (MgSO₄), and concentrated to give a yellow solid. The solid was purified by SiO₂ chromatography eluting with an EtOAc/hexane gradient (0% to 30% EtOAc) to afford 0.847 g (51 %) of the amide as a yellow solid. The amide (500 mg) was dissolved in EtOH (20 mL), 10% Pd/C (50 mg) was added, and the reaction was placed under H₂ (50 psi) and shaken for 2 h. Filtration of the catalyst and concentration of the filtrate afforded 0.320 g (73%) of 138 as a light yellow oil (0.320g, 73%)

step 2 - Condensation of 94c and 138 was carried out as described in step 9 of example 1 which afforded **1-178.**

### Example 16

### 2-[3-(3-Bromo-5-chloro-phenoxy)-4-ethyl-phenyl]-N-(4-sulfamoyl-phenyl)-acetamide (I-15)

step 1- A dried round bottom purged with nitrogen was charged with [3-(3-bromo-5-chloro-phenoxy)-4-ethyl-phenyl]-acetic acid ethyl ester (**124a** (example 10), 0.200 g; 0.5 mmol) and THF (6 mL) and maintained under a stream of nitrogen. To the reaction vessel was added LiOH (0.036 g;1.5 mmol) and deionized water (2 mL). The reaction was allowed to stir for 1 h under N₂. The homogeneous mixture cooled to 0° C and 10% aqueous HCl was added. The reaction mixture was stirred for an additional 15 min. The crude mixture was extracted with DCM and washed with water and brine. The organic layers were dried (Na₂SO₄), filtered and the solvent removed *in vacuo* to yield [3-(3-bromo-5-chloro-phenoxy)-4-ethyl-phenyl]-acetic acid as a crude oil which was used without any further purification.

step 2 - The acid chloride was prepared from the acid in step 1 as described in step 8 of Example 1.

step 3 - The acid chloride (0.25 mmol) from step 2 was dissolved in acetone (3 mL) and purged with nitrogen. NaHCO₃ (0.042g; 0.5 mmol) was added followed by 4-amino-benzenesulfonamide (0.043 g; 0.25 mmol) and water (6 mL). The mixture was sonicated for 5 min and allowed to stir for 12 h at RT. The reaction mixture was filtered and the crude product was washed sequentially with water and diethyl ether to afford 1-22.

Compound 1-16 was prepared in the same manner except 4-amino-benzenesulfonamide was replaced with 2-chloro-phenylamine.

### Example 17

### 2-[3-(2-Bromo-5-chloro-phenoxy)-4-ethyl-phenyl]-N-(4-sulfamoyl-phenyl)-acetamide (I-15)

step 1 - To a solution of ethyl 4-ethyl-3-hydroxyphenylacetate (**12a**, 1.000 g; 4.8 mmol) and NMP (10 mL) was added K₂CO₃ (1.99 g; 14.4 mmol) and 1-bromo-4-chloro-2-fluorobenzene (1.106 g; 5.28 mmol). The reaction was heated to 120° C and monitored by TLC. After 8 h the reaction was cooled to RT and 10% HCl was added. The mixture was extracted with EtOAc and the combined extracts were washed with H₂O and brine. The extracts were dried (Na₂SO₄) filtered and evaporated. The crude product was chromatographed with SiO₂ and eluted with a gradient of hexane/EtOAc (100:0 to 60:40) to afford [3-(2-bromo-5-chloro-phenoxy)-4-ethyl-phenyl]-acetic acid ethyl ester.

Steps 2 and 3 - Hydrolysis and formation of the acid chloride were carried as described in step 7and 8 of Example 1 and used without additional purification.

step 4 - [3-(2-Bromo-5-chloro-phenoxy)-4-ethyl-phenyl]-acetic acid ethyl ester (0.5 mmol) was dissolved in acetone (2 mL) and the flask was purged with N₂. NaHCO₃ (0.084 g;1.0 mmol) was added followed by 4-amino-benzenesulfonamide (0.086 g; 0.5 mmol) and water (6 mL). The mixture was sonicated for 5 min and allowed to stir for 12 h at RT. The reaction mixture was filtered and the crude product was washed sequentially with water and diethyl ether to afford **I-15.**

### Example 18

### 2-[3-(5-Chloro-2-cyano-phenoxy)-4-ethyl-phenyl]-N-(4-sulfamoyl-phenyl)-acetamide (I-17)

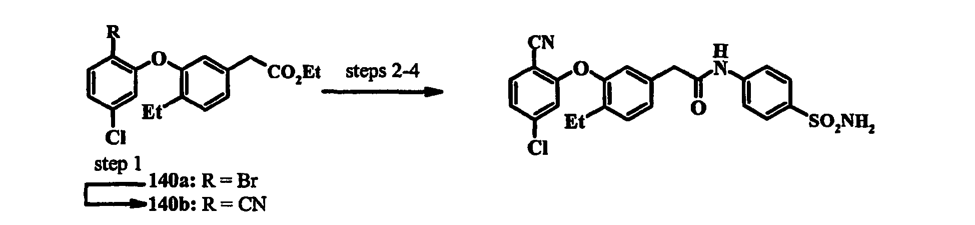

step 1 - To a solution of **140b** (step 1 of example 17, 0.629 g; 1.58 mmol) in DMF (14 ml) was added Zn(CN)₂ (0.929 g; 7.9 mmol) and Pd(PPh₃)₄ (0.366 g; 0.32 mmol). The solution was purged with N₂ and heated at 85° C overnight. The reaction mixture was partitioned between H₂O and EtOAc and the mixture stirred for 30 min. The suspension was filtered and the combined filtrate extracted with EtOAc. The organic extracts were washed with H₂O and brine, dried (Na₂SO₄) and evaporated. The crude product was purified by SiO₂ chromatography and eluted with a gradient of hexane/EtOAc (100:0 to 70:30) to afford **140b.**

Steps 2 and 3 - Hydrolysis and formation of the acid chloride were carried as described in step 7and 8 of Example 1 and used without additional purification.

step 4 - The acid chloride from step 3 (0.29 mmol) was dissolved in acetone (1 mL) of acetone and purged with nitrogen. NaHCO₃ (0.049 g; 0.58 mmol) was added followed by 4-amino-benzenesulfonamide (0.050 g; 0.29 mmol) and water (2 mL). The mixture was sonicated for 5 min and allowed to stir for 12 h at RT. The reaction mixture was filtered and the crude product was washed sequentially with water and diethyl ether to afford **I-17.**

Compound **I-18** was prepared in the same manner except 4-amino-benzenesulfonamide was replaced by 2-chloro-phenylamine.

### Example 19

### 2-[3-(2,5-Dichloro-phenoxy)-4-ethyl-phenyl]-N-(4-sulfamoyl-phenyl)-acetamide (I-19)

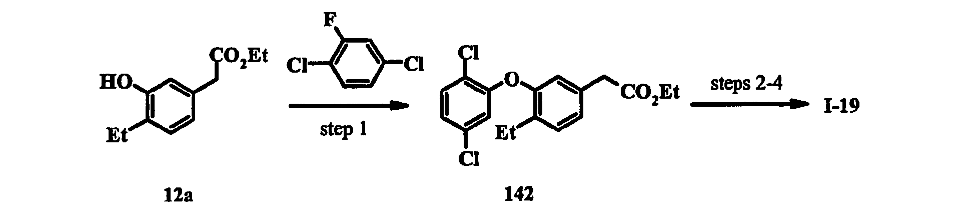

step 1- To a solution of **12a** (0.200 g; 0.960 mmol) and NMP (4 mL) was added K₂CO₃ (0.398 g; 2.88 mmol) and 1,4-dichloro-2-fluoro-benzene (0.124 mL; 1.056 mmol). The reaction was heated to 120° C and monitored by TLC. After 8 h the reaction was cooled to RT and 10% HCl was added. The mixture was extracted with EtOAc and the combined extracts were washed with H₂O and brine. The extracts were dried (Na₂SO₄) filtered and evaporated. The crude product was purified by SiO₂ chromatography eluting with a gradient of hexane/EtOAc (100:0 to 60:40) to afford **142.**

steps 2 and 3 - Hydrolysis and formation of the acid chloride were carried as described in step 7and 8 of Example 1 and used without additional purification.

step 4 - The acid chloride from step 3 (0.415 mmol) was dissolved in acetone (2 mL) and the flask and purged with nitrogen. NaHCO₃ (0.070 g; 0.83 mmol) was added followed by 4-amino-benzenesulfonamide (0.072 g; 0.415 mmol) and water (4 mL). The mixture was sonicated for 5 min and allowed to stir for 12 h at RT. The reaction mixture was filter and the crude product was washed sequentially with water and diethyl ether to afford **I-19**

Compound **I-21** was prepared in the same manner except 4-amino-benzenesulfonamide was replaced with 2-chloro-phenylamine.

### Example 20

### 2-[3-(2,6-Dichloro-phenoxy)-4-ethyl-phenyl]-N-(4-sulfamoyl-phenyl)-acetamide (I-20)

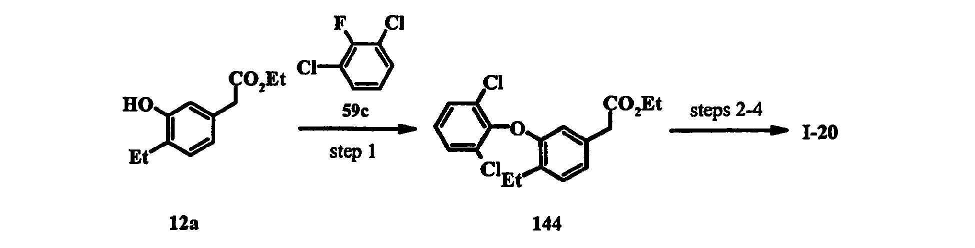

step 1- To a solution of **12a** (0.200 g; 0.960 mmol) and NMP (4 mL) was added K₂CO₃ (0.398 g; 2.88 mmol) and 1,3-dicholoro-2-fluoro-benzene (0.174 g; 1.056 mmol). The reaction was heated to 120° C and monitored by TLC. After 8 h the reaction was cooled to RT and 10% HCl was added. The mixture was extracted with EtOAc and the combined extracts were washed with H₂O and brine. The extracts were dried (Na₂SO₄) filtered and evaporated. The crude product was purified by SiO₂ chromatography eluting with a gradient of hexane/EtOAc (100:0 to 60:40) to afford **144.**

steps 2 and 3 - Hydrolysis and formation of the acid chloride were carried as described in step 7and 8 of Example 1 and used without additional purification.

step 4 - The acid chloride from step 3 (0.14 mmol) was dissolved in acetone (2 mL) and the flask was purged with nitrogen. NaHCO₃ (0.024 g; 0.28 mmol) was added followed by 4-amino-benzenesulfonamide (0.024 g; 0.14 mmol) and water (4 mL). The mixture was sonicated for 5 min and allowed to stir for 12 hours at room temperature. The reaction mixture was filtered and the crude product was washed sequentially with water and diethyl ether to afford **I-20.**

Compound **I-23** was prepared in the same manner except 4-amino-benzenesulfonamide was replaced by 2-chloro-phenylamine.

### Example 21

### 2-[3-(3-Bromo-2,5-dichloro-phenoxy)-4-ethyl-phenyl]-N-(4-sulfamoyl-phenyl)-acetamide (I-35)

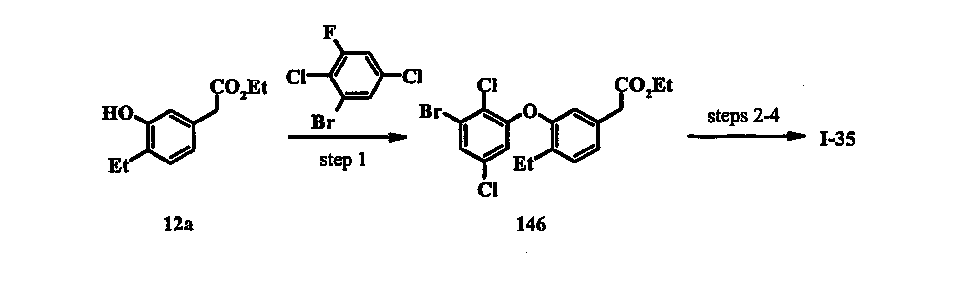

step 1 - To a solution of **12a** (0.450 g; 2.160 mmol) and NMP (5 mL) was added K₂CO₃ (0.896 g; 6.48 mmol) and 1-bromo-2,5-dichloro-3-fluoro-benzene (0.580 g; 2.38 mmol). The reaction was heated to 120° C and monitored by TLC. After 8 h the reaction was cooled to RT and 10% HCl was added. The mixture was extracted with EtOAc and the combined extracts were washed with H₂O and brine. The extracts were dried (Na₂SO₄) filtered and evaporated. The crude product was purified by SiO₂ chromatography eluting with a gradient of hexane/EtOAc (100:0 to 60:40) to afford 146.

steps 2 and 3 - Hydrolysis and formation of the acid chloride were carried as described in step 7and 8 of Example 1 and used without additional purification.

step 4 - The acid chloride from step 3 (0.112 mmol) was dissolved in acetone (1 mL) and the flask was purged with nitrogen. NaHCO₃ (0.019 g; 0.224 mmol) was added followed by 4-amino-benzenesulfonamide (0.019 g; 0.112 mmol) and water (2 mL). The mixture was sonicated for 5 min and allowed to stir for 12 h at RT. The reaction mixture was filtered and the crude product was washed sequentially with water and diethyl ether to afford **I-35.**

Compound **I-36** was prepared in the same manner except 4-amino-benzenesulfonamide was replaced with 4-amino-3-methyl-benzenesulfonamide.

Compound **I-37** was prepared in the same manner except 4-amino-benzenesulfonamide was replaced with 2-chloro-phenylamine.

### Example 22

### N-(2-Chloro-phenyl)-2-[3-(3,5-dibromo-2-chloro-phenoxy)-4-ethyl-phenyl]-acetamide (I-38)

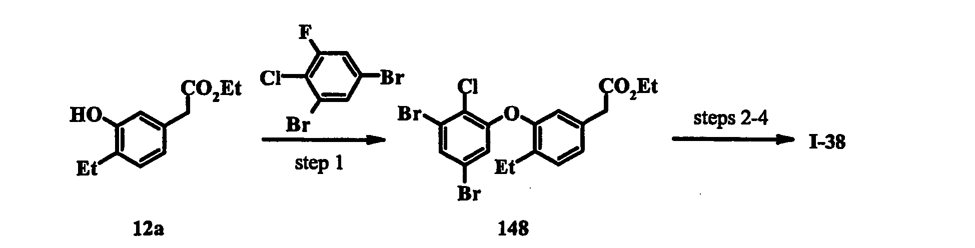

step 1 - To a solution of **12a** (0.500 g; 2.4 mmol) and NMP (5 mL) was added K₂CO₃ (0.995 g; 7.2 mmol) and 2-chloro-1,5-dibromo-3-fluoro-benzene (0.762 g; 2.64 mmol). The reaction was heated to 120° C and monitored by TLC. After 8 h the reaction was cooled to RT and 10% HCl was added. The mixture was extracted with EtOAc and the combined extracts were washed with H₂O and brine. The extracts were dried (Na₂SO₄), filtered and evaporated. The crude product was purified by SiO₂ chromatography eluting with a gradient of hexane/EtOAc (100:0 to 60:40) to afford 148.

steps 2 and 3 - Hydrolysis and formation of the acid chloride were carried as described in step 7and 8 of Example 1 and used without additional purification.

step 4 - The acid chloride from step 3 (0.155 mmol) was dissolved in acetone (1 mL) and purged with nitrogen. NaHCO₃ (0.026 g; 0.31 mmol) was added followed by (0.027 g; 0.155 mmol) and water (2 mL). The mixture was sonicated for 5 min and allowed to stir for 12 h at RT. The reaction mixture was filtered and the crude product was washed sequentially with water and diethyl ether to afford **I-38.**

### Example 23

### 2-[3-(2-Chloro-3,5-dicyano-phenoxy)-4-ethyl-phenyl]-N-(4-sulfamoyl-phenyl)-acetamide (I-52)

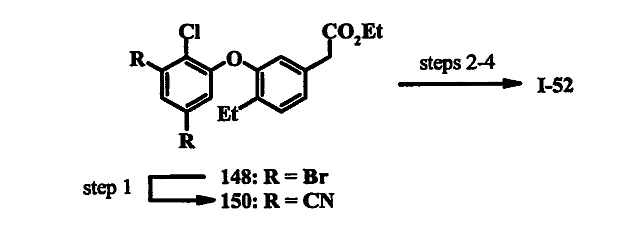

step 1- To a solution of the ester **148** (0.660 g; 1.43 mmol) in THF (7 ml) was added Zn(CN)₂ (0.673 g; 5.73 mmol) and Pd(PPh₃)₄ (0.248 g; 0.215 mmol). The solution was purged with N₂ and heated at 85°C overnight. The reaction mixture was partitioned between H₂O and EtOAc and the mixture stirred for 30 min. The suspension was filtered and the combined filtrate extracted with EtOAc. The organic extracts were washed with H₂O and brine, dried (Na₂SO₄) and evaporated. The crude product was purified by SiO₂ chromatography eluting with a gradient of hexane/EtOAc (100:0 to 70:30) to afford 150.

steps 2 and 3 - Hydrolysis and formation of the acid chloride were carried as described in step 7and 8 of Example 1 and used without additional purification.

step 4 - The acid chloride from step 3 (0.12 mmol) was dissolved in acetone (1 mL) and the flask was purged with nitrogen. NaHCO₃ (0.020 g; 0.24 mmol) was added followed by 4-amino-benzenesulfonamide (0.021 g; 0.12 mmol) and water (2 mL). The mixture was sonicated for 5 min and allowed to stir for 12 h at RT. The reaction mixture was filtered and the crude product was washed sequentially with water and diethyl ether to afford **I-52.**

Compound 1-53 was prepared in the same manner except in step 4, 4-amino-benzenesulfonamide was replaced with 4-amino-3-methyl-benzenesulfonamide.

Compound I-54 was prepared in the same manner except in step 4, 4-amino-benzenesulfonamide was replaced with 4-amino-3-chloro-benzenesulfonamide.

Compound 1-60 was prepared in the same manner except in step 4, 4-amino-benzenesulfonamide was replaced with 2-chlorophenylamine.

Compound 1-208 was prepared in the same manner except in step 4, 4-amino-benzenesulfonamide was replaced with phenylamine.

### Example 24

### 2-[3-(2-Chloro-5-cyano-3-fluoro-phenoxy)-4-ethyl-phenyl]-N-(4-sulfamoyl-phenyl)-acetamide (I-49)

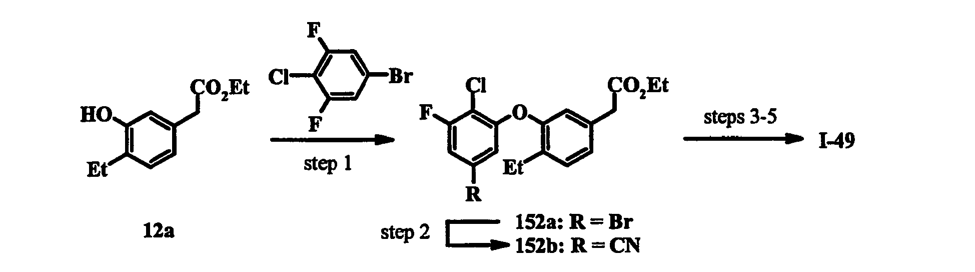

step 1 - To a solution of 12a (0.400 g; 1.920 mmol) and NMP (5 mL) was added K₂CO₃ (0.796 g; 5.76) and 5-bromo-2-chloro-1,3-difluoro-benzene (0.481 g; 2.11 mmol). The reaction was heated to 120°C and monitored by TLC. After 8 h the reaction was cooled to RT and 10% HCl was added. The mixture was extracted with EtOAc and the combined extracts were washed with H₂O and brine. The extracts were dried (Na₂SO₄), filtered and evaporated. The crude product was purified by SiO₂ chromatography eluting with a gradient of hexane/EtOAc (100:0 to 60:40) to afford **152a**.

step 2 - To a solution of **152a** (0.420 g; 1.01 mmol) in DMF (5 ml) was added Zn(CN)₂ (0.475 g; 4.04 mmol) and Pd(PPh₃)₄ (0.175 g; 0.152 mmol). The solution was purged with N₂ and heated at 85°C overnight. The reaction mixture was cooled to RT and partitioned between H₂O and EtOAc and the mixture stirred for 30 min. The suspension was filtered and the combined filtrate extracted with EtOAc. The organic extracts were washed with H₂O and brine, dried (Na₂SO₄) and evaporated. The crude product was purified by SiO₂ chromatograph eluting with a gradient of hexane/EtOAc (100:0 to 70:30) to afford 152b.

steps 3 and 4 - Hydrolysis and formation of the acid chloride were carried as described in step 7and 8 of Example 1 and used without additional purification.

step 5 - The acid chloride from step 4 (0.1 mmol) was dissolved in acetone (1 mL) and the flask was purged with nitrogen. NaHCO₃ (0.017 g; 0.2 mmol) was added followed by 4-amino-benzenesulfonamide (0.017 g; 0.1 mmol) and water (2 mL). The mixture was sonicated for 5 min and allowed to stir for 12 h at RT. The reaction mixture was filtered and the crude product was washed sequentially with water and diethyl ether to afford **I-49.**

Compound **I-50** was prepared in the same manner except 4-amino-benzenesulfonamide was replaced with 4-amino-3-methyl-benzenesulfonamide.

Compound **I-59** was prepared in the same manner except 4-amino-benzenesulfonamide was replaced with 2-chlorophenylamine.

### Example 25

### 2-[3-(5-Bromo-2-chloro-3-fluoro-phenoxy)-4-ethyl-phenyl]-N-(4-sulfamoyl-phenyl)-acetamide (I-39)

**I-39** was prepared as described in the previous example except cyanide displacement of the aryl bromide (step 2) was omitted.

Compound **I-40** was prepared in the same manner except in step 4, 4-amino-benzenesulfonamide was replaced by 4-amino-3-methyl-benzenesulfonamide.

Compound **I-41** was prepared in the same manner except in step 4, 4-amino-benzenesulfonamide was replaced with 2-chlorophenylamine.

### Example 26

### 2-[3-(3-Cyano-5-difluoromethyl-phenoxy)-4-ethyl-phenyl]-N-(4-sulfamoyl-phenyl)-acetamide (I-91, see SCHEME 8)

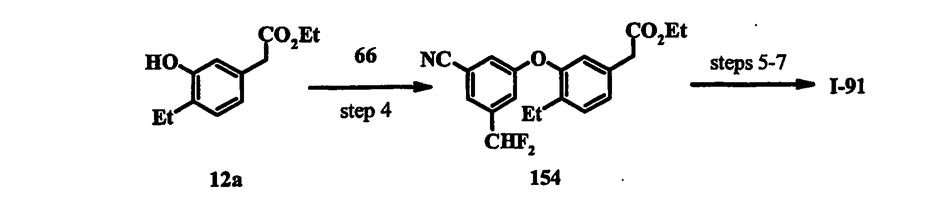

step 1 - To a solution of **64a** (25.39 g, 0.1 mol) and anhydrous Et₂O (125 mL)cooled to -78°C and maintained under an Ar atmosphere was added dropwise over 30 min n-BuLi (40 mL, 0.1 mol, 2.5M in hexane). The yellow solution was stirred at -78°C for 10 min. To the reaction mixture was added dropwise dry DMF (8.52 mL, 2.2 mmol) over 5 min and the reaction stirred at -78°C for 10 min before the cooling bath was removed and the reaction allowed to warm to -30° C over 30 min. The reaction vessel was placed in an ice-water bath and warmed to -10° C. The mixture was slowly added to an ice cold saturated aqueous NH₄Cl solution (400 mL). The organic layer was separated and the aqueous phase thrice extracted with Et₂O. The combined extracts were washed with water, dried (MgSO₄), filtered and evaporated to afford an oil which solidified on standing. The crude product was purified by SiO₂ chromatography eluting with a hexane/EtOAc gradient (97:3 to 95:5) to afford 15 g of **64b**.

step 2 - To a solution of 64b (32.4 g, 0.15 mol) and DCM (160 mL) cooled to -10°C in an ice/MeOH/water bath under an Ar atmosphere in a septum-capped I L Nalgene narrow-neck bottle is added dropwise DAST (35.85 mL. 0.27 mol). The reaction mixture was stirred overnight. The reaction mixture was added dropwise over a 30 min period to saturated aqueous NaHCO₃ (400 mL) cooled to 0°C. Additional saturated NaHCO₃ was added to maintain the reaction at a slightly basic pH. The phases were separated and the aqueous phase was extracted twice with Et₂O and the combined extracts dried (MgSO₄) and concentrated at 30°C under house vacuum to afford 36 g of an orange oil which was purified by bulb-to-bulb distillation in a Kugel-Rohr at 100°C under house vacuum to afford 30.65 g of **64c**.

step 3 - A solution of **64c** (41.6 g, 0.182 mol), Pd[P(Ph)₃]₄(0) (15 g, 13 mmol), and zinc cyanide (12.82 g, 0.109 mol) in dry DMF (400 mL) under nitrogen was heated to 80° C for 5.5 h. The reaction mixture was cooled to RT, the yellow solid filtered and the filtrate added to water (500 mL). The filtrate was thrice extracted with Et₂O and the combined extracts washed twice with water, dried (MgSO₄), filtered and evaporated at 30°C. The crude was purified by SiO₂ chromatography eluting with a hexane/EtOAc gradient (100:0 to 95:5 to 90:10) to provide 26.3 g of 66 as a colorless oil which partially crystallizes.

step 4 - To a solution of **12a** (0.400 g; 1.920 mmol) and NMP (4 mL) was added K₂CO₃ (0.796 g; 5.76) and 3-fluoro-5-(difluoromethyl)-benzonitrile (66, 0.362 g; 2.11 mmol). The reaction was heated to 120° C and monitored by TLC. After 8 h the reaction was cooled to RT and 10% HCl was added. The mixture was extracted with EtOAc and the combined extracts were washed with H₂O and brine. The extracts were dried (Na₂SO₄) filtered and evaporated. The crude product was purified by SiO₂ chromatographed eluting with a gradient of hexane/EtOAc (100:0 to 60:40) to afford **154**.

steps 5 and 6 - Hydrolysis and formation of the acid chloride were carried as described in step 7and 8 of Example 1 and used without additional purification.

step 7 - The acid chloride from step 6 (0.25 mmol) was dissolved in acetone (1 mL) and purged with nitrogen. NaHCO₃ (0.042 g; 0.5 mmol) was added followed by 4-amino-benzenesulfonamide (0.043 g; 0.25 mmol) and water (2 mL). The mixture was sonicated for 5 min and allowed to stir for 12 h at RT. The reaction mixture was filtered and the crude product was washed sequentially with water and diethyl ether to afford **I-91.**

Compound **I-101** was prepared in the same manner except in step 7,4-amino-benzenesulfonamide was replaced with 4-amino-3-methyl-benzenesulfonamide.

Compound **I-140** was prepared in the same manner except in step 7,4-amino-benzenesulfonamide was replaced with 4-amino-3-chloro-benzenesulfonamide.

### Example 27

### N-(2-Chloro-phenyl)-2-[3-(3,5-dicyano-phenoxy)-4-ethyl-phenyl]-acetamide (I-25)

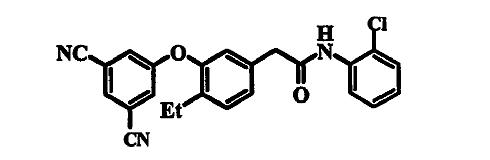

step 1 - Coupling of 5-fluoroisophthalonitrile and 12a was carried out as described in step 1 of example 7.

steps 2 and 3 - Hydrolysis and formation of the acid chloride were carried as described in step 7and 8 of Example 1 and used without additional purification.

step 4 - To a solution of the acid chloride from step 3 (0.15 mmol), TEA (22.7 µL, 0.225 mmol) and DCM (4 mL) cooled in an ice bath and maintained under a nitrogen atmosphere was added a solution of 2-chloro-phenylamine (15 µL, 0.0182 g; 0.1425 mmol) was added dropwise. The mixture was stirred for 12 h at RT. The solvents were removed *in vacuo* and the residue partitioned between H₂O. The aqueous phase was extracted with EtOAc and the combined extracts sequentially washed with I N HCL, saturated NaHCO₃ and brine. The organic extracts were dried (Na₂SO₄), filtered and the solvents remove *in vacuo.* The solid was triturated with Et₂O and filtered 1-25.

### Example 28

### 2-[3-(3-Chloro-5-cyano-phenoxy)-4-methoxy-phenyl]-N-(4-sulfamoyl-phenyl)-acetamide (I-62)

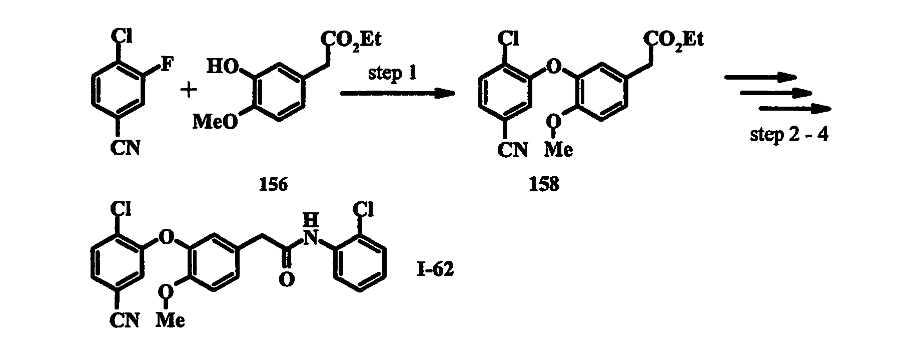

step 1 - To a solution of ethyl 4-methoxy-3-hydroxyphenylacetate (156,1.0 g; 4.46 mmol) and NMP (10 mL) was added K₂CO₃ (1.85 g; 13.38) and 4-chloro-3-fluoro-benzenonitrile (0.0.763 g; 4.9 mmol). The reaction was heated to 120° C and stirred overnight. The reaction was cooled to RT and 10% HCl was added. The mixture was extracted with EtOAc and the combined extracts were washed with H₂O and brine. The extracts were dried (Na₂SO₄), filtered and evaporated. The crude product was purified by SiO₂ chromatography eluting with a gradient of hexane/EtOAc (100:0 to 60:40) to afford **158.**

steps 2 and 3 - Hydrolysis and formation of the acid chloride were carried as described in step 7and 8 of Example 1 and used without additional purification.

step 4 - To a solution of the acid chloride from step 3 (0.36 mmol), TEA (75.2 µL, 0.0546 g, 0.54 mmol) and DCM (3 mL) cooled in an ice bath and maintained under a nitrogen atmosphere was added 2-chloro-phenylamine (36 µL, 0.0436 g; 0.342 mmol) was added dropwise. The mixture was stirred for 12 h at RT. The solvents were removed *in vacuo* and the residue partitioned between EtOAc and H₂O. The aqueous phase was extracted with EtOAc and the combined extracts sequentially washed with 1 N HCl, saturated NaHCO₃ and brine. The organic extracts were dried (Na₂SO₄), filtered and the solvents remove *in vacuo.* The solid was triturated with Et₂O and filtered to afford 1-62.

Compound **I-65** was prepared in the same manner except in step 4, 2-chloro-phenylamine was replaced by 4-amino-benzenesulfonamide.

Compound **I-66** was prepared in the same manner except in step 4, 2-chloro-phenylamine was replaced by 4-amino-3-methyl-benzenesulfonamide.

Compound **I-67** was prepared in the same manner except in step 4, 2-chloro-phenylamine was replaced by 4-amino-3-chloro-benzenesulfonamide.

### Example 29

### 2-[3-(3-Chloro-5-cyano-phenoxy)-4-methoxy-phenyl]-N-(4-sulfamoyl-phenyl)-acetamide (I-61)

Compound **I-61** was prepared by the procedures described in the previous example except in step 1, 4-chloro-3-fluoro-benzenonitrile was replaced by 3-chloro-5-fluoro-benzonitrile.

Compound **I-57** was prepared in the same manner except in step 4, 2-chloro-phenylamine was replaced with 4-amino-benzenesulfonamide.

Compound **I-64** was prepared in the same manner except in step 4, 2-chloro-phenylamine was replaced with 4-amino-3-chloro-benzenesulfonamide.

Compound **I-210** was prepared in the same manner except in step 4, 2-chlorophenylamine was replaced with phenylamine.

### Example 30

### 2-[3-(3-Cyano-5-difluoromethyl-phenoxy)-4-methoxy-phenyl]-N-(4-sulfamoyl-phenyl)-acetamide (I-92)

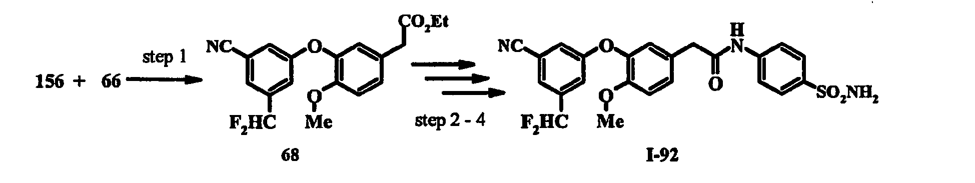

step 1 - Coupling of **66** and **156** to afford **68** was carried out as described in step 4 of example 26.

steps 2 and 3 - Hydrolysis and formation of the acid chloride were carried as described in step 7and 8 of Example 1 and used without additional purification.

step 4 - was carried out as described in step 4 of example 26 to afford **I-92.**

Compound **I-93** was prepared in the same manner except in step 7, 4-aminobenzenesulfonate was replaced with 4-amino-3-methyl-benzenesulfonamide.

Compound **I-94** was prepared in the same manner except in step 7, 4-aminobenzenesulfonate was replaced with 4-amino-3-chloro-benzenesulfonamide.

### Example 31

### 2-[4-Chloro-3-(3-chloro-5-cyano-phenoxy)-2-fluoro-phenyl]-N-(2-methyl-4-sulfamoyl-phenyl)-acetamide (I-42)

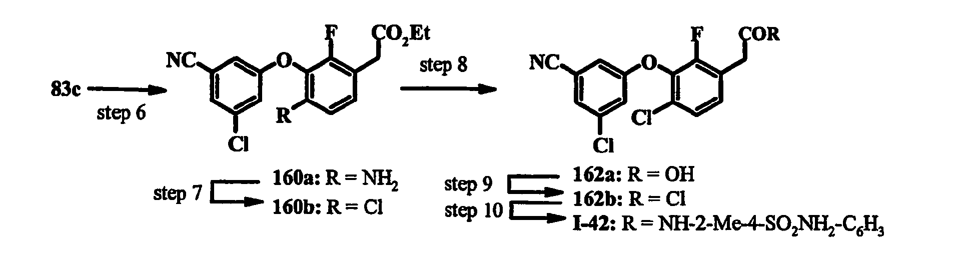

step 1 - (Steps 1-5 are depicted in Scheme 9) A 100 ml round bottom was charged under a stream of nitrogen with 3,5-dichlorobenzonitrile (**81a**, 7.0 g, 40.69 mmol) and anhydrous DMF (75 mL). To the solution was added sodium methoxide (2.26 g, 44.76 mmol) and resulting solution was stirred further at RT for 24 h. When the reaction was complete, aqueous 10% hydrochloric acid added drop wise to the reaction vessel. The crude mixture was extracted with EtOAc and sequentially washed with aqueous acid, water and brine. The EtOAc extracts were dried (Na₂SO₄), filtered and the solvent was removed *in vacuo* to afford a crude solid which was recrystallized from hexane/acetone to afford 5.9 g (86%) of **81b.**

step 2 - A 250 mL flask was charged with 81b (7.0 g, 41.766 mmol) and 2,4,6-collidine (100mL). The mixture was heated to 170°C and LiI (16.76 g, 125.298 mmol) was added and the reaction mixture was heated for 4 h. When 81b was consumed the reaction was cooled to RT and quenched with 10% aqueous HCl. The resulting mixture was extracted with EtOAc and washed with water and brine. The EtOAc extract was dried over (Na₂SO₄) and filtered. The solvent was removed *in vacuo* to afford a yellow oil which was purified by silica gel chromatography eluting with EtOAc/hexane (10:90) to afford 6.0 g (94%) of **81c.**

step 3 - A 250 mL round-bottom flask was charged with 81c (6.0 g, 39.070 mmol) and anhydrous THF (100 mL) and the solution was cooled to 0°C. To the cooled solution was added sodium *tert*-butoxide (46.89 g, 4.51 mmol) and the resulting solution stirred for 1 h. 2,3,4-Trifluoro-nitro-benzene (6.92 g, 39.070 mmol) was added dropwise while maintaining the reaction at 0° C until phenol was completely consumed. The mixture was quenched by addition of 10% aqueous HCl and the resulting mixture was stirred for an additional hour. The mixture was extracted with EtOAc and washed with water and brine. The EtOAc was dried (Na₂SO₄) and filtered. The solvent was removed *in vacuo* to yield a yellow oil which was purified by SiO₂ column chromatography eluting with hexane/EtOAc (92:8) to afford 10 g (82%) of 83a.

step 4 - To a solution of *tert-*butyl ethyl malonate (10.31 g, 54.80 mmol) and anhydrous NMP (200ml) cooled to 0° C and stirred under a nitrogen atmosphere. To this solution was added NaH 40% in mineral oil (1.84 g, 76.70 mmol). The mixture was allowed to stir at 0° C for an additional 1 h. The *bis*-aryl ether 83a (15.00 g, 49.80 mmol) was then added to the reaction vessel and stirred under nitrogen at RT until the reaction was complete. The mixture was quenched by addition of aqueous 10% HCl at RT. The mixture was extracted with EtOAc and washed with water and brine. The EtOAc was dried (Na₂SO₄) and filtered. The solvent was removed *in vacuo* to afford 83b as a light yellow oil which was used in the next step without any further purification.

step 5 - The diester 83b (24.0 g, 50.117 mmol) was dissolved in dichloroethane (300 mL) and TFA (6.29 g,55.13 mmol) and heated to 75°C for 24 h. The mixture was cooled to RT and solvent and excess TFA were removed *in vacuo.* The crude oil was redissolved in DCM and cooled to 0°C and aqueous NaHCO₃ was added. The mixture was extracted with DCM and washed with water and brine. The DCM was dried (Na₂SO₄), filtered and the solvent was removed *in vacuo* to afford a yellow oil. The crude oil was purified by SiO₂ chromatography eluting with hexane/EtOAc (90:10) to afford 15.0 g (80%) of 83c

step 6 - A 250 mL round bottom flask was charged with 83c (8.0, 21.12 mmol) and absolute EtOH. To the reaction vessel was added ammonium chloride (2.26 g, 42.244 mmol), water (30mL) and iron (1.17 g, 21.12 mmol). The reaction was stirred and heated to 80°C for 4 h. When 83c was consumed, the heterogeneous mixture was filtered through a pad of CELITE^{®} and the filter cake was washed with EtOAc. The aqueous filtrate was extracted with EtOAc and washed with water and brine. The combined EtOAc extracts were dried over (Na₂SO₄) and filtered. The solvent was removed *in vacuo* to afford a pale oil which was purified by SiO₂ chromatography eluting with hexane:EtOAc (85: 15) to afford 6.0 g (87%) of 160a.

step 7 - A 100 mL round bottom flask was charged with anhydrous MeCN (15 mL) under a continuous stream of nitrogen. To this mixture was added Cu(II)Cl₂ (0.083 g, 0.624 mmol) and *tert*-butyl nitrite (0.064 g, 0.624mmol). The mixture was heated to 70° C 30 min. To this mixture was added 160a (0.100 g, 0.624mmol) in a single portion and stirring continued for an additional 2 h. Upon consumption of starting materials the mixture was cooled to RT and reaction mixture quenched with aqueous 10% HCl. The mixture was extracted with EtOAc and the combined extracts were washed with water and brine. The EtOAc extract was dried (Na₂SO₄) and filtered. The solvent was removed *in vacuo* to afford a light brown oil which was purified by SiO₂ chromatography eluting with hexane/EtOAc (96:4) to afford 0.080 g (76%) of 160b.

step 8 - A dried 100 mL round bottom flask purged with nitrogen and charged with **160b** (2.0 g; 5.43 mmol) and dissolved in THF (20 mL) and stirred under a stream of nitrogen. To the reaction vessel was added LiOH (0.46 g; 10.86 mmol) followed by 5 mL, deionized water. The reaction was stirred for 1 h under a continuous stream of nitrogen. The homogeneous mixture was quenched at 0°C with 10% aqueous HCl. The reaction mixture was stirred for an additional 15 minutes. The crude mixture was extracted with EtOAc and washed with water and brine. The organic extracts were dried (Na₂SO₄) and filtered. The solvent was removed *in vacuo* and the crude acid 162a was used without any further purification.

step 9 - A 100 mL round bottom was charged with **162a** (0.200 g, 0.520 mmol) and 5 mL of DCM and the solution was stirred under nitrogen at RT. To the solution was added thionyl chloride (0.061 g, 0.520 mmol) dropwise followed by a single drop of DMF. The reaction was stirred for 1 h at RT. Excess solvent and thionyl chloride were removed *in vacuo* to afford the carboxylic acid **162b** as a crude yellow oil which was used in the next reaction without any further purification.

step 10 - A 100 mL round bottom was charged with 4-amino-3-methyl-benzenesulfonamide (0.260 g, 1.40 mmol) and dissolved in 5 mL of acetone and NaHCO₃ (0.117 g, 1.40 mmol) under nitrogen. To the stirred mixture was added dropwise 162b (0.500 g, 1.40 mmol) dissolved in 7 mL of acetone and resulting mixture stirred for 24 h at RT. When starting material was consumed the reaction mixture was cooled to 0°C and quenched with 10% aqueous HCl. The reaction mixture was extracted with EtOAc and washed with aqueous 10% HCl, water, and brine. The organic extracts were dried (Na₂SO₄) and filtered. The solvent was removed *in vacuo* to yield a crude solid which was purified by SiO₂ chromatography eluting with DCM/MeOH (93:7) to afford 0.64 g (90%) of **I-42**: ms (MW: 508), (M-H) = 507; mp. :250.1-252.3° C; Elemental Analysis: calcd; C 51.98, H 3.17, N 8.27; found: C 51.20, H 3.01, N 8.10 (with 0.4M H₂O).

Compound **I-98** was prepared in the same manner except in step 10, 4-amino-benzenesulfonamide was replaced by 2-chlorophenylamine.

Compound **I-100** was prepared in the same manner except in step 10, 4-amino-benzenesulfonamide was replaced by 4-amino-3-chloro-benzenesulfonamide.

Compound **I-108** was prepared in the same manner except in step 10, 4-amino-benzenesulfonamide was replaced by 4-amino-3,N-dimethyl-benzenesulfonamide.

Compound **1-239** was prepared in the same manner except in step 10, 4-amino-benzenesulfonamide was replaced by 4-amino-3-methyl-benzamide.

Compound **I-111** was prepared in the same manner except in step 10, 4-amino-benzenesulfonamide was replaced by 3-methyl-4-methylamino-benzenesulfonamide.

Compound **I-168** was prepared in the same manner except in step 10, 4-amino-benzenesulfonamide was replaced by 4-amino-3-chloro-N-methyl-benzenesulfonamide.

### Example 32

### N-[4-(3-Amino-propionylsulfamoyl)-2-chloro-phenyl]-2-[4-chloro-3-(3-chloro-5-cyano-phenoxy)-2-fluoro-phenyl]-acetamide; compound with hydrochloric acid (I-153)

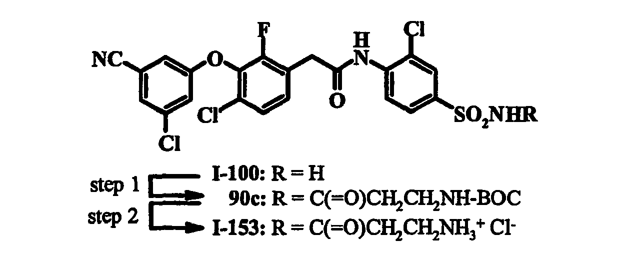

step 1 - To a solution of **I-100** (0.100 g, 0.189 mmol) and Boc-β-alanine (0.05 g, 0.264 mmol) and DCM (10 mL) was added EDCI hydrochloride (0.04 g, 0.21 mmol) and DMAP (0.01 g, 0.09 mmol). The reaction was stirred at RT for 2 h. The reaction mixture was cooled to 0°C and 25 mL of ice was added. The reaction mixture was allowed to warm to RT and extracted with DCM. The organic extracts were washed sequentially with 10% HCl, water and brine. The DCM solution was dried (Na₂SO₄), filtered and evaporated *in vacuo.* The solid was purified by recrystallization with EtOAc/acetone to afford **90c.**

step 2 - **90c** was dissolved in Et₂O (10 mL) and 1 N HCl (10 mL) was added and the reaction stirred until deprotection of the BOC protecting group was compete. The solvents were evaporated *in vacuo* and the resulting solid triturated in toluene to afford **I-153.**

Compound **I-77** was prepared in the same manner except 2-[4-chloro-3-(3-chloro-5-cyano-phenoxy)-2-fluoro-phenyl]-N-(2-methyl-4-sulfamoyl-phenyl)-acetamide **(I-42)** replaced **I-100** and BOC-NH-Val-OH replaced BOC-β-Ala.

Compound **I-127** was prepared in the same manner except BOC-NH-Val-OH replaced BOC-β-Ala.

Compound **I-181** was prepared in the same manner except 2-[4-chloro-3-(3-chloro-5-cyano-phenoxy)-2-fluoro-phenyl]-N-(2-chloro-4-methylsulfamoyl-phenyl)-acetamide **(1-168)** replaced **I-100.**

### Example 33

### 2-[4-Chloro-3-(3-chloro-5-cyano-phenoxy)-2-fluoro-phenyl]-N-(2-chloro-4-pentanoylsulfamoyl-phenyl)-acetamide; sodium salt (I-173)

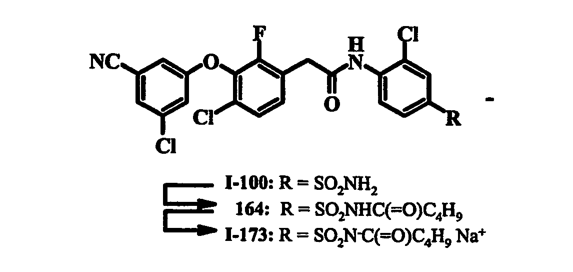

To a solution of the sulfonamide **I-100** (0.166 g, 0.314 mmol), THF (2 mL) and DCE (2 mL) was added valeric anhydride (0.064 g, 0.345 mmol) followed by a single crystal of DMAP. The solution was stirred for 24 h and partitioned between water and DCM. The organic phase was washed sequentially with 10% HCl, water and brine. The organic phase was dried (Na₂SO₄), filtered and the solvents removed *in vacuo.* The residue was triturated with Et₂O and filtered to afford 2-[4-chloro-3-(3-chloro-5-cyano-phenoxy)-2-fluoro-phenyl]-N-(2-chloro-4-pentanoylsulfamoyl-phenyl)-acetamide **(164).**

The acyl sulfonamide **164** was suspended in THF and stirred until the solution was homogenous. To the solution cooled to 0°C was added 1 equivalent of 1M NaOH. The reaction was stirred for 10 min then allowed to warm to RT and the solvents were removed in *vacuo.* The resulting material was triturated with Et₂O and EtOAc to afford **I-173** as a crystalline solid which was dried at 100°C for 24 h.

Compound **I-51** was prepared in the same manner except 2-[4-chloro-3-(3-chloro-5-cyano-phenoxy)-2-fluoro-phenyl]-N-(2-methyl-4-sulfamoyl-phenyl)-acetamide **(I-42)** replaced **I-100** and propionic anhydride replaced valeric anhydride.

Compound **I-87** was prepared in the same manner except 2-[4-chloro-3-(3-chloro-5-cyano-phenoxy)-2-fluoro-phenyl]-N-(2-methyl-4-sulfamoyl-phenyl)-acetamide **(I-42)** replaced **I-100** and nicotinoyl chloride hydrochloride replaced valeric anhydride. The compound was isolated as the hydrochloride salt rather then as a sodium salt

Compound **I-99** was prepared by alkylation of the sodium salt **of I-126** in DMF. Sodium salts of the acylsulfonamides are prepared by adding 1 equivalent of aqueous NaOH to a THF solution of **I-99** and evaporating the solvent.

Compound **I-126** was prepared in the same manner except valeric anhydride was replaced with propionic anhydride.

Compound **I-138** was prepared in the same manner except valeric anhydride was replaced with butyric anhydride.

Compound **I-139** was prepared by alkylation of the sodium salt **I-138** with methyl iodide in DMF.

Compound **I-155** was prepared in the same manner except **I-100** methoxyacetic acid replaced valeric anhydride. The EDIC coupling from the previous example was used.

Compound **I-156** was prepared in the same manner except acetoxyacetyl chloride replaced valeric anhydride and the acetoxy ester was subsequently cleaved by basic hydrolysis.

Compound **I-162** was prepared by alkylation of the sodium salt of **I-126** with methyl iodide in DMF.

Compound **I-171** was prepared by alkylation of the sodium salt of **I-155** with methyl iodide in DMF.

Compound **I-172** was prepared by alkylation of the sodium salt of **I-164** with methyl iodide in DMF.

Compound **I-174** was prepared in the same manner except isovaleric acid replaced valeric anhydride. The EDIC coupling from the previous procedure was used.

Compound **I-175** was prepared in the same manner except N,N-diethylglycine hydrochloride replaced valeric anhydride. The EDIC coupling from the previous procedure was used.

Compound **I-176** was prepared in the same manner except 4-methylvaleric acid replaced valeric anhydride. The EDIC coupling from the previous procedure was used.

Compound **I-177** was prepared in the same manner except morpholin-4-yl-acetic acid replaced valeric anhydride. The EDIC coupling from the previous procedure was used.

Compound **I-188** was prepared in the same manner except succinic acid replaced valeric anhydride.

### Example 34

### 2-[4-Chloro-3-(3,5-dicyano-phenoxy)-2-fluoro-phenyl]-N-(4-sulfamoyl-phenyl)-acetamide (I-9)

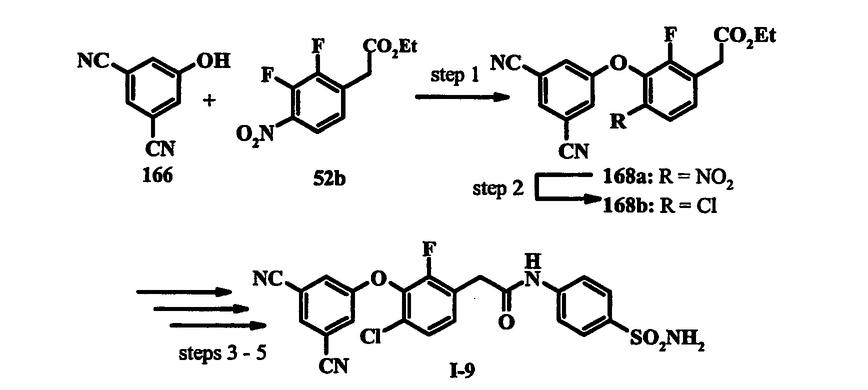

### step 1

A solution of anhydrous THF (100mL) and 5-hydroxyisophthalonitrile (**166**, 10.00 g, 69.38 mmol) cooled to 0°C was treated with sodium *tert*-butoxide (7.34 g, 76.32mmol). The mixture was stirred for 30 min at 0°C then **52b** (17.01, 69.38 mmol) was added and allowed to stir for an additional 3 h. The reaction was quenched with 10% aqueous HCl. The crude mixture was extracted with EtOAc and the combined extracts washed with water and brine. The organic phase was dried (Na₂SO₄) and filtered. The solvent was removed *in vacuo* to afford a crude oil which was purified by SiO₂ chromatography eluting with hexanes:EtOAc (90:10) to afford 20 g (78%) of **168a.**

Introduction of the chloro substituent (step 2) was carried out as described in steps 6 and 7 of Example 31. Steps 3-5 were carried out by the procedure described in steps 7-9 of Example 1 which afforded **I-9.**

Compound **I-28** was prepared in the same manner except in step 5 of the present example, 4-amino-3-methyl-benzenesulfonamide was replaced with 2-chlorophenylamine.

Compound **I-32** was prepared in the same manner except in step 5 of the present example, 4-amino-3-methyl-benzenesulfonamide was utilized.

### Example 35

### 2-[4-Chloro-3-(3-cyano-5-difluoromethyl-phenoxy)-2-fluoro-phenyl]-N-(4-sulfamoyl-phenyl)-acetamide (I-13)

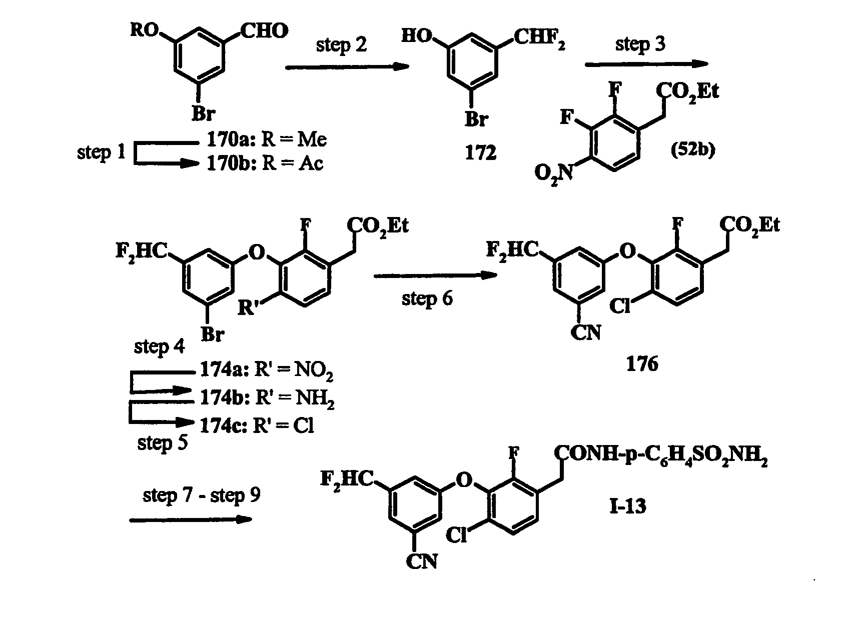

step 1 - A solution of BBr₃ (29.1 mL of a 1.0 M solution in DCM, 29.1 mmol) was added slowly to a solution of **170a** (2.5 g, 11.62 mmol) in anhydrous DCM (25 mL) maintained under N₂ at -78°C. The orange solution was warmed to RT, stirred for 2 h, and poured onto ice. The mixture was extracted with CH₂Cl₂ (100 mL), and the organic layer was washed with H₂O (50 mL) and brine (50 mL). The solvents were evaporated, and the remaining oil was purified by flash chromatography on silica gel eluting with a EtOAc/hexanes gradient (0% to 20% EtOAc) to provide the desired phenol. To a solution of this phenol in pyridine (10 mL) under argon was slowly added acetic anhydride (0.6 mL, 6.33 mmol). After 2 h, the volatile materials were removed to provide 3-bromo-5-formyl-phenyl acetate **(170b,**1.02 g, 40 %).

step 2 - DAST (1.02 mL, 7.69 mmol) was added to a solution of the 3-bromo-5-formyl-phenyl acetate (**170b**, 1.1 g, 4.52 mmol) in DCM (5 mL) under nitrogen contained in a NALGENE^{®} bottle. EtOH (0.013 mL, 0.23 mmol) was added, and the mixture was stirred for 16 h. The reaction mixture was then added slowly to an aqueous solution of saturated NaHCO₃. After the bubbling was finished, DCM (50 mL) was added and the layers were separated. The organic layer was washed with brine (30 mL) and dried with anhydrous MgSO₄. The solvent was removed to provide a yellow oil that was placed in a mixture of THF (15 mL) and H₂O (4 mL). LiOH monohydrate (474 mg, 11.3 mmol) was added, and the reaction mixture was stirred at RT for 2 h. The solution was then added dropwise to 5% aqueous HCl (50 mL), and the mixture was extracted with EtOAc (3 x 30 mL). The combined organic fractions were washed with brine (30 mL), and dried with anhydrous MgSO₄. Evaporation of the volatile materials gave an oil that was purified by flash chromatography on silica gel (0% to 25% EtOAc/hexanes) to provide 800 mg (79%) of 3-bromo-5-difluoromethylphenol **(172).**

The phenol **172** was condensed with ethyl 2,3-difluoro-4-nitro-phenyl acetate **(52b,** step 3) as described in step 1 of Example 34. Reduction of the nitro group and diazotization and displacement of the diazonium salt by chloride (steps 4 and 5) were carried out as described in steps 6 and 7 of Example 31 to afford **174c.**

Step 6 - A solution of **174c** (757 mg, 1.73 mmol), Pd[P(Ph)₃]₄(0) (300 mg, 0.26 mmol), and zinc cyanide (122 mg, 1.04 mmol) in DMF (8 mL) under nitrogen was heated to 80°C for 4 h. The reaction mixture was cooled to RT and added to 2 M aqueous NH₄OH. The solution was extracted with 1:1 EtOAc/hexanes (3 x 30 mL), and the combined organic fractions were washed with H₂O (3 x 20 mL) and dried (MgSO₄). The solvent was evaporated, and the remaining oil was purified by SiO₂ chromatography eluting with an EtOAc/hexanes gradient (0% to 25% EtOAc) to provide 580 mg (87%) of [4-chloro-3-(3-cyano-5-difluoromethyl-phenoxy)-2-fluoro-phenyl] -acetic acid ethyl ester **(176).**

Steps 7-9 were carried out by the procedure described in steps 7-9 of Example 1 which afforded **I-13.**

Compound **I-14** was prepared in the same manner except in step 9 of the present example, 2-chlorophenylamine was utilized.

Compound **I-75** was prepared in the same manner except in step 9 of the present example, 4-amino-3-methyl-benzenesulfonamide was utilized.

### Example 36

### 2-[4-Chloro-3-(3-cyano-5-difluoromethoxy-phenoxy)-2-fluoro-phenyl]-N-(4-sulfamoyl-phenyl)acetamide (I-166)

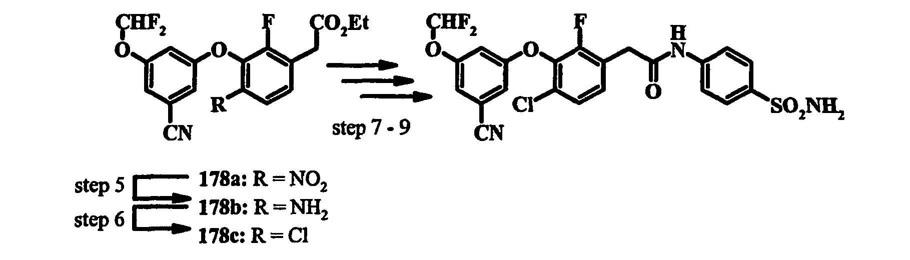

Steps 1-4 are depicted in SCHEME 10

step 1 - Acetic anhydride (30 mL, 4 equiv) was added to a solution of 3,5-dihydroxybenzonitrile (**84a**, 10.36 g, 77 mmol) in anhydrous pyridine (60 mL) cooled to 0° C and blanketed with nitrogen. The reaction was warmed to RT and stirred for 16 h. The volatile materials were removed *in vacuo,* and the remaining oil was dissolved in EtOAc, washed with water, 5% HCl solution, brine, and dried (MgSO₄). The volatile materials were removed to afford 14.5 g (86%) of the diacetate. The diacetate (14 g, 64 mmol) was dissolved in a mixture of EtOH (100 mL) and benzene (100 mL) and cooled to 0° C. A solution of KOH (3.6 g, 1 equiv) in EtOH was added dropwise. After 1 h, the solution was added to an ice-cold solution of saturated ammonium chloride, extracted with ether, and washed with brine. The Et₂O extracts were concentrated and purified by SiO₂ chromatography eluting with a hexane/EtOAc gradient (0% to 25% EtOAc) which afforded 10 of 84b (88%).

step 2 - (2-Trimethylsilyl-ethoxy)-methyl-chloride (2.2 mL, 1.1 equiv) was added to a solution of the **84b** (2.0 g, 11.3 mmol) and DIPEA (2.4 mL, 1.2 equiv) in DCM (50 mL) cooled to 0°C. The solution was warmed to RT, stirred for 16 h, and poured into a saturated sodium bicarbonate solution. The aqueous solution was extracted with DCM, and the combined organic extracts washed with water and brine and dried (MgSO₄). The solvents were removed *in vacuo* and the acetylated product was dissolved in a mixture of water (8 mL) and THF (32 mL). LiOH.H₂O (0.71 g, 1.5 equiv) was added. The mixture was stirred for 2 h, acidified to pH 5 and extracted with ether. The organic layer was dried (MgSO₄) and evaporated to provide 2.5 g (80%) of the **84c.**

step 3 - F₂ClCCO₂Na (2.84 g, 2.3 equiv) was added to a solution of Cs₂CO₃ (3.69 g, 1.4 equiv), **84b** (2.26 g, 8.09 mmol), DMF (32 mL) and water (2 mL). The solution was heated to 100° C for 2 h, cooled to RT, and poured into a solution of ammonium chloride. The solution was extracted with a mixture of EtOAc and hexanes, and the organic layer was washed with brine and dried (MgSO₄). The crude product was purified by SiO₂ chromatography eluting with a EtOAc/hexane gradient (0% to 10%) which afforded 1.83 g (70%) of **86a**. The difluoromethyl ether 86a was dissolved in MeOH (30 mL), and 5.6 mL of a 1.0 M solution of HCl was added. The solution was heated to 50°C for 5 h, and stirred at RT for 16 h. The volatile materials were evaporated, and the aqueous residue was partitioned between DCM and water. The aqueous layer was extracted with DCM, and the combined extracts were washed with water and brine. The volatile materials were removed *in vacuo* to afford 780 mg (73%) of **86b.**

step 4 - Potassium *t*-butoxide (4.29 mL of a 1 M solution, 1 equiv) was added to a solution of **86b** (795 mg, 4.3 mmol) in THF (10 mL) cooled to 0°C under nitrogen atmosphere. The solution was stirred for 10 min, and a solution of the 52b (1.05 g, 1 equiv) in THF (8 mL) was added dropwise. The solution was warmed to RT, stirred overnight, and poured into a saturated ammonium chloride solution. The mixture was extracted with EtOAc, washed with water and brine and dried (MgSO₄). The solvents were evaporated and the crude product purified by SiO₂ chromatography elution with an EtOAc/hexane gradient (0% to 25%) to afford 1.6 g (93%) of **178a.**

step 5 - A solution of **178a** (1.59 g, 3.98 mmol), iron filings (0.93 g, 4.2 equiv), NH₄Cl (0.89 g, 4.2 equiv) in EtOH (25 mL) and water (15 mL) was heated to reflux for 4 h. The solution was cooled, diluted with methylene chloride, and filtered through a pad of SiO₂. Evaporation of the volatile materials afforded 1.43 g (94%) of **178b.**

step 6 - A solution of the **178b** (1.43 g, 3.76 mmol) in dry MeCN(15 mL) was added slowly to a rapidly stirred solution of CuCl₂ (1.01 g, 2 equiv) and *tert*-butyl nitrite (0.89 mL of a 90% solution, 1.8 equiv) that had been heated to 60 C under a N₂ atmosphere. After 1h, the reaction was cooled to RT, and 5 mL of 5% HCl was added. The solution was partitioned between EtOAc and brine. The organic extracts were dried (MgSO₄), filtered and concentrated *in vacuo.* The crude product was purified by SiO₂ chromatography and eluted with an EtOAc/hexane gradient (0% to 35%) to afford 1.04 g (69%) of **178c.**

Steps 7 to 9 were carried out as described in steps 7-9 of Example 1 to afford **I-166.**

Compound **I-167** was prepared in the same manner except in step 9 of the present example, 4-amino-3-choro-benzenesulfonamide replaced 4-amino-benzenesulfonamide.

### Example 37

### 2-{4-Chloro-3- [3-cyano-5-(1,1-difluoro-ethyl)-phenoxy] -2-fluoro-phenyl}-N-(4-sulfamoyl-phenyl)-acetamide (I-95)

Steps 1-7 are depicted in reaction C of SCHEME 8

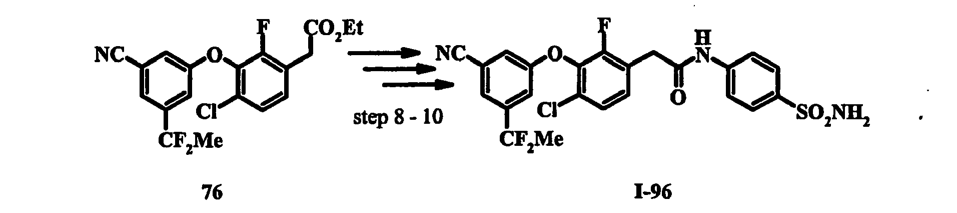

step 1 - *n*-BuLi (13.4 mL of a 1.6 M solution, 1.1 equiv) was added slowly to a solution of the tert-butyl-(3,5-dibromo-phenoxy)-dimethyl-silane (70, 7.16 g, 19.5 mmol) in Et₂O (60 mL) cooled to -78° C under a blanket of N₂. The solution was stirred for 25 min, and N-methoxy-N-methyl-acetamide was added *via* syringe. The solution was warmed slowly to RT, added to saturated ammonium chloride, and extracted with ether. The combined organic layers were washed with brine and dried (MgSO₄), filtered and concentrated *in vacuo.* The crude product material was dissolved in THF (50 mL), and a solution of Bu₄NF (approximately 1.2 equiv) was added. The solution was stirred for 2 h and partitioned between EtOAc and brine. The organic layer was washed with water, brine, and dried (MgSO₄) to afford 3.35 g (80%) of **72a**.

step 2 - Acetic anhydride (1.1 mL, 1.2 equiv) was added to a solution of the 72a (2.0 g, 9.3 mmol) in pyridine (30 mL) cooled to 0° C. The solution was warmed to RT, stirred for 2h, and the volatile materials were removed *in vacuo.* The residue was dissolved in ether, and washed with 5% HCl solution, water, brine, and dried (MgSO₄). The solvents were evaporated to afford 2.3 g (98%) of **72b.**

step 3 - A suspension of **72b** (2.35 g, 9.1 mmol) and Deoxy-Fluor^{®} (2.9 mL, 1.7 equiv) (Caution: EXPLOSIVE REAGENT) under N₂ atmosphere in a Teflon bottle was heated to 85°C. The solution was stirred for 20 h at 85°C then for 24 h at RT. The resulting solution was added slowly to a cooled solution of saturated NaHCO₃. The aqueous solution was extracted with DCM, and the organic layer was washed with brine. Evaporation of the volatile materials afforded crude 72c which was dissolved in THF (22 mL) and water (6 mL). LiOH.H₂O (1.12 g, ca. 3 equiv) was added, and the solution was stirred for 2 h. The mixture was then poured into a 5% HCl aqueous solution, extracted with ether, and the organic layer was washed with brine. Evaporation of the volatile solvents afforded the crude residue which was purified by SiO₂ chromatography eluting with an EtOAc/hexane gradient (3% to 10%) which afforded 0.95 g (44%) of **72c.**

Steps 4 to 6 were carried out by the procedure described in steps 4 and 6 of example 36 to afford **74c.**

step 7 - A solution of the **74c** (0.78 g, 1.73 mmol), Zn(CN)₂ (0.12 g, 0.6 equiv), and (Ph₃P)₄P (0.30 g, 0.15 equiv) in DMF (7 mL) was heated to 80° C for 4 h. The solution was cooled, added to 2 M ammonium hydroxide solution and extracted with ether. The organic extract was washed with brine and dried (MgSO₄) and concentrated *in vacuo.* The crude product was purified by SiO₂ chromatography eluting with a EtOAc/hexane gradient (0% to 25%) to afford 0.55 g (80%) of 76.

Steps 8 to 10 were carried out as described in steps 7-9 of the example 1 to afford I-95.

Compound **I-96** was prepared in the same manner except in step 10 of the present example, 4-amino-3-methyl-benzenesulfonamide replaced 4-amino-benzenesulfonamide.

Compound **I-97** was prepared in the same manner except in step 10 of the present example, 4-amino-3-chloro-benzenesulfonamide replaced 4-amino-benzenesulfonamide.

### Example 38

### 2-[3-(3-Bromo-5-cyano-phenoxy)-4-chloro-2-fluoro-phenyl]-N-(2-methyl-4-sulfamoyl-phenyl)-acetamide (I-185)

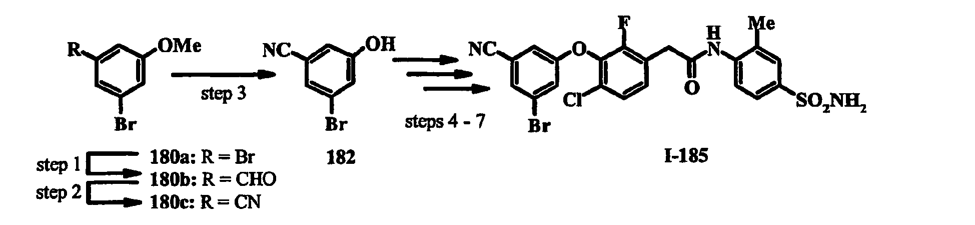

step 1 - *n*-BuLi (2.6 mL of a 1.6 M solution, 1.1 equiv) was added slowly to a solution of the 180a (1.0 g, 3.8 mmol) in Et₂O (20 mL) cooled to -78° C under an N₂ atmosphere. The solution was stirred for 45 min, and DMF was added *via* syringe. The solution was warmed slowly to RT, added to saturated ammonium chloride, and extracted with ether. The organic phase was washed with brine and dried (MgSO₄), filtered and evaporated to afford 0.80 g (98%) of **180b.**

step 2 - A solution of the aldehyde **180b** (12.0 g, 56 mmol), hydroxylamine hydrochloride (19.4 g, 5 equiv), EtOH (100 mL) and pyridine (10 mL) was heated to 65° C for 16 h. The mixture was cooled to RT, and partitioned between 50% EtOAc/hexanes and water. The organic layer was washed with brine and dried (MgSO₄). The volatile materials were evaporated to afford 12.4 g (97%) of the oxime. This material was dissolved in anhydrous dioxane (100 mL) and pyridine (26 mL, 6 equiv). The solution was cooled to 0° C, TFAA (15 mL, 2 equiv) was added, and the mixture was allowed to warm to RT. The solution was stirred for 2 d, and warmed to 60 C for 1 h. The mixture was cooled to RT, and added carefully to ice water. The mixture was extracted with methylene chloride, and the combined organic layers were washed with water, 1 M HCl, and brine. The organic layer was dried (MgSO₄) and evaporated to afford 10.4 g (90%) of **180c**

step 3 - Anhydrous collidine (100 mL) was added to a dry flask containing 180c (10.4 g, 49 mmol) and LiI (19.6 g, 3 equiv). The solution was heated under nitrogen to 150°C overnight, cooled to RT, and poured into an ice cold 1 M HCl solution. The mixture was extracted with a 1:1 EtOAc/hexanes solution, washed with water, and dried (MgSO₄). Concentration in *vacuo* afforded 8.7 g (89%) of **182.**

Step 4 - The condensation of 182 and **52b** was carried out as described in step 4 of example 36. Steps 5 to 7 were carried out as described in steps 7 to 9 of example 1 except in the present case 4-amino-3-methyl-benzensulfonamide replaced 4amino-benzenesulfonamide.

### Example 39

### 2-[4-Bromo-3-(3-chloro-5-cyano-phenoxy)-2-fluoro-phenyl]-N-(2-chloro-4-sulfamoyl-phenyl)-acetamide (I-189)

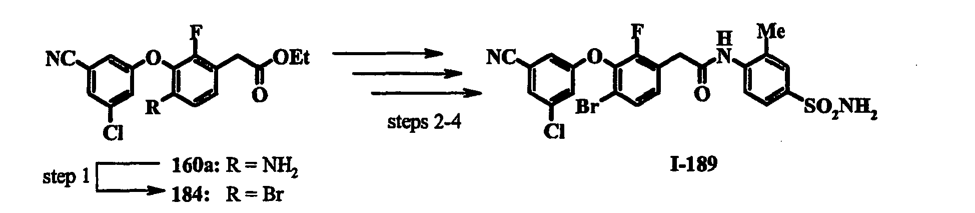

The phenyl acetic acid **160a** was prepared as described in steps 1-6 of example 31

step 1 - A 150 mL three-neck round bottom flask was charged with MeCN (50 mL), CuBr (2.8 g, 12.61 mmol) and *t*-butyl nitrite (1.4 g, 13.76 mmol), degassed and maintained under an Ar atmosphere and heated to 70° C. To the mixture was added dropwise a solution of **160a** (4.0 g, 11.47 mmol) dissolved MeCN (20 mL). The reaction mixture was stirred at 70°C for 4 h and then cooled to 0°C. The reaction was quenched by addition of 10 % HCl (30 mL) and extracted with EtOAc. The combined extracts were sequentially washed with 10% HCl and brine. The organic extract was dried (Na₂SO₄), filtered and the volatile solvents removed in *vacuo* to yield a black oil which was purified by flash chromatography on silica gel (hexanes:EtOAc 95:5) to afford 2.5 g (52.8% theory) of **184.**

Steps 2 and 4 were carried out as described steps 7 and 8 of Example 1.

step 5 - A 100mL round bottom flask was charged with 4-amino-3-methyl-benzenesulfonamide (0.092 g, 0.495 mmol), dissolved in 2 mL of acetone and NaHCO₃ (0.040 g, 0.495 mmol) was added. To the stirred suspension under a nitrogen atmosphere was added dropwise a solution of the acid chloride from step 4 (0.200 g, 0.495 mmol) dissolved in 3mL acetone and the reaction was stirred for 24 h at RT. The reaction mixture was cooled to 0°C and quenched with 10% aqueous HCl. The aqueous phase was extracted with EtOAc and the combined extracts were washed with aqueous 10% HCl, water, and brine. The organic phase was dried over (Na₂SO₄), filtered and the solvent was removed *in vacuo.* The crude product was purified by SiO₂ chromatography eluting with DCM/MeOH (93:7) to afford 0.240 g (87%) of **I-189**: ms (M-H) = 551, mp 244.0-245.1, CHN; calcd C 47.80, H 2.92, N 7.60; found C 47.51, H 2.80, N 7.49.

### Example 40

### 2-[4-Bromo-3-(3-cyano-5-difluoromethyl-phenoxy)-2-fluoro-phenyl]-N-(2-chloro-4-sulfamoyl-phenyl)-acetamide (I-169)

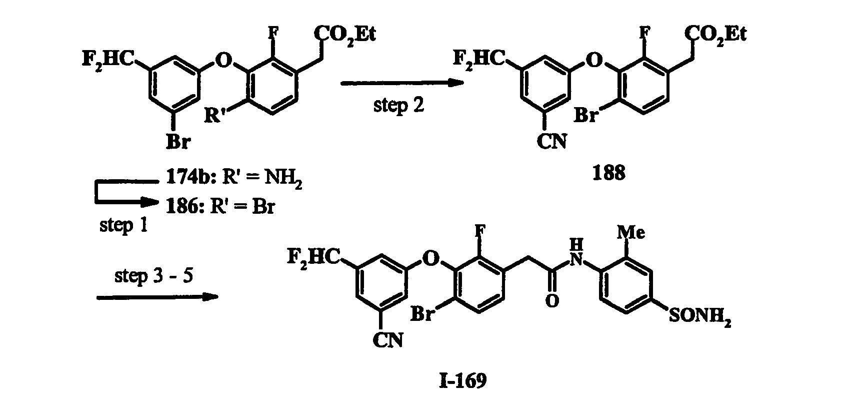

Step 1 was carried out by the procedure described in step 1 of example 39. Step 2 was carried out by the procedure described in step 7 of example 37. Steps 3 to 5 were carried out by the procedures described in steps 7 to 9 of Example 1 except in step 9, 4-amino-benzenesulfonamide was replaced by 4-amino-3-methyl-bezenesulfonamide which afforded in **I-169.**

### Example 41

### 2-[3-(3-Cyano-5-difluoromethyl-phenoxy)-2-fluoro-4-methyl-phenyl]-N-(2-methyl-4-sulfamoyl-phenyl)-acetamide (I-85) and 2-[3-(3-Cyano-5-difluoromethyl-phenoxy)-4-ethyl-2-fluoro-phenyl]-N-(2-methyl-4-sulfamoyl-phenyl)-acetamide (I-180)

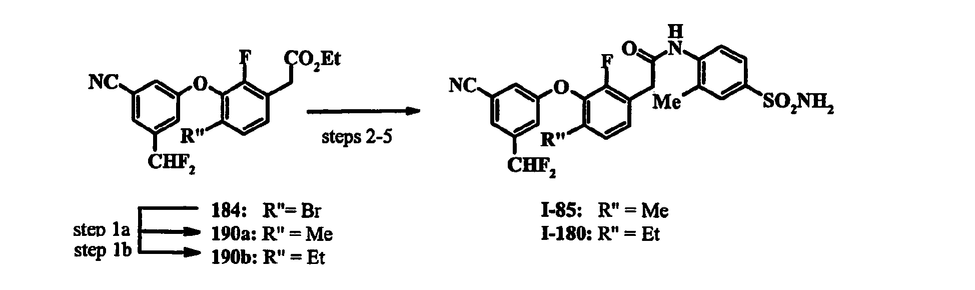

step 1a - To a degassed ice-cold solution of THF (15mL), Pd(dppf)Cl₂ (0.09 g, 0.121 mmol) was added DIBAL-H (0.012 mmol; 1M in toluene). The reaction mixture was allowed to warm to RT. A solution of **184** (1.0 g, 2.42 mmol) was added followed by Me₂Zn (1M in THF, 4.240 mmol). The reaction was heated to 65°C for 4 h, cooled to RT and quenched with aqueous NH₄Cl. The resulting mixture was extracted with EtOAc and washed sequentially with NH₄Cl and brine. The EtOAc extract was dried (Na₂SO₄), filtered and the volatile solvent removed *in vacuo* to yield a dark brown oil that was purified by flash chromatography on silica gel eluting with hexanes:EtOAc (95:5) to yield **190a** (0.50 g, 59% theory).

step 1b - **190b** was prepared by a procedure identical to that described in step 1a except Me₂Zn was replaced with Et₂Zn. The product was purified by flash chromatography on silica gel eluting with hexanes:EtOAc (95:5) to afford 0.62 g (74%) of **190b.**

**I-85** and **I-180** were prepared from **190a** and **190b** respectively following the procedure described in steps 7-9 of example 1 except in step 5 of the present example 4-amino-benzenesulfonamide was replaced by 4-amino-3-methyl-bezenesulfonamide.

Compound **I-84** was prepared in the same manner from **I-190a** except in step 5 of the present, example 4-amino-benzenesulfonamide was replaced by 4-amino-3-chloro-bezenesulfonamide.

The crude product recrystallized from EtOAc/hexane to afford 0.182 g (28%) **of I-84**: MS: (M-H) = 522, mp. 218.0-218.7, Elemental Analysis Found: C: 52.54; 3.04; 7.99

Compound **I-86** was prepared in the same manner from **I-190a** except in step 5 of the present example, 4-amino-benzenesulfonamide was replaced by 2-chloro-phenylamine.

Compound **I-110** was prepared in the same manner from **I-190a** except in step 5 of the present, example 4-amino-benzenesulfonamide was replaced by methyl 4-amino-3-chloro-benzoate and in a subsequent step the benzoic acid methyl ester was converted to the corresponding benzoic acid present in **I-110.**

Compound **I-112** was prepared in the same manner except in step 8 of the present example, 4-amino-3-chloro-benzenesulfonamide was replaced with 2-chloro-4-methanesulfonyl-phenylamine.

### Example 42

### 2-[3-(3-Chloro-5-cyano-phenoxy)-2-fluoro-4-methyl-phenyl]-N-(2-chloro-4-sulfamoyl-phenyl)-acetamide (I-83)

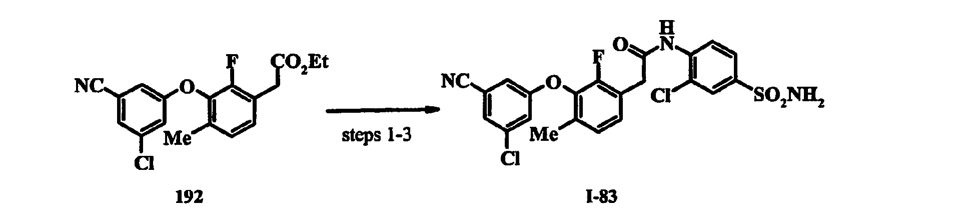

[3-(3-Chloro-5-cyano-phenoxy)-2-fluoro-4-methyl-phenyl]-acetic acid ethyl ester (192) was prepared from **160** (example 31) by reduction of the nitro group followed by the diazotization/bromination and Negishi methylation of **160** as described in step 1a of example 41. Steps 1-3 of the present example were carried out as described in steps 7 to 9 of example 1 except in the present example 4-amino-benzenesulfonamide was replaced by 4-amino-3-chloro-benzenesulfonamide to afford **I-83.**

### Example 43

2-[3-(3-Chloro-5-cyano-phenoxy)-2-fluoro-4-methoxy-phenyl]-N-(2-chloro-4- 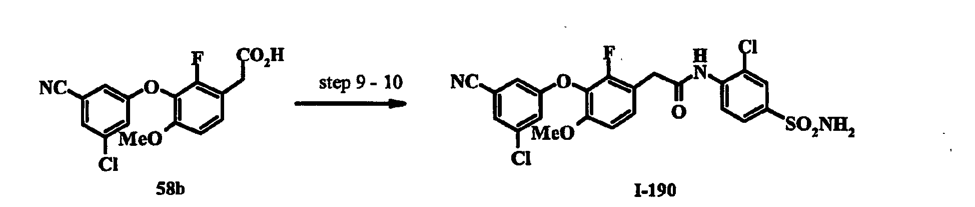 sulfamoyl-phenyl)-acetamide **(I-190)**

See SCHEME 7 for steps 1 to 7.

step 1 - To a solution of di-*iso*-propylamine (150 mL,108.3 g,1.07 mol) in THF (500 mL) cooled to -78°C and maintained under a N₂ atmosphere was added over a 15 min period, *n*-BuLi (100 mL, 1.00 mol, 10M in hexanes). The resulting mixture was stirred for 30 min at -78° C. A mixture **of 54a** (45 mL, 52.110 g, 0.457 mol) and chlorotrimethylsilane (130.0 mL, 111.28 g, 1.024 mol) was added at a rate which maintained the internal reaction temperature below -50°C. The solution was stirred at - 78° C for 1 h. The reaction was quenched at -78°C by addition of 1M H₂SO₄, diluted with MTBE and the mixture was saturated with solid NaCl. The phases were separated and the aqueous phase was extracted with MTBE (300 mL). The combined organic extracts were dried (MgSO₄), filtered and the solvents evaporated to afford 118 g (100%) of 54b as a white solid.

step 2 - To neat bromine (76.9 mL, 1.50 mol) cooled to 0°C in an ice bath was added portion wise solid **54b** (126.23 g, 0.500 mol) while maintaining the internal temperature between 20-45°C (caution: exothermic !). The reaction mixture was stirred at 58° C for 2 h. After 1 h of this period had elapsed additional bromine (45.48 g) was added and the addition funnel was rinse with cyclohexane (10 mL). The reaction mixture was cooled to 0° C and slowly poured into ice-cold saturated NaHSO₃ solution. After the addition the resulting mixture was saturated with solid NaCl, extracted with MTBE (500 mL and 200 mL), dried (MgSO₄) and concentrated *in vacuo* to afford 191 g of **54c**. The reaction mixture was distilled at ca. 60 mbar which afforded 161.53 g of colorless liquid which boiled at 110° C and contained about 11% of the monobromo derivative. The product was redistilled through a bubble ball column at *ca.* 50 mbar which afforded 141.3 (78.5%) of 54c with a boiling point of 93-94° C which was >99.6 pure.

step 3 - Preparation of *iso*-PrMgCl.LiCl - A sample of LiCl (4.56 g, 107.6 mmol) was dried under high vacuum with a heat gun for 10 min. To the dry solid under a N₂ atmosphere at 23° C was added *iso*-PrMgCl (53.8 mL, 107.6 mmol, 2M solution in THF) and the resulting mixture was stirred at 23°C for 3 days.

To a solution of **54c** (1.29 mL, 10 mmol) in THF (5 mL) at -40°C was added the *iso*-PrMgCl.LiCl solution (5.5 mL, 11 mmol, 2.0M in THF) at a rate that maintained the reaction temperature below -30°C. Stirring was continued at -35 to -30°C for 1 h then warmed to -7°C for an additional 1 h. The reaction mixture was cooled to -30°C and DMF (1.00 mL, 13 mmol) was added in one portion (temperature rose to -23°C) and stirring continued for 3.5 h at -25 to +15°C. The reaction mixture was poured into 1M H₂SO₄ and ice and the resulting mixture was saturated with solid NaCl and twice extracted with MTBE. The combined extracts were dried (MgSO₄), filtered and concentrated *in vacuo* to afford 2.17 g (98%) of 54d as a white solid.

step 4 - To a solution of 3-chloro-5-hydroxy-benzonitrile (3.84 g), K₂CO₃ powder (4.2 g) and n-butyl nitrile was added **54d** (5.57 g). The reaction mixture was heated to reflux for 4.5 h when the reaction appeared complete by gc/ms. The reaction mixture was cooled and poured into water, then EtOAc was added. The resulting mixture was allowed to stand until the layers separated. Some crystals were present at the interface and along the walls of the upper layer which were filtered and washed with water and hexanes. The filtrate was evaporated *in vacuo,* the residue taken up in IPA and re-evaporated. The solid was triturated with hexane and filtered. The mother liquor was evaporated and the residue purified by SiO₂ chromatography eluting with hexane/EtOAc (80:20). The product was triturated with IPA, filtered and washed with hexanes and the product fractions combined to afford 1.45 g (83%) of **56a.**

step 5 - Trifluoroacetic anhydride (8.88,4.231 mmol) was added to a 100 mL round bottom and stirred at 0°C. 30% Hydrogen peroxide (0.290, 8.46 mmol) was then added dropwise to the reaction vessel and stirred for 2 hours at zero to produce trifluoroperacetic acid (TFPA).

To a solution of **56a** (2.0, 5.64 mmol) in DCM (20 mL) stirred at 0° C was added KH₂PO₄ (15.35 g, 112.82 mmol). To this suspension was added dropwise at 0° C the TFPA. The reaction was stirred for 48 h. Upon consumption of starting material reaction mixture was cooled to 0° C, and diluted with brine, and quenched with aqueous 10% sodium bisulfite. The resulting mixture was extracted with DCM and washed with brine, dried (Na₂SO₄), filtered and the solvent removed *in vacuo* to yield a yellow solid which was purified by SiO₂ chromatography eluting with hexane/EtOAc (92:8) to afford 1.8 g (94%) of **56b.**

step 6 - To a solution of **56b** (1.8 g, 5.26 mmol) in DMF (15 mL) was added Cs₂CO₃ (3.43,10.52 mmol) and iodomethane ( 0.74 g, 5.26 mmol). The reaction mixture was stirred at 85°C for 12 h. When 56b was consumed, the reaction mixture was cooled to RT and the cured mixture extracted with EtOAc and the combined extracts washed with water and brine. The EtOAc was dried (Na₂SO₄), filtered and concentrated *in vacuo* to afford 56c as a yellow oil which was used in the next step without additional purification.

step 7 - A dry 100 mL round bottom was purged with nitrogen and charged with 56c (1.6 g, 4.50 mmol) and anhydrous THF (20 mL). The mixture was cooled to -20°C and a solution of *iso*-PrMgCl.LiCl (5.40 ml, 5.40mol, 2M in THF, see step 3) was added dropwise. The reaction was stirred for 2 h at -20°C and a solution of CuCN LiCl (0.100 mL, 0.100 mol 1M in THF) was added and stirred continued at -20 C. To this mixture was added allyl bromide (1.08 g, 9.0 mmol) and the mixture stirred for an additional two h. The reaction was quenched by addition of aqueous NH₄Cl. The mixture extracted with EtOAc and washed with water and brine. The extracts were dried (Na₂SO₄), filtered and the solvent was removed *in vacuo* to yield a yellow oil. The crude product was purified by SiO₂ chromatography eluting with hexane/EtOAc (95:5) to afford 1 g (70%) of **58a.**

step 8 - To a solution of **58a** (0.100 g, 0.315 mmol), EtOAc (2mL), MeCN (2mL) and water (3mL) was added NaIO₄ (0.437 g, 2.050 mmol) and RuCl₃ (0.001 g, 0.006 mmol). When **58a** was consumed, the crude mixture was filtered through a pad of CELITE^{®}, washed with EtOAc and the combined EtOAc washes were washed with brine, dried (Na₂SO₄) filtered and evaporated *in vacuo* to afford 0.090g (85%) of **58b** as a yellow solid. extracted with ethyl acetate, and washed with brine. The ethyl acetate was dried over sodium sulfate and filtered. Solvent was removed *in vacuo* to yield **58b** as a yellow solid (0.090 g, 85%).

Step 9 and 10 were carried as described in steps 8 and 9 of Example 1 except in step 9, 4-amino-3-methyl-benzenesulfonamide was replaced by 4-amino-3-chloro-benzenesulfonamide to afford **I-190.**

Compound 1-191 was made by a similar procedure except in step 10 4-amino-3-chloro-benzenesulfonamide was replaced by 4-amino-3-chloro-benzenesulfonamide

### Example 44

### 2-[3-(2-Chloro-5-cyano-phenoxy)-5-methoxy-phenyl]-N-(4-sulfamoyl-phenyl)-acetamide (I-102)

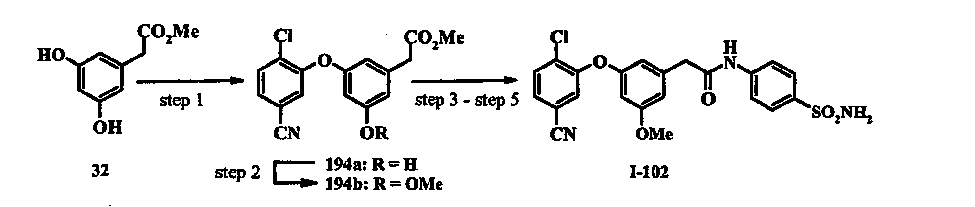

step 1 - A solution of 4-chloro-3-fluoro-benzonitrile (5.977 g, 38.42 mmol), methyl 3,5-dihydroxy-phenylacetate (7.0 g, 38.42 mmol), K₂CO₃ (15.9 g, 0.115 mol) and NMP (70 mL) was stirred and heated to 120° C for 12 h. The reaction mixture was cooled to RT and diluted with H₂O (150 mL), acidified with 10% aqueous HCl and twice extracted with EtOAc. The combined organic extracts were washed sequentially with water (6 times) and brine, dried (Na₂SO₄), filtered and concentrated *in vacuo.* The crude product was purified by SiO₂ column chromatography eluting with a EtOAc/hexane gradient (0 to 25% EtOAc) to afford **194a.**

step 2 - A solution of **194a** (0.500 g,1.57 mmol), methyl iodide (196 □L, 0.447 g, 3.15 mmol), K₂CO₃ (0.594 g, 3.93 mmol) and NMP (5 mL) was stirred at 85° C for 1 h. The reaction mixture was cooled to RT, diluted with H₂O (25 mL) and twice extracted with EtOAc. The combined organic extracts were washed sequentially with water (6 times) and brine, dried (Na₂SO₄), filtered and concentrated *in vacuo.* The crude product was purified by SiO₂ column chromatography eluting with EtOAc/hexane to afford **194b**.

Steps 3 to 5 were carried out by the procedure described in steps 7-9 of Example 1 which afforded of **I-102**.

Compound **I-103** was prepared using a similar procedure except in step 5 of the present example, 4-amino-benzenesulfonamide was replaced with 4-amino-2-methyl-benzenesulfonamide.

Compound **I-113** was prepared using a similar procedure except in step 5 of the present example, 4-amino-benzenesulfonamide was replaced with 4-amino-2-chloro-benzenesulfonamide.

### Example 45

### 2-[3-(3-Chloro-5-cyano-phenoxy)-5-methoxy-phenyl]-N-(4-sulfamoyl-phenyl)-acetamide (I-132)

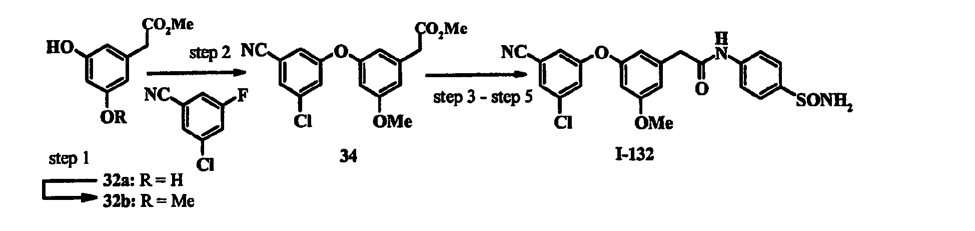

step 1- A solution of methyl iodide (10.25 mL, 23.374 g, 0.165 mol), methyl 3,5-dihydroxy-phenylacetate (**32a**, 30.0 g, 0.165 mol), K₂CO₃ (34.15 g, 0.247 mol) and NMP (300 mL) was stirred and heated to 85° C for 2 h. An additional 5 mL of methyl iodide and 10 g of K₂CO₃ was added and stirred for an additional 2 h. The reaction mixture was cooled to RT and diluted with H₂O (150 mL), acidified with 10% aqueous HCl and twice extracted with EtOAc. The combined organic extracts were washed sequentially with water (6 times) and brine, dried (Na₂SO₄), filtered and concentrated *in vacuo.* The crude product was purified by SiO₂ column chromatography eluting with a EtOAc/hexane gradient (0 to 25% EtOAc) to afford **32b**.

step 2 - A solution of methyl 3-hydroxy-5-methoxy-phenyl acetate (**32b**, 0.500 g, 2.55 mmol), 3-chloro-5-fluorobenzonitrile (0.3964 g, 2.55 mmol), K₂CO₃ (1.057 g, 7.65 mmol) and NMP (5 mL) was stirred and heated at 120° C for 12 h. The reaction mixture was cooled to RT and diluted with H₂O (35 mL), adjusted to pH 11 with 1N NaOH and twice extracted with EtOAc. The combined organic extracts were washed sequentially with water (6 times) and brine, dried (Na₂SO₄), filtered and concentrated *in vacuo.* The crude product was purified by SiO₂ column chromatography eluting with a EtOAc/hexane gradient (0 to 25% EtOAc) to afford **34**.

Steps 3 to 5 were carried out by the procedure described in steps 7-9 of Example 1 which afforded **I-132**.

Compound **I-133** was prepared using a similar procedure except in step 5 of the present example, 4-amino-benzenesulfonamide was replaced with 4-amino-2-methyl-benzenesulfonamide.

Compound **I-136** was prepared using a similar procedure except in step 5 of the present example, 4-amino-benzenesulfonamide was replaced with 4-amino-2-chloro-benzenesulfonamide.

### Example 46

### 2-[3-(3-Chloro-5-cyano-phenoxy)-5-methyl-phenyl] -N-(4-sulfamoyl-phenyl)-acetamide (I-141)

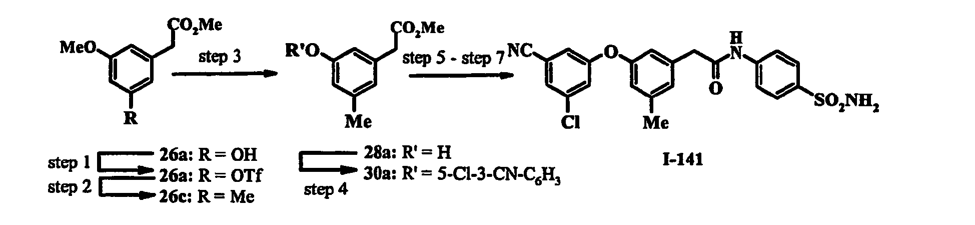

step 1- A solution of **26a** (4.8 g, 024.46 mmol) was dissolved in DCM (50 mL) and pyridine (3.957 mL, 3,896 g, 48.92 mmol) was added. The reaction mixture was cooled to 0° C and triflic anhydride (4.83 g, 7.593 g, 26.9 mmol) was added dropwise and the reaction mixture stirred for 1 h. The cold reaction mixture was transferred to a separatory funnel and washed quickly with ice-cold 1 N HCl, cold saturated bicarbonate, water and brine. The resulting DCM solution was dried (Na₂SO₄), filtered and the volatile solvents removed *in vacuo.* The resulting crude triflate **26b** was used directly in step 2.

step 2 - A three-neck round bottom flask was flame-dried and flushed with Ar. The flask was charged with Pd(dppf)Cl₂ (0.649 g, 7.95 mmol) and anhydrous THF (30 mL). The reaction mixture was cooled to 0° C and DIBAL-H was added dropwise (1.1 mL, 0.2261 g, 1.59 mmol). The reaction mixture was warmed to RT and stirred for 1 h. A solution of **26a** (5.22 g, 0.0159 mmol) and THF (30 mL) was added and the resulting solution was stirred at RT for 3h. Me₂Zn (7.95 mL, 0.159 mmol, 2.0 M solution in toluene) was added dropwise and the reaction mixture was stirred at 50° C for 1 h. The reaction was carefully quenched with a small quantity of water, filtered through a pad of CELITE^{®} and the solids washed thoroughly with EtOAc. The combined organic extracts were washed with water and brine, dried (MgSO₄) and the solvent evaporated. The crude product was purified by SiO₂ chromatography eluting with a EtOAc/hexane gradient (0 to 25% EtOAc) to afford **26c.**

step 3 - The ester **26c** (2.64 g, 0.136 mmol) was dissolved in DCM (20 mL) and the reaction mixture was cooled to -78° C. A solution of BBr₃ (67.96 mL, 67.96 mmol, 1.0 M in DCM) was added dropwise. After the addition was completed the reaction mixture was allowed to warm to RT and stirred for 2 h. The reaction mixture was recooled to -78 °C and the reaction quenched aqueous NaHCO₃ then warmed to RT and the organic phase washed with water, saturated NaHCO₃ and brine. The organic phase was dried (MgSO₄) and the solvent evaporated to afford **28a.**

step 4 - A solution of **28a** (0.500g, 2.77 mmol), 3-chloro-5-fluoro-benzonitrile (0.4316 g, 2.77 mmol), K₂CO₃ (1.150 g, 8.32 mmol) and NMP (5 mL) was stirred and heated for 8 h at 120° C. The reaction mixture was cooled to RT and diluted with H₂O (150 mL), acidified with 10% aqueous HCl and twice extracted with EtOAc. The combined organic extracts were washed sequentially with water (6 times) and brine, dried (Na₂SO₄), filtered and concentrated *in vacuo.* The crude product was purified by SiO₂ column chromatography eluting with EtOAc/hexane gradient (0 to 25% EtOAc) to afford 30a.

Steps 5 to 7 were carried out by the procedure described in steps 7-9 of Example 1 which afforded **I-141.**

Compound **I-142** was prepared using a similar procedure except in step 7 of the present example, 4-amino-benzenesulfonamide was replaced with 4-amino-2-methyl-benzenesulfonamide.

Compound **I-143** was prepared using a similar procedure except in step 7 of the present example, 4-amino-benzenesulfonamide was replaced with 4-amino-2-chloro-benzenesulfonamide.

Compound **I-146** was prepared using a similar procedure except in step 7 of the present example, 4-amino-benzenesulfonamide was replaced with 4-chloro-3-fluorobenzonitrile.

Compound **I-147** was prepared using a similar procedure except in step 4 of the present example, 3-chloro-5-fluoro-benzonitrile was replaced with 4-chloro-3-fluorobenzonitrile and in step 7, 4-amino-benzenesulfonamide was replaced with 4-amino-2-methyl-benzenesulfonamide.

Compound **I-148** was prepared using a similar procedure except in step 4 of the present example, 3-chloro-5-fluoro-benzonitrile was replaced with 4-chloro-3-fluorobenzonitrile and in step 7, 4-amino-benzenesulfonamide was replaced with 4-amino-2-chloro-benzenesulfonamide.

Compound **I-105** was prepared using a similar procedure except in step 2 of the present example, dimethylzinc was replaced with diethylzinc to afford **26** (R = Et) and in step 4, 3-chloro-5-fluoro-benzonitrile was replaced with 4-chloro-3-fluorobenzonitrile.

Compound **I-106** was prepared using a similar procedure except in step 2 of the present example, dimethylzinc was replaced with diethylzinc to afford **26** (R = Et), in step 4, 3-chloro-5-fluoro-benzonitrile was replaced with 4-chloro-3-fluorobenzonitrile and in step 7, 4-amino-benzenesulfonamide was replaced with 4-amino-2-methyl-benzenesulfonamide.

Compound **I-107** was prepared using a similar procedure except in step 2 of the present example, dimethylzinc was replaced with diethylzinc to afford **26** (R = Et), in step 4, 3-chloro-5-fluoro-benzonitrile was replaced with 4-chloro-3-fluorobenzonitrile and in step 7, 4-amino-benzenesulfonamide was replaced with 4-amino-2-chloro-benzenesulfonamide.

### Example 47

### 2-[7-(3-Cyano-phenoxy)-benzofuran-5-yl]-N-(2-methyl-4-sulfamoyl-phenyl)-acetamide (I-145)

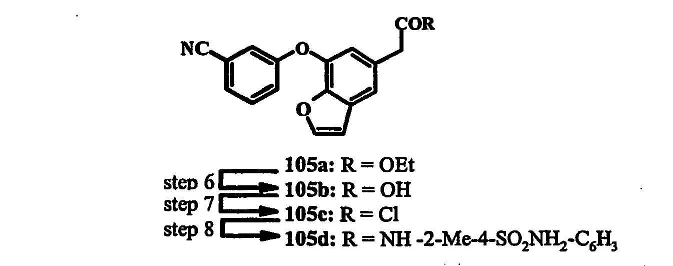

step 1 - To a solution of **101a** (5.0g; 24.2 mmol; SCHEME 14) and anhydrous DCM (75 mL) was added sequentially acetyl chloride ((2.42 mL; 33.9 mmol) and SnCl₄ (5.39 mL; 46.1 mmol; 1 M solution in DCM). The reaction was stirred at RT for 50 minutes and poured into a mixture of ice and 2 N HCl (200 mL). The organic phase was separated and diluted with about 50 mL of CH₂Cl₂ and thrice washed with water (100 mL) and once with brine (100 mL). The organic phase was dried (MgSO₄), filtered and evaporated to yield **101b** (6.0 g) which contained about 10% of **101a.** The crude product was used without further purification.

step 2 - To an ice-cold solution of **101b** (6.01 g; 24.2 mmol) and DCM (100 mL) under a nitrogen atmosphere was added sequentially a solution of MCPBA (11.9 g; 48.4 mmol) and DCM (12 mL) followed by TFA (2.14 mL; 27.8 mmol). The reaction mixture was stirred at RT overnight. The reaction mixture was cooled to 0°C and a 5% aqueous Na₂SO₃ solution (150 mL) was added slowly with stirring. The mixture was stirred for 5 minutes after addition was completed and precipitated *m*-chlorobenzoic acid was filtered. The solid was washed with DCM and the combined filtrates were washed with 10% NaOH (2 x 250 mL), 2 N HCl (200 mL), water and brine. The resulting solution was dried (MgSO₄), filtered through a pad of CELITE and concentrated *in vacuo* to yield **101c** (4.1g).

step 3 - A solution of **101c** (10.3 g; 39.3 mmol), EtOH (250 mL) and saturated NaHCO₃ (100 mL) were heated to reflux for 1 h. The reaction mixture was cooled to room temperature and the EtOH removed *in vacuo.* Ice was added to the residue aqueous solution and the reaction carefully acidified to about pH 2 with 2 N HCl. The resulting mixture was extracted with EtOAc (2 x 300 mL) and the combined organic phase washed with brine, dried (NaSO₄), filtered and evaporated to yield a brown oil (8.8 g). The crude product was run through a silica gel column with 15% EtOAc:hexane to yield **101d** (5.44g; 62.9%) as a white solid.

step 4 - A dry 50 mL round bottom flask was charged with **101d** (0.5 g, 2.27 mmol), 3-bromobenzonitrile (0.620 g, 3.4 mmol), Cs₂CO₃ (1.48 g, 4.54 mmol), Cu(I)Cl (0.112 g, 1.14 mmol), 2,2,6,6-tetramethyl-3,5-heptanedione (50 µL, 0.23 mmol) and NMP (10 mL). The reaction was heated at 120° C under an Ar atmosphere for 55 h. The reaction mixture was partitioned between EtOAc and 10 % aqueous HCl. The aqueous extracts were diluted with hexane and the hexane solution washed thoroughly with water, dried (MgSO₄), filtered and evaporated. The crude product was purified by SiO₂ chromatography eluting with 20% EtOAc/hexane to afford 0.373 g of 103.

step 5 - To a solution of **103** (0.350 g; 1.052.mmol) and CCl₄ (15 mL) was added NBS (0.197 g;1.104 mmol) and AIBN (0.0035 g). The reaction was heated at reflux for 50 min, an additional 5 mg of AIBN was added and refluxing continued for an additional 0.5 h. The reaction mixture was cooled to RT, diluted with DCM, washed with 10% NaHSO₄, water and brine. The resulting solution was dried, filtered and evaporated in *vacuo.* The crude product was purified by flash chromatography eluting with 5% EtOAc/hexane to afford 0.277 g of **105a**.

Steps 6 to 8 were carried out by the procedure described in steps 7-9 of Example 1 except 4-amino-3-methyl-benzenesulfonamide replaced 4-amino-benzenesulfonamide which **I-145.**

Compound **I-144** was prepared using a similar procedure except in step 8 of the present example, 4-amino-2-methyl-benzenesulfonamide was replaced with 4-amino-benzenesulfonamide.

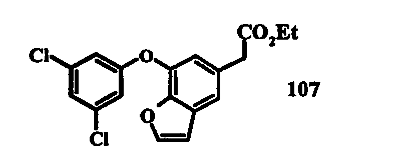

Compound **1-149** was prepared from **107** which was prepared by Cu(II)(OAc)₂ mediated coupling of **101d** and 3,5-dichlorobenzeneboronic acid. Conversion of **107** to **I-149** was carried out by the procedure described in steps 7-9 of Example 1 except 4-amino-3-chloro-benzenesulfonamide replaced 4-amino-benzene-sulfonamide.

### Example 48

### 2-[3-(3-Chloro-5-cyano-phenoxy)-4,5-dimethyl-phenyl] -N-(2-methyl-4-sulfamoyl-phenyl)-acetamide (I-183)

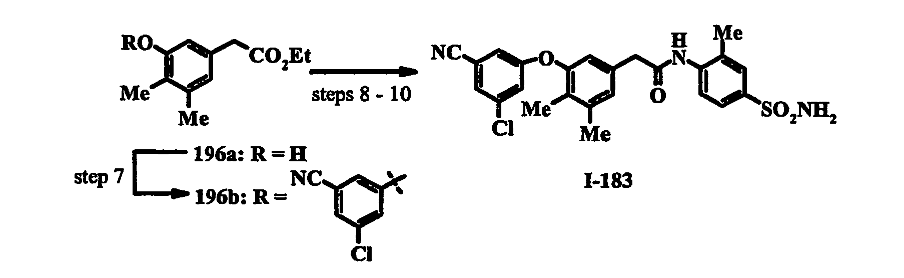

step 1- A mixture of 4-hydroxy-3-methoxyphenylacetic acid (20;1.0 g; 5.49 mmol, SCHEME 2) and hexamethylenetetramine (0.808 g; 5.76 mmol) and TFA (7 mL) were stirred and heated at 90° C for 4 h. The reaction was cooled and excess TFA removed *in vacuo* and 35 mL of ice and water was added to the residue. The resulting dark brown solution was stirred at rt for 20 min. The aqueous solution was extracted with Et₂O (40 mL) and the extract was dried (Na₂SO₄), filtered and evaporated to afford 0.70 g of **22a** (61%): ms (M+H)⁺ = 211.13; mw = 210

step 2 - To a solution of **22a** (4.0 g; 19.03 mmol) in EtOH (80 mL) was added con H₂SO₄ (1 mL). The reaction was heated at reflux for 6 h. Approximately 80% of the EtOH was removed *in vacuo* and the residue partitioned between EtOAc/H₂O (1:1) the organic phase residue washed with 10% NaHCO₃, water (100 mL), dried (Na₂SO₄), filtered and evaporated to afford a brown oil **22b** (88%): ms (M+H)⁺= 239.19; mw = 238.3.

step 3 - A mixture of **22b** (3.70 g; 15.53 mmol), 5% Pd/C (0.350 g), HOAc (45 mL) were shaken under a H₂ atmosphere (40 psi) for 8 h. TLC showed product and the corresponding benzyl alcohol. An additional 300 mg of Pd/C in 25 mL HOAc was added and hydrogenation continued for another 8 h. A second portion of 0.15 g of Pd/C in HOAc (15 mL) was added and reaction continued for another 12 h. The mixture was diluted with EtOAc and filtered through a pad of CELITE®. The catalyst was washed with EtOAc and the combined organic extracts dried (Na₂SO₄) and evaporated. The product was purified by silica gel chromatography and eluted with CH₂Cl₂:hexane (4:1) to afford 2.64 g of **22c** (75.8%).

step 4 - To a solution of **22c** (5.87 g; 26.175 mmol) in CH₂Cl₂ cooled to 0 °C was added pyridine (3.60 mL; 44.51 mmol) followed by dropwise addition of triflic anhydride (6.605 mL; 39.26 mmol) over about 20 min. The reaction was stirred at 0 °C for 3.5 h. The reaction mixture was extracted with dilute HCl and half-saturated NaHCO₃, dried (Na₂SO₄) and evaporated to yield 9.41 g of **24a** as a brown oil (100%).

step 5 - To a suspension of PdCl₂(dppf) (0.650 g; 0.785 mmol) in THF (40 mL) cooled to 0 °C was added dropwise a solution of DIBAL-H (1.0 M in PhMe; 1.57 mL; 1.57 mmol). The resulting mixture was stirred at 0 °C for 5 minutes and a solution of 24a in 5 mL of THF was added followed by Me₂Zn (23 mL; 46.0 mmol; 1.0 M in PhMe). The mixture was stirred at 0 °C for 5 m and heated at reflux for 2.5 h then cooled to rt for 30 m. The reaction was poured into dilute HCl and extracted with EtOAc (2 x 100 mL), dried (Na₂SO₄), and evaporated. The crude product was purified by silica gel chromatography and eluted with CH₂Cl₂:hexane (1:2 to 1:1 to 2:1 v/v) to yield 5.1 g (87.6%) of **24b.**

step 6 - A solution of ethyl 3,4-dimethyl-5-methoxyphenylacetate (24b; 0.560 g; 2.519 mmol) and CH₂Cl₂ (40 mL) was cooled to -78 °C and a solution of BBr₃ (10.1 mL; 10.1 mmol; 1.0 M in CH₂Cl₂) dropwise over 10 min. After 1 h at -78°C the reaction was allowed to warm to rt and stirred for 12 h. The reaction was cooled in an ice-water bath and the reaction quenched with 15 mL of ice/water. The aqueous phase was extracted with CH₂Cl₂:EtOAc (3:1 v/v), dried (Na₂SO₄), filtered and evaporated to yield **24c** (0.52 g; 99%): ms 209.21 (M+H)⁺.

Steps 7-10 were carried out as described in steps 6 to 9 of Example 1 except in the final step of the present example, 4-amino-3-methyl-benzenesulfonamide replaced 4-amino-3-benzenesulfonamide.

Compound **I-182** was prepared in the same manner except in the final step of the present example, 4-amino-3-methyl-benzenesulfonamide was replaced with 4-amino-benzenesulfonamide.

Compound **I-184** was prepared in the same manner except in the final step of the present example, 4-amino-3-methyl-benzenesulfonamide was replaced with 4-amino-3-chloro-benzenesulfonamide.

### Example 49

### 2-[3-(2-Bromo-5-chloro-phenoxy)-4-isopropyl-phenyl]-N-(4-sulfamoyl-phenyl)-acetamide (I-26)

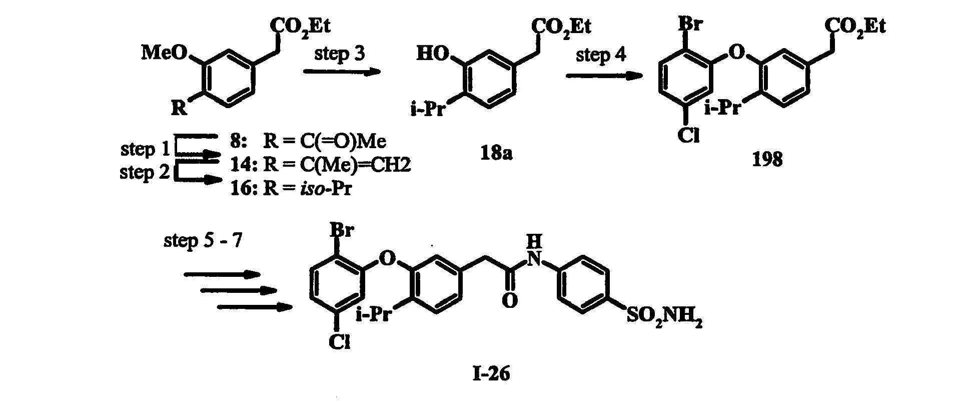

### step 1

To a suspension of PPh₃CH₃⁺ Br⁻ (36.29 g; 101.6 mmol) in THF (150 mL) cooled to -40°C was added dropwise *n*-BuLi (40.6 mL; 1.6M in hexanes) and the resulting solution was allowed to warm to -10 °C for 10 m and re-cooled to -40 °C. To the resulting solution was added in one portion ethyl 4-acetyl-3-methoxyphenylacetate (8, see Example 10; ) and the reaction mixture was stirred at 0° C for 30 m and warmed to rt and stirred for an additional 2 h. The reaction mixture was diluted with hexane filtered through a pad of CELITE ® and the solids washed with hexane:Et₂O (5:1 v/v; 60 mL). The combined organic layers were washed with water (50 mL) and brine (50 mL), dried (Na₂SO₄), filtered and evaporated to yield a yellow oil. The product was purified by silica gel chromatography and eluted with CH₂Cl₂:hexane (50 to 66% DCM) to yield 9.1 g of 14.

### step 2

A suspension of 14 (9.0 g; 38.41 mmol), 5% Pd/C (380 mg) in 50 mL HOAc and 50 mL EtOH was shaken under a hydrogen atmosphere (50 psi) for 7 h. The mixture was filtered through a pad of CELITE^{®} and the filtered catalyst was washed with EtOAc. The solvents were evaporated under reduced pressure and the residue dissolved in MTBE and carefully washed with sat'd HaHCO₃, water and brine. The resulting solution was dried (Na₂SO4), filtered and evaporated to yield ethyl 4-*iso*-propyl-3-methoxyphenylacetate (**16**; 9.0 g) as a yellow oil.

### step 3

A solution of **16** (3.38 g; 14.30 mmol) and CH₂Cl₂ (150 mL) were cooled to -78°C and a solution of BBr₃ (5.41 mL; 57.22 mmol) in 130 mL of CH₂Cl₂ were added dropwise over a 30 m period. The reaction mixture was stirred at -78°C for 1 h, allowed to warm to rt for 4 h and re-cooled to -78 °C and carefully quenched with sat'd. NaHCO₃ (80 mL). The aqueous layer was extracted with CH₂Cl₂ (1 x 100 mL), EtOAc (50 mL) and the combined aqueous layers washed with water and brine, dried (Na₂SO₄) and evaporated to yield a light brown oil. The phenol was purified by silica gel chromatography and eluted with CH₂Cl₂:hexane (3:1) to CH₂Cl₂ to CH₂Cl₂:EtOAc (100:4) to yield ethyl 4-*iso*-propyl-3-hydroxyphenylacetate (**18a**; 3.0 g; 94%)

Introduction of the aryloxy linkage (step 4) was accomplished by reacting **18a** and 1-bromo-4-chloro-2-fluorobenzene as described in step 1 of example 17 to afford **198**. Steps 5 to 7 were carried out by the procedure described in steps 7-9 of example 1 to afford **I-26**.

Compound **I-27** was prepared in the same manner except in the final step of the present example 2-chloro-benzeneamine replaced 4-amino-benzenesulfonamide.

### Example 50

### 2-[4,5-Dibromo-3-(3-cyano-5-difluoromethyl-phenoxy)-2-fluoro-phenyl]-N-(2-methyl-4-sulfamoyl-phenyl)-acetamide (I-104)

[4,5-Dibromo-3-(3-cyano-5-difluoromethyl-phenoxy)-2-fluoro-phenyl] -acetic acid ethyl ester (**200**) was isolated as a byproduct in the Sandmeyer reaction used in the preparation of **186** described in step 1 of example 40. The dibromo compound was converted to **I-104** by the procedure described in steps 7 to 9 of example 1 except in the present example, 4-amino-3-methyl-benzenesulfonamide replaced 4-amino-benzenesulfonamide.

### Example 51

### (4-{2-[3-(3-Chloro-5-cyano-phenoxy)-4-methyl-phenyl]-acetylamino}-3-methyl-phenoxy)-acetic acid (I-234)

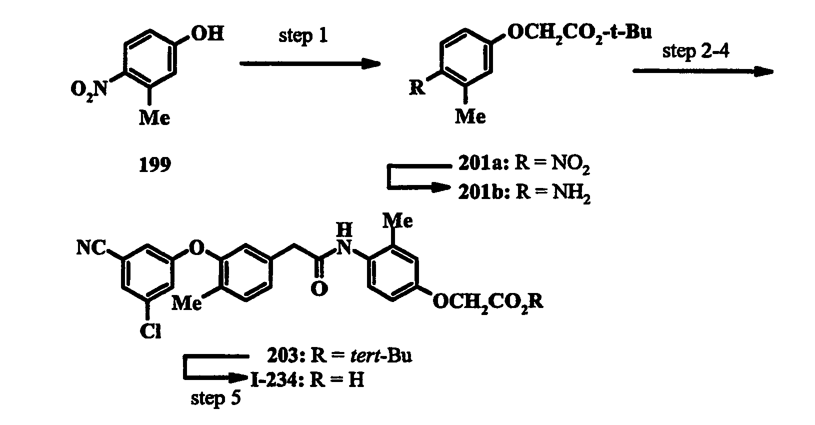

K₂CO₃ (2.7 g, 1 equiv) was added to a solution of **199** (3.0 g, 19.6 mmol) and DMF (20 mL). To this mixture was added the *tert*-butyl bromoacetate (4.09 g, 1.1 equiv). The reaction mixture was stirred for 2 h, poured into NH₄Cl solution, and extracted with EtOAc. The organic layer was washed with water, dried (MgSO₄), filtered, and concentrated to provide a brown oil. This oil was purified by SiO₂ chromatography eluting with a EtOAc/hexane gradient (0% to 20% EtOAc) to afford 3.67 g (71%) of the 201a as a white solid. The solid was dissolved in EtOH, 10% Pd/C (0.3 g) was added, and the reaction was placed under a hydrogen atmosphere(45 psi). After 2 h, the catalyst was filtered and the filtrate concentrated *in vacuo* to afford **201b.**

Hydroysis of the ester, formation of the acid chloride and condensation with 201b were carried out as described in steps 7 to 9 of Example 1 which afforded **203**. The ester 203 (0.22 g, 0.42 mmol) was stirred in formic acid (4.6 mL) under N₂. After 2 h, the reaction was diluted with EtOAc and poured into water. The organic layer was washed with NaHC03. The combined organic layers were (MgSO₄) and concentrated *in vacuo* to afford 0.103 g (53%) of **I-234** as a white solid.

### Example 52

### 2-[3-(4-Chloro-benzoyl)-5-methyl-phenyl]-N-(2-methyl-4-sulfamoyl-phenyl)-acetamide (I-255)

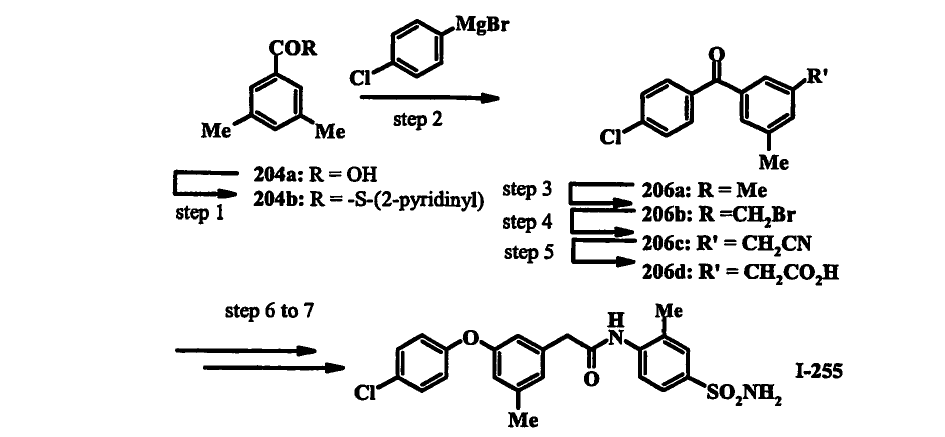

step 1- A solution of **204a** (25 g, 0.166 mmol), triphenylphosphine (43.66 g, 0.166 mmol), 2,2'-dipyridyl disulfide (36.67g, 0.166 mmol) and MeCN ( 250 mL) was heated under an Ar atmosphere for 2 h. The reaction mixture was cooled to RT and partitioned between EtOAc and H₂O. The aqueous phase was extracted with EtOAc and the combined EtOAc extracts were dried (MgSO₄), filtered and concentrated *in vacuo.* The crude product was purified by SiO₂ chromatography eluting with a EtOAc/hexane gradient (10 to 30% EtOAc) which afforded 37 g of **204b**.

step 2 - A dry flask was charged with fine Mg turnings (1.48 g, 60.8 mmol) and dry THF (50 mL) and a crystal of iodine was added. A solution of 1-bromo-4-chlorobenzene (11.64 g, 60.8 mmol) and dry THF was added dropwise. If the Grignard reagent fails to form spontaneously the flask was gently warmed until the reaction began and stirring was continued until the Mg was consumed. To a solution of **204b** (7.4 g, 30.4mmol) and dry THF (50 mL) cooled to 0° C and maintained under an Ar atmosphere was added dropwise the solution of 1-chloro-1-bromomagnesium-benzene over a 30 min period. After the reaction was complete the solution was warmed to RT and stirred overnight. The reaction mixture was cooled to 0° C and the reaction was quenched with saturated NH₄Cl. The solution was extracted with EtOAc, dried (MgSO₄), filtered and evaporated. The crude product was purified by SiO₂ chromatography eluting with a EtOAc/hexane gradient (10 to 30% EtOAc) to afford 3.46 g of **206a**.

step 3 - A solution of **206a** (3.46g, 14.1 mmol), NBS (2.52 g, 14.1 mmol) and benzoyl peroxide (0.035 g) and CCl₄ (75 mL) was heated to reflux for 1.5 h while exposed to a 300 watt lamp. The solution was cooled and the precipitated succinimde was removed by filtration. The solvent was removed *in vacuo* and purified by flash chromatography eluting with a EtOAc/hexane gradient (1 to 2% EtOAc) to afford **206b**.

step 4 - A solution of **206b** (1.97 g, 6.09 mmol), sodium cyanide (0.59 g, 12.1 mmol) and EtOH/H₂O (50 mL, 90% EtOAc was stirred at RT overnight. The reaction mixture was concentrated *in vacuo* and partitioned between EtOAc and H₂O. The aqueous phase was extracted with EtOAc and the combined extracts dried (MgSO₄), filtered and evaporated. The crude solid was recrystallized from hexane/EtOAc to afford 1.36 g of **206c**.

step 5 - A mixture of **206c** (1.36 g, 5.04 mmol) and concentrated HCl/HOAc (50 mL, 1:1) was stirred at 100° C for 2 h. The reaction mixture was concentrated *in vacuo,* H₂O was added and the aqueous phase extracted twice with EtOAc. The combined extracts were dried (MgSO₄), filtered and evaporated to afford 1.2 g of **206d** as a white powder.

Steps 6 and 7 were carried out by the procedure in steps 8 and 9 of example 1 except in step 7 of the present example 4-amino-3-methyl-benzenesulfonamide replaced 4-amino-benzenesulfonamide to afford **I-255.**

Compound **I-254** was prepared as described for **I-255** except in step 7, 4-amino-3-methyl-benzenesulfonamide was replaced by 4-amino-benzenesulfonamide.

Compound **I-256** was prepared as described for **I-255** except in step 2, 2-bromo-1-chloro-benzene was used instead of 4-bromo-1-chloro-benzene.

Compound **I-257** was prepared as described for **I-255** except in step 7, 4-amino-3-chloro-benzenesulfonamide was used in place of 4-amino-3-methyl-benzenesulfonamide.

Compound **I-258** was prepared as described for **I-255** except in step 7, 2-chloro-phenylamine was used in place of 4-amino-3-methyl-benzenesulfonamide.

Compound **I-259** was prepared as described for **I-256** except in step 7,2-chloro-phenylamine was used in place of 4-amino-3-methyl-benzenesulfonamide.

Compound **I-260** was prepared as described for **I-255** except in step 2, 1-bromo-3-chlorobenzene was used in place of 1-bromo-4-chlorobenzene.

Compound **I-261** was prepared as described for I-256 except in step 7, 4-amino-benzenesulfonamide replaced 4-amino-3-methyl-benzenesulfonamide.

Compound **I-262** was prepared as described for I-260 except in step 7, 2-chloro-phenylamine replaced 4-amino-3-methyl-benzenesulfonamide.

Compound **I-232** was prepared as described for **I-255** except in step 2, 1-bromo-4-chlorobenzene was replaced by 1-bromo-3,5-dichloro-benzene.

Compound **I-231** was prepared as described for **I-232** except in step 7, 4-amino-3-methyl-benzenesulfonamide was replaced by 2-chloro-phenylamine.

Compound **I-247** was prepared as described for **I-255** except in step 12-chloro-5-methyl benzoic acid was used in place of 3,5-dimethylbenzoic acid, in step 2,1-bromo-3,5-dichloro-benzene was used instead of 4-bromo-1-chloro-benzene and in step 7, 4-amino-benzenesulfonamide was replaced by 2-chloro-phenylamine.

Compound **I-252** was prepared as described for **I-247** except in step 7, 4-amino-3-methyl-benzenesulfonamide was used in place of 2-chloro-phenylamine

Compound **I-248** was prepared as described for **I-247** except in step 2, 2-bromo-1,4-dichloro-benzene was used instead of 1-bromo-3,5-dichloro-benzene and in step 7, 2-chloro-phenylamine was used instead of 4-amino-benzenesulfonamide.

Compound **I-249** was prepared as described for **I-248** except in step 7, 4-amino-3-methyl-benzenesulfonamide was used in place of 2-chloro-phenylamine

Compound **I-250** was prepared as described for **I-248** except in step 7, 4-amino-benzenesulfonamide was used in place of 2-chloro-phenylamine.

Compound **I-253** was prepared as described for **I-248** except in step 7, 4-amino-3-chloro-benzenesulfonamide was used in place of 2-chloro-phenylamine

Compound **I-251** was prepared as described for **I-248** except in step 7, phenylamine was used in place of 2-chloro-phenylamine

### Example 53

### 2-[3-(2-Bromo-5-chloro-phenylsulfanyl)-4-chloro-phenyl]-N-(4-sulfamoyl-phenyl)-acetamide (I-243)

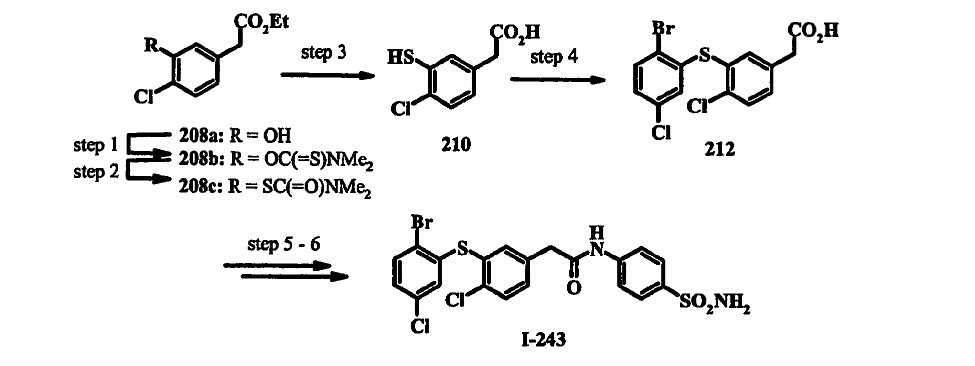

step 1- A solution of **208a** (10.11 g, 47 mmol), dimethylthiocarbamoyl chloride (8.73 g, 70.6 mmol), DABCO (10.56 g, 94.2 mmol) and DMF (75 mL) was stirred at RT for 20 min then at 75° C for an additional 30 min. The reaction mixture was cooled to RT, diluted with H₂O, and twice extracted with EtOAc. The combined EtOAc extracts were thrice washed with H₂O, dried (MgSO₄), filtered and evaporated. The impure product was purified by SiO₂ chromatography elution with an EtOAc/hexane gradient (10 to 20% EtOAc) to afford 11.27 g of **208b.**

step 2 - A flask was charged with **208b** (11.27 g) and the melt was heated at 220° C overnight. The crude product was purified by SiO₂ chromatography eluting with an EtOAc/hexane gradient (10 to 20% EtOAc) to afford 7.08 g of **208c.** An additional 2.19 g was isolated form the column which contained a small amount of an impurity.

step 3 - To a solution of **208c** (7.08 g, 23.4 mmol) was added a solution of NaOH (3.75 g, 93.8 mmol) and H₂O (25 mL). The solution was heated for 1 h at 60° C under an Ar atmosphere. The reaction mixture was cooled to RT and acidified with 1 N HCl. The resulting solid was filtered, washed well with water and dried to afford 4.6 g of product. The crude product along with an earlier batch was purified by SiO₂ chromatograph eluting with EtOAc/hexane/HOAc (39.5:60:0.5) to afford 5.26 g of **210**.

step 4 - A solution of **210** (0.26 g, 1.28 mmol), 1-bromo-2-fluoro-4-chloro-benzene (0.16 mL, 1.28 mmol), K₂CO₃ (0.35 g, 2.56 mmol) and DMF (12 mL) maintained under an Ar atmosphere was warmed to 60° C overnight. The reaction mixture was cooled to RT, diluted with H₂O (20 mL) and extracted with EtOAc. The aqueous phase was adjusted to pH 2 with dilute HCl and the resulting precipitate was collected by filtration and washed with H₂O. The crude product was preabsorbed onto SiO₂ and purified by flash chromatography elution 50% EtOAc/hexane to afford 0.053 g of 212.

Steps 5 and 6 were carried out as described for steps 8 and 9 of Example 1 to afford **I-243**.

Compound **I-244** prepared as described for **I-243** except in step 7, 4-amino-benzenesulfonamide was replaced with 2-chlorophenylamine.

Compound **I-242** prepared as described for **I-243** except in step 4, 1-bromo-2-fluoro-4-chloro-benzene was replaced by 5-fluoroisophthalonitrile.

### Example 54

### (3-Chloro-4-{2-[3-(3-chloro-5-cyano-phenoxy)-4-methyl-phenyl]-acetylamino}-phenyl)-acetic acid methyl ester (I-235)

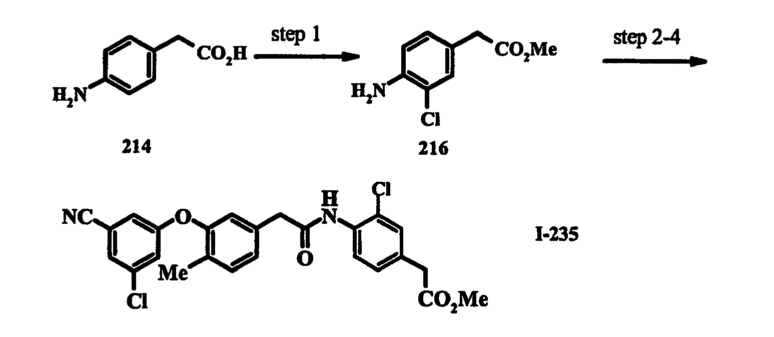

### step 1

The aniline **214**(21.4 g, 140 mmol) was dissolved in MeOH (140 mL), and 1 mL of concentrated sulfuric acid was added. The solution was heated to reflux for 3 d, cooled, and poured into saturated NaHCO₃ solution. The aqueous solution was extracted with a 1:1 EtOAc/hexane, dried (MgSO₄) and evaporated *in vacuo.* Six grams of the resulting crude product was purified by SiO₂ chromatography eluting with a hexane/EtOAc gradient (10% to 50% EtOAc) to provide 5.5 grams of methyl ester. The methyl ester (0.50 g, 3.0 mmol) was dissolved in MeCN (10 mL). The solution was warmed to 30° C, and NCS (0.42 g,1.05 equiv) was added in one portion. After 1 h, the reaction was warmed to 50 C. After an addition hour, the reaction was cooled to RT and the solvent was evaporated. The remaining oil was dissolved in DCM, and the organic layer was washed with a 5% NaOH solution, brine, and dried (MgSO₄). Evaporation of the volatile materials and SiO₂ chromatography of the residue provided 0.28 g (45%) of **216**.

Steps 2-4 were carried out as described in steps 7 to 9 of example 1 except in the final step 4-amino-benzenesulfonate was replaced by **216**.

The ester was deprotected by adding LiOH (1.1 mL of a 2M solution, 4 equiv) to a solution of the ester (0.29 g, 0.55 mmol) in THF (4 mL). The solution was stirred for 4 h. The mixture was quenched with 2 M HCl solution, and the aqueous layer was extracted with ethyl acetate. The combined organic extracts were washed with brine and dried over magnesium sulfate. Evaporation of the volatile materials afforded **I-235**.

### Example 55

### (4-{2-[3-(3-Chloro-5-cyano-phenoxy)-4-methyl-phenyl]-acetylamino}-3-methyl-phenoxy)-acetic acid (I-234)

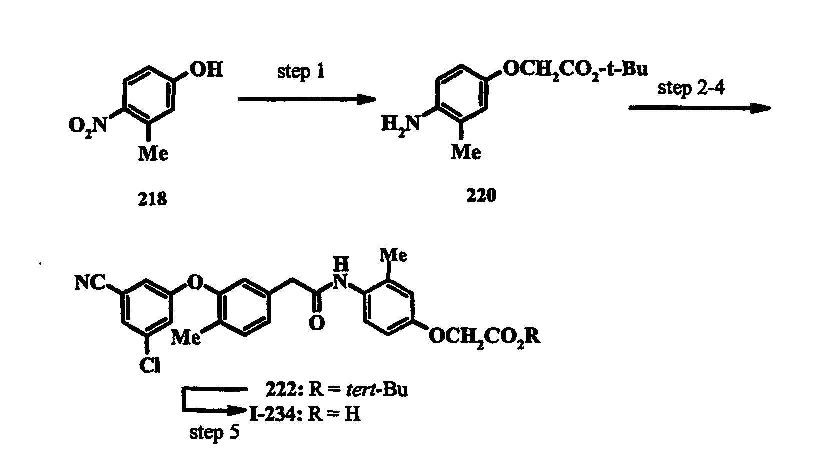

step 1- K₂CO₃ (2.7 g, 1 equiv) was added to a solution of **218** (3.0 g, 19.6 mmol) and DMF (20 mL). To this mixture was added the *tert*-butyl bromoacetate (4.09 g, 1.1 equiv). The reaction mixture was stirred for 2 h, poured into NH₄Cl solution, and extracted with EtOAc. The organic layer was washed with water, dried (MgSO₄), filtered, and concentrated to provide a brown oil. This oil was purified by SiO₂ chromatography eluting with a EtOAc/hexane gradient (0% to 20% EtOAc) to afford 3.67 g (71%) of the nitro ether as a white solid. This solid was dissolved in EtOH, 10% Pd/C (0.3 g) was added, and the reaction was placed under a hydrogen atmosphere (45 psi). After 2 h, the catalyst was filtered and the filtrate concentrated *in vacuo* to afford **220**

Steps 2-4 were carried out as described in steps 7 to 9 of example 1 except in the final step 4-amino-benzenesulfonate was replaced by 220.

The ester **220** (0.22 g, 0.42 mmol) was stirred in formic acid (4.6 mL) under N₂. After 2 h, the reaction was diluted with EtOAc and poured into water. The organic layer was washed with NaHC03. The combined organic layers were dried (MgSO₄) and concentrated *in vacuo* to afford 0.103 g (53%) of **I-234** as a white solid.

### Example 56

### 2-[4-Chloro-3-(4-cyano-6-methyl-pyridin-2-yloxy)-2-fluoro-phenyl]-N-(2-methyl-4-sulfamoyl-phenyl)-acetamide (I-233)

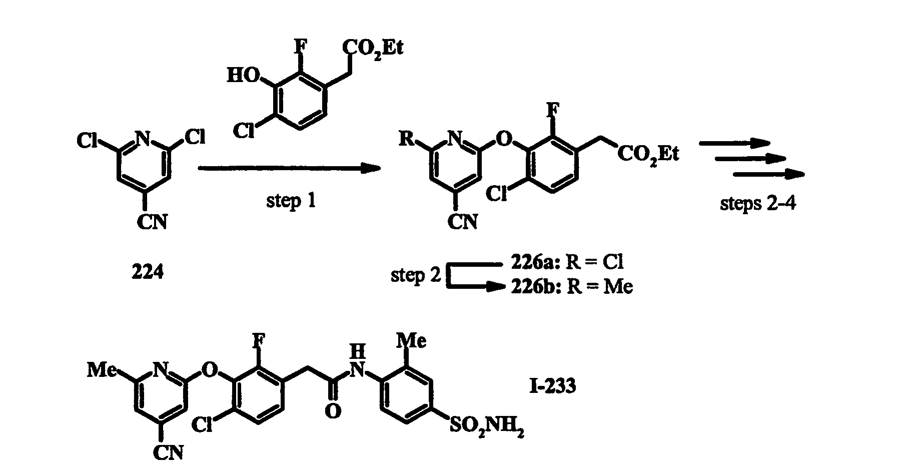

step 1 - To a stirred mixture containing 2,6-dichloro-isonicotinonitrile (**224**, 0.95 g, 5.49 mmol), Cs₂CO₃ (1.97 g, 6.04 mmol) in dimethylacetamide (20 mL) was added (4-chloro-2-fluoro-3-hydroxy-phenyl)-acetic acid ethyl ester (1.20 g, 5.16 mmol). The flask was flushed with argon and heated at 100 °C. After stirring for 3 h the reaction mixture was cooled to RT and diluted with EtOAc/hexanes (3:1). The organic phase was washed with saturated NH₄Cl solution and brine solution, dried (MgSO₄) and concentrated *in vacuo.* The crude product was purified by SiO₂ chromatography eluting with a EtOAc/hexane gradient (0 to 20%) to afford 0.78 g (39%) of **226a** as a white solid.

step 2 - Me₂Zn solution (1.58 mL, 3.17 mmol, 2M in PhMe) was added to a mixture (0° C) containing 226a (0.78 g, 2.11 mmol), (Ph₃P)₂Pd(II)Cl₂ (0.15 g, 0.211 mmol), N,N-dimethylethanolamine (43 mL, 0.422 mmol) cooled to 0° C. After stirring for 20 min the reaction mixture was warmed to RT and stirred for 2 h then poured onto ice/aqueous saturated ammonium chloride solution. The organics were extracted with EtOAc, washed with brine, dried (MgSO₄) and concentrated *in vacuo.* The crude product was purified by SiO₂ chromatography eluting with a EtOAc/hexane gradient (0 to 15%) to afford 0.67 g (91%) of 226b as a white solid.

The phenyl acetic acid ester **226b** was converted to **I-124** (steps 2-4) utilizing the procedures described in steps 7-9 of Example 1 except in the final step 4-amino-3-methyl-benzenesulfonamide replaced 4-aminobenzenesulfonamide.

### Example 57

### 2-[7-(4-Chloro-benzoyl)-2,3-dihydro-benzofuran-5-yl]-N-(4-sulfamoyl-phenyl)-acetamide (I-241)

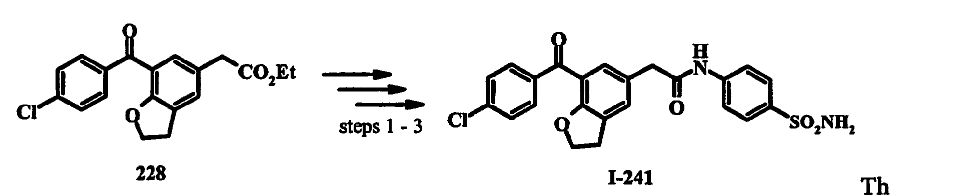

The dihydrobenzofuran 228 was prepared as described by J. Dunn et al. J. Med Chem 1986 29:2326. Steps 1-3 were carried out as described in steps 7-9 of example 1 to afford **I-241**.

Compound **I-240** was prepared as described for **I-241** except in step 3, 2-chloro-phenylamine was used in place of 2-amino-benzenesulfonamide

### Example 58

### 2-[4-Chloro-3-(2,5-dichloro-benzyl)-phenyl]-N-(2-chloro-4-sulfamoyl-phenyl)-acetamide (I-209)

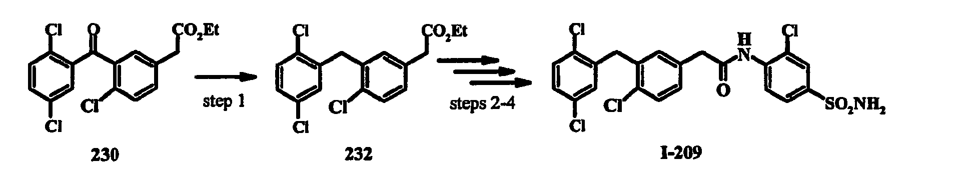

step 1- A solution of 230 (0.521 g,1.47 mmol) and triethylsilane (0.64 mL, 3.98 mmol) and TFA (10 mL) were stirred overnight at RT. The reaction mixture was diluted with H₂O (20 mL) and extracted twice with EtOAc. The combine extracts were dried, filtered and evaporated to afford **232**.

Steps 2-4 were carried out as described in steps 7-9 of example 1 except in the final step 4-amino-benzenesulfonamide was replaced by 4-amino-3-chloro-benzenesulfonamide to afford **I-209.**

### Example 59

### 2-[3-(5-Cyano-2-ethyl-benzoyl)-4-ethyl-phenyl]-N-(2-methyl-4-sulfamoyl-phenyl)-acetamide (I-268)

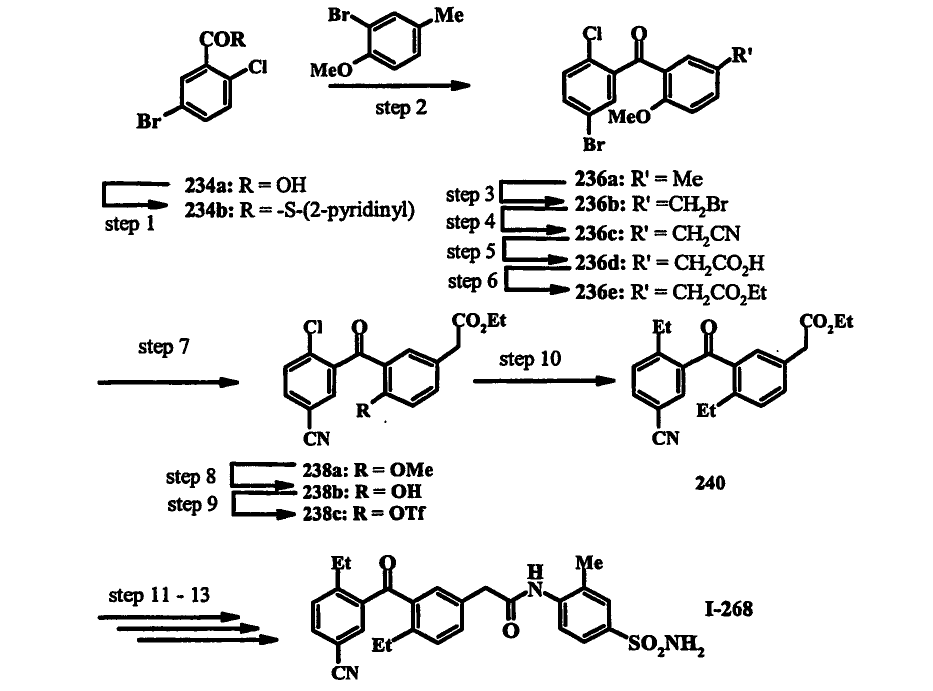

Steps 1 5 were carried out as described in steps 1 to 5 Example 52 except in the present example 5-chloro-2-bromo-benzoic acid was used in place of 3,5-dimethylbenzoic acid.

step 6 - A solution of **236d** (6.09 g, 15.9 mmol), EtOH (75 mL) and con H₂SO₄ were heated at 80° C overnight. Approximately 75% of the EtOH was evaporated and the residue diluted with H₂O and twice extracted with EtOAc. The combined extracts were washed with saturated NaHCO₃, dried (MgSO₄), filtered and evaporated to yield 5.54 g of 236e as a white solid.

step 7 - A flask was charged with **236e** (5.54 g, 13.4 mmol), Zn(CN)₂ (1.57 g, 13.4 mmol), Pd[P(Ph)₃]₄ (1.55 g, 13.34 mmol) and dry DMF (70 mL) and the flask thrice evacuated and flushed with Ar. The reaction mixture was heated at 80° C for 1.5 h. The reaction was cooled to RT and diluted with EtOAc/hexane (300 mL 1:1) and twice extracted with EtOAc/hexane. The EtOAc extracts were washed once with H₂O, dried (MgSO₄), filtered and evaporated. The resulting orange oil was purified by SiO₂ chromatography eluting with an EtOAc/hexane gradient (20 to 30% EtOAc) to afford 1.1 g of **238a** as white solid.

step 8 - To a solution of **238a** (3.1 g, 8.66 mmol) and DME (40 mL) cooled to -78° C was added dropwise over 5 min, a solution of BBr₃ and DCM (43.3 mL of a 1.0 M solution in DMC, 43.3 mmol). After the addition was complete the reaction mixture was stirred at RT for 2 h, poured onto ice. The aqueous layer was separated and extracted with DCM and the combined DCM solutions washed with brine, dried (MgSO₄), filtered and evaporated. The crude product was purified by SiO₂ chromatography eluting with 20% EtOAc/hexane to afford 2.63 g of **238b** as a white solid.

step 9 - The conversion of **238b** to the triflate **238c** was carried out as described in step 1 of example 6.

step 10 - A flask was charged with **238c** (1.2 g, 2.52 mmol), Pd(dppf).CH₂Cl₂ (0.103 g, 0.126 mmol), TEA (3.4 mL, 3.78 mmol). DIBAL-H (75 µL, 0.113 mmol) was added at 0° C, warmed to RT and Et₂Zn was added as described in step 2 of example 6 to afford 0.86 g of **240**.

Stepe 11-13 were carried out as described for steps 7-9 of example 1 except in the present example 4-amino-3-methyl-benzenesulfonamide was used in place of 4-amino-benzenesulfonamide which afford **I-268**.

### Example 60

### HIV Reverse Transcriptase Assay: Inhibitor IC₅₀ determination

HIV-1 RT assay was carried out in 96-well Millipore MultiScreen MADVNOB50 plates using purified recombinant enzyme and a poly(rA)/oligo(dT)₁₆ template-primer in a total volume of 50 µl. The assay constituents were 50 mM Tris/HCl, 50 mM NaCl, 1 mM EDTA, 6 mM MgCl₂, 5 µM dTTP, 0.15 µCi [³H] dTTP, 5 µg/ml poly (rA) pre annealed to 2.5 µg/ml oligo (dT)₁₆ and a range of inhibitor concentrations in a final concentration of 10% DMSO. Reactions were initiated by adding 4 nM HIV-1 RT and after incubation at 37°C for 30 min, they were stopped by the addition of 50 µl ice cold 20%TCA and allowed to precipitate at 4°C for 30 min. The precipitates were collected by applying vacuum to the plate and sequentially washing with 3 x 200 µl of 10% TCA and 2 x 200 µl 70% ethanol. Finally, the plates were dried and radioactivity counted in a Packard TopCounter after the addition of 25 µl scintillation fluid per well. IC₅₀'s were calculated by plotting % inhibition versus log₁₀ inhibitor concentrations.

### Example 61

### Anti-viral assay

Anti-viral assays were carried out by the method described by R. E. Pauwels et al. J. Virol. Methods 1988 20(4):309-322.

| Table 2 | | |
|---|---|---|
| Compound # | RT inhibition IC50 | Anti-Viral Assay EC₅₀ |
| I-138 | 0.0045 | - |
| I-63 | 0.0058 | - |
| I-127 | 0.0059 | - |
| I-179 | 0.0074 | 0.0004 |
| I-83 | 0.0081 | 0.0009 |
| I-109 | 0.0136 | 0.0013 |
| I-180 | 0.0091 | - |
| I-84 | 0.014 | 0.0004 |
| I-140 | 0.021 | 0.0004 |
| I-165 | 0.021 | 0.0004 |
| I-169 | 0.027 | 0.0004 |

### EXAMPLE 61

### Pharmaceutical Compositions

Pharmaceutical compositions of the subject Compounds for administration via several routes were prepared as described in this Example.

### Composition for Oral Administration (A)

| Ingredient | % wt./wt. |
|---|---|
| Active ingredient | 20.0% |
| Lactose | 79.5% |
| Magnesium stearate | 0.5% |

The ingredients are mixed and dispensed into capsules containing about 100 mg each; one capsule would approximate a total daily dosage.

### Composition for Oral Administration (B)

| Ingredient | % wt./wt. |
|---|---|
| Active ingredient | 20.0% |
| Magnesium stearate | 0.5% |
| Crosscarmellose sodium | 2.0% |
| Lactose | 76.5% |
| PVP (polyvinylpyrrolidine) | 1.0% |

The ingredients are combined and granulated using a solvent such as methanol. The formulation is then dried and formed into tablets (containing about 20 mg of active compound) with an appropriate tablet machine.

### Composition for Oral Administration (C)

| **Ingredient** | **% wt./wt.** |
|---|---|
| Active compound | 1.0 g |
| Fumaric acid | 0.5 g |
| Sodium chloride | 2.0 g |
| Methyl paraben | 0.15 g |
| Propyl paraben | 0.05 g |
| Granulated sugar | 25.5 g |
| Sorbitol (70% solution) | 12.85 g |
| Veegum K (Vanderbilt Co.) | 1.0 g |
| Flavoring | 0.035 ml |
| Colorings | 0.5 mg, |
| Distilled water | q.s. to 100 ml |

The ingredients are mixed to form a suspension for oral administration.

### Parenteral Formulation (D)

| **Ingredient** | **% wt/wt.** |
|---|---|
| Active ingredient | 0.25 g |
| Sodium Chloride | qs to make isotonic |
| Water for injection to | 100 ml |

The active ingredient is dissolved in a portion of the water for injection. A sufficient quantity of sodium chloride is then added with stirring to make the solution isotonic. The solution is made up to weight with the remainder of the water for injection, filtered through a 0.2 micron membrane filter and packaged under sterile conditions.

### Suppository Formulation (E)

| **Ingredient** | **% wt./wt.** |
|---|---|
| Active ingredient | 1.0% |
| Polyethylene glycol 1000 | 74.5% |
| Polyethylene glycol, 4000 | 24.5% |

The ingredients are melted together and mixed on a steam bath, and poured into molds containing 2.5 g total weight.

### Topical Formulation (F)

| Ingredients | grams |
|---|---|
| Active compound | 0.2-2 |
| Span 60 | 2 |
| Tween 60 | 2 |
| Mineral oil | 5 |
| Petrolatum | 10 |
| Methyl paraben | 0.15 |
| Propyl paraben | 0.05 |
| BHA (butylated hydroxy | 0.01 |
| Water | q.s. 100 |

All of the ingredients, except water, are combined and heated to about 60°C with stirring. A sufficient quantity of water at about 60°C is then added with vigorous stirring to emulsify the ingredients, and water then added q.s. about 100 g.

The features disclosed in the foregoing description, or the following claims, expressed in their specific forms or in terms of a means for performing the disclosed function, or a method or process for attaining the disclosed result, as appropriate, may, separately, or in any combination of such features, be utilized for realizing the invention in diverse forms thereof.

## Claims

1. Use of compounds of formula I 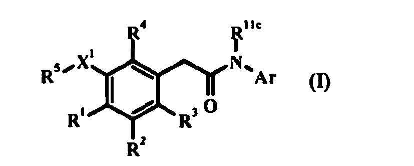 wherein
X¹ is selected from the group consisting of -O-, -S-, -CH₂-, -C(O)-;
R¹ and R² are each independently selected from the group consisting of hydrogen, C₁₋₆ alkyl, C₁₋₆ haloalkyl, C₃₋₈ cycloalkyl, C₁₋₆ alkoxy, C₁₋₆ alkylthio, C₁₋₆ alkylsulfinyl, C₁₋₆ sulfonyl, C₁₋₆ haloalkoxy, C₁₋₆ haloalkylthio, halogen, amino, alkylamino, dialkylamino, acylamino, nitro and cyano; or together R¹ and R² are -O-CH=CH- or -O-CH₂CH₂-;
R³ and R⁴ are independently selected from the group consisting of hydrogen, C₁₋₆ alkyl, C₁₋₆ haloalkyl, C₁₋₆ alkoxy, C₁₋₆ haloalkoxy C₁₋₆ alkylthio, C₁₋₆ haloalkylthio, halogen, amino, nitro and cyano;
R⁵ is selected from the group consisting of C₁₋₆ alkyl, C₁₋₆ haloalkyl, C₃₋₈ cycloalkyl, aryl or heteroaryl selected from the group consisting of pyridinyl, N-hydroxypyridine, pyrimidinyl, indole, pyrazinyl and pyrrolyl; wherein, said aryl and said heteroaryl are optionally substituted with one to three substituents independently selected from the group consisting of C₁₋₆ alkyl, C₂₋₆ alkenyl, C₂₋₆ alkynyl, C₁₋₆ haloalkyl, C₃₋₈ cycloalkyl, C₁₋₆ alkoxy, C₁₋₆ alkylthio, C₁₋₆ alkylsulfinyl, C₁₋₆ sulfonyl, C₁₋₆ haloalkoxy, C₁₋₆ haloalkylthio, hydroxy, halogen, amino, C₁₋₆ alkylamino, C₁₋₆ dialkylamino, acylamino, acyl, C₁₋₆ alkoxycarbonyl, carbamoyl, C₁₋₆ N- alkylcarbamoyl, C₁₋₆N,N-dialkylcarbamoyl, alkynol, -C≡CCH₂OH, -C≡CCH₂N(CH₃)₂, nitro and cyano, said alkyl and said cycloalkyl are optionally substituted with one or two substituents independently selected from the group consisting of alkyl, hydroxy, alkoxy, thiol, alkylthio, halogen, amino, alkylamino, amino alkyl, alkylaminoalkyl, and dialkylamino;
Ar is phenyl optionally substituted with 1 to 3 substituents independently selected in each incidence from the group consisting of C₁₋₆ alkyl, C₃₋₈ cycloalkyl, C₁₋₃ haloalkyl, C₁₋₆ alkoxy, C₁₋₆ haloalkoxy, halogen, cyano, C₁₋₆ alkylthio, C₁₋₆ alkylsulfinyl, C₁₋₆ sulfonyl, amino C₁₋₆ alkylsulfonyl, C₁₋₆ haloalkoxy, C₁₋₆ haloalkylthio, C₁₋₆ acyl, nitro, C₁₋₆ heteroalkyl, C₁₋₆ heteroalkoxy, hydroxyl, -X²(CH₂)ₚS(O)ₙNR⁸R⁹;-(CH₂)ₚCOOR¹¹,⁻-X²(CH₂)ₚNHC(O)NHR⁸R⁹, X²(CH₂)ₚCONR⁸R⁹,-SO₂R¹³,-NR^{8a}R^{9a}, X²(CH₂)ₚNR¹¹S(O)₂NR⁸R⁹, -X²(CH₂)ₚNHCOOR¹⁰, -X⁶(CH₂)ₚCOOR¹⁰, -X²(CH₂)ₚCN, -OR¹⁵ and C(=O)CH₂N[(CH₂)₂]₂X⁴; or,
heteroaryl ring selected from the group consisting of pyridinyl, pyridine N-oxide, pyridazinyl, pyrimidyl, pyrazyl, triazinyl, pyrrolyl, pyrazolyl, imidazolyl, (1,2,3,)- and (1,2,4)-triazolyl, pyrazinyl, pyrimidinyl, tetrazolyl, furyl, thienyl, isoxazolyl, thiazolyl, thienyl, isoxazolyl, indole, indole N-oxide, quinoline, quinoline N-oxide and oxazolyl said heteroaryl ring optionally substituted with one to three substituents selected from the group consisting of C₁₋₆ alkyl, C₁₋₆ alkoxy, C₁₋₆ alkylthio, halogen, C₁₋₆ aminoacyl cyano and NR^{8b}R^{9b};
R⁸ and R⁹ taken independently, one of R⁸ and R⁹ is hydrogen or C₁₋₆ alkyl and the other of R⁸ and R⁹ is selected from the group consisting of hydrogen, -C(=O)R¹⁴, -C(=O)CHR¹²NH₂, -(CH₂)₂N[(CH₂)₂]₂O, COCO₂Me, C₃₋₈ cycloalkyl said cycloalkyl optionally substituted with one or two hydroxyl substituents, pyranyl, C₁₋₆ alkyl and aryl said alkyl and said aryl groups optionally substituted with one or two substituents independently selected from the group consisting of hydroxy, C₁₋₆ alkoxy, thiol, C₁₋₆ alkylthio, C₁₋₆ alkylsulfinyl, C₁₋₆ sulfonyl, and halogen; or, (ii) R⁸ and R⁹ taken together are (CH₂)₂-X⁵-(CH₂)₂ or -(CH₂)ₒ- optionally substituted with one or two substituents selected from the group consisting of halogen, hydroxyl and NR^{11a}R^{11b};
R^{8a} and R^{9a} taken independently are selected from the group consisting of hydrogen, C₁₋₆ alkyl, C(=O)CO₂R¹¹ and SO₂R¹⁰, or (ii) taken together are (CH₂)ᵣSO₂, (CH₂)₂S(O)ₚ(CH₂)₂;
R¹⁰ is C₁₋₆ alkyl;
R¹¹ is hydrogen or C₁₋₆ alkyl;
R^{11a}, R^{11b} and R^{11c} are independently R¹¹;
R¹² is the side chain of a naturally occurring α-amino acid;
R¹³ is C₁₋₆ alkyl; -(CH₂)ₛCO₂R¹¹, -(CH₂)₂CN, -(CH₂)₂NH₂, -(CH₂)ᵤOH;
R¹⁴ is C₁₋₆ alkyl, -(CH₂)ₛNHR^{11a}R^{11b}, (CH₂)ₛOR¹¹, -CH₂CH(OH)CH₃, CH₂N[(CH₂)₂]₂O, -(CH₂)₂CO₂R¹¹, optionally substituted phenyl or pyridinyl;
R¹⁵ is C₁₋₆ alkyl optionally substituted with one to three hydroxyl groups;
X² is -O- or a bond;
X⁴ is -O- or -NMe-;
X⁵ is -O-, -S(O)ₙ- or NR¹¹;
X⁶ is -O- or -S(O)ₙ-;
n is an integer from 0 to 2;
o is an integer from 4 to 6;
p is an integer from 0 to 6;
r is an integer from 3 to 4
s is an integer from 1 to 2;
u is an integer from 2 to 3; and,
hydrates, solvates and salts thereof;
for the manufacture of a medicament for treating an HIV infection, or preventing an HIV infection, or treating AIDS or ARC.

2. Use of compounds of formula I according to claim 1 wherein
X¹ is selected from the group consisting of -O-, -S -, -CH₂-, -C(O)-;
R¹ and R² are each independently selected from the group consisting of hydrogen, C₁₋₆ alkyl, C₁₋₆ haloalkyl, C₃₋₈ cycloalkyl, C₁₋₆ alkoxy, C₁₋₆ alkylthio, C₁₋₆ alkylsulfinyl, C₁₋₆ sulfonyl, C₁₋₆ haloalkoxy, C₁₋₆ haloalkylthio, halogen, amino, alkylamino, dialkylamino, acylamino, nitro and cyano; or together R¹ and R² are -O-CH=CH- or -O-CH₂CH₂-;
R³ and R⁴ are independently selected from the group consisting of hydrogen, C₁₋₆ alkyl, C₁₋₆ haloalkyl, C₁₋₆ alkoxy, C₁₋₆ haloalkoxy, C₁₋₆ alkylthio, C₁₋₆ haloalkylthio, halogen, amino, nitro and cyano;
R⁵ is selected from the group consisting of C₁₋₆ alkyl, C₁₋₆ haloalkyl, C₃₋₈ cycloalkyl, aryl or heteroaryl selected from the group consisting of pyridinyl, N-hydroxypyridine, pyrimidinyl, indole, pyrazinyl and pyrrolyl; wherein, said aryl and said heteroaryl are optionally substituted with one to three substituents independently selected from the group consisting of C₁₋₆ alkyl, C₂₋₆ alkenyl, C₂₋₆ alkynyl, C₁₋₆ haloalkyl, C₃₋₈ cycloalkyl, C₁₋₆ alkoxy, C₁₋₆ alkylthio, C₁₋₆ alkylsulfinyl, C₁₋₆ sulfonyl, C₁₋₆ haloalkoxy, C₁₋₆ haloalkylthio, hydroxy, halogen, amino, C₁₋₆ alkylamino, C₁₋₆ dialkylamino, acylamino, acyl, C₁₋₆ alkoxycarbonyl, carbamoyl, C₁₋₆ N- alkylcarbamoyl, C₁₋₆N,N-dialkylcarbamoyl, nitro and cyano, said alkyl and said cycloalkyl are optionally substituted with one or two substituents independently selected from the group consisting of alkyl, hydroxy, alkoxy, thiol, alkylthio, halogen, amino, alkylamino, amino alkyl, alkylaminoalkyl, and dialkylamino;
Ar is phenyl optionally substituted with 1 to 3 substituents independently selected in each incidence from the group consisting of C₁₋₆ alkyl, C₃₋₈ cycloalkyl, C₁₋₃ haloalkyl, C₁₋₆ alkoxy, C₁₋₆ haloalkoxy, halogen, cyano, C₁₋₆ alkylthio, C₁₋₆ alkylsulfinyl,
C₁₋₆ sulfonyl, amino C₁₋₆ alkylsulfonyl, C₁₋₆ haloalkoxy, C₁₋₆ haloalkylthio, C₁₋₆ acyl, nitro, C₁₋₆ heteroalkyl, C₁₋₆ heteroalkoxy, hydroxyl, -X²(CH₂)ₚS(O)ₙNR⁸R⁹; -(CH₂)ₚCOOR¹¹₋,-X²(CH₂)ₚNHC(O)NHR⁸R⁹, X²(CH₂)ₚCONR⁸R⁹,-SO₂R¹³, -NR^{8a}R^{9a}, X²(CH₂)ₚNR¹¹S(O)₂NR⁸R⁹, -X²(CH₂)ₚNHCOOR¹⁰, -X⁶(CH₂)ₚCOOR¹⁰, -X²(CH₂)ₚCN, -OR¹⁵ and C(=O)CH₂N[(CH₂)₂]₂X⁴; or,
heteroaryl ring selected from the group consisting of pyridinyl, pyridine N-oxide, pyridazinyl, pyrimidyl, pyrazyl, triazinyl, pyrrolyl, pyrazolyl, imidazolyl, (1,2,3,)-and (1,2,4)-triazolyl, pyrazinyl, pyrimidinyl, tetrazolyl, furyl, thienyl, isoxazolyl, thiazolyl, thienyl, isoxazolyl, indole, indole N-oxide, quinoline, quinoline N-oxide and oxazolyl said heteroaryl ring optionally substituted with one to three substituents selected from the group consisting of C₁₋₆ alkyl, C₁₋₆ alkoxy, C₁₋₆ alkylthio, halogen, C₁₋₆ aminoacyl and NR^{8b}R^{9b};
R⁸ and R⁹ taken independently, one of R⁸ and R⁹ is hydrogen or C₁₋₆ alkyl and the other of R⁸ and R⁹ is selected from the group consisting of hydrogen, -C(=O)R¹⁴, -C(=O)CHR¹²NH₂, -(CH₂)₂N[(CH₂)₂]₂O, COCO₂Me, C₃₋₈ cycloalkyl said cycloalkyl optionally substituted with one or two hydroxyl substituents, pyranyl, C₁₋₆ alkyl and aryl said alkyl and said aryl groups optionally substituted with one or two substituents independently selected from the group consisting of hydroxy, C₁₋₆ alkoxy, thiol, C₁₋₆ alkylthio, C₁₋₆ alkylsulfinyl, C₁₋₆ sulfonyl, and halogen; or, (ii) R⁸ and R⁹ taken together are (CH₂)₂-X⁵-(CH₂)₂ or -(CH₂)ₒ- optionally substituted with one or two substituents selected from the group consisting of halogen, hydroxyl and NR^{11a}R^{11b};
R^{8a} and R^{9a} taken independently are selected from the group consisting of hydrogen, C₁₋₆ alkyl, C(=O)CO₂R¹¹ and SO₂R¹⁰, or (ii) taken together are (CH₂)ᵣSO₂, (CH₂)₂S(O)ₚ(CH₂)₂;
R¹⁰ is C₁₋₆ alkyl;
R¹¹ is hydrogen or C₁₋₆ alkyl;
R^{11a} R^{11b} and R^{11c} are independently R¹¹;
R¹² is the side chain of a naturally occurring α-amino acid;
R¹³ is C₁₋₆ alkyl; -(CH₂)ₛCO₂R¹¹, -(CH₂)₂CN, -(CH₂)₂NH₂, -(CH₂)ᵤOH;
R¹⁴ is C₁₋₆ alkyl, -(CH₂)ₛNHR^{11a}R^{11b}, (CH₂)ₛOR¹¹, -CH₂CH(OH)CH₃, CH₂N[(CH₂)₂]₂O, -(CH₂)₂CO₂R¹¹, optionally substituted phenyl or pyridinyl;
R¹⁵ is C₁₋₆ alkyl optionally substituted with one to three hydroxyl groups;
X² is -O- or a bond;
X⁴ is -O- or -NMe-;
X⁵ is -O-, -S(O)ₙ- or NR¹¹_{;}
X⁶ is O- or -S(O)ₙ-;
n is an integer from 0 to 2;
o is an integer from 4 to 6;
p is an integer from 0 to 6;
r is an integer from 3 to 4
s is an integer from 1 to 2;
u is an integer from 2 to 3; and,
hydrates, solvates and salts thereof;
for the manufacture of a medicament for treating an HIV infection, or preventing an HIV infection, or treating AIDS or ARC.

3. Use of compounds of formula I according to claim 1 or 2, wherein
X¹ is selected from the group consisting of -O-, -S -, -CH₂-, -C(O)-;
R¹ and R² are each independently selected from the group consisting of hydrogen, C₁₋₆ alkyl, C₁₋₆ alkoxy and halogen; or together R¹ and R² are -O-CH=CH- or -O-CH₂CH₂-;
R³ and R⁴ are independently selected from the group consisting of hydrogen and halogen;
R⁵ is aryl or heteroaryl selected from the group consisting of pyridinyl, N-hydroxypyridine, pyrimidinyl, indole, pyrazinyl and pyrrolyl; wherein, said aryl and said heteroaryl are unsubstituted or substituted with one to three substituents independently selected from the group consisting of C₁₋₆ alkyl, C₁₋₆ haloalkyl, C₁₋₆ haloalkoxy, C₁₋₆ haloalkoxy, halogen, -C≡CCH₂OH, -C≡CCH₂N(CH₃)₂ and cyano.
Ar is phenyl optionally substituted with 1 to 3 substituents independently selected from the group consisting of C₁₋₆ alkyl, C₃₋₈ cycloalkyl, C₁₋₃ haloalkyl, C₁₋₆ alkoxy, halogen, cyano, C₁₋₆ alkylsulfinyl, C₁₋₆ sulfonyl, amino C₁₋₆ alkylsulfonyl, C₁₋₆ haloalkoxy, C₁₋₆ haloalkylthio, C₁₋₆ acyl, nitro, C₁₋₆ heteroalkyl, C₁₋₆ heteroalkoxy, hydroxyl, -X²(CH₂)ₚS(O)ₙNR⁸R⁹; -(CH₂)ₚCOOR¹¹, -X²(CH₂)ₚNHC(O)NHR⁸R⁹, X²(CH₂)ₚCONR⁸R⁹,-SO₂R¹³ -NR^{8a}R^{9a}, -X²(CH₂)ₚNR¹¹S(O)₂NR⁸R⁹, -X²(CH₂)ₚNHCOOR¹⁰, -X⁶(CH₂)ₚCOOR¹⁰, -X²(CH₂)pCN, -OR¹⁵ and C(=O)CH₂N[(CH₂)₂]₂X⁴; or,
heteroaryl ring selected from the group consisting of pyridinyl, pyridine N-oxide, pyridazinyl, pyrimidyl, pyrazyl, triazinyl, pyrrolyl, pyrazolyl, imidazolyl, (1,2,3,)- and (1,2,4)-triazolyl, pyrazinyl, pyrimidinyl, tetrazolyl, furyl, thienyl, isoxazolyl, thiazolyl, thienyl, isoxazolyl, indole, indole N-oxide, quinoline, quinoline N-oxide and oxazolyl said heteroaryl ring optionally substituted with one to three substituents selected from the group consisting of C₁₋₆ alkyl, C₁₋₆alkoxy, C₁₋₆ alkylthio, halogen, C₁₋₆ aminoacyl, cyano and NR^{8b}R^{9b};
R⁸ and R⁹ taken independently, one of R⁸ and R⁹ is hydrogen or C₁₋₆ alkyl and the other of R⁸ and R⁹ is selected from the group consisting of hydrogen, -C(=O)R¹⁴, -C(=O)CHR¹²NH₂, -(CH₂)₂N[(CH₂)₂]₂O, COCO₂Me, C₃₋₈ cycloalkyl said cycloalkyl optionally substituted with one or two hydroxyl substituents, pyranyl, C₁₋₆ alkyl and aryl said alkyl and said aryl groups optionally substituted with one or two substituents independently selected from the group consisting of hydroxy, C₁₋₆ alkoxy, thiol, C₁₋₆ alkylthio, C₁₋₆ alkylsulfinyl, C₁₋₆ sulfonyl, and halogen; or, (ii) R⁸ and R⁹ taken together are (CH₂)₂-X⁵-(CH₂)₂ or -(CH₂)ₒ- optionally substituted with one or two substituents selected from the group consisting of halogen, hydroxyl and NR^{11a}R^{11b};
R^{8a} and R^{9a} taken independently are selected from the group consisting of hydrogen, C₁₋₆ alkyl, C(=O)CO₂R¹¹ and SO₂R¹⁰, or (ii) taken together are (CH₂)ᵣSO₂, (CH₂)₂5(O)ₚ(CH₂)₂;
R¹⁰ is C₁₋₆ alkyl;
R¹¹ is hydrogen or C₁₋₆ alkyl;
R^{11a}, R^{11b} and R^{11c} are independently R¹¹;
R¹² is the side chain of a naturally occurring α-amino acid;
R¹³ is C₁₋₆ alkyl; -(CH₂)ₛCO₂R¹¹, -(CH₂)₂CN, -(CH₂)₂NH₂, -(CH₂)ᵤOH;
R¹⁴ is C₁₋₆ alkyl, -(CH₂)ₛNHR^{11a}R^{11b}, (CH₂)ₛOR¹¹, -CH₂CH(OH)CH₃, CH₂N[(CH₂)₂]₂O, -(CH₂)₂CO₂R¹¹, optionally substituted phenyl or pyridinyl;
R¹⁵ is C₁₋₆ alkyl optionally substituted with one to three hydroxyl groups;
X² is -O- or a bond;
X⁴ is -O- or -NMe-;
X⁵ is -O-, -S(O)ₙ- or NR¹¹;
X⁶ is O- or -S(O)ₙ-;
n is an integer from 0 to 2;
o is an integer from 4 to 6;
p is an integer from 0 to 6;
r is an integer from 3 to 4
s is an integer from 1 to 2;
u is an integer from 2 to 3; and,
hydrates, solvates and salts thereof;
for the manufacture of a medicament for treating an HIV infection, or preventing an HIV infection, or treating AIDS or ARC.

4. Use of compounds of formula I 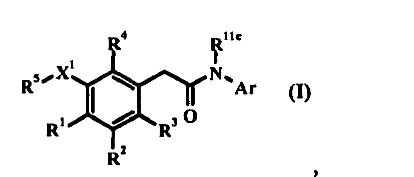 wherein
X¹ is selected from the group consisting of -O-, -S -, -CH₂-, -C(O)-;
R¹ and R² are each independently selected from the group consisting of hydrogen, methyl, ethyl, i-propyl, methoxy, chloro and bromo; or together R¹ and R² are -O-CH=CH- or -O-CH₂CH₂-;
R³ and R⁴ are independently selected from the group consisting of hydrogen and fluoro;
R⁵ is phenyl, wherein said phenyl is unsubstituted or substituted with one to three substituents independently selected from the group consisting of methyl, ethyl, CHF₂, CF₃, -CF₂CH₃, -OCHF₂, chloro, bromo, fluoro, -C≡CCH₂OH, -C≡CCH₂N(CH₃)₂ and cyano.
or pyridinyl, wherein said pyridinyl is unsubstituted or substituted with one to three substituents independently selected from the group consisting of methyl and cyano.
Ar is phenyl, wherein said phenyl is unsubstituted or substituted with 1 to 3 substituents selected from the group consisting of methyl, ethyl, i-propyl, 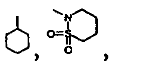 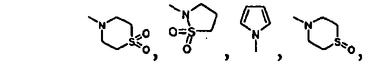 -CF₃, methoxy, chloro, fluoro, cyano, hydroxyl, -SCH₃, -SO₂NH -SO₂NHCH₃, -SO₂NCH₃CH₂CH₂OH, -SO₂NHCH₂CHCH₃OH, -SO₂NHCH₂CH₂CH₂OH, -SO₂NHCH₂CH₂ 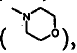 -SO₂ 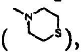 -SO₂N(CH₃)₂, -SO₂ 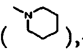 -SO₂ 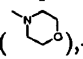 -SO₂ 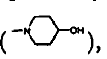 -SO₂NH 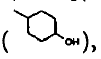 -SO₂ 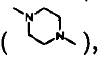 -SO₂ 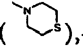 - SO₂NHCOCH₂CH₃, -SO₂NHCOCH₂CH₂CH₃, -SO₂NHCOCH(NH₂)CH(CH₃)₂, -SO₂NHCOCH₂ 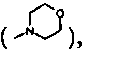 -SO₂NCH₃COCH₂CH₂CH₃, -SO₂NHCOCH₂CH₂CH₂CH₃, -SO₂NHCOCH₂CH(CH₃)₂, -SO₂NHCOCH₂CH(CH₂CH₃)₂, -SO₂NHCOCH₂CH₂CH(CH₃)₂, -SO₂NHCOCH₂CH₂NH₂, -SO₂NCH₃COCH₂CH₂NH₂, -SO₂NHCOCH₂OCH₃, -SO₂NHCO 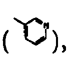 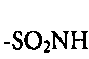 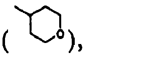 -SO₂NHCOCH(i-propyl)NH₂, -SO₂NHCH₂CH₂SCH₃, -SO₂NCH₃COCH₂CH₃, -SO₂NCH₃COCH₂OCH₃, -SO₂NCH₃COCH₂CH₂OCH₃, -SO₂NHCOCH₂OCH₃, -SO₂NHCOCH₂OH, -SO₂NHCOCH₂CH₂COOH, -SO₂CH₂CH₂NH₂, -SO₂CH₂COOH, -SO₂CH₂CH₂COOH, -SO₂CH₃, -SO₂CH₂CH₂CN, -SO₂CH₂CH₂CH₂OH, -O(CH₂)₃SO₂NH₂, -OCH₂COOH, -OCH₂COOCH₃, -OCH₂CN, -OCH₂CHOHCH₂OH, -OCH₂CH₂CHOHCH₂OH, -SCH₂COOCH₃, -COOCH₃, -COOH, -COCH₂ 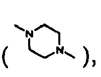 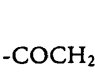 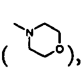 -CH₂COOCH₃, -CH₂COOH, -CH₂CH₂COOCH₃, CH₂CH₂CH₂COOCH₃, -N(CH₃)₂, -NHCOCOOCH₂CH₃, -NHSO₂CH₃ and -NCH₃SO₂CH₃; or
heteroaryl ring selected from the group consisting of pyridinyl, pyrazolyl and triazolyl said heteroaryl is unsubstituted or substituted with one or two substituents selected from the group consisting of methyl, methoxy, -SCH₃, and -NHCOOCH₃;
R^{11c} is hydrogen or methyl; and
Hydrates, solvates and salts thereof.

5. Use of compound of formula I according to claim 1 or 4, which compound is
2-[4-Chloro-3-(3-chloro-5-cyano-phenoxy)-phenyl]-N-(4-sulfamoyl-phenyl)-acetamide;
2- [4-Chloro-3-(3,5-dicyano-phenoxy)-phenyl]-N-(4-sulfamoyl-phenyl)-acetamide;
2-[3-(2-Bromo-5-chloro-phenoxy)-4-chloro-phenyl]-N-[2-methyl-4-(3-sulfamoyl-propoxy)-phenyl] -acetamide;
2-[3-(2-Bromo-5-chloro-phenoxy)-4-chloro-phenyl]-N-(4-sulfamoyl-phenyl)-acetamide;
2- [4-Chloro-3- (3,5-dicyano-phenoxy)-phenyl] - N-(2-chloro-phenyl)-acetamide;
2-[4-Chloro-3-(3,5-dicyano-phenoxy)-phenyl]-N-(4-dimethylamino-phenyl)-acetamide;
2- [3-(2-Chloro-5-cyano-phenoxy)-4-ethyl-2-fluoro-phenyl]-N-(4-sulfamoyl-phenyl)-acetamide;
2-[4-Chloro-3-(2,5-dicyano-phenoxy)-2-fluoro-phenyl]-N-(4-sulfamoyl-phenyl)-acetamide;
2-[4-Chloro-3-(3,5-dicyano-phenoxy)-2-fluoro-phenyl]-N-(4-sulfamoyl-phenyl)-acetamide;
2-[4-Chloro-2-fluoro-3-(2,3,5-trichloro-phenoxy)-phenyl]-N-(4-sulfamoyl-phenyl)-acetamide;
2- [3-(3-Cyano-5-fluoro-phenoxy)-4-methyl-phenyl] -N-(4-sulfamoyl-phenyl)-acetamide;
N-(2-Chloro-phenyl)-2-[3-(3-cyano-5-fluoro-phenoxy)-4-methyl-phenyl]-acetamide;
2- [4-Chloro-3-(3-cyano-5-difluoromethyl-phenoxy)-2-fluoro-phenyl] -N-(4-sulfamoyl-phenyl)-acetamide;
2- [4-Chloro-3-(3-cyano-5-difluoromethyl-phenoxy) -2-fluoro-phenyl] -N-(2-chloro-phenyl)-acetamide;
2-[3-(2-Bromo-5-chloro-phenoxy)-4-ethyl-phenyl]-N-(4-sulfamoyl-phenyl)-acetamide;
2-[3-(2-Bromo-5-chloro-phenoxy)-4-ethyl-phenyl]-N-(2-chloro-phenyl)-acetamide;
2-[3-(5-Chloro-2-cyano-phenoxy)-4-ethyl-phenyl]-N-(4-sulfamoyl-phenyl)-acetamide;
2-[3-(5-Chloro-2-cyano-phenoxy)-4-ethyl-phenyl]-N-(2-chloro-phenyl)-acetamide;
2-[3-(2,5-Dichloro-phenoxy)-4-ethyl-phenyl]-N-(4-sulfamoyl-phenyl)-acetamide;
2-[3-(2,6-Dichloro-phenoxy)-4-ethyl-phenyl]-N-(4-sulfamoyl-phenyl)-acetamide;
N-(2-Chloro-phenyl)-2-[3-(2,5-dichloro-phenoxy)-4-ethyl-phenyl]-acetamide;
2-[3-(3-Bromo-5-chloro-phenoxy)-4-ethyl-phenyl]-N-(4-sulfamoyl-phenyl)-acetamide;
N-(2-Chloro-phenyl)-2-[3-(2,6-dichloro-phenoxy)-4-ethyl-phenyl]-acetamide;
2-[3-(3-Bromo-5-chloro-phenoxy)-4-ethyl-phenyl]-N-(2-chloro-phenyl)-acetamide;
N-(2-Chloro-phenyl)-2-[3-(3,5-dicyano-phenoxy)-4-ethyl-phenyl]-acetamide;
2-[3-(2-Bromo-5-chloro-phenoxy)-4-isopropyl-phenyl]-N-(4-sulfamoyl-phenyl)-acetamide;
2-[3-(2-Bromo-5-chloro-phenoxy)-4-isopropyl-phenyl]-N-(2-chloro-phenyl)-acetamide;
2-[4-Chloro-3-(3,5-dicyano-phenoxy)-2-fluoro-phenyl]-N-(2-chloro-phenyl)-acetamide;
2- [4-Chloro-3-(3,5-dicyano-phenoxy)-phenyl]-N-[2-methyl-4-(3-sulfamoyl-propoxy)-phenyl]-acetamide;
2-[3-(2-Bromo-5-chloro-phenoxy)-4-methyl-phenyl]-N-(4-sulfamoyl-phenyl)-acetamide;
2-[3-(2-Bromo-5-chloro-phenoxy)-4-methyl-phenyl]-N-(2-methyl-4-sulfamoyl-phenyl)-acetamide;
2-[4-Chloro-3-(3,5-dicyano-phenoxy)-2-fluoro-phenyl]-N-(2-methyl-4-sulfamoyl-phenyl)-acetamide;
2-[3-(2-Bromo-5-chloro-phenoxy)-4-methyl-phenyl]-N-(2-chloro-phenyl)-acetamide;
N-(2-Chloro-phenyl)-2-[3-(3,5-dicyano-phenoxy)-4-methyl-phenyl]-acetamide;
2-[3-(3-Bromo-2,5-dichloro-phenoxy)-4-ethyl-phenyl]-N-(4-sulfamoyl-phenyl)-acetamide;
2-[3-(3-Bromo-2,5-dichloro-phenoxy)-4-ethyl-phenyl]-N-(2-methyl-4-sulfamoyl-phenyl)-acetamide;
2-[3-(3-Bromo-2,5-dichloro-phenoxy)-4-ethyl-phenyl]-N-(2-chloro-phenyl)-acetamide;
N-(2-Chloro-phenyl)-2-[3-(3,5-dibromo-2-chloro-phenoxy)-4-ethyl-phenyl]-acetamide;
2-[3-(5-Bromo-2-chloro-3-fluoro-phenoxy)-4-ethyl-phenyl] -N-(4-sulfamoyl-phenyl)-acetamide;
2-[3-(5-Bromo-2-chloro-3-fluoro-phenoxy)-4-ethyl-phenyl]-N-(2-methyl-4-sulfamoyl-phenyl)-acetamide;
2-[3-(5-Bromo-2-chloro-3-fluoro-phenoxy)-4-ethyl-phenyl]-N-(2-chloro-phenyl)-acetamide;
2-[4-Chloro-3-(3-chloro-5-cyano-phenoxy)-2-fluoro-phenyl]-N-(2-methyl-4-sulfamoyl-phenyl)-acetamide;
2-[3-(3,5-Dicyano-phenoxy)-4-methyl-phenyl]-N-(2-methyl-4-sulfamoyl-phenyl)-acetamide;
N-(2-Chloro-4-sulfamoyl-phenyl)-2-[3-(3,5-dicyano-phenoxy)-4-methyl-phenyl]-acetamide;
2-[3-(3-Chloro-5-cyano-phenoxy)-4-ethyl-phenyl]-N-(4-sulfamoyl-phenyl)-acetamide;
2- [3-(3-Chloro-5-cyano-phenoxy)-4-ethyl-phenyl] -N-(2-methyl-4-sulfamoyl-phenyl)-acetamide;
2-[3-(3-chloro-5-cyano-phenoxy)-4-ethyl-phenyl]-N-(2-chloro-4-sulfamoyl-phenyl)-acetamide;
2-[3-(3-Chloro-5-cyano-phenoxy)-4-ethyl-phenyl]-N-(4-methylsulfamoyl-phenyl)-acetamide;
2-[3-(2-Chloro-5-cyano-3-fluoro-phenoxy)-4-ethyl-phenyl]-N-(4-sulfamoyl-phenyl)-acetamide;
2-[3-(2-Chloro-5-cyano-3-fluoro-phenoxy)-4-ethyl-phenyl]-N-(2-methyl-4-sulfamoyl-phenyl)-acetamide;
2-[4-Chloro-3-(3-chloro-5-cyano-phenoxy)-2-fluoro-phenyl] -N-(2-methyl-4-propionylsulfamoyl-phenyl)-acetamide;
2-[3-(2-Chloro-3,5-dicyano-phenoxy)-4-ethyl-phenyl]-N-(4-sulfamoyl-phenyl)-acetamide;
2-[3-(2-Chloro-3,5-dicyano-phenoxy)-4-ethyl-phenyl]-N-(2-methyl-4-sulfamoyl-phenyl)-acetamide;
2-[3-(2-Chloro-3,5-dicyano-phenoxy)-4-ethyl-phenyl]-N-(2-chloro-4-sulfamoyl-phenyl)-acetamide;
2-[4-Chloro-3-(4-cyano-2,6-dimethyl-phenoxy)-phenyl]-N-(4-sulfamoyl-phenyl)-acetamide;
2- [4-Chloro-3-(4-cyano-2,6-dimethyl-phenoxy)-phenyl] -N-(2-methyl-4-sulfamoyl-phenyl)-acetamide;
2-[3-(3-Chloro-5-cyano-phenoxy)-4-methoxy-phenyl]-N-(4-sulfamoyl-phenyl)-acetamide;
2-[3-(3-Chloro-5-cyano-phenoxy)-4-ethyl-phenyl]-N-(2-chloro-phenyl)-acetamide;
2-[3-(2-Chloro-5-cyano-3-fluoro-phenoxy)-4-ethyl-phenyl]-N-(2-chloro-phenyl)-acetamide;
2-[3-(2-Chloro-3,5-dicyano-phenoxy)-4-ethyl-phenyl]-N-(2-chloro-phenyl)-acetamide;
2-[3-(3-Chloro-5-cyano-phenoxy)-4-methoxy-phenyl]-N-(2-chloro-phenyl)-acetamide;
2-[3-(2-Chloro-5-cyano-phenoxy)-4-methoxy-phenyl]-N-(2-chloro-phenyl)-acetamide;
2-[4-Bromo-3-(3-chloro-5-cyano-phenoxy)-2-fluoro-phenyl]-N-(2-chloro-4-propionylsulfamoyl-phenyl)-acetamide; sodium salt;
2-[3-(3-Chloro-5-cyano-phenoxy)-4-methoxy-phenyl]-N-(2-chloro-4-sulfamoyl-phenyl)-acetamide;
2-[3-(2-Chloro-5-cyano-phenoxy)-4-methoxy-phenyl]-N-(4-sulfamoyl-phenyl)-acetamide; .
2-[3-(2-Chloro-5-cyano-phenoxy)-4-methoxy-phenyl]-N-(2-methyl-4-sulfamoyl-phenyl) -acetamide;
2-[3-(2-Chloro-5-cyano-phenoxy)-4-methoxy-phenyl]-N-(2-chloro-4-sulfamoyl-phenyl)-acetamide;
2-[4-Chloro-3-(4-cyano-2,6-dimethyl-phenoxy)-phenyl]-N-(2-chloro-phenyl)-acetamide;
2-[4-Chloro-3-(3,5-dicyano-phenoxy)-phenyl]-N-(4-methylsulfamoyl-phenyl)-acetamide;
2- [4-Chloro-3-(3,5-dicyano-phenoxy)-phenyl]-N-(4-dimethylsulfamoyl-phenyl)-acetamide;
2-[4-Chloro-3-(3,5-dicyano-phenoxy)-phenyl]-N-[4-(piperidine-1-sulfonyl)-phenyl]-acetamide;
2-[4-Chloro-3-(3,5-dicyano-phenoxy)-phenyl]-N-[4-(morpholine-4-sulfonyl)-phenyl]-acetamide;
2-[3-(3-Chloro-5-cyano-phenoxy)-4-methoxy-phenyl]-N-(2-methyl-4-sulfamoyl-phenyl)-acetamide;
2-[3-(3-Chloro-5-cyano-phenoxy)-2-fluoro-4-methyl-phenyl]-N-(2-methyl-4-sulfamoyl-phenyl)-acetamide;
2-[4-Chloro-3-(3-cyano-5-difluoromethyl-phenoxy)-2-fluoro-phenyl]-N-(2-methyl-4-sulfamoyl-phenyl)-acetamide;
2-[4-Chloro-3-(3,5-dicyano-phenoxy)-phenyl]-N-(2-chloro-4-methanesulfonyl-phenyl)-acetamide;
N-[4-((S)-2-Amino-3-methyl-butyrylsulfamoyl)-2-methyl-phenyl]-2-[4-chloro-3-(3-chloro-5-cyano-phenoxy)-2-fluoro-phenyl]-acetamide; compound with hydrochloric acid;
2-[4-Chloro-3-(3,5-dicyano-phenoxy)-phenyl]-N-(4-cyanomethoxy-phenyl)-acetamide;
2-[4-Chloro-3-(3,5-dicyano-phenoxy)-phenyl]-N-(4-methanesulfonyl-phenyl)-acetamide;
3-Chloro-4-{2-[4-chloro-3-(3,5-dicyano-phenoxy)-phenyl]-acetylamino}-benzoic acid methyl ester;
2-[4-Chloro-3-(3,5-dicyano-phenoxy)-phenyl]-N-{4-[(2-hydroxy-ethyl)-methyl-sulfamoyl] -phenyl}-acetamide;
2-[4-Chloro-3-(3,5-dicyano-phenoxy)-phenyl]-N-[4-(4-hydroxy-piperidine-1-sulfonyl)-phenyl] -acetamide;
2-[3-(3-Chloro-5-cyano-phenoxy)-2-fluoro-4-methyl-phenyl]-N-(2-chloro-4-sulfamoyl-phenyl)-acetamide;
N-(2-Chloro-4-sulfamoyl-phenyl)-2-[3-(3-cyano-5-difluoromethyl-phenoxy)-2-fluoro-4-methyl-phenyl] -acetamide;
2-[3-(3-Cyano-5-difluoromethyl-phenoxy)-2-fluoro-4-methyl-phenyl]-N-(2-methyl-4-sulfamoyl-phenyl)-acetamide;
N-(2-Chloro-phenyl)-2-[3-(3-cyano-5-difluoromethyl-phenoxy)-2-fluoro-4-methyl-phenyl] -acetamide;
2-[4-Chloro-3-(3-chloro-5-cyano-phenoxy)-2-fluoro-phenyl]-N-{2-methyl-4-[(pyridine-3-carbonyl)-sulfamoyl]-phenyl}-acetamide; compound with hydrochloric acid;
2- [4-Chloro-3-(3-cyano-5-trifluoromethyl-phenoxy)-phenyl]-N-(4-sulfamoyl-phenyl)-acetamide;
2-[4-Chloro-3-(3-cyano-5-trifluoromethyl-phenoxy)-phenyl]-N-(2-methyl-4-sulfamoyl-phenyl)-acetamide;
2-[4-Chloro-3-(3-cyano-5-trifluoromethyl-phenoxy)-phenyl]-N-(2-chloro-4-sulfamoyl-phenyl)-acetamide;
2-[3-(3-Cyano-5-difluoromethyl-phenoxy)-4-ethyl-phenyl]-N-(4-sulfamoyl-phenyl)-acetamide;
2-[3-(3-Cyano-5-difluoromethyl-phenoxy)-4-methoxy-phenyl]-N-(4-sulfamoyl-phenyl)-acetamide;
2-[3-(3-Cyano-5-difluoromethyl-phenoxy)-4-methoxy-phenyl]-N-(2-methyl-4-sulfamoyl-phenyl)-acetamide;
N-(2-Chloro-4-sulfamoyl-phenyl)-2-[3-(3-cyano-5-difluoromethyl-phenoxy)-4-methoxy-phenyl]-acetamide;
2-{4-Chloro-3-[3-cyano-5-(1,1-difluoro-ethyl)-phenoxy]-2-fluoro-phenyl}-N-(4-sulfamoyl-phenyl)-acetamide;
2-{4-Chloro-3-[3-cyano-5-(1,1-difluoro-ethyl)-phenoxy]-2-fluoro-phenyl}-N-(2-methyl-4-sulfamoyl-phenyl)-acetamide;
2-{4-Chloro-3-[3-cyano-5-(1,1-difluoro-ethyl)-phenoxy]-2-fluoro-phenyl}-N-(2-chloro-4-sulfamoyl-phenyl)-acetamide;
2-[4-Chloro-3-(3-chloro-5-cyano-phenoxy)-2-fluoro-phenyl]-N-(2-chloro-phenyl)-acetamide;
2-[4-Chloro-3-(3-chloro-5-cyano-phenoxy)-2-fluoro-phenyl]-N-[2-methyl-4-(methylpropionyl-sulfamoyl)-phenyl]-acetamide;
2-[4-Chloro-3-(3-chloro-5-cyano-phenoxy)-2-fluoro-phenyl]-N-(2-chloro-4-sulfamoyl-phenyl)-acetamide;
2-[3-(3-Cyano-5-difluoromethyl-phenoxy)-4-ethyl-phenyl]-N-(2-methyl-4-sulfamoyl-phenyl)-acetamide;
2-[3-(2-Chloro-5-cyano-phenoxy)-5-methoxy-phenyl]-N-(4-sulfamoyl-phenyl)-acetamide;
2-[3-(2-Chloro-5-cyano-phenoxy)-5-methoxy-phenyl]-N-(2-methyl-4-sulfamoyl-phenyl)-acetamide;
2-[4,5-Dibromo-3-(3-cyano-5-difluoromethyl-phenoxy)-2-fluoro-phenyl]-N-(2-methyl-4-sulfamoyl-phenyl)-acetamide;
2-[3-(2-Chloro-5-cyano-phenoxy)-5-ethyl-phenyl]-N-(4-sulfamoyl-phenyl)-acetamide;
2-[3-(2-Chloro-5-cyano-phenoxy)-5-ethyl-phenyl]-N-(2-methyl-4-sulfamoyl-phenyl)-acetamide;
2- [3-(2-Chloro-5-cyano-phenoxy)-5-ethyl-phenyl] -N-(2-chloro-4-sulfamoyl-phenyl)-acetamide;
2- [4-Chloro-3-(3-chloro-5-cyano-phenoxy)-2-fluoro-phenyl] -N-(2-methyl-4-methylsulfamoyl-phenyl)-acetamide;
2-[4-Bromo-3-(3-chloro-5-cyano-phenoxy)-2-fluoro-phenyl]-N-(2-chloro-4-sulfamoyl-phenyl)-acetamide;
3-Chloro-4-{2-[3-(3-cyano-5-difluoromethyl-phenoxy)-2-fluoro-4-methyl-phenyl]-acetylamino}-benzoic acid;
2-[4-Chloro-3-(3-chloro-5-cyano-phenoxy)-2-fluoro-phenyl]-N-(2-chloro-phenyl)-N-methyl-acetamide;
N-(2-Chloro-4-methanesulfonyl-phenyl)-2-[3-(3-cyano-5-difluoromethyl-phenoxy)-2-fluoro-4-methyl-phenyl]-acetamide;
2-[3-(2-Chloro-5-cyano-phenoxy)-5-methoxy-phenyl]-N-(2-chloro-4-sulfamoyl-phenyl)-acetamide;
2-[4,5-Dibromo-3-(3-cyano-5-difluoromethyl-phenoxy)-2-fluoro-phenyl]-N-(4-sulfamoyl-phenyl)-acetamide;
2-[3-(3-Chloro-5-cyano-phenoxy)-4-methyl-phenyl]-N-[2-methyl-4-(morpholine-4-sulfonyl)-phenyl] -acetamide;
2-[3-(3-Chloro-5-cyano-phenoxy)-4-methyl-phenyl]-N-[2-methyl-4-(4-methyl-piperazine-1-sulfonyl)-phenyl]-acetamide;
2-[3-(3-Chloro-5-cyano-phenoxy)-4-methyl-phenyl]-N-[2-methyl-4-(thiomorpholine-4-sulfonyl)-phenyl]-acetamide;
2-[3-(3-Chloro-5-cyano-phenoxy)-4-methyl-phenyl]-N-[2-methyl-4-(2-morpholin-4-yl-ethylsulfamoyl)-phenyl]-acetamide;
2-[3-(3-Chloro-5-cyano-phenoxy)-4-methyl-phenyl]-N-[4-(3-hydroxy-propylsulfamoyl)-2-methyl-phenyl]-acetamide;
2-[4-Chloro-3-(3-cyano-5-trifluoromethyl-phenoxy)-2-fluoro-phenyl] -N-(2-methyl-4-sulfamoyl-phenyl)-acetamide;
2-[3-(3-Chloro-5-cyano-phenoxy)-4-methyl-phenyl] -N-[4-(4-hydroxy-cyclohexylsulfamoyl)-2-methyl-phenyl]-acetamide;
2-[3-(3-Chloro-5-cyano-phenoxy)-4-methyl-phenyl]-N-(2-methyl-4-sulfamoyl-phenyl)-acetamide;
2-[3-(3-Chloro-5-cyano-phenoxy)-4-methyl-phenyl]-N-(2-chloro-4-sulfamoyl-phenyl)-acetamide;
3-(4-{2-[3-(3-Chloro-5-cyano-phenoxy)-4-methyl-phenyl]-acetylamino}-3-methyl-benzenesulfonyl)-propionic acid;
2-[4-Chloro-3-(3-chloro-5-cyano-phenoxy)-2-fluoro-phenyl]-N-(2-chloro-4-propionylsulfamoyl-phenyl)-acetamide; sodium salt;
N-[4-((S)-2-Amino-3-methyl-butyrylsulfamoyl)-2-chloro-phenyl]-2-[4-chloro-3-(3-chloro-5-cyano-phenoxy)-2-fluoro-phenyl]-acetamide; sodium salt;
2-[3-(3-Chloro-5-cyano-phenoxy)-4-methyl-phenyl]-N-[2-methyl-4-(tetrahydro-pyran-4-ylsulfamoyl)-phenyl]-acetamide;
2-[3-(3-Chloro-5-cyano-phenoxy)-4-methyl-phenyl]-N-[4-(2-hydroxy-propylsulfamoyl)-2-methyl-phenyl]-acetamide;
2-[3-(3-Chloro-5-cyano-phenoxy)-4-methyl-phenyl]-N-[2-methyl-4-(2-methylsulfanyl-ethylsulfamoyl)-phenyl]-acetamide;
2-[3-(3-Chloro-5-cyano-phenoxy)-4-methyl-phenyl]-N-[2-methyl-4-(3-methylsulfanyl-propylsulfamoyl)-phenyl] -acetamide;
2-[3-(3-Chloro-5-cyano-phenoxy)-5-methoxy-phenyl]-N-(4-sulfamoyl-phenyl)-acetamide;
2-[3-(3-Chloro-5-cyano-phenoxy)-5-methoxy-phenyl]-N-(2-methyl-4-sulfamoyl-phenyl)-acetamide;
N-(2-Chloro-4-sulfamoyl-phenyl)-2-[4,5-dibromo-3-(3-cyano-5-difluoromethyl-phenoxy)-2-fluoro-phenyl]-acetamide;
2-[4-Chloro-3-(4-cyano-2,6-dimethyl-phenoxy)-phenyl]-N-(2-chloro-4-sulfamoyl-phenyl)-acetamide;
2- [3-(3-Chloro-5-cyano-phenoxy)-5-methoxy-phenyl] -N-(2-chloro-4-sulfamoyl-phenyl)-acetamide;
2-[3-(3-Chloro-5-cyano-phenoxy)-4-methyl-phenyl]-N-[4-(2-cyano-ethanesulfonyl)-2-methyl-phenyl] -acetamide;
N-(4-Butyrylsulfamoyl-2-chloro-phenyl)-2-[4-chloro-3-(3-chloro-5-cyano-phenoxy)-2-fluoro-phenyl]-acetamide; sodium salt;
N-[4-(Butyryl-methyl-sulfamoyl)-2-chloro-phenyl]-2-[4-chloro-3-(3-chloro-5-cyano-phenoxy)-2-fluoro-phenyl]-acetamide;
N-(2-Chloro-4-sulfamoyl-phenyl)-2-[3-(3-cyano-5-difluoromethyl-phenoxy)-4-ethylphenyl]-acetamide;
2-[3-(3-Chloro-5-cyano-phenoxy)-5-methyl-phenyl]-N-(4-sulfamoyl-phenyl)-acetamide;
2-[3-(3-Chloro-5-cyano-phenoxy)-5-methyl-phenyl]-N-(2-methyl-4-sulfamoyl-phenyl)-acetamide;
2-[3-(3-Chloro-5-cyano-phenoxy)-5-methyl-phenyl]-N-(2-chloro-4-sulfamoyl-phenyl)-acetamide;
2-[7-(3-Cyano-phenoxy)-benzofuran-5-yl]-N-(4-sulfamoyl-phenyl)-acetamide;
2-[7-(3-Cyano-phenoxy)-benzofuran-5-yl]-N-(2-methyl-4-sulfamoyl-phenyl)-acetamide;
2-[3-(2-Chloro-5-cyano-phenoxy)-5-methyl-phenyl]-N-(4-sulfamoyl-phenyl)-acetamide;
2-[3-(2-Chloro-5-cyano-phenoxy)-5-methyl-phenyl]-N-(2-methyl-4-sulfamoyl-phenyl)-acetamide;
2-[3-(2-Chloro-5-cyano-phenoxy)-5-methyl-phenyl]-N-(2-chloro-4-sulfamoyl-phenyl)-acetamide;
N-(2-Chloro-4-sulfamoyl-phenyl)-2-[7-(3,5-dichloro-phenoxy)-benzofuran-5-yl]-acetamide;
2-[3-(3-Chloro-5-cyano-phenoxy)-4-methyl-phenyl]-N-[4-((S)-2,3-dihydroxy-propoxy)-2-methyl-phenyl]-acetamide;
2-[3-(3-Chloro-5-cyano-phenoxy)-4-methyl-phenyl]-N-[4-((R)-3,4-dihydroxy-butoxy)-2-methyl-phenyl] -acetamide;
(4-{2-[3-(3-Chloro-5-cyano-phenoxy)-4-methyl-phenyl]-acetylamino}-3-methyl-benzenesulfonyl)-acetic acid;
N-[4-(3-Amino-propionylsulfamoyl)-2-chloro-phenyl]-2-[4-chloro-3-(3-chloro-5-cyano-phenoxy)-2-fluoro-phenyl]-acetamide; compound with hydrochloric acid;
N-[4-(2-Amino-ethanesulfonyl)-2-methyl-phenyl]-2-[3-(3-chloro-5-cyano-phenoxy)-4-methyl-phenyl] -acetamide;
2-[4-Chloro-3-(3-chloro-5-cyano-phenoxy)-2-fluoro-phenyl]-N-[2-chloro-4-(2-methoxy-acetylsulfamoyl)-phenyl]-acetamide; sodium salt;
2-[4-Chloro-3-(3-chloro-5-cyano-phenoxy)-2-fluoro-phenyl]-N-[2-chloro-4-(2-hydroxy-acetylsulfamoyl)-phenyl]-acetamide; sodium salt;
2-[3-(3-Chloro-5-cyano-phenoxy)-4-methyl-phenyl]-N-[4-(3-hydroxy-propane-1-sulfonyl)-2-methyl-phenyl]-acetamide;
2-[3-(3-Chloro-5-cyano-phenoxy)-4-methyl-phenyl]-N-[4-(1,1-dioxo-1λ6-[1,2] thiazinan-2-yl)-2-methyl-phenyl]-acetamide;
2-[3-(3-Chloro-5-cyano-phenoxy)-4-methyl-phenyl]-N-{2-methyl-4-[2-(4-methyl-piperazin-1-yl)-acetyl]-phenyl}-acetamide;
2-[3-(3-Chloro-5-cyano-phenoxy)-4-methyl-phenyl]-N-[2-methyl-4-(1-oxo-1λ4-thiomorpholin-4-yl)-phenyl] -acetamide;
2-[3-(3-Chloro-5-cyano-phenoxy)-4-methyl-phenyl]-N-[4-(1,1-dioxo-1λ6-thiomorpholin-4-yl)-2-methyl-phenyl]-acetamide;
2-[4-Chloro-3-(3-chloro-5-cyano-phenoxy)-2-fluoro-phenyl]-N-[2-chloro-4-(methylpropionyl-sulfamoyl)-phenyl] -acetamide;
2-[3-(3-Chloro-5-cyano-phenoxy)-4-methyl-phenyl]-N-[2-methyl-4-(2-morpholin-4-yl-acetyl)-phenyl]-acetamide; compound with trifluoro-acetic acid;
2-[4-Chloro-3-(3-chloro-5-cyano-phenoxy)-2-fluoro-phenyl]-N-[2-chloro-4-(3-methoxy-propionylsulfamoyl)-phenyl]-acetamide; sodium salt;
2-[4-Bromo-3-(3-cyano-5-difluoromethyl-phenoxy)-2-fluoro-phenyl]-N-(2-chloro-4-sulfamoyl-phenyl)-acetamide;
2-[4-Chloro-3-(3-cyano-5-difluoromethoxy-phenoxy)-2-fluoro-phenyl]-N-(4-sulfamoyl-phenyl)-acetamide;
2-[4-Chloro-3-(3-cyano-5-difluoromethoxy-phenoxy)-2-fluoro-phenyl]-N-(2-chloro-4-sulfamoyl-phenyl)-acetamide;
2-[4-Chloro-3-(3-chloro-5-cyano-phenoxy)-2-fluoro-phenyl]-N-(2-chloro-4-methylsulfamoyl-phenyl)-acetamide;
2-[4-Bromo-3-(3-cyano-5-difluoromethyl-phenoxy)-2-fluoro-phenyl]-N-(2-methyl-4-sulfamoyl-phenyl)-acetamide;
2-[3-(3-Chloro-5-cyano-phenoxy)-4-methyl-phenyl]-N-[4-(1,1-dioxo-1λ6-isothiazolidin-2-yl)-2-methyl-phenyl]-acetamide;
2-[4-Chloro-3-(3-chloro-5-cyano-phenoxy)-2-fluoro-phenyl]-N-{2-chloro-4-[(2-methoxy-acetyl)-methyl-sulfamoyl]-phenyl}-acetamide;
2-[4-Chloro-3-(3-chloro-5-cyano-phenoxy)-2-fluoro-phenyl]-N-{ 2-chloro-4- [(3-methoxy-propionyl)-methyl-sulfamoyl]-phenyl}-acetamide;
2-[4-Chloro-3-(3-chloro-5-cyano-phenoxy)-2-fluoro-phenyl]-N-(2-chloro-4-pentanoylsulfamoyl-phenyl)-acetamide; sodium salt;
2-[4-Chloro-3-(3-chloro-5-cyano-phenoxy)-2-fluoro-phenyl]-N-[2-chloro-4-(3-methyl-butyrylsulfamoyl)-phenyl]-acetamide; sodium salt;
2-[4-Chloro-3-(3-chloro-5-cyano-phenoxy)-2-fluoro-phenyl]-N-[2-chloro-4-(2-diethylamino-acetylsulfamoyl)-phenyl] -acetamide; compound with hydrochloric acid;
2-[4-Chloro-3-(3-chloro-5-cyano-phenoxy)-2-fluoro-phenyl]-N-[2-chloro-4-(4-methyl-pentanoylsulfamoyl)-phenyl]-acetamide; sodium salt;
2-[4-Chloro-3-(3-chloro-5-cyano-phenoxy)-2-fluoro-phenyl]-N-[2-chloro-4-(2-morpholin-4-yl-acetylsulfamoyl)-phenyl]-acetamide; compound with hydrochloric acid;
N-(4-{ 2- [3-(3-Chloro-5-cyano-phenoxy)-4-methyl-phenyl] -acetylamino }-3-methyl-phenyl)-oxalamic acid ethyl ester;
N-(2-Chloro-4-sulfamoyl-phenyl)-2-[3-(3-cyano-5-difluoromethyl-phenoxy)-4-ethyl-2-fluoro-phenyl]-acetamide;
2-[3-(3-Cyano-5-difluoromethyl-phenoxy)-4-ethyl-2-fluoro-phenyl]-N-(2-methyl-4-sulfamoyl-phenyl)-acetamide;
N-{4-[(3-Amino-propionyl)-methyl-sulfamoyl]-2-chloro-phenyl}-2-[4-chloro-3-(3-chloro-5-cyano-phenoxy)-2-fluoro-phenyl]-acetamide; compound with hydrochloric acid;
2-[3-(3-Chloro-5-cyano-phenoxy)-4,5-dimethyl-phenyl]-N-(4-sulfamoyl-phenyl)-acetamide;
2-[3-(3-Chloro-5-cyano-phenoxy)-4,5-dimethyl-phenyl]-N-(2-methyl-4-sulfamoyl-phenyl)-acetamide;
2-[3-(3-Chloro-5-cyano-phenoxy)-4,5-dimethyl-phenyl]-N-(2-chloro-4-sulfamoyl-phenyl)-acetamide;
2-[3-(3-Bromo-5-cyano-phenoxy)-4-chloro-2-fluoro-phenyl]-N-(2-methyl-4-sulfamoyl-phenyl)-acetamide;
2-{4-Chloro-3-[3-cyano-5-(3-hydroxy-prop-1-ynyl)-phenoxyl-2-fluoro-phenyl}-N-(2-methyl-4-sulfamoyl-phenyl)-acetamide;
2-{4-Chloro-3-[3-cyano-5-(3-dimethylamino-prop-1-ynyl)-phenoxy]-2-fluoro-phenyl}-N-(2-methyl-4-sulfamoyl-phenyl)-acetamide;
4-(3-Chloro-4-{2-[4-chloro-3-(3-chloro-5-cyano-phenoxy)-2-fluoro-phenyl]-acetylamino}-benzenesulfonylamino)-4-oxo-butyric acid;
2-[4-Bromo-3-(3-chloro-5-cyano-phenoxy)-2-fluoro-phenyl]-N-(2-methyl-4-sulfamoyl-phenyl)-acetamide;
2-[3-(3-Chloro-5-cyano-phenoxy)-2-fluoro-4-methoxy-phenyl]-N-(2-chloro-4-sulfamoyl-phenyl)-acetamide;
2-[3-(3-Chloro-5-cyano-phenoxy)-2-fluoro-4-methoxy-phenyl]-N-(2-methyl-4-sulfamoyl-phenyl)-acetamide;
2-[4-Chloro-3-(3-chloro-phenoxy)-phenyl]-N-phenyl-acetamide;
2-[3-(2-Bromo-5-chloro-phenoxy)-4-chloro-phenyl]-N-(4-sulfamoyl-phenyl)-acetamide;
2-[4-Chloro-3-(3,5-dicyano-phenoxy)-phenyl]-N-(2-pyrrol-1-yl-phenyl)-acetamide;
2-[4-Chloro-3-(3,5-dicyano-phenoxy)-phenyl]-N-(2-fluoro-phenyl)-acetamide;
2-[4-Chloro-3-(3,5-dicyano-phenoxy)-phenyl]-N-(2-methoxy-phenyl)-acetamide;
2-[4-Chloro-3-(3,5-dicyano-phenoxy)-phenyl]-N-(2-isopropyl-phenyl)-acetamide;
2-[4-Chloro-3-(3,5-dicyano-phenoxy)-phenyl]-N-o-tolyl-acetamide;
2-[4-Chloro-3-(3,5-dicyano-phenoxy)-phenyl]-N-(2-ethyl-phenyl)-acetamide;
2-[4-Chloro-3-(3,5-dicyano-phenoxy)-phenyl]-N-(2,3-dimethyl-phenyl)-acetamide;
2-[4-Chloro-3-(3,5-dicyano-phenoxy)-phenyl]-N-(4-methoxy-2-methyl-phenyl)-acetamide;
2-[4-Chloro-3-(3,5-dicyano-phenoxy)-phenyl]-N-(4-cyclohexyl-phenyl)-acetamide;
2-[4-Chloro-3-(3,5-dicyano-phenoxy)-phenyl]-N-(2-cyano-phenyl)-acetamide;
2-[4-Chloro-3-(3,5-dicyano-phenoxy)-phenyl]-N-(2-trifluoromethyl-phenyl)-acetamide;
2-{2-[4-Chloro-3-(3,5-dicyano-phenoxy)-phenyl]-acetylamino}-4,5-dimethoxy-benzoic acid methyl ester;
2-[3-(3-Chloro-5-cyano-phenoxy)-4-ethyl-phenyl]-N-phenyl-acetamide;
2-[3-(2-Chloro-3,5-dicyano-phenoxy)-4-ethyl-phenyl]-N-phenyl-acetamide;
2-[4-Chloro-3-(2,5-dichloro-benzyl)-phenyl]-N-(2-chloro-4-sulfamoyl-phenyl)-acetamide;
2-[3-(3-Chloro-5-cyano-phenoxy)-4-methoxy-phenyl]-N-phenyl-acetamide;
2-[4-Chloro-3-(3,5-dicyano-phenoxy)-phenyl]-N-phenyl-acetamide;
2-[4-Chloro-3-(3,5-dicyano-phenoxy)-phenyl]-N-(4-methyl-3-sulfamoyl-phenyl)-acetamide;
2-[4-Chloro-3-(3,5-dicyano-phenoxy)-phenyl]-N-(3-methanesulfonyl-phenyl)-acetamide;
(4-{2-[4-Chloro-3-(3,5-dicyano-phenoxy)-phenyl]-acetylamino}-phenoxy)-acetic acid methyl ester;
3-(4-{2-[4-Chloro-3-(3,5-dicyano-phenoxy)-phenyl]-acetylamino}-phenyl)-propionic acid methyl ester;
(4-{2-[4-Chloro-3-(3,5-dicyano-phenoxy)-phenyl]-acetylamino}-phenylsulfanyl)-acetic acid methyl ester;
4-(4-{2-[4-Chloro-3-(3,5-dicyano-phenoxy)-phenyl]-acetylamino}-phenyl)-butyric acid methyl ester;
2-[4-Chloro-3-(3,5-dicyano-phenoxy)-phenyl]-N-(2-fluoro-5-methanesulfonyl-phenyl)-acetamide;
(4-{2-[4-Chloro-3-(3,5-dicyano-phenoxy)-phenyl]-acetylamino}-phenyl)-acetic acid methyl ester;
2-[4-Chloro-3-(3,5-dicyano-phenoxy)-phenyl]-N-(4-cyano-phenyl)-acetamide;
4-{2-[4-Chloro-3-(3,5-dicyano-phenoxy)-phenyl]-acetylamino}-benzoic acid methyl ester;
2-[4-Chloro-3-(3,5-dicyano-phenoxy)-phenyl]-N-(4-hydroxy-phenyl)-acetamide;
2-[3-(3-Chloro-5-cyano-phenoxy)-4-methyl-phenyl]-N-(4-methyl-pyridin-3-yl)-acetamide;
2-[3-(3-Chloro-5-cyano-phenoxy)-4-methyl-phenyl]-N-(3-methyl-pyridin-2-yl)-acetamide;
2-[3-(3-Chloro-5-cyano-phenoxy)-4-methyl-phenyl]-N-(2,5-dimethyl-2H-pyrazol-3-yl)-acetamide;
2- [3-(3-Chloro-5-cyano-phenoxy)-4-methyl-phenyl] -N-(2-methyl-2H-pyrazol-3-yl)-acetamide;
N-(6-Acetylamino-4-methyl-pyridin-3-yl)-2- [3-(3-chloro-5-cyano-phenoxy)-4-methyl-phenyl] -acetamide;
2-[3-(3-Chloro-5-cyano-phenoxy)-4-methyl-phenyl]-N-(6-methoxy-2-methyl-pyridin-3-yl)-acetamide;
2-[3-(3-Chloro-5-cyano-phenoxy)-4-methyl-phenyl]-N-(2-methyl-5-methylsulfanyl-2H-[1,2,4]triazol-3-yl)-acetamide;
N-(2-Chloro-phenyl)-2-[3-(3,5-dichloro-benzoyl)-5-methyl-phenyl] -acetamide;
2-[3-(3,5-Dichloro-benzoyl)-5-methyl-phenyl]-N-(2-methyl-4-sulfamoyl-phenyl)-acetamide;
(4-{2-[3-(3-Chloro-5-cyano-phenoxy)-4-methyl-phenyl]-acetylamino}-3-methyl-phenoxy)-acetic acid;
(3-Chloro-4-{2-[3-(3-chloro-5-cyano-phenoxy)-4-methyl-phenyl]-acetylamino}-phenyl)-acetic acid methyl ester;
(3-Chloro-4-{2-[3-(3-chloro-5-cyano-phenoxy)-4-methyl-phenyl]-acetylamino}-phenyl)-acetic acid;
2-[3-(3-Chloro-5-cyano-phenoxy)-4-methyl-phenyl]-N-(4-methanesulfonylamino-2-methyl-phenyl) -acetamide;
2-[3-(3-Chloro-5-cyano-phenoxy)-4-methyl-phenyl]-N-[4-(methanesulfonyl-methyl-amino) -2-methyl-phenyl] -acetamide;
4-{2-[4-Chloro-3-(3-chloro-5-cyano-phenoxy)-2-fluoro-phenyl]-acetylamino}-3-methyl-benzamide;
2-[7-(4-Chloro-benzoyl)-2,3-dihydro-benzofuran-5-yl]-N-(2-chloro-phenyl)-acetamide;
2-[7-(4-Chloro-benzoyl)-2,3-dihydro-benzofuran-5-yl] -N-(4-sulfamoyl-phenyl)-acetamide;
2-[4-Chloro-3-(3,5-dicyano-phenylsulfanyl)-phenyl]-N-(4-sulfamoyl-phenyl)-acetamide;
2-[3-(2-Bromo-5-chloro-phenylsulfanyl)-4-chloro-phenyl]-N-(4-sulfamoyl-phenyl)-acetamide;
2-[3-(2-Bromo-5-chloro-phenylsulfanyl)-4-chloro-phenyl]-N-(2-chloro-phenyl)-acetamide;
2-[4-Chloro-3-(3,5-dichloro-benzoyl)-phenyl]-N-(2-chloro-phenyl)-acetamide;
2-[4-Chloro-3-(2,5-dichloro-benzoyl)-phenyl]-N-(2-chloro-phenyl)-acetamide;
2-[4-Chloro-3-(2,5-dichloro-benzoyl)-phenyl]-N-(2-methyl-4-sulfamoyl-phenyl)-acetamide;
2-[4-Chloro-3-(2,5-dichloro-benzoyl)-phenyl]-N-(4-sulfamoyl-phenyl)-acetamide;
2-[4-Chloro-3-(2,5-dichloro-benzoyl)-phenyl]-N-phenyl-acetamide;
2-[4-Chloro-3-(3,5-dichloro-benzoyl)-phenyl]-N-(2-methyl-4-sulfamoyl-phenyl)-acetamide;
2-[4-Chloro-3-(2,5-dichloro-benzoyl)-phenyl]-N-(2-chloro-4-sulfamoyl-phenyl)-acetamide;
2- [3-(4-Chloro-benzoyl)-5-methyl-phenyl] -N-(4-sulfamoyl-phenyl)-acetamide;
2- [3-(4-Chloro-benzoyl)-5-methyl-phenyl] -N-(2-methyl-4-sulfamoyl-phenyl)-acetamide;
2-[3-(2-Chloro-benzoyl)-5-methyl-phenyl]-N-(2-methyl-4-sulfamoyl-phenyl)-acetamide;
2-[3-(4-Chloro-benzoyl)-5-methyl-phenyl]-N-(2-chloro-4-sulfamoyl-phenyl)-acetamide;
2-[3-(4-Chloro-benzoyl)-5-methyl-phenyl]-N-(2-chloro-phenyl)-acetamide;
2-[3-(2-Chloro-benzoyl)-5-methyl-phenyl]-N-(2-chloro-phenyl)-acetamide;
2-[3-(3-Chloro-benzoyl)-5-methyl-phenyl]-N-(2-methyl-4-sulfamoyl-phenyl)-acetamide;
2-[3-(3-Chloro-benzoyl)-5-methyl-phenyl]-N-(4-sulfamoyl-phenyl)-acetamide;
2-[3-(3-Chloro-benzoyl)-5-methyl-phenyl]-N-(2-chloro-phenyl)-acetamide;
2-[3-(2-Chloro-benzoyl)-5-methyl-phenyl]-N-(4-sulfamoyl-phenyl)-acetamide;
2-[3-(5-Bromo-2-chloro-benzoyl)-4-methoxy-phenyl]-N-(2-methyl-4-sulfamoyl-phenyl)-acetamide;
2-[3-(5-Cyano-2-methyl-benzoyl)-4-methyl-phenyl]-N-(2-methyl-4-sulfamoyl-phenyl)-acetamide;
2-[3-(5-Cyano-2-methyl-benzoyl)-4-methyl-phenyl]-N-(4-sulfamoyl-phenyl)-acetamide;
N-(2-Chloro-phenyl)-2-[3-(5-cyano-2-methyl-benzoyl)-4-methyl-phenyl]-acetamide;
2-[3-(5-Cyano-2-ethyl-benzoyl)-4-ethyl-phenyl]-N-(2-methyl-4-sulfamoyl-phenyl)-acetamide;
2-[3-(3,5-Dichloro-benzoyl)-5-methyl-phenyl]-N-(4-sulfamoyl-phenyl)-acetamide; or
2-[3-(3-Chloro-5-cyano-phenoxy)-2-fluoro-4-methyl-phenyl]-N-(2-chloro-4-propionylsulfamoyl-phenyl)-acetamide; sodium salt.

6. Use of compound of formula I according to claim 5, which is
2-[4-chloro-3-(3-chloro-5-cyano-phenoxy)-2-fluoro-phenyl]-N-(2-chloro-4-sulfamoyl-phenyl)-acetamide;
2-[4-chloro-3-(3-chloro-5-cyano-phenoxy)-2-fluoro-phenyl]-N-(2-chloro-4-propionylsulfamoyl-phenyl)-acetamide; sodium salt;
2-[3-(3-chloro-5-cyano-phenoxy)-2-fluoro-4-methyl-phenyl]-N-(2-chloro-4-sulfamoyl-phenyl)-acetamide;
2-[4-bromo-3-(3-chloro-5-cyano-phenoxy)-2-fluoro-phenyl]-N-(2-chloro-4-sulfamoyl-phenyl)-acetamide;
2-[4-bromo-3-(3-chloro-5-cyano-phenoxy)-2-fluoro-phenyl] -N-(2-chloro-4-propionylsulfamoyl-phenyl)-acetamide; sodium salt;
N-(4-butyrylsulfamoyl-2-chloro-phenyl)-2-[4-chloro-3-(3-chloro-S-cyano-phenoxy)-2-fluoro-phenyl]-acetamide; sodium salt;
N-[4-((S)-2-amino-3-methyl-butyrylsulfamoyl)-2-chloro-phenyl]-2-[4-chloro-3-(3-chloro-5-cyano-phenoxy)-2-fluoro-phenyl]-acetamide; sodium salt;
N-(2-chloro-4-sulfamoyl-phenyl)-2-[3-(3-cyano-5-difluoromethyl-phenoxy)-4-ethyl-2-fluoro-phenyl]-acetamide;
2-[3-(3-cyano-5-difluoromethyl-phenoxy)-4-ethyl-2-fluoro-phenyl]-N-(2-methyl-4-sulfamoyl-phenyl)-acetamide;
2-[4-bromo-3-(3-chloro-5-cyano-phenoxy)-2-fluoro-phenyl]-N-(2-methyl-4-sulfamoyl-phenyl)-acetamide;
2-[4-bromo-3-(3-cyano-5-difluoromethyl-phenoxy)-2-fluoro-phenyl]-N-(2-methyl-4-sulfamoyl-phenyl)-acetamide;
2-[4-bromo-3-(3-cyano-5-difluoromethyl-phenoxy)-2-fluoro-phenyl]-N-(2-chloro-4-sulfamoyl-phenyl)-acetamide;
2-[4-chloro-3-(3-cyano-5-difluoromethyl-phenoxy)-2-fluoro-phenyl]-*N*-(2-methyl-4-sulfamoyl-phenyl)-acetamide;
2-[3-(3-chloro-5-cyano-phenoxy)-2-fluoro-4-methoxy-phenyl]-N-(2-chloro-4-sulfamoyl-phenyl)-acetamide;
2-[3-(3-chloro-5-cyano-phenoxy)-2-fluoro-4-methoxy-phenyl] -N-(2-methyl-4-sulfamoyl-phenyl)-acetamide; or,
2-[3-(3-chloro-5-cyano-phenoxy)-2-fluoro-4-methyl-phenyl]-N-(2-chloro-4-propionylsulfamoyl-phenyl)-acetamide; sodium salt.

7. A compound according to formula Ia 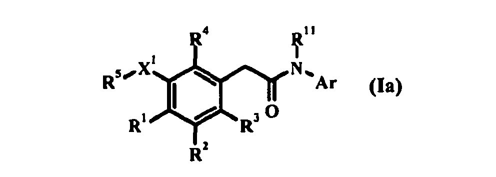 wherein
X¹ is -O-;
R¹ and R² are each independently selected from the group consisting of hydrogen, C₁₋₆ alkyl, C₁₋₆haloalkyl, C₃₋₈ cycloalkyl, C₁₋₆ alkoxy, C₁₋₆ alkylthio, C₁₋₆ alkylsulfinyl, C₁₋₆ sulfonyl, C₁₋₆ haloalkoxy, C₁₋₆ haloalkylthio, halogen, amino, alkylamino, dialkylamino, aminoacyl, nitro and cyano; or, (ii) together R¹ and R² are -O-CH=CH- or -O-CH₂CH₂- provided that R¹ is not hydrogen;
R³ and R⁴ are independently selected from the group consisting of hydrogen, C₁₋₆ alkyl, C₁₋₆ haloalkyl, C₁₋₆ alkoxy, C₁₋₆ haloalkoxy C₁₋₆ alkylthio, C₁₋₆ haloalkylthio, halogen, amino, nitro and cyano;
R⁵ is aryl substituted with one to three substituents independently selected from the group consisting of C₁₋₆alkyl, C₂₋₆ alkenyl, C₂₋₆alkynyl, -C≡CCH₂OH, -C≡CCH₂NMe₂, C₁₋₆ haloalkyl, C₃₋₈ cycloalkyl, C₁₋₆ alkoxy, C₁₋₆ alkylthio, C₁₋₆ alkylsulfinyl, C₁₋₆ sulfonyl, C₁₋₆ haloalkoxy, C₁₋₆ haloalkylthio, hydroxy, halogen, , nitro and cyano, said alkyl and said cycloalkyl are optionally substituted with one or two substituents independently selected from the group consisting of alkyl, hydroxy, alkoxy, thiol, alkylthio, halogen, amino, alkylamino, amino alkyl, alkylaminoalkyl, and dialkylamino;
Ar is a substituted phenyl ring according to formula IIa with the proviso that R^{7a} and R^{7c} are not both hydrogen or if R^{7c} is hydrogen, then R^{7a} is chlorine: 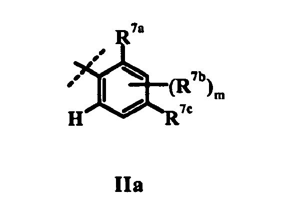
R^{7a} is selected from the group consisting of hydrogen, C₁₋₆ alkyl, C₁₋₃ haloalkyl, C₁₋₆ alkoxy, C₁₋₆ haloalkoxy, halogen and cyano;
R^{7b} is independently selected from the group consisting of C₁₋₆ haloalkyl, C₁₋₆ alkoxy, C₁₋₆ alkylthio, C₁₋₆ alkylsulfinyl, C₁₋₆ sulfonyl, amino C₁₋₆ alkylsulfonyl, SO₂NR^{11a}R^{11b}, C₁₋₆ haloalkoxy, C₁₋₆ haloalkylthio, hydroxy, amino, C₁₋₆ alkylamino, C₁₋₆ dialkylamino, aminoacyl, acyl, CONR⁸R⁹, nitro, cyano, C₁₋₆ heteroalkoxy, -X²(CH₂)ₚS(O)₂NR⁸R⁹, -X²(CH₂)ₚNHC(O)NHR⁸R⁹, -X²(CH₂)ₚNHS(O)₂NR⁸R⁹ and -X²(CH₂)ₚNHCOOR¹⁰;
R^{7c} is selected from the group consisting of hydrogen, C₁₋₆ heteroalkoxy, -S(O)₂NR⁸R⁹, -X²CH₂(CH₂)ₚS(O)₂NR⁸R⁹; -X²(CH₂)ₚNHC(O)NHR⁸R⁹, X⁶(CH₂)ᵥCONR⁸R⁹, -SO₂R¹³, -NR⁸R⁹, X²(CH₂)ₚNR¹¹S(O)₂NR⁸R⁹, -X²(CH₂)ₚNHCOOR¹⁰, -X⁶(CH₂)ᵥCOOR¹⁰, -X⁶(CH₂)ᵥCN, -OR¹⁵ and C(=O)CH₂N[(CH₂)₂]₂X⁴;
R⁸ and R⁹ taken independently, one of R⁸ and R⁹ is hydrogen or C₁₋₆ alkyl and the other of R⁸ and R⁹ is selected from the group consisting of hydrogen, -C(=O)R¹⁴, -C(=O)CR¹²NH₂, -(CH₂)₂N[(CH2)₂]₂O, COCO₂Me, C₃₋₈ cycloalkyl said cycloalkyl optionally substituted with one or two hydroxyl substituents, pyranyl, C₁₋₆ alkyl and aryl said alkyl and said aryl groups optionally substituted with one or two substituents independently selected from the group consisting of hydroxy, C₁₋₆ alkoxy, thiol, C₁₋₆ alkylthio, C₁₋₆ alkylsulfinyl, C₁₋₆ sulfonyl, and halogen; or, taken together are (CH₂)₂-X⁵-(CH₂)₂, -(CH₂)ₒ- or (CH₂)ᵣS(O)ₙ optionally substituted with one or two substituents selected from the group consisting of halogen, hydroxyl and NR^{11a}R^{11b};
R¹⁰ is C₁₋₆ alkyl;
R¹¹ is hydrogen or C₁₋₆ alkyl;
R^{11a} and R^{11b} are independently R¹¹;
R¹² is the sidechain of a naturally occurring α-amino acid;
R¹³ is C₁₋₆ alkyl; -(CH₂)ₛCO₂R¹¹, -(CH₂)₂CN, -(CH₂)₂NH₂, -(CH₂)ᵤOH;
R¹⁴ is C₁₋₁₀ alkyl, -(CH₂)ₛNHR^{11a}R^{11b}, -(CH₂)ₛOR¹¹, -CH₂CH(OH)CH₃, -CH₂N[(CH₂)₂]₂O, -(CH₂)₂CO₂R¹¹, optionally substituted phenyl or pyridinyl;
R¹⁵ is C₁₋₆ alkyl substituted with one to three hydroxyl groups;
X² is -O- or a bond;
X⁴ is -O- or -NMe-;
X⁵ is -O-, -S(O)ₙ- or NR¹¹;
X⁶ is **O-** or -S(O)ₙ-;
m is an integer from 0 to 2;
n is an integer from 0 to 2;
o is an integer from 4 to 6;
p is an integer from 0 to 6;
r is an integer from 3 to 4
s is an integer from 1 to 2;
u is an integer from 2 to 3;
v is an integer from 2 to 6; and,
hydrates, solvates, and acid addition salts or salts of the conjugate base thereof.

8. Compound of formula Ia according to claim 7, wherein
X¹ is -O-;
R¹ and R² are each independently selected from the group consisting of hydrogen, C₁₋₆ alkyl, C₁₋₆ alkoxy and halogen; or, together R¹ and R² are -O-CH=CH-, provided that R¹ is not hydrogen;
R³ and R⁴ are independently selected from the group consisting of hydrogen, and halogen;
R⁵ is aryl substituted with one to three substituents independently selected from the group consisting of C₁₋₆ alkyl, -C≡CCH₂OH, -C≡CCH₂NMe₂, C₁₋₆ haloalkyl, C₁₋₆ haloalkoxy, cyano and halogen;
Ar is a substituted phenyl ring according to formula IIa with the proviso that R^{7a} and R^{7c} are not both hydrogen or if R^{7c} is hydrogen, then R^{7a} is chlorine: 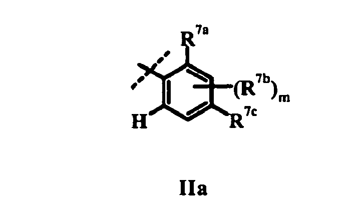
R^{7a} is selected from the group consisting of hydrogen, C₁₋₆ alkyl, C₁₋₃ haloalkyl, C₁₋₆ alkoxy, C₁₋₆ haloalkoxy, halogen and cyano;
R^{7b} is independently selected from the group consisting of C₁₋₆ haloalkyl, C₁₋₆ alkoxy, C₁₋₆ alkylthio, C₁₋₆ alkylsulfinyl, C₁₋₆ sulfonyl, amino C₁₋₆ alkylsulfonyl, SO₂NR¹¹R^{11b}, C₁₋₆ haloalkoxy, C₁₋₆ haloalkylthio, hydroxy, amino, C₁₋₆ alkylamino, C₁₋₆ dialkylamino, aminoacyl, acyl, CONR⁸R⁹, nitro, cyano, C₁₋₆ heteroalkoxy, -X²(CH₂)ₚS(O)₂NR⁸R⁹, -X²(CH₂)ₚNHC(O)NHR⁸R⁹, X²(CH₂)ₚNHS(O)₂NR⁸R⁹ and -X²(CH₂)pNHCOOR¹⁰;
R^{7c} is selected from the group consisting of hydrogen C₁₋₆ heteroalkoxy, -S(O)₂NR⁸R⁹, -X²CH₂(CH₂)ₚS(O)₂NR⁸R⁹; -X²(CH₂)ₚNHC(O)NHR⁸R⁹, X⁶(CH₂)ᵥCONR⁸R⁹, -SO₂R¹³, -NR⁸R⁹, X²(CH₂)ₚNR¹¹S(O)₂NR⁸R⁹, -X²(CH₂)ₚNHCOOR¹⁰, -X⁶(CH₂)ᵥCOOR¹⁰, -X⁶(CH₂)ᵥCN, -OR¹⁵ and C(=O)CH₂N[(CH₂)₂]₂X⁴;
R⁸ and R⁹ taken independently, one of R⁸ and R⁹ is hydrogen or C₁₋₆ alkyl and the other of R⁸ and R⁹ is selected from the group consisting of hydrogen, -C(=O)R¹⁴, -C(=O)CR¹²NH₂, -(CH₂)₂N[(CH₂)₂]₂O, COCO₂Me, C₃₋₈ cycloalkyl said cycloalkyl optionally substituted with one or two hydroxyl substituents, pyranyl, C₁₋₆ alkyl and aryl said alkyl and said aryl groups optionally substituted with one or two substituents independently selected from the group consisting of hydroxy, C₁₋₆ alkoxy, thiol, C₁₋₆ alkylthio, C₁₋₆ alkylsulfinyl, C₁₋₆ sulfonyl, and halogen; or, taken together are (CH₂)₂-X⁵-(CH₂)₂, -(CHF₂)ₒ- or (CH₂)ᵣS(O)ₙ optionally substituted with one or two substituents selected from the group consisting of halogen, hydroxyl and NR^{11a}R^{11b};
R¹⁰ is C₁₋₆ alkyl;
R¹¹ is hydrogen or C₁₋₆ alkyl;
R^{11a} and R^{11b} are independently R¹¹;
R¹² is the sidechain of a naturally occurring α-amino acid;
R¹³ is C₁₋₆ alkyl; -(CH₂)CO₂R¹¹, -(CH₂)₂CN, -(CH₂)₂NH₂, -(CH₂)ᵤOH;
R¹⁴ is C₁₋₁₀ alkyl, -(CH₂)ₛNHR^{11a}R^{11b}, -(CH₂)ₛOR¹¹, -CH₂CH(OH)CH₃, -CH₂N[(CH₂)₂]₂O, -(CH₂)₂CO₂R¹¹, optionally substituted phenyl or pyridinyl;
R¹⁵ is C₁₋₆ alkyl substituted with one to three hydroxyl groups;
X² is -O- or a bond;
X⁴ is -O- or -NMe-;
X⁵ is -O-, -S(O)ₙ- or NR¹¹;
X⁶ is **O-** or -S(O)ₙ-;
m is an integer from 0 to 2;
n is an integer from 0 to 2;
o is an integer from 4 to 6;
p is an integer from 0 to 6;
r is an integer from 3 to 4
s is an integer from 1 to 2;
u is an integer from 2 to 3;
v is an integer from 2 to 6; and,
hydrates, solvates, and acid addition salts or salts of the conjugate base thereof.

9. Compound of formula Ia according to claim 8, wherein
X¹ is -O-;
R¹ and R² are each independently selected from the group consisting of hydrogen, methyl, ethyl, i-propyl, methoxy, chloro and bromo; or together R¹ and R² are -O-CH=CH-;
R³ and R⁴ are independently selected from the group consisting of hydrogen and fluoro;
R⁵ is phenyl, wherein said phenyl is unsubstituted or substituted with one to three substituents independently selected from the group consisting of methyl, ethyl, -CHF₂, -CF₃, -CF₂CH₃, -OCHF₂, chloro, bromo, fluoro, -C≡CCH₂OH, -C≡CCH₂N(CH₃)₂ and-cyano.
Ar is phenyl, wherein said phenyl is substituted with 1 to 3 substituents selected from the group consisting of methyl, halogen, 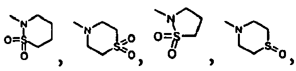 -SO₂NH₂,-SO₂NHCH₃, -SO₂NCH₃CH₂CH₂OH, -SO₂NHCH₂CHCH₃OH,-SO₂NHCH₂CH₂ 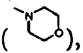 -SO₂NHCH₂CH₂CH₂OH, -SO₂ 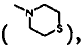 -SO₂ 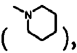 -SO₂ 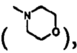 -SO₂ 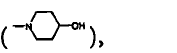 -SO₂NH 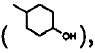 -SO₂ 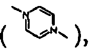 - SO₂NHCOCH₂CH₃, -SO₂NHCOCH₂CH₂CH₃, -SO₂NHCOCH(NH₂)CH(CH₃)₂, -SO₂NHCOCH₂CH₂CH₃, -SO₂NHCOCH₂CH₂CH₂CH₃, -SO₂NHCOCH₂CH(CH₃)₂, -SO₂NHCOCH₂CH(CH₂CH₃)₂, -SO₂NHCOCH₂CH₂CH(CH₃)₂, -SO₂NHCOCH₂CH₂NH₂, -SO₂NCH₃COCH₂CH₂NH₂, -SO₂NHCOCH₂OCH₃, -SO₂NHCO 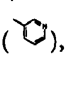 -SO₂NH 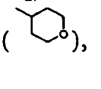 -SO₂NHCOCH(i-propyl)NH₂, -SO₂NHCH₂CH₂SCH₃, -SO₂NCH₃COCH₂CH₃, -SO₂NCH₃COCH₂OCH₃, -SO₂NCH₃COCH₂CH₂OCH₃, -SO₂NHCOCH₂OH, -SO₂NHCOCH₂CH₂COOH, -SO₂CH₂COOH, -SO₂CH₂CH₂COOH, -SO₂CH₃, -SO₂CH₂CH₂NH₂, -SO₂CH₂CH₂CN, -SO₂CH₂CH₂CH₂OH, -O(CH₂)₃SO₂NH₂, -OCH₂CN, -OCH₂CHOHCH₂OH, -OCH₂CH₂CHOHCH₂OH, -COOCH₃, -COOH, -COCH₂ 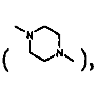 -COCH₂ 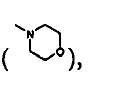 -N(CH₃)₂, and -NHCOCOOCH₂CH₃;
R¹¹ is hydrogen or methyl; and
hydrates, solvates and salts thereof.

10. Compound of formula Ia according to claim 7, which is
2-[4-Chloro-3-(3-chloro-5-cyano-phenoxy)-phenyl]-N-(4-sulfamoyl-phenyl)-acetamide;
2-[4-Chloro-3-(3,5-dicyano-phenoxy)-phenyl]-N-(4-sulfamoyl-phenyl)-acetamide;
2-[3-(2-Bromo-5-chloro-phenoxy)-4-chloro-phenyl]-N-[2-methyl-4-(3-sulfamoyl-propoxy)-phenyl] -acetamide;
2-[3-(2-Bromo-5-chloro-phenoxy)-4-chloro-phenyl]-N-(4-sulfamoyl-phenyl)-acetamide;
2-[4-Chloro-3-( 3,5-dicyano-phenoxy)-phenyl]-N-(2-chloro-phenyl)-acetamide;
2-[4-Chloro-3-(3,5-dicyano-phenoxy)-phenyl]-N-(4-dimethylamino-phenyl)-acetamide;
2-[3-(2-Chloro-5-cyano-phenoacy)-4-ethyl-2-fluoro-phenyl]-N-(4-sulfamoyl-phenyl)-acetamide;
2-[4-Chloro-3-(2,5-dicyano-phenoxy)-2-fluoro-phenyl]-N-(4-sulfamoyl-phenyl)-acetamide;
2-[4-Chloro-3-(3,5-dicyano-phenoxy)-2-fluoro-phenyl]-N-(4-sulfamoyl-phenyl)-acetamide;
2-[4-Chloro-2-fluoro-3-(2,3,5-trichloro-phenoxy)-phenyl]-N-(4-sulfamoyl-phenyl)-acetamide;
2- [3-(3-Cyano-5-fluoro-phenoxy)-4-methyl-phenyl] -N-(4-sulfamoyl-phenyl)-acetamide;
N-(2-Chloro-phenyl)-2-[3-(3-cyano-5-fluoro-phenoxy)-4-methyl-phenyl]-acetamide;
2-[4-Chloro-3-(3-cyano-5-difluoromethyl-phenoxy)-2-fluoro-phenyl]-N-(4-sulfamoyl-phenyl)-acetamide;
2-[4-Chloro-3-(3-cyano-5-difluoromethyl-phenoxy)-2-fluoro-phenyl]-N-(2-chloro-phenyl)-acetamide;
2-[3-(2-Bromo-5-chloro-phenoxy)-4-ethyl-phenyl]-N-(4-sulfamoyl-phenyl)-acetamide;
2-[3-(2-Bromo-5-chloro-phenoxy)-4-ethyl-phenyl]-N-(2-chloro-phenyl)-acetamide;
2-[3-(5-Chloro-2-cyano-phenoxy)-4-ethyl-phenyl]-N-(4-sulfamoyl-phenyl)-acetamide;
2-[3-(5-Chloro-2-cyano-phenoxy)-4-ethyl-phenyl]-N-(2-chloro-phenyl)-acetamide;
2-[3-(2,5-Dichloro-phenoxy)-4-ethyl-phenyl]-N-(4-sulfamoyl-phenyl)-acetamide;
2-[3-(2,6-Dichloro-phenoxy)-4-ethyl-phenyl]-N-(4-sulfamoyl-phenyl)-acetamide;
N-(2-Chloro-phenyl)-2-[3-(2,5-dichloro-phenoxy)-4-ethyl-phenyl]-acetamide;
2-[3-(3-Bromo-5-chloro-phenoxy)-4-ethyl-phenyl]-N-(4-sulfamoyl-phenyl)-acetamide;
N-(2-Chloro-phenyl)-2-[3-(2,6-dichloro-phenoxy)-4-ethyl-phenyl]-acetamide;
2-[3-(3-Bromo-5-chloro-phenoxy)-4-ethyl-phenyl]-N-(2-chloro-phenyl)-acetamide;
N-(2-Chloro-phenyl)-2-[ 3-(3,5-dicyano-phenoxy)-4-ethyl-phenyl]-acetamide;
2-[3-(2-Bromo-5-chloro-phenoxy)-4-isopropyl-phenyl]-N-(4-sulfamoyl-phenyl)-acetamide;
2-[3-(2-Bromo-5-chloro-phenoxy)-4-isopropyl-phenyl]-N-(2-chloro-phenyl)-acetamide;
2-[4-Chloro-3-(3,5-dicyano-phenoxy)-2-fluoro-phenyl]-N-(2-chloro-phenyl)-acetamide;
2-[4-Chloro-3-(3,5-dicyano-phenoxy)-phenyl]-N-[2-methyl-4-(3-sulfamoyl-propoxy)-phenyl]-acetamide;
2-[3-(2-Bromo-5-chloro-phenoxy)-4-methyl-phenyl]-N-(4-sulfamoyl-phenyl)-acetamide;
2-[3-(2-Bromo-5-chloro-phenoxy)-4-methyl-phenyl]-N-(2-methyl-4-sulfamoyl-phenyl)-acetamide;
2-[4-Chloro-3-(3,5-dicyano-phenoxy)-2-fluoro-phenyl]-N-(2-methyl-4-sulfamoyl-phenyl)-acetamide;
2-[3-(2-Bromo-5-chloro-phenoxy)-4-methyl-phenyl]-N-(2-chloro-phenyl)-acetamide;
N-(2-Chloro-phenyl)-2-[3-(3,5-dicyano-phenoxy)-4-methyl-phenyl]-acetamide;
2-[3-(3-Bromo-2,5-dichloro-phenoxy)-4-ethyl-phenyl]-N-(4-sulfamoyl-phenyl)-acetamide;
2-[3-(3-Bromo-2,5-dichloro-phenoxy)-4-ethyl-phenyl]-N-(2-methyl-4-sulfamoyl-phenyl)-acetamide;
2-[3-(3-Bromo-2,5-dichloro-phenoxy)-4-ethyl-phenyl]-N-(2-chloro-phenyl)-acetamide;
N-(2-Chloro-phenyl)-2-[3-(3,5-dibromo-2-chloro-phenoxy)-4-ethyl-phenyl]-acetamide;
2-[3-(5-Bromo-2-chloro-3-fluoro-phenoxy)-4-ethyl-phenyl]-N-(4-sulfamoyl-phenyl)-acetamide;
2-[3-(5-Bromo-2-chloro-3-fluoro-phenoxy)-4-ethyl-phenyl]-N-(2-methyl-4-sulfamoyl-phenyl)-acetamide;
2-[3-(5-Bromo-2-chloro-3-fluoro-phenoxy)-4-ethyl-phenyl]-N-(2-chloro-phenyl)-acetamide;
2-[4-Chloro-3-(3-chloro-5-cyano-phenoxy)-2-fluoro-phenyl]-N-(2-methyl-4-sulfamoyl-phenyl)-acetamide;
2- [3-(3,5-Dicyano-phenoxy)-4-methyl-phenyl]-N-(2-methyl-4-sulfamoyl-phenyl)-acetamide;
N-(2-Chloro-4-sulfamoyl-phenyl)-2-[3-(3,5-dicyano-phenoxy)-4-methyl-phenyl] - acetamide;
2-[3-(3-Chloro-5-cyano-phenoxy)-4-ethyl-phenyl]-N-(4-sulfamoyl-phenyl)-acetamide;
2-[3-(3-Chloro-5-cyano-phenoxy)-4-ethyl-phenyl]-N-(2-methyl-4-sulfamoyl-phenyl)-acetamide;
2-[3-(3-Chloro-5-cyano-phenoxy)-4-ethyl-phenyl]-N-(2-chloro-4-sulfamoyl-phenyl)-acetamide;
2-[3-(3-Chloro-5-cyano-phenoxy)-4-ethyl-phenyl]-N-(4-methylsulfamoyl-phenyl)-acetamide;
2-[3-(2-Chloro-5-cyano-3-fluoro-phenoxy)-4-ethyl-phenyl]-N-(4-sulfamoyl-phenyl)-acetamide;
2-[3-(2-Chloro-5-cyano-3-fluoro-phenoxy)-4-ethyl-phenyl]-N-(2-methyl-4-sulfamoyl-phenyl)-acetamide;
2-[4-Chloro-3-(3-chloro-5-ryano-phenoxy)-2-fluoro-phenyl]-N-(2-methyl-4-propionylsulfamoyl-phenyl)-acetamide;
2-[3-(2-Chloro-3,5-dicyano-phenoxy)-4-ethyl-phenyl]-N-(4-sulfamoyl-phenyl)-acetamide;
2-[3-(2-Chloro-3,5-dicyano-phenoxy)-4-ethyl-phenyl]-N-(2-methyl-4-sulfamoyl-phenyl)-acetamide;
2-[3-(2-Chloro-3,5-dicyano-phenoxy)-4-ethyl-phenyl]-N-(2-chloro-4-sulfamoyl-phenyl)-acetamide;
2-[4-Chloro-3-(4-cyano-2,6-dimethyl-phenoxy)-phenyl]-N-(4-sulfamoyl-phenyl)-acetamide;
2-[4-Chloro-3-(4-cyano-2,6-dimethyl-phenoxy)-phenyl]-N-(2-methyl-4-sulfamoyl-phenyl)-acetamide;
2-[3-(3-Chloro-5-cyano-phenoxy)-4-methoxy-phenyl]-N-(4-sulfamoyl-phenyl)-acetamide;
2-[3-(3-Chloro-5-cyano-phenoxy)-4-ethyl-phenyl]-N-(2-chloro-phenyl)-acetamide;
2-[3-(2-Chloro-5-cyano-3-fluoro-phenoxy)-4-ethyl-phenyl]-N-(2-chloro-phenyl)-acetamide;
2-[3-(2-Chloro-3,5-dicyano-phenoxy)-4-ethyl-phenyl]-N-(2-chloro-phenyl)-acetamide;
2-[3-(3-Chloro-5-cyano-phenoxy)-4-methoxy-phenyl]-N-(2-chloro-phenyl)-acetamide;
2-[3-(2-Chloro-5-cyano-phenoxy)-4-methoxy-phenyl]-N-(2-chloro-phenyl)-acetamide;
2-[4-Bromo-3-(3-chloro-5-cyano-phenoxy)-2-fluoro-phenyl]-N-(2-chloro-4-propionylsulfamoyl-phenyl)-acetamide; sodium salt;
2- [3-(3-Chloro-5-cyano-phenoxy)-4-methoxy-phenyl] -N-(2-chloro-4-sulfamoyl-phenyl)-acetamide;
2-[3-(2-Chloro-5-cyano-phenoxy)-4-methoxy-phenyl]-N-(4-sulfamoyl-phenyl)-acetamide;
2- [3-(2-Chloro-5-cyano-phenoxy)-4-methoxy-phenyl] -N-(2-methyl-4-sulfamoyl-phenyl)-acetamide;
2-[3-(2-Chloro-5-cyano-phenoxy)-4-methoxy-phenyl]-N-(2-chloro-4-sulfamoyl-phenyl)-acetamide;
2-[4-Chloro-3-(4-cyano-2,6-dimethyl-phenoxy)-phenyl]-N-(2-chloro-phenyl)-acetamide;
2-[4-Chloro-3-(3,5-dicyano-phenoxy)-phenyl]-N-(4-methylsulfamoyl-phenyl)-acetamide;
2-[4-Chloro-3-(3,5-dicyano-phenoxy)-phenyl]-N-(4-dimethylsulhmoyl-phenyl)-acetamide;
2- [4-Chloro-3-(3,5-dicyano-phenoxy)-phenyl]-N- [4-(piperidine-1-sulfonyl)-phenyl]-acetamide;
2-[4-Chloro-3-(3,5-dicyano-phenoxy)-phenyl]-N-[4-(morpholine-4-sulfonyl)-phenyl]-acetamide;
2-[3-(3-Chloro-5-cyano-phenoxy)-4-methoxy-phenyl]-N-(2-methyl-4-sulfamoyl-phenyl)-acetamide;
2-[3-(3-Chloro-5-cyano-phenoxy)-2-fluoro-4-methyl-phenyl]-N-(2-methyl-4-sulfamoyl-phenyl)-acetamide;
2-[4-Chloro-3-(3-cyano-5-difluoromethyl-phenoxy)-2-fluoro-phenyl]-N-(2-methyl-4-sulfamoyl-phenyl)-acetamide;
2-[4-Chloro-3-(3,5-dicyano-phenoxy)-phenyl]-N-(2-chloro-4-methanesulfonyl-phenyl)-acetamide;
N-[4-((S)-2-Amino-3-methyl-butyrylsulfamoyl)-2-methyl-phenyl]-2-[4-chloro-3-(3-chloro-5-cyano-phenoxy)-2-fluoro-phenyl]-acetamide; compound with hydrochloric acid;
2-[4-Chloro-3-(3,5-dicyano-phenoxy)-phenyl]-N-(4-cyanomethoxy-phenyl)-acetamide;
2-[4-Chloro-3-(3,5-dicyano-phenoxy)-phenyl]-N-(4-methanesulfonyl-phenyl)-acetamide;
3-Chloro-4-{2-[4-chloro-3-(3,5-dicyano-phenoxy)-phenyl]-acetylamino}-benzoic acid methyl ester;
2-[4-Chloro-3-(3,5-dicyano-phenoxy)-phenyl]-N-{4-[(2-hydroxy-ethyl)-methyl-sulfamoyl]-phenyl}-acetamide;
2- [4-Chloro-3-(3,5-dicyano-phenoxy)-phenyl]-N-[4-(4-hydroxy-piperidine-1-sulfonyl)-phenyl]-acetamide;
2-[3-(3-Chloro-5-cyano-phenoxy)-2-fluoro-4-methyl-phenyl]-N-(2-chloro-4-sulfamoyl-phenyl)-acetamide;
N-(2-Chloro-4-sulfamoyl-phenyl)-2-[3-(3-cyano-5-difluoromethyl-phenoxy)-2-fluoro-4-methyl-phenyl] -acetamide;
2-[3-(3-Cyano-5-difluoromethyl-phenoxy)-2-fluoro-4-methyl-phenyl]-N-(2-methyl-4-sulfamoyl-phenyl)-acetamide;
N-(2-Chloro-phenyl)-2-[3-(3-ryano-5-difluoromethyl-phenoxy)-2-fluoro-4-methyl-phenyl]-acetamide;
2- [4-Chloro-3-(3-chloro-5-cyano-phenoxy)-2-fluoro-phenyl]-N-{2-methyl-4-[(pyridine-3-carbonyl)-sulfamoyl]-phenyl}-acetamide; compound with hydrochloric acid;
2-[4-Chloro-3-(3-cyano-5-trifluoromethyl-phenoxy)-phenyl]-N-(4-sulfamoyl-phenyl)-acetamide;
2- [4-Chloro-3-(3-cyano-5-trifluoromethyl-phenoxy)-phenyl]-N-(2-methyl-4-sulfamoyl-phenyl)-acetamide;
2-[4-Chloro-3-(3-cyano-5-trifluoromethyl-phenoxy)-phenyl]-N-(2-chloro-4-sulfamoyl-phenyl)-acetamide;
2- [3-(3-Cyano-5-difluoromethyl-phenoxy)-4-ethyl-phenyl] -N-(4-sulfamoyl-phenyl)-acetamide;
2- [3-(3-Cyano-5-difluoromethyl-phenoxy)-4-methoxy-phenyl] -N-(4-sulfamoyl-phenyl)-acetamide;
2-[3-(3-Cyano-5-difluoromethyl-phenoxy)-4-methoxy-phenyl]-N-(2-methyl-4-sulfamoyl-phenyl)-acetamide;
N-(2-Chloro-4-sulfamoyl-phenyl)-2-[3-(3-cyano-5-difluoromethyl-phenoxy)-4-methoxy-phenyl] -acetamide;
2-{4-Chloro-3-[3-cyano-5-(1,1-difluoro-ethyl)-phenoxy]-2-fluoro-phenyl}-N-(4-sulfamoyl-phenyl)-acetamide;
2-{4-Chloro-3-[3-cyano-5-(1,1-difluoro-ethyl)-phenoxy]-2-fluoro-phenyl}-N-(2-methyl-4-sulfamoyl-phenyl)-acetamide;
2-{4-Chloro-3-[3-cyano-5-(1,1-difluoro-ethyl)-phenoxy]-2-fluoro-phenyl}-N-(2-chloro-4-sulfamoyl-phenyl)-acetamide;
2-[4-Chloro-3-(3-chloro-S-cyano-phenoxy)-2-fluoro-phenyl]-N-(2-chloro-phenyl)-acetamide;
2-[4-Chloro-3-(3-chloro-5-cyano-phenoxy)-2-fluoro-phenyl]-N-[2-methyl-4-(methylpropionyl-sulfamoyl)-phenyl]-acetamide;
2-[4-Chloro-3-(3-chloro-5-cyano-phenoxy)-2-fluoro-phenyl] -N-(2-chloro-4-sulfamoyl-phenyl)-acetamide;
2- [3-(3-Cyano-5-difluoromethyl-phenoxy)-4-ethyl-phenyl] -N-(2-methyl-4-sulfamoyl-phenyl)-acetamide;
2- [3-(2-Chloro-5-cyano-phenoxy)-5-methoxy-phenyl] -N-(4-sulfamoyl-phenyl)-acetamide;
2-[3-(2-Chloro-5-cyano-phenoxy)-5-methoxy-phenyl]-N-(2-methyl-4-sulfamoyl-phenyl)-acetamide;
2-[4,5-Dibromo-3-(3-cyano-5-difluoromethyl-phenoxy)-2-fluoro-phenyl]-N-(2-methyl-4-sulfamoyl-phenyl)-acetamide;
2-[3-(2-Chloro-5-cyano-phenoxy)-5-ethyl-phenyl]-N-(4-sulfamoyl-phenyl)-acetamide;
2-[3-(2-Chloro-5-cyano-phenoxy)-5-ethyl-phenyl]-N-(2-methyl-4-sulfamoyl-phenyl)-acetamide;
2-[3-(2-Chloro-5-cyano-phenoxy)-5-ethyl-phenyl] -N-(2-chloro-4-sulfamoyl-phenyl)-acetamide;
2-[4-Chloro-3-(3-chloro-5-ryano-phenoxy)-2-fluoro-phenyl]-N-(2-methyl-4-methylsulfamoyl-phenyl)-acetamide;
2-[4-Bromo-3-(3-chloro-5-cyano-phenoxy)-2-fluoro-phenyl]-N-(2-chloro-4-sulfamoyl-phenyl)-acetamide;
3-Chloro-4-{2- [3-(3-cyano-5-difluoromethyl-phenoxy)-2-fluoro-4-methyl-phenyl] - acetylamino}-benzoic acid;
2-[4-Chloro-3-(3-chloro-5-cyano-phenoxy)-2-fluoro-phenyl]-N-(2-chloro-phenyl)-N-methyl-acetamide;
N-(2-Chloro-4-methanesulfonyl-phenyl)-2-[3-(3-cyano-5-difluoromethyl-phenoacy)-2-fluoro-4-methyl-phenyl]-acetamide;
2- [3-(2-Chloro-5-cyano-phenoxy)-5-methoxy-phenyl] -N-(2-chloro-4-sulfamoyl-phenyl)-acetamide;
2-[4,5-Dibromo-3-(3-cyano-5-difluoromethyl-phenoxy)-2-fluoro-phenyl]-N-(4-sulfamoyl-phenyl)-acetamide;
2- [3-(3-Chloro-5-cyano-phenoxy)-4-methyl-phenyl] -N- [2-methyl-4-((morpholine-4-sulfonyl)-phenyl] -acetamide;
2- [3-(3-Chloro-5-cyano-phenoxy)-4-methyl-phenyl]-N-[2-methyl-4-(4-methyl-piperazine-1-sulfonyl)-phenyl] -acetamide;
2- [3-(3-Chloro-5-cyano-phenoxy)-4-methyl-phenyl]-N- [2-methyl-4-(thiomorpholine-4-sulfonyl)-phenyl]-acetamide;
2- [3-(3-Chloro-5-cyano-phenoxy)-4-methyl-phenyl] -N- [2-methyl-4-(2-morpholin-4-yl-ethylsulfamoyl)-phenyl] -acetamide;
2-[3-(3-Chloro-5-cyano-phenoxy)-4-methyl-phenyl]-N-[4-(3-hydroxy-propylsulfamoyl)-2-methyl-phenyl] -acetamide;
2- [4-Chloro-3-(3-cyano-5-trifluoromethyl-phenoxy)-2-fluoro-phenyl] -N-(2-methyl-4-sulfamoyl-phenyl)-acetamide;
2- [3-(3-Chloro-5-cyano-phenoxy)-4-methyl-phenyl] -N- [4-(4-hydroxy-cyclohexylsulfamoyl)-2-methyl-phenyl]-acetamide;
2-[3-(3-Chloro-5-cyano-phenoxy)-4-methyl-phenyl]-N-(2-methyl-4-sulfamoyl-phenyl)-acetamide;
2- [3-(3-Chloro-5-cyano-phenoxy)-4-methyl-phenyl] -N-(2-chloro-4-sulfamoyl-phenyl)-acetamide;
2-[4-Chloro-3-(4-cyano-6-methyl-pyridin-2-yloxy)-2-fluoro-phenyl]-N-(2-methyl-4-sulfamoyl-phenyl)-acetamide;
3-(4-{2-[3-(3-Chloro-5-cyano-phenoxy)-4-methyl-phenyl]-acetylamino}-3-methyl-benzenesulfonyl)-propionic acid;
2-[4-Chloro-3-(3-chloro-5-cyano-phenoxy)-2-fluoro-phenyl]-N-(2-chloro-4-propionylsulfamoyl-phenyl)-acetamide; sodium salt;
N-[4-((S)-2-Amino-3-methyl-butyrylsulfamoyl)-2-chloro-phenyl]-2-[4-chloro-3-(3-chloro-5-cyano-phenoxy)-2-fluoro-phenyl]-acetamide; sodium salt;
2-[3-(3-Chloro-5-cyano-phenoxy)-4-methyl-phenyl]-N-[2-methyl-4-(tetrahydro-pyran-4-ylsulfamoyl)-phenyl] -acetamide;
2-[3-(3-Chloro-5-cyano-phenoxy)-4-methyl-phenyl]-N-[4-(2-hydroxy-propylsulfamoyl)-2-methyl-phenyl]-acetamide;
2-[3-(3-Chloro-5-cyano-phenoxy)-4-methyl-phenyl]-N-[2-methyl-4-(2-methylsulfanyl-ethylsulfamoyl)-phenyl] -acetamide;
2- [3-(3-Chloro-5-cyano-phenoxy)-4-methyl-phenyl]-N-[2-methyl-4-(3-methylsulfanyl-propylsulfamoyl)-phenyl] -acetamide;
2- [3-(3-Chloro-5-cyano-phenoxy)-5-methoxy-phenyl]-N-(4-sulfamoyl-phenyl)-acetamide;
2- [3-(3-chloro-5-cyano-phenoxy)-5-methoxy-phenyl] -N-(2-methyl-4-sulfamoyl-phenyl)-acetamide;
N-(2-Chloro-4-sulfamoyl-phenyl)-2-[4,5-dibromo-3-(3-cyano-5-difluoromethyl-phenoxy)-2-fluoro-phenyl] -acetamide;
2- [4-Chloro-3-(4-cyano-2,6-dimethyl-phenoxy)-phenyl]-N-(2-chloro-4-sulfamoyl-phenyl)-acetamide;
2- [3-(3-chloro-5-cyano-phenoxy)-5-methoxy-phenyl]-N-(2-chloro-4-sulfamoyl-phenyl)-acetamide;
2-[3-(3-Chloro-5-cyano-phenoxy)-4-methyl-phenyl]-N-[4-(2-cyano-ethanesulfonyl)-2-methyl-phenyl] -acetamide;
N-(4-Butyrylsulfamoyl-2-chloro-phenyl)-2-[4-chloro-3-(3-chloro-5-cyano-phenoxy)-2-fluoro-phenyl]-acetamide; sodium salt;
N- [4-(Butyryl-methyl-sulfamoyl)-2-chloro-phenyl] -2-[4-chloro-3-(3-chloro-5-cyano-phenoxy)-2-fluoro-phenyl] -acetamide;
N-(2-Chloro-4-sulfamoyl-phenyl)-2-[3-(3-cyano-5-difluoromethyl-phenoxy)-4-ethylphenyl] -acetamide;
2-[3-(3-Chloro-5-ryano-phenoxy)-5-methyl-phenyl]-N-(4-sulfamoyl-phenyl)-acetamide;
2-[3-(3-Chloro-5-cyano-phenoxy)-5-methyl-phenyl]-N-(2-methyl-4-sulfamoyl-phenyl)-acetamide;
2-[3-(3-Chloro-5-cyano-phenoxy)-5-methyl-phenyl)-N-(2-chloro-4-sulfamoyl-phenyl)-acetamide;
2-[7-(3-Cyano-phenoxy)-benzofuran-5-yl]-N-(4-sulfamoyl-phenyl)-acetamide;
2-[7-(3-Cyano-phenoxy)-benzofuran-5-yl]-N-(2-methyl-4-sulfamoyl-phenyl)-acetamide;
2-[3-(2-Chloro-5-cyano-phenoxy)-5-methyl-phenyl]-N-(4-sulfamoyl-phenyl)-acetamide;
2-[3-(2-Chloro-5-cyano-phenoxy)-5-methyl-phenyl]-N-(2-methyl-4-sulfamoyl-phenyl)-acetamide;
2-[3-(2-Chloro-5-cyano-phenoxy)-5-methyl-phenyl]-N-(2-chloro-4-sulfamoyl-phenyl)-acetamide;
N-(2-Chloro-4-sulfamoyl-phenyl)-2-[7-(3,5-dichloro-phenoxy)-benzofuran-5-yl]-acetamide;
2- [3-(3-Chloro-5-cyano-phenoxy)-4-methyl-phenyl]-N- [4-((S)-2,3-dihydroxy-propoxy)-2-methyl-phenyl]-acetamide;
2-[3-(3-Chloro-5-cyano-phenoxy)-4-methyl-phenyl]-N-[4-((R)-3,4-dihydroxy-butoxy)-2-methyl-phenyl]-acetamide;
(4-{2-3-(3-Chloro-5-cyano-phenoxy)-4-methyl-phenyl]-acetylamino}-3-methyl-benzenesulfonyl)-acetic acid;
N-[4-(3-Amino-propionylsulfamoyl)-2-chloro-phenyl]-2-[4-chloro-3-(3-chloro-5-cyano-phenoxy)-2-fluoro-phenyl]-acetamide; compound with hydrochloric acid;
N-[4-(2-Amino-ethanesulfonyl)-2-methyl-phenyl]-2-[3-(3-chloro-5-cyano-phenoxy)-4-methyl-phenyl] -acetamide;
2- [4-Chloro-3-(3-chloro-5-cyano-phenoxy)-2-fluoro-phenyl] -N- [2-chloro-4-(2-methoxy-acetylsulfamoyl)-phenyl]-acetamide; sodium salt;
2-[4-Chloro-3-(3-chloro-5-cyano-phenoxy)-2-fluoro-phenyl]-N-[2-chloro-4-(2-hydroxy-acetylsulfamoyl)-phenyl]-acetamide; sodium salt;
2- [3-(3-Chloro-5-cyano-phenoxy)-4-methyl-phenyl] -N- [4-(3-hydroxy-propane-1-sulfonyl)-2-methyl-phenyl] -acetamide;
2-[3-(3-Chloro-5-cyano-phenoxy)-4-methyl-phenyl]-N-[4-(1,1-dioxo-1λ6-[1,2] thiazinan-2-yl)-2-methyl-phenyl]-acetamide;
2-[3-(3-Chloro-5-cyano-phenoxy)-4-methyl-phenyl]-N-{2-methyl-4-[2-(4-methyl-piperazin-1-yl)-acetyl] -phenyl} -acetamide;
2-[3-(3-Chloro-5-cyano-phenoxy)-4-methyl-phenyl]-N-[2-methyl-4-(1-oxo-1λ4-thiomorpholin-4-yl)-phenyl]-acetamide;
2- [3-(3-Chloro-5-cyano-phenoxy)-4-methyl-phenyl] -N- [4-(1,1-dioxo-1λ6-thiomorpholin-4-yl)-2-methyl-phenyl]-acetamide;
2- [4-Chloro-3-(3-chloro-5-cyano-phenoxy)-2-fluoro-phenyl]-N- [2-chloro-4-(methylpropionyl-sulfamoyl)-phenyl] -acetamide;
2- [3-(3-Chloro-5-cyano-phenoxy)-4-methyl-phenyl]-N-[2-methyl-4-(2-morpholin-4-yl-acetyl)-phenyl]-acetamide; compound with trifluoro-acetic acid;
2- [4-Chloro-3-(3-chloro-5-ryano-phenoxy)-2-fluoro-phenyl]-N-[2-chloro-4-(3-methoxy-propionylsulfamoyl)-phenyl]-acetamide; sodium salt;
2-[4-Bromo-3-(3-cyano-5-difluoromethyl-phenoxy)-2-fluoro-phenyl]-N-(2-chloro-4-sulfamoyl-phenyl)-acetamide;
2-[4-Chloro-3-(3-cyano-5-difluoromethoxy-phenoxy)-2-fluoro-phenyl]-N-(4-sulfamoyl-phenyl)-acetamide;
2- [4-Chloro-3-(3-cyano-5-difluoromethoxy-phenoxy)-2-fluoro-phenyl] -N-(2-chloro-4-sulfamoyl-phenyl)-acetamide;
2-[4-Chloro-3-(3-chloro-5-cyano-phenoxy)-2-fluoro-phenyl]-N-(2-chloro-4-methylsulfamoyl-phenyl)-acetamide;
2-[4-Bromo-3-(3-cyano-5-difluoromethyl-phenoxy)-2-fluoro-phenyl]-N-(2-methyl-4-sulfamoyl-phenyl)-acetamide;
2-[3-(3-Chloro-5-cyano-phenoxy)-4-methyl-phenyl]-N-[4-(1,1-dioxo-1λ6-isothiazolidin-2-yl)-2-methyl-phenyl]-acetamide;
2-[4-Chloro-3-(3-chloro-5-ryano-phenoxy)-2-fluoro-phenyl]-N-{2-chloro-4-[(2-methoxy-acetyl)-methyl-sulfamoyl]-phenyl}-acetamide;
2-[4-Chloro-3-(3-chloro-5-cyano-phenoxy)-2-fluoro-phenyl]-N-{2-chloro-4-[(3-methoxy-propionyl)-methyl-sulfamoyl]-phenyl}-acetamide;
2- [4-Chloro-3-(3-chloro-5-cyano-phenoxy)-2-fluoro-phenyl] -N-(2-chloro-4-pentanoylsulfamoyl-phenyl)-acetamide; sodium salt;
2-[4-Chloro-3-(3-chloro-5-cyano-phenoxy)-2-fluoro-phenyl]-N-[2-chloro-4-(3-methyl-butyrylsulfamoyl)-phenyl]-acetamide; sodium salt;
2- [4-Chloro-3-(3-chloro-5-cyano-phenoxy)-2-fluoro-phenyl] -N- [2-chloro-4-(2-diethylamino-acetylsulfamoyl)-phenyl]-acetamide; compound with hydrochloric acid;
2-[4-Chloro-3-(3-chloro-5-ryano-phenoxy)-2-fluoro-phenyl]-N-[2-chloro-4-(4-methyl-pentanoylsulfamoyl)-phenyl]-acetamide; sodium salt;
2- [4-Chloro-3-(3-chloro-5-cyano-phenoxy)-2-fluoro-phenyl] -N- [2-chloro-4-(2-morpholin-4-yl-acetylsulfamoyl)-phenyl]-acetamide; compound with hydrochloric acid;
N-(4-{2-[3-(3-Chloro-5-cyano-phenoxy)-4-methyl-phenyl]-acetylamino}-3-methyl-phenyl)-oxalamic acid ethyl ester;
N-(2-Chloro-4-sulfamoyl-phenyl)-2- [3-(3-cyano-5-difluoromethyl-phenoxy)-4-ethyl-2-fluoro-phenyl] -acetamide;
2- [3-(3-Cyano-5-difluoromethyl-phenoxy)-4-ethyl-2-fluoro-phenyl]-N-(2-methyl-4-sulfamoyl-phenyl)-acetamide;
N-{4-[(3-Amino-propionyl)-methyl-sulfamoyl] -2-chloro-phenyl}-2- [4-chloro-3-(3-chloro-5-cyano-phenoxy)-2-fluoro-phenyl]-acetamide; compound with hydrochloric acid;
2-[3-(3-Chloro-5-cyano-phenoxy)-4,5-dimethyl-phenyl]-N-(4-sulfamoyl-phenyl)-acetamide;
2-[3-(3-Chloro-5-cyano-phenoxy)-4,5-dimethyl-phenyl]-N-(2-methyl-4-sulfamoyl-phenyl)-acetamide;
2-[3-(3-Chloro-5-cyano-phenoxy)-4,5-dimethyl-phenyl]-N-(2-chloro-4-sulfamoyl-phenyl)-acetamide;
2-[3-(3-Bromo-5-cyano-phenoxy)-4-chloro-2-fluoro-phenyl]-N-(2-methyl-4-sulfamoyl-phenyl)-acetamide;
2-{4-Chloro-3-[3-cyano-5-(3-hydroxy-prop-1-ynyl)-phenoxy]-2-fluoro-phenyl}-N-(2-methyl-4-sulfamoyl-phenyl)-acetamide;
2-{4-Chloro-3-[3-cyano-5-(3-dimethylamino-prop-1-ynyl)-phenoxy]-2-fluoro-phenyl}-N-(2-methyl-4-sulfamoyl-phenyl)-acetamide;
4-(3-Chloro-4-{2-[4-chloro-3-(3-chloro-5-cyano-phenoxy)-2-fluoro-phenyl]-acetylamino}-benzenesulfonylamino)-4-oxo-butyric acid;
2- [4-Bromo-3-(3-chloro-5-cyano-phenoxy)-2-fluoro-phenyl] -N-(2-methyl-4-sulfamoyl-phenyl)-acetamide;
2-[3-(3-Chloro-5-ryano-phenoxy)-2-fluoro-4-methoxy-phenyl]-N-(2-chloro-4-sulfamoyl-phenyl)-acetamide;
2-[3-(3-Chloro-5-cyano-phenoxy)-2-fluoro-4-methoxy-phenyl]-N-(2-methyl-4-sulfamoyl-phenyl)-acetamide; or
2-[4-Chloro-3-(3-chloro-phenoxy)-phenyl]-N-phenyl-acetamide.

11. Compound of formula Ia according to claim 9, which is
2-[4-Chloro-3-(3-chloro-5-cyano-phenoxy)-2-fluoro-phenyl]-N-(2-chloro-4-sulfamoyl-phenyl)-acetamide;
2-[4-Chloro-3-(3-chloro-5-cyano-phenoxy)-2-fluoro-phenyl]-N-(2-chloro-4-propionylsulfamoyl-phenyl)-acetamide; sodium salt;
2-[3-(3-Chloro-5-cyano-phenoxy)-2-fluoro-4-methyl-phenyl]-N-(2-chloro-4-sulfamoyl-phenyl)-acetamide;
2-[4-Bromo-3-(3-chloro-5-cyano-phenoxy)-2-fluoro-phenyl]-N-(2-chloro-4-sulfamoyl-phenyl)-acetamide;
2- [4-Bromo-3-(3-chloro-5-cyano-phenoxy)-2-fluoro-phenyl]-N-(2-chloro-4-propionylsulfamoyl-phenyl)-acetamide; sodium salt;
N-(4-Butyrylsulfamoyl-2-chloro-phenyl)-2-[4-chloro-3-(3-chloro-5-cyano-phenoxy)-2-fluoro-phenyl]-acetamide; sodium salt;
N- [4-((S)-2-Amino-3-methyl-butyrylsulfamoyl)-2-chloro-phenyl]-2- [4-chloro-3-(3-chloro-5-cyano-phenoxy)-2-fluoro-phenyl]-acetamide; sodium salt;
N-(2-Chloro-4-sulfamoyl-phenyl)-2-[3-(3-cyano-5-difluoromethyl-phenoxy)-4-ethyl-2-fluoro-phenyl]-acetamide;
2-[3-(3-Cyano-5-difluoromethyl-phenoxy)-4-ethyl-2-fluoro-phenyl]-N-(2-methyl-4-sulfamoyl-phenyl)-acetamide;
2-[4-Bromo-3-(3-chloro-5-cyano-phenoxy)-2-fluoro-phenyl]-N-(2-methyl-4-sulfamoyl-phenyl)-acetamide;
2-[4-Bromo-3-(3-cyano-5-difluoromethyl-phenoxy)-2-fluoro-phenyl]-N-(2-methyl-4-sulfamoyl-phenyl)-acetamide;
2-[4-Bromo-3-(3-cyano-5-difluoromethyl-phenoxy)-2-fluoro-phenyl]-N-(2-chloro-4-sulfamoyl-phenyl)-acetamide;
2-[4-Chloro-3-(3-cyano-5-difluoromethyl-phenoxy)-2-fluoro-phenyl] -N-(2-methyl-4-sulfamoyl-phenyl)-acetamide;
2-[3-(3-Chloro-5-cyano-phenoxy)-2-fluoro-4-methoxy-phenyl]-N-(2-chloro-4-sulfamoyl-phenyl)-acetamide;
2-[3-(3-Chloro-5-cyano-phenoxy)-2-fluoro-4-methoxy-phenyl]-N-(2-methyl-4-sulfamoyl-phenyl)-acetamide; and,
2-[3-(3-Chloro-5-cyano-phenoxy)-2-fluoro-4-methyl-phenyl]-N-(2-chloro-4-propionylsulfamoyl-phenyl)-acetamide; sodium salt.

12. Compound of formula Ia according to any one of claims 7 to 11 for use as medicament.

13. Use of the compound of formula Ia according to any one of claims 7 to 11 for the manufacture of a medicament for the treatment of diseases mediated by the human immunodeficiency virus (HIV)

14. A pharmaceutical composition comprising a pharmaceutically effective amount of compound or a pharmaceutically acceptable salt thereof as defined in any one of claim 1 to 11 and a pharmaceutical inert carrier.

15. A pharmaceutical composition according to claim 14 for the use in the treatment of diseases mediated by the human immunodeficiency virus (HIV).

## Patentansprüche

1. Verwendung von Verbindungen der Formel I 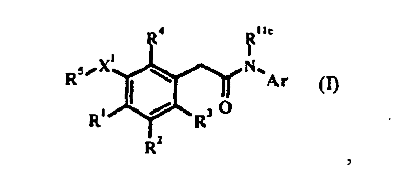 worin
X¹ ausgewählt ist aus der Gruppe, bestehend aus -O-, -S-, -CH₂-, -C(O)-;
R¹ und R² jeweils unabhängig voneinander ausgewählt sind aus der Gruppe, bestehend aus Wasserstoff, C₁₋₆-Alkyl, C₁₋₆-Halogenalkyl, C₃₋₈-Cycloalkyl, C₁₋₆-Alkoxy, C₁₋₆-Alkylthio, C₁₋₆-Alkylsulfinyl, C₁₋₆-Sulfonyl, C₁₋₆-Halogenalkoxy, C₁₋₆-Halogenalkylthio, Halogen, Amino, Alkylamino, Dialkylamino, Acylamino, Nitro und Cyano; oder R¹ und R² zusammen -O-CH=CH- oder -O-CH₂CH₂- sind;
R³ und R⁴ unabhängig voneinander ausgewählt sind aus der Gruppe, bestehend aus Wasserstoff, C₁₋₆-Alkyl, C₁₋₆-Halogenalkyl, C₁₋₆-Alkoxy, C₁₋₆-Halogenalkoxy, C₁₋₆-Alkylthio, C₁₋₆-Halogenalkylthio, Halogen, Amino, Nitro und Cyano;
R⁵ ausgewählt ist aus der Gruppe, bestehend aus C₁₋₆-Alkyl, C₁₋₆-Halogenalkyl, C₃₋₈-Cycloalkyl, Aryl oder Heteroaryl, ausgewählt aus der Gruppe, bestehend aus Pyridinyl, N-Hydroxypyridin, Pyrimidinyl, Indol, Pyrazinyl und Pyrrolyl; wobei das Aryl und das Heteroaryl gegebenenfalls mit ein bis drei Substituenten, unabhängig voneinander ausgewählt aus der Gruppe, bestehend aus C₁₋₆-Alkyl, C₂₋₆-Alkenyl, C₂₋₆-Alkinyl, C₁₋₆-Halogenalkyl, C₃₋₈-Cycloalkyl, C₁₋₆-Alkoxy, C₁₋₆-Alkylthio, C₁₋₆-Alkylsulfinyl, C₁₋₆-Sulfonyl, C₁₋₆-Halogenalkoxy, C₁₋₆-Halogenalkylthio, Hydroxy, Halogen, Amino, C₁₋₆-Alkylamino, C₁₋₆-Dialkylamino, Acylamino, Acyl, C₁₋₆-Alkoxycarbonyl, Carbamoyl, C₁₋₆-N-Alkylcarbamoyl, C₁₋₆-N,N-Dialkylcarbamoyl, Alkinol, -C≡CCH₂OH, -C≡CCH₂N(CH₃)₂, Nitro und Cyano, substituiert sind, das Alkyl und das Cycloalkyl gegebenenfalls mit einem oder zwei Substituenten, unabhängig voneinander ausgewählt aus der Gruppe, bestehend aus Alkyl, Hydroxy, Alkoxy, Thiol, Alkylthio, Halogen, Amino, Alkylamino, Aminoalkyl, Alkylaminoalkyl und Dialkylamino, substituiert sind;
Ar Phenyl, gegebenenfalls substituiert mit 1 bis 3 Substituenten, unabhängig voneinander bei jedem Vorkommen ausgewählt aus der Gruppe, bestehend aus C₁₋₆-Alkyl, C₃₋₈-Cycloalkyl, C₁₋₃-Halogenalkyl, C₁₋₆-Alkoxy, C₁₋₆-Halogenalkoxy, Halogen, Cyano, C₁₋₆-Alkylthio, C₁₋₆-Alkylsulfinyl, C₁₋₆-Sulfonyl, Amino-C₁₋₆-alkylsulfonyl, C₁₋₆-Halogen-alkoxy, C₁₋₆-Halogenalkylthio, C₁₋₆-Acyl, Nitro, C₁₋₆-Heteroalkyl, C₁₋₆-Heteroalkoxy, Hydroxyl, -X²(CH₂)ₚS(O)ₙNR⁸R⁹; -(CH₂)ₚCOOR¹¹; -X²(CH₂)ₚNHC(O)NHR⁸R⁹, X²(CH₂)ₚCONR⁸R⁹, -SO₂R¹³, -NR^{8a}R^{9a}, X²(CH₂)ₚNR¹¹S(O)₂NR⁸R⁹, -X²(CH₂)ₚNHCOOR¹⁰, -X⁶(CH₂)ₚCOOR¹⁰, -X²(CH₂)ₚCN, -OR¹⁵ und C(=O)CH₂N[(CH₂)₂]₂X⁴; oder
ein Heteroarylring, ausgewählt aus der Gruppe, bestehend aus Pyridinyl, Pyridin-N-oxid, Pyridazinyl, Pyrimidyl, Pyrazyl, Triazinyl, Pyrrolyl, Pyrazolyl, Imidazolyl, (1,2,3)- und (1,2,4)-Triazolyl, Pyrazinyl, Pyrimidinyl, Tetrazolyl, Furyl, Thienyl, Isoxazolyl, Thiazolyl, Thienyl, Isoxazolyl, Indol, Indol-N-oxid, Chinolin, Chinolin-N-oxid und Oxazolyl, wobei der Heteroarylring gegebenenfalls mit ein bis drei Substituenten, ausgewählt aus der Gruppe, bestehend aus C₁₋₆-Alkyl, C₁₋₆-Alkoxy, C₁₋₆-Alkylthio, Halogen, C₁₋₆-Aminoacylcyano und NR^{8b}R^{9b}, substituiert ist, ist;
R⁸ und R⁹ voneinander unabhängig sind, einer von R⁸ und R⁹ Wasserstoff oder C₁₋₆-Akyl ist und der andere von R⁸ und R⁹ ausgewählt ist aus der Gruppe, bestehend aus Wasserstoff, -C(=O)R¹⁴, -C(=O)CHR¹²NH₂, -(CH₂)₂N[(CH₂)₂]₂O, COCO₂Me, C₃₋₈-Cycloalkyl, wobei das Cycloalkyl gegebenenfalls substituiert ist mit einem oder zwei Hydroxylsubstituenten, Pyranyl, C₁₋₆-Alkyl und Aryl, wobei die Alkyl- und Arylgruppen gegebenenfalls substituiert sind mit einem oder zwei Substituenten, unabhängig voneinander ausgewählt aus der Gruppe, bestehend aus Hydroxy, C₁₋₆-Alkoxy, Thiol, C₁₋₆-Alkylthio, C₁₋₆-Alkylsulfinyl, C₁₋₆-Sulfonyl und Halogen; oder
(ii) R⁸ und R⁹ zusammengenommen (CH₂)₂-X⁵-(CH₂)₂ oder -(CH₂)ₒ-, gegebenenfalls substituiert mit einem oder zwei Substituenten, ausgewählt aus der Gruppe, bestehend aus Halogen, Hydroxyl und NR^{11a}R^{11b}, sind;
R^{8a} und R^{9a} unabhängig voneinander ausgewählt sind aus der Gruppe, bestehend aus Wasserstoff, C₁₋₆-Alkyl, C(=O)CO₂R¹¹ und SO₂R¹⁰, oder (ii) zusammengenommen (CH₂)ᵣSO₂, (CH₂)₂S(O)ₚ(CH₂)₂ sind;
R¹⁰ C₁₋₆-Alkyl ist;
R¹¹ Wasserstoff oder C₁₋₆-Alkyl ist;
R^{11a}, R^{11b} und R^{11c} unabhängig voneinander R¹¹ sind;
R¹² die Seitenkette einer natürlich vorkommenden α-Aminosäure ist;
R¹³ C₁₋₆-Alkyl; -(CH₂)ₛCO₂R¹¹; -(CH₂)₂CN, -(CH₂)₂NH₂, -(CH₂)ᵤOH ist;
R¹⁴ C₁₋₆-Alkyl, -(CH₂)ₛNHR^{11a}R^{11b}, (CH₂)ₛOR¹¹, -CH₂CH(OH)CH₃, CH₂N[(CH₂)₂]₂O, -(CH₂)₂CO₂R¹¹, gegebenenfalls substituiertes Phenyl oder Pyridinyl ist;
R¹⁵ C₁₋₆-Alkyl, gegebenenfalls substituiert mit ein bis drei Hydroxylgruppen, ist;
X² -O- oder eine Bindung ist;
X⁴ -O- oder -NMe- ist;
X⁵ -O-, -S(O)ₙ- oder NR¹¹ ist;
X⁶ -O- oder -S(O)ₙ- ist;
n eine ganze Zahl von 0 bis 2 ist;
o eine ganze Zahl von 4 bis 6 ist;
p eine ganze Zahl von 0 bis 6 ist;
r eine ganze Zahl von 3 bis 4 ist;
s eine ganze Zahl von 1 bis 2 ist;
u eine ganze Zahl von 2 bis 3 ist;
und Hydraten, Solvaten und Salzen davon
zur Herstellung eines Medikaments zur Behandlung einer HIV-Infektion oder Vorbeugung einer HIV-Infektion oder Behandlung von AIDS oder ARC.

2. Verwendung von Verbindungen der Formel I nach Anspruch 1, worin
X¹ ausgewählt ist aus der Gruppe, bestehend aus -O-, -S-, -CH₂-, -C(O)-;
R¹ und R² jeweils unabhängig voneinander ausgewählt sind aus der Gruppe, bestehend aus Wasserstoff, C₁₋₆-Alkyl, C₁₋₆-Halogenalkyl, C₃₋₈-Cycloalkyl, C₁₋₆-Alkoxy, C₁₋₆-Alkylthio, C₁₋₆-Alkylsulfinyl, C₁₋₆-Sulfonyl, C₁₋₆-Halogenalkoxy, C₁₋₆-Halogenalkylthio, Halogen, Amino, Alkylamino, Dialkylamino, Acylamino, Nitro und Cyano; oder R¹ und R² zusammen -O-CH=CH- oder -O-CH₂CH₂- sind;
R³ und R⁴ unabhängig voneinander ausgewählt sind aus der Gruppe, bestehend aus Wasserstoff, C₁₋₆-Alkyl, C₁₋₆-Halogenalkyl, C₁₋₆-Alkoxy, C₁₋₆-Halogenalkoxy, C₁₋₆-Alkylthio, C₁₋₆-Halogenalkylthio, Halogen, Amino, Nitro und Cyano;
R⁵ ausgewählt ist aus der Gruppe, bestehend aus C₁₋₆-Alkyl, C₁₋₆-Halogenalkyl, C₃₋₈-Cycloalkyl, Aryl oder Heteroaryl, ausgewählt aus der Gruppe, bestehend aus Pyridinyl, N-Hydroxypyridin, Pyrimidinyl, Indol, Pyrazinyl und Pyrrolyl; wobei das Aryl und das Heteroaryl gegebenenfalls mit ein bis drei Substituenten, unabhängig voneinander ausgewählt aus der Gruppe, bestehend aus C₁₋₆-Alkyl, C₂₋₆-Alkenyl, C₂₋₆-Alkinyl, C₁₋₆-Halogenalkyl, C₃₋₈-Cycloalkyl, C₁₋₆-Alkoxy, C₁₋₆-Alkylthio, C₁₋₆-Alkylsulfinyl, C₁₋₆-Sulfonyl, C₁₋₆-Halogenalkoxy, C₁₋₆-Halogenalkylthio, Hydroxy, Halogen, Amino, C₁₋₆-Alkylamino, C₁₋₆-Dialkylamino, Acylamino, Acyl, C₁₋₆-Alkoxycarbonyl, Carbamoyl, C₁₋₆-N-Alkylcarbamoyl, C₁₋₆-N,N-Dialkylcarbamoyl, Nitro und Cyano, substituiert sind, das Alkyl und das Cycloalkyl gegebenenfalls mit einem oder zwei Substituenten, unabhängig voneinander ausgewählt aus der Gruppe, bestehend aus Alkyl, Hydroxy, Alkoxy, Thiol, Alkylthio, Halogen, Amino, Alkylamino, Aminoalkyl, Alkylaminoalkyl und Dialkylamino, substituiert sind;
Ar Phenyl, gegebenenfalls substituiert mit 1 bis 3 Substituenten, unabhängig voneinander bei jedem Vorkommen ausgewählt aus der Gruppe, bestehend aus C₁₋₆-Alkyl, C₃₋₈-Cycloalkyl, C₁₋₃-Halogenalkyl, C₁₋₆-Alkoxy, C₁₋₆-Halogenalkoxy, Halogen, Cyano, C₁₋₆-Alkylthio, C₁₋₆-Alkylsulfinyl, C₁₋₆-Sulfonyl, Amino-C₁₋₆-alkylsulfonyl, C₁₋₆-Halogen-alkoxy, C₁₋₆-Halogenalkylthio, C₁₋₆-Acyl, Nitro, C₁₋₆-Heteroalkyl, C₁₋₆-Heteroalkoxy, Hydroxyl, -X²(CH₂)ₚS(O)ₙNR⁸R⁹; -(CH₂)ₚCOOR¹¹; -X²(CH₂)ₚNHC(O)NHR⁸R⁹, X²(CH₂)ₚCONR⁸R⁹, -SO₂R¹³, -NR^{8a}R^{9a}, X²(CH₂)ₚNR¹¹S(O)₂NR⁸R⁹, -X²(CH₂)ₚNHCOOR¹⁰, -X⁶(CH₂)ₚCOOR¹⁰, -X²(CH₂)ₚCN, -OR¹⁵ und C(=O)CH₂N[(CH₂)₂]₂X⁴; oder
ein Heteroarylring, ausgewählt aus der Gruppe, bestehend aus Pyridinyl, Pyridin-N-oxid, Pyridazinyl, Pyrimidyl, Pyrazyl, Triazinyl, Pyrrolyl, Pyrazolyl, Imidazolyl, (1,2,3)- und (1,2,4)-Triazolyl, Pyrazinyl, Pyrimidinyl, Tetrazolyl, Furyl, Thienyl, Isoxazolyl, Thiazolyl, Thienyl, Isoxazolyl, Indol, Indol-N-oxid, Chinolin, Chinolin-N-oxid und Oxazolyl, wobei der Heteroarylring gegebenenfalls mit einem bis drei Substituenten, ausgewählt aus der Gruppe, bestehend aus C₁₋₆-Alkyl, C₁₋₆-Alkoxy, C₁₋₆-Alkylthio, Halogen, C₁₋₆-Aminoacyl und NR^{8b}R^{9b}, substituiert ist, ist;
R⁸ und R⁹ voneinander unabhängig sind, einer von R⁸ und R⁹ Wasserstoff oder C₁₋₆-Akyl ist und der andere von R⁸ und R⁹ ausgewählt ist aus der Gruppe, bestehend aus Wasserstoff, -C(=O)R¹⁴, -C(=O)CHR¹²NH₂, -(CH₂)₂N[(CH₂)₂]₂O, COCO₂Me, C₃₋₈-Cycloalkyl, wobei das Cycloalkyl gegebenenfalls substituiert ist mit einem oder zwei Hydroxylsubstituenten, Pyranyl, C₁₋₆-Alkyl und Aryl, wobei die Alkyl- und Arylgruppen gegebenenfalls substituiert sind mit einem oder zwei Substituenten, unabhängig voneinander ausgewählt aus der Gruppe, bestehend aus Hydroxy, C₁₋₆-Alkoxy, Thiol, C₁₋₆-Alkylthio, C₁₋₆-Alkylsulfinyl, C₁₋₆-Sulfonyl und Halogen; oder
(ii) R⁸ und R⁹ zusammengenommen (CH₂)₂-X⁵-(CH₂)₂ oder -(CH₂)ₒ-, gegebenenfalls substituiert mit einem oder zwei Substituenten, ausgewählt aus der Gruppe, bestehend aus Halogen, Hydroxyl und NR^{11a} R^{11b}, sind;
R^{8a} und R^{9a} unabhängig voneinander ausgewählt sind aus der Gruppe, bestehend aus Wasserstoff, C₁₋₆-Alkyl, C(=O)CO₂R¹¹ und SO₂R¹⁰ oder (ii) zusammengenommen (CH₂)ᵣSO₂, (CH₂)₂S(O)ₚ(CH₂)₂ sind;
R¹⁰ C₁₋₆-Alkyl ist;
R¹¹ Wasserstoff oder C₁₋₆-Alkyl ist;
R^{11a}, R^{11b} und R^{11c} unabhängig voneinander R¹¹ sind;
R¹² die Seitenkette einer natürlich vorkommenden α-Aminosäure ist;
R¹³ C₁₋₆-Alkyl; -(CH₂)ₛCO₂R¹¹, -(CH₂)₂CN, -(CH₂)₂NH₂, -(CH₂)ᵤOH ist;
R¹⁴ C₁₋₆-Alkyl, -(CH₂)ₛNHR^{11a}R^{11b}, (CH₂)ₛOR¹¹, -CH₂CH(OH)CH₃, CH₂N[(CH₂)₂]₂O, -(CH₂)₂CO₂R¹¹, gegebenenfalls substituiertes Phenyl oder Pyridinyl ist;
R¹⁵ C₁₋₆-Alkyl, gegebenenfalls substituiert mit ein bis drei Hydroxylgruppen, ist;
X² -O- oder eine Bindung ist;
X⁴ -O- oder -NMe- ist;
X⁵ -O-, -S(O)ₙ- oder NR¹¹ ist;
X⁶ -O- oder -S(O)ₙ- ist;
n eine ganze Zahl von 0 bis 2 ist;
o eine ganze Zahl von 4 bis 6 ist;
p eine ganze Zahl von 0 bis 6 ist;
r eine ganze Zahl von 3 bis 4 ist;
s eine ganze Zahl von 1 bis 2 ist;
u eine ganze Zahl von 2 bis 3 ist;
und Hydraten, Solvaten und Salzen davon
zur Herstellung eines Medikaments zur Behandlung einer HIV-Infektion oder Vorbeugung einer HIV-Infektion oder Behandlung von AIDS oder ARC.

3. Verwendung von Verbindungen der Formel I nach Anspruch 1 oder 2, wobei
X¹ ausgewählt ist aus der Gruppe, bestehend aus -O-, -S-, -CH₂-, -C(O)-;
R¹ und R² jeweils unabhängig voneinander ausgewählt sind aus der Gruppe, bestehend aus Wasserstoff, C₁₋₆-Alkyl, C₁₋₆-Alkoxy und Halogen, oder R¹ und R² zusammen -O-CH=CH- oder -O-CH₂CH₂- sind;
R³ und R⁴ unabhängig voneinander ausgewählt sind aus der Gruppe, bestehend aus Wasserstoff und Halogen;
R⁵ Aryl oder Heteroaryl, ausgewählt aus der Gruppe, bestehend aus Pyridinyl, N-Hydroxypyridin, Pyrimidinyl, Indol, Pyrazinyl und Pyrrolyl, ist; wobei das Aryl und das Heteroaryl unsubstituiert oder mit ein bis drei Substituenten, unabhängig voneinander ausgewählt aus der Gruppe, bestehend aus C₁₋₆-Alkyl, C₁₋₆-Halogenalkyl, C₁₋₆-Halogenalkoxy, C₁₋₆-Halogenalkoxy, Halogen, -C≡CCH₂OH, -C≡CCH₂N(CH₃)₂ und Cyano, substituiert sind;
Ar Phenyl, gegebenenfalls substituiert mit 1 bis 3 Substituenten, unabhängig voneinander ausgewählt aus der Gruppe, bestehend aus C₁₋₆-Alkyl, C₃₋₈-Cycloalkyl, C₁₋₃-Halogenalkyl, C₁₋₆-Alkoxy, Halogen, Cyano, C₁₋₆-Alkylsulfinyl, C₁₋₆-Sulfonyl, Amino-C₁₋₆-alkylsulfonyl, C₁₋₆-Halogenalkoxy, C₁₋₆-Halogenalkylthio, C₁₋₆-Acyl, Nitro, C₁₋₆-Heteroalkyl, C₁₋₆-Heteroalkoxy, Hydroxyl, -X²(CH₂)ₚS(O)ₙNR⁸R⁹; -(CH₂)ₚCOOR¹¹; -X²(CH₂)ₚNHC(O)NHR⁸R⁹, X²(CH₂)ₚCONR⁸R⁹, -SO₂R¹³, -NR^{8a}R^{9a}, -X²(CH₂)ₚNR¹¹S(O)₂NR⁸R⁹, -X²(CH₂)ₚNHCOOR¹⁰, -X⁶(CH₂)ₚCOOR¹⁰, -X²(CH₂)ₚCN, -OR¹⁵ und C(=O)CH₂N[(CH₂)₂]₂X⁴; oder
ein Heteroarylring, ausgewählt aus der Gruppe, bestehend aus Pyridinyl, Pyridin-N-oxid, Pyridazinyl, Pyrimidyl, Pyrazyl, Triazinyl, Pyrrolyl, Pyrazolyl, Imidazolyl, (1,2,3)- und (1,2,4)-Triazolyl, Pyrazinyl, Pyrimidinyl, Tetrazolyl, Furyl, Thienyl, Isoxazolyl, Thiazolyl, Thienyl, Isoxazolyl, Indol, Indol-N-oxid, Chinolin, Chinolin-N-oxid und Oxazolyl, wobei der Heteroarylring gegebenenfalls mit ein bis drei Substituenten, ausgewählt aus der Gruppe, bestehend aus C₁₋₆-Alkyl, C₁₋₆-Alkoxy, C₁₋₆-Alkylthio, Halogen, C₁₋₆-Aminoacylcyano und NR^{8b}R^{9b}, substituiert ist, ist;
R⁸ und R⁹ voneinander unabhängig sind, einer von R⁸ und R⁹ Wasserstoff oder C₁₋₆-Akyl ist und der andere von R⁸ und R⁹ ausgewählt ist aus der Gruppe, bestehend aus Wasserstoff, -C(=O)R¹⁴, -C(=O)CHR¹²NH₂, -(CH₂)₂N[(CH₂)₂]₂O, COCO₂Me, C₃₋₈-Cycloalkyl, wobei das Cycloalkyl gegebenenfalls substituiert ist mit einem oder zwei Hydroxylsubstituenten, Pyranyl, C₁₋₆-Alkyl und Aryl, wobei die Alkyl- und Arylgruppen gegebenenfalls substituiert sind mit einem oder zwei Substituenten, unabhängig voneinander ausgewählt aus der Gruppe, bestehend aus Hydroxy, C₁₋₆-Alkoxy, Thiol, C₁₋₆-Alkylthio, C₁₋₆-Alkylsulfinyl, C₁₋₆-Sulfonyl und Halogen; oder
(ii) R⁸ und R⁹ zusammengenommen (CH₂)₂-X⁵-(CH₂)₂ oder -(CH₂)ₒ-, gegebenenfalls substituiert mit einem oder zwei Substituenten, ausgewählt aus der Gruppe, bestehend aus Halogen, Hydroxyl und NR^{11a}R^{11b}, sind;
R^{8a} und R^{9a} unabhängig voneinander ausgewählt sind aus der Gruppe, bestehend aus Wasserstoff, C₁₋₆-Alkyl, C(=O)CO₂R¹¹ und SO₂R¹⁰, oder (ii) zusammengenommen (CH₂)ᵣSO₂, (CH₂)₂S(O)ₚ(CH₂)₂ sind;
R¹⁰ C₁₋₆-Alkyl ist;
R¹¹ Wasserstoff oder C₁₋₆-Alkyl ist;
R^{11a}, R^{11b} und R^{11c} unabhängig voneinander R¹¹ sind;
R¹² die Seitenkette einer natürlich vorkommenden α-Aminosäure ist;
R¹³ C₁₋₆-Alkyl; -(CH₂)ₛCO₂R¹¹, -(CH₂)₂CN, -(CH₂)₂NH₂, -(CH₂)ᵤOH ist;
R¹⁴ C₁₋₆-Alkyl, -(CH₂)ₛNHR^{11a}R^{11b}, (CH₂)ₛOR¹¹, -CH₂CH(OH)CH₃, CH₂N[(CH₂)₂]₂O, -(CH₂)₂CO₂R¹¹, gegebenenfalls substituiertes Phenyl oder Pyridinyl ist;
R¹⁵ C₁₋₆-Alkyl, gegebenenfalls substituiert mit ein bis drei Hydroxylgruppen, ist;
X² -O- oder eine Bindung ist;
X⁴ -O- oder -NMe- ist;
X⁵ -O-, -S(O)ₙ- oder NR¹¹ ist;
X⁶ -O- oder -S(O)ₙ- ist;
n eine ganze Zahl von 0 bis 2 ist;
o eine ganze Zahl von 4 bis 6 ist;
p eine ganze Zahl von 0 bis 6 ist;
r eine ganze Zahl von 3 bis 4 ist;
s eine ganze Zahl von 1 bis 2 ist;
u eine ganze Zahl von 2 bis 3 ist;
und Hydraten, Solvaten und Salzen davon
zur Herstellung eines Medikaments zur Behandlung einer HIV-Infektion oder Vorbeugung einer HIV-Infektion oder Behandlung von AIDS oder ARC.

4. Verwendung von Verbindungen der Formel I 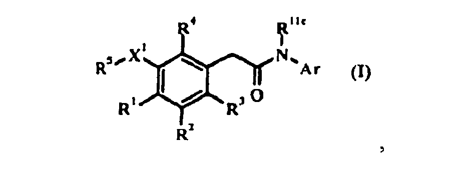 worin
X¹ ausgewählt ist aus der Gruppe, bestehend aus -O-, -S-, -CH₂-, -C(O)-;
R¹ und R² unabhängig voneinander ausgewählt sind aus der Gruppe, bestehend aus Wasserstoff, Methyl, Ethyl, i-Propyl, Methoxy, Chlor und Brom; oder R¹ und R² zusammen -O-CH=CH- oder -O-CH₂CH₂- sind;
R³ und R⁴ unabhängig voneinander ausgewählt sind aus der Gruppe, bestehend aus Wasserstoff und Fluor;
R⁵ Phenyl, wobei das Phenyl unsubstituiert oder mit ein bis drei Substituenten, unabhängig voneinander ausgewählt aus der Gruppe, bestehend aus Methyl, Ethyl, CHF₂, CF₃, -CF₂CH₃, -OCHF₂, Chlor, Brom, Fluor, -C≡CCH₂OH, -C≡CCH₂N(CH₃)₂ und Cyano, substituiert ist,
oder Pyridinyl, wobei das Pyridinyl unsubstituiert oder mit ein bis drei Substituenten, unabhängig voneinander ausgewählt aus der Gruppe, bestehend aus Methyl und Cyano, substituiert ist, ist;
Ar Phenyl, wobei das Phenyl unsubstituiert oder mit 1 bis 3 Substituenten, ausgewählt aus der Gruppe, bestehend aus Methyl, Ethyl, i-Propyl, 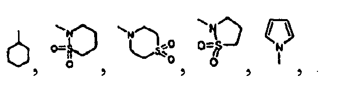 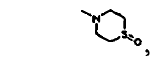 -CF₃, Methoxy, Chlor, Fluor, Cyano, Hydroxyl, -SCH₃, -SO₂NH₂, -SO₂NHCH₃, -SO₂NCH₃CH₂CH₂OH, -SO₂NHCH₂CHCH₃OH, -SO₂NHCH₂CH₂CH₂OH, -SO₂NHCH₂CH₂ 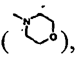 -SO₂ 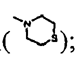 -SO₂N(CH₃)₂, -SO₂ 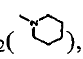 -SO₂ 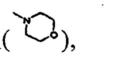 -SO₂ 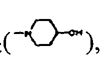 -SO₂NH 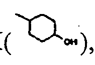 -SO₂ 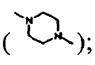 -SO₂ 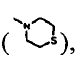 -SO₂NHCOCH₂CH₃, -SO₂NHCOCH₂CH₂CH₃, -SO₂NHCOCH(NH₂)CH(CH₃)₂, -SO₂NHCOCH₂ 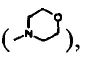 -SO₂NCH₃COCH₂CH₂CH₃, -SO₂NHCOCH₂CH₂CH₂CH₃, -SO₂NHCOCH₂CH(CH₃)₂, -SO₂NHCOCH₂CH(CH2CH₃)₂, -SO₂NHCOCH₂CH₂CH(CH₃)₂, -S0₂NHCOCH₂CH₂NH₂, -SO₂NCH₃COCH₂CH₂NH₂, -SO₂NHCOCH₂OCH₃, -SO₂NHCO 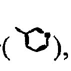 -SO₂NH 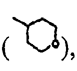 -SO₂NHCOCH(i-Propyl)NH₂, -SO₂NHCH₂CH₂SCH₃, -SO₂NCH₃COCH₂CH₃, -SO₂NCH₃COCH₂OCH₃, -SO₂NCH₃COCH₂CH₂OCH₃, -SO₂NHCOCH₂OCH₃, -SO₂NHCOCH₂OH, -SO₂NHCOCH₂CH₂COOH, -SO₂CH₂CH₂NH₂, -SO₂CH₂COOH, -SO₂CH₂CH₂COOH, -SO₂CH₃, -SO₂CH₂CH₂CN, -SO₂CH₂CH₂CH₂OH, -O(CH₂)₃SO₂NH₂, -OCH₂COOH, -OCH₂COOCH₃, -OCH₂CN, -OCH₂CHOHCH₂OH, -OCH₂CH₂CHOHCH₂OH, -SCH₂COOCH₃, -COOCH₃, -COOH, -COCH₂ 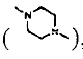 , -COCH₂ 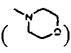 , -CH₂COOCH₃, -CH₂COOH, -CH₂CH₂COOCH₃, CH₂CH₂CH₂COOCH₃, -N(CH₃)₂, -NHCOCOOCH₂CH₃, -NHSO₂CH₃ und -NCH₃SO₂CH₃, substituiert ist; oder
ein Heteroarylring, ausgewählt aus der Gruppe, bestehend aus Pyridinyl, Pyrazolyl und Triazolyl, wobei das Heteroaryl unsubstituiert oder mit einem oder zwei Substituenten, ausgewählt aus der Gruppe, bestehend aus Methyl, Methoxy, -SCH₃ und -NHCOOCH₃, substituiert ist, ist;
R^{11c} Wasserstoff oder Methyl ist;
und Hydraten, Solvaten und Salzen davon.

5. Verwendung einer Verbindung der Formel I nach Anspruch 1 oder 4, wobei die Verbindung
2-[4-Chlor-3-(3-chlor-5-cyano-phenoxy)-phenyl]-N-(4-sulfamoyl-phenyl)-acetamid;
2-[4-Chlor-3-(3,5-dicyano-phenoxy)-phenyl]-N-(4-sulfamoyl-phenyl)-acetamid;
2-[3-(2-Brom-5-chlor-phenoxy)-4-chlor-phenyl]-N-[2-methyl-4-(3-sulfamoyl-propoxy)-phenyl]-acetamid;
2-[3-(2-Brom-5-chlor-phenoxy)-4-chlor-phenyl]-N-(4-sulfamoyl-phenyl)-acetamid;
2-[4-Chlor-3-(3,5-dicyano-phenoxy)-phenyl]-N-(2-chlor-phenyl)-acetamid;
2-[4-Chlor-3-(3,5-dicyano-phenoxy)-phenyl]-N-(4-dimethylamino-phenyl)-acetamid;
2-[3-(2-Chlor-5-cyano-phenoxy)-4-ethyl-2-fluor-phenyl]-N-(4-sulfamoyl-phenyl)-acetamid;
2-[4-Chlor-3-(2,5-dicyano-phenoxy)-2-fluor-phenyl]-N-(4-sulfamoyl-phenyl)-acetamid;
2-[4-Chlor-3-(3,5-dicyano-phenoxy)-2-fluor-phenyl]-N-(4-sulfamoyl-phenyl)-acetamid;
2-[4-Chlor-2-fluor-3-(2,3,5-trichlor-phenoxy)-phenyl]-N-(4-sulfamoyl-phenyl)-acetamid;
2-[3-(3-Cyano-5-fluor-phenoxy)-4-methyl-phenyl]-N-(4-sulfamoyl-phenyl)-acetamid;
2-(2-Chlor-phenyl)-2-[3-(3-cyano-5-fluor-phenoxy)-4-methyl-phenyl]-acetamid;
2-[4-Chlor-3-(3-cyano-5-difluormethyl-phenoxy)-2-fluor-phenyl]-N-(4-sulfamoyl-phenyl)-acetamid;
2-[4-Chlor-3-(3-cyano-5-difluormethyl-phenoxy)-2-fluor-phenyl]-N-(2-chlor-phenyl)-acetamid;
2-[3-(2-Brom-S-chlor-phenoxy)-4-ethyl-phenyl]-N-(4-sulfamoyl-phenyl)-acetamid;
2-[3-(2-Brom-5-chlor-phenoxy)-4-ethyl-phenyl]-N-(2-chlor-phenyl)-acetamid;
2-[3-(5-Chlor-2-cyano-phenoxy)-4-ethyl-phenyl]-N-(4-sulfamoyl-phenyl)-acetamid;
2-[3-(5-Chlor-2-cyano-phenoxy)-4-ethyl-phenyl]-N-(2-chlor-phenyl)-acetamid;
2-[3-(2,5-Dichlor-phenoxy)-4-ethyl-phenyl]-N-(4-sulfamoyl-phenyl)-acetamid;
2-[3-(2,6-Dichlor-phenoxy)-4-ethyl-phenyl]-N-(4-sulfamoyl-phenyl)-acetamid;
N-(2-Chlor-phenyl)-2-[3-(2,5-dichlor-phenoxy)-4-ethyl-phenyl]-acetamid;
2-[3-(3-Brom-5-chlor-phenoxy)-4-ethyl-phenyl]-N-(4-sulfamoyl-phenyl)-acetanlid;
N-(2-Chlor-phenyl)-2-[3-(2,6-dichlor-phenoxy)-4-ethyl-phenyl]-acetanlid;
2-[3-(3-Brom-5-chlor-phenoxy)-4-ethyl-phenyl]-N-(2-chlor-phenyl)-acetamid;
N-(2-Chlor-phenyl)-2-[3-(3,5-dicyano-phenoxy)-4-ethyl-phenyl]-acetamid;
2-[3-(2-Brom-5-chlor-phenoxy)-4-isopropyl-phenyl]-N-(4-sulfamoyl-phenyl)-acetamid;
2-[3-(2-Brom-5-chlor-phenoxy)-4-isopropyl-phenyl]-N-(2-chlor-phenyl)-acetamid;
2-[4-Chlor-3-(3,5-dicyano-phenoxy)-2-fluor-phenyl]-N-(2-chlor-phenyl)-acetamid;
2-[4-Chlor-3-(3,5-dicyano-phenoxy)-phenyl]-N-[2-methyl-4-(3-sulfamoyl-propoxy)-phenyl]-acetamid;
2-[3-(2-Brom-5-chlor-phenoxy)-4-methyl-phenyl]-N-(4-sulfamoyl-phenyl)-acetamid;
2-[3-(2-Brom-5-chlor-phenoxy)-4-methyl-phenyl]-N-(2-methyl-4-sulfamoyl-phenyl)₋acetamid;
2-[4-Chlor-3-(3,5-dicyano-phenoxy)-2-fluor-phenyl]-N-(2-methyl-4-sulfamoyl-phenyl)-acetamid;
2-[3-(2-Brom-5-chlor-phenoxy)-4-methyl-phenyl]-N-(2-chlor-phenyl)-acetamid;
N-(2-Chlor-phenyl)-2-[3-(3,5-dicyano-phenoxy)-4-methyl-phenyl]-acetamid;
2-[3-(3-Brom-2,5-dichlor-phenoxy)-4-ethyl-phenyl]-N-(4-sulfamoyl-phenyl)-acetamid;
2-[3-(3-Brom-2,5-dichlor-phenoxy)-4-ethyl-phenyl]-N-(2-methyl-4-sulfamoyl-phenyl)-acetamid;
2-[3-(3-Brom-2,5-dichlor-phenoxy)-4-ethyl-phenyl]-N-(2-chlor-phenyl)-acetamid;
N-(2-Chlor-phenyl)-2-[3-(3,5-dibrom-2-chlor-phenoxy)-4-ethyl-phenyl]-acetamid;
2-[3-(5-Brom-2-chlor-3-fluor-phenoxy)-4-ethyl-phenyl]-N-(4-sulfamoyl-phenyl)-acetamid;
2-[3-(5-Brom-2-chlor-3-fluor-phenoxy)-4-ethyl-phenyl]-N-(2-methyl-4-sulfamoyl-phenyl)-acetamid;
2-[3-(5-Brom-2-chlor-3-fluor-phenoxy)-4-ethyl-phenyl]-N-(2-chlor-phenyl)-acetamid;
2-[4-Chlor-3-(3-chlor-5-cyano-phenoxy)-2-fluor-phenyl]-N-(2-methyl-4-sulfamoyl-phenyl)-acetamid:
2-[3-(3,5-Dicyano-phenoxy)-4-methl-phenyl]-N-(2-methyl-4-sulfamoyl-phenyl)-acetamid;
N-(2-Chlor-4-sulfamoyl-phenyl)-2-[3-(3,5-dicyano-phenoxy)-4-methyl-phenyl]-acetamid;
2-[3-(3-Chlor-cynano-phenoxy)-4-ethyl-phenyl]-N-(4-sulfamoyl-phenyl)-acetamid;
2-[3-(3-Chlor-5-cyano-phenoxy)-4-ethyl-phenyl]-N-(2-methyl-4-sulfamoyl-phenyl)-acetamid;
2-[3-(3-Chlor-5-cyano-phenoxy)-4-ethyl-phenyl]-N-(2-chlor-4-sulfamoyl-phenyl)-acetamid;
2-[3-(3-Chlor-5-cyano-phenoxy)-4-ethyl-phenyl]-N-(4-methylsulfamoyl-phenyl)-acetamid;
2-[3-(2-Chlor-5-cyano-3-fluor-phenoxy)-4-ethyl-phenyl]-N-(4-sulfamoyl-phenyl)-acetamid;
2-[3-(2-Chlor-5-cyano-3-fluor-phenoxy)-4-ethyl-phenyl]-N-(2-methyl-4-sulfamoyl-phenyl)-acetamid;
2-[4-Chlor-3-(3-chlor-5-cyano-phenoxy)-2-fluor-phenyl]-N-(2-methyl-4-propionylsulfamoyl-phenyl)-acetamid;
2-[3-(2-Chlor-3,5-dicyano-phenoxy)-4-ethyl-phenyl]-N-(4-sulfamoyl-phenyl)-acetamid;
2-[3-(2-Chlor-3,5-dicyano-phenoxy)-4-ethyl-phenyl]-N-(2-methyl-4-sulfamoyl-phenyl)-acetamid;
2-[3-(2-Chlor-3,5-dicyano-phenoxy)-4-ethyl-phenyl]-N-(2-chlor-4-sulfamoyl-phenyl)-acetamid;
2-[4-Chlor-3-(4-cyano-2,6-dimethyl-phenoxy)-phenyl]-N-(4-sulfamoyl-phenyl)-acetamid;
2-[4-Chlor-3-(4-cyano-2,6-dimethyl-phenoxy)-phenyl]-N-(2-methyl-4-sulfamoyl-phenyl)-acetamid;
2-[3-(3-Chlor-5-cyano-phenoxy)-4-methoxy-phenyl]-N-(4-sulfamoyl-phenyl)-acetamid;
2-[3-(3-Chlor-5-cyano-phenoxy)-4-ethyl-phenyl]-N-(2-chlor-phenyl)-acetamid;
2-[3-(2-Chlor-5-cyano-3-fluor-phenoxy)-4-ethyl-phenyl]-N-(2-chlor-phenyl)-acetamid;
2-[3-(2-Chlor-3,5-dicyano-phenoxy)-4-ethyl-phenyl]-N-(2-chlor-phenyl)-acetamid;
2-[3-(3-Chlor-5-cyano-phenoxy)-4-methoxy-phenyl]-N-(2-chlor-phenyl)-acetamid;
2-[3-(2-Chlor-5-cyano-phenoxy)-4-methoxy-phenyl]-N-(2-chlor-phenyl)-acetamid;
2-[4-Brom-3-(3-chlor-5-cyano-phenoxy)-2-fluor-phenyl]-N-(2-chlor-4-propionylsulfamoyl-phenyl)-acetamid; Natriumsalz;
2-[3-(3-Chlor-5-cyano-phenoxy)-4-methoxy-phenyl]-N-(2-chlor-4-sulfamoyl-phenyl)-acetamid;
2-[3-(2-Chlor-5-cyano-phenoxy)-4-methoxy-phenyl]-N-(4-sulfamoyl-phenyl)-acetamid;
2-[3-(2-Chlor-5-cyano-phenoxy)-4-methoxy-phenyl]-N-(2-methyl-4-sulfamoyl-phenyl)-acetamid;
2-[3-(2-Chlor-5-cyano-phenoxy)-4-methoxy-phenyl]-N-(2-chlor-4-sulfamoyl-phenyl)-acetamid;
2-[4-Chlor-3-(4-cyano-2,6-dimethyl-phenoxy)-phenyl]-N-(2-chlor-phenyl)acetanlid;
2-[4-Chlor-3-(3,5-dicyano-phenoxy)-phenyl]-N-(4-methylsulfamoyl-phenyl)-acetamid;
2-[4-Chlor-3-(3,5-dicyano-phenoxy)-phenyl]-N-(4-dimethylsulfamoyl-phenyl)-acetamid;
2-[4-Chlor-3-(3,5-dicyano-phenoxy)-phenyl]-N-[4-(piperidin-1-sulfonyl)-phenyl]-acetamid;
2-[4-Chlor-3-(3,5-dicyano-phenoxy)-phenyl]-N-[4-(morpholin-4-sulfonyl)-phenyl]-acetamid;
2-[3-(3-Chlor-5-cyano-phenoxy)-4-methoxy-phenyl]-N-(2-methyl-4-sulfamoyl-phenyl)-acetamid;
2-[3-(3-Chlor-5-cyano-phenoxy)-2-fluor-4-methyl-phenyl]-N-(2-methyl-4-sulfamoyl-phenyl)-acetamid;
2-[4-Chlor-3-(3-cyano-5-difluormethyl-phenoxy)-2-fluor-phenyl]-N-(2-methyl-4-sulfamoyl-phenyl)-acetamid;
2-[4-Chlor-3-(3,5-dicyano-phenoxy)-phenyl]-N-(2-chlor-4-methansulfonyl-phenyl)acetamid;
N-[4-((S)-2-Amino-3-methyl-butyrylsulfamoyl)-2-methyl-phenyl]-2-[4-chlor-3-(3-chlor-5-cyano-phenoxy)-2-fluor-phenyl]-acetamid; Verbindung mit Salzsäure;
2-[4-Chlor-3-(3,5-dicyano-phenoxy)-phenyl]-N-(4-cyanomethoxy-phenyl)-acetamid;
2-[4-Chlor-3-(3,5-dicyano-phenoxy)-phenyl]-N-(4-methansulfonyl-phenyl)-acetamid;
3-Chlor-4-{2-[4-chlor-3-(3,5-dicyano-phenoxy)-phenyl]-acetylamino}-benzoesäure-methylester;
2-[4-Chlor-3-(3,5-dicyano-phenoxy)-phenyl]-N- {4-[(2-hydroxy-ethyl)-methyl-sulfamoyl]-phenyl} -acetamid;
2-[4-Chlor-3-(3,5-dicyano-phenoxy)-phenyl]-N-[4-(4-hydroxy-piperidin-1-sulfonyl)-phenyl]-acetamid;
2-[3-(3-Chlor-5-cyano-phenoxy)-2-fluor-4-methyl-phenyl]-N-(2-chlor-4-sulfamoyl-phenyl)-acetamid;
N-(2-Chlor-4-sulfamoyl-phenyl)-2-[3-(3-cyano-5-difluormethyl-phenoxy)-2-fluor-4-methyl-phenyl]-acetamid;
2-[3-(3-Cyano-5-difluormethyl-phenoxy)-2-fluor-4-methyl-phenyl]-N-(2-methyl-4-sulfamoyl-phenyl)-acetamid;
N-(2-Chlor-phenyl)-2-[3-(3-cyano-5-difluormethyl-phenoxy)-2-fluor-4-methyl-phenyl]-acetamid;
2-[4-Chlor-3-(3-chlor-5-cyano-phenoxy)-2-fluor-phenyl]-N-{2-methyl-4-[(pyridin-3-carbonyl)-sulfamoyl]-phenyl}-acetamid; Verbindung mit Salzsäure;
2-[4-Chlor-3-(3-cyano-5-trifluormethyl-phenoxy)-phenyl]-N-(4-sulfamoyl-phenyl)-acetamid;
2-[4-Chlor-3-(3-cyano-5-trifluormethyl-phenoxy)-phenyl]-N-(2-methyl-4-sulfamoyl-phenyl)-acetamid;
2-[4-Chlor-3-(3-cyano-5-trifluormethyl-phenoxy)-phenyl]-N-(2-chlor-4-sulfamoyl-phenyl)-acetamid;
2-[3-(3-Cyano-5-difluormethyl-phenoxy)-4-ethyl-phenyl]-N-(4-sulfamoyl-phenyl)-acetamid;
2-[3-(3-Cyano-5-difluormethyl-phenoxy)-4-methoxy-phenyl]-N-(4-sulfamoyl-phenyl)-acetamid;
2-[3-(3-Cyano-5-difluormethyl-phenoxy)-4-methoxy-phenyl]-N-(2-methyl-4-sulfamoyl-phenyl)-acetamid;
N-(2-Chlor-4-sulfamoyl-phenyl)-2-[3-(3-cyano-5-difluormethyl-phenoxy)-4-methoxy-phenyl]-acetamid;
2- {4-Chlor-3-[3-cyano-5-(1,1-difluor-ethyl)-phenoxy]-2-fluor-phenyl}-N-(4-sulfamoyl-phenyl)-acetamid;
2- {4-Chlor-3-[3-cyano-5-(1,1-difluor-ethyl)-phenoxy]-2-fluor-phenyl}-N-(2-methyl-4-sulfamoyl-phenyl)-acetamid;
2-{4-Chlor-3-[3-cyano-5-(1,1-difluor-ethyl)-phenoxy]-2-fluor-phenyl}-N-(2-chlor-4-sulfamoyl-phenyl)-acetamid;
2-[4-Chlor-3-(3-chlor-5-cyano-phenoxy)-2-fluor-phenyl]-N-(2-chlor-phenyl)-acetamid;
2-[4-Chlor-3-(3-chlor-5-cyano-phenoxy)-2-fluor-phenyl]-N-[2-methyl-4-methyl-propionyl-sulfamoyl)-phenyl]-acetamid;
2-[4-Chlor-3-(3-chlor-5-cyano-phenoxy)-2-fluor-phenyl]-N-(2-chlor-4-sulfanloyl-phenyl)-acetamid;
2-[3-(3-Cyano-5-difluormethyl-phenoxy)-4-ethyl-phenyl]-N-(2-methyl-4-sulfanloyl-phenyl)-acetamid;
2-[3-(2-Chlor-5-cyano-phenoxy)-5-methoxy-phenyl]-N-(4-sulfamoyl-phenyl)-acetamid;
2-[3-(2-Chlor-5-cyano-phenoxy)-5-methoxy-phenyl]-N-(2-methyl-4-sulfanloyl-phenyl)-acetamid;
2-[4,5-Dibrom-3-(3-cyano-5-difluormethyl-phenoxy)-2-fluor-phenyl]-N-(2-methyl-4-sulfamoyl-phenyl)-acetamid;
2-[3-(2-Chlor-5-cyano-phenoxy)-5-ethyl-phenyl]-N-(4-sulfamoyl-phenyl)-acetamid;
2-[3-(2-Chlor-5-cyano-phenoxy)-5-ethyl-phenyl]-N-(2-methyl-4-sulfamoyl-phenyl)-acetamid;
2-[3-(2-Chlor-5-cyano-phenoxy)-5-ethyl-phenyl]-N-(2-chlor-4-sulfamoyl-phenyl)-acetamid;
2-[4-Chlor-3-(3-chlor-5-cyano-phenoxy)-2-fluor-phenyl]-N-(2-methyl-4-methylsulfamoyl-phenyl)-acetamid;
2-[4-Brom-3-(3-chlor-5-cyano-phenoxy)-2-fluor-phenyl]-N-(2-chlor-4-sulfamoyl-phenyl)-acetamid;
3-Chlor-4-(2-[3-(3-cyano-5-difluormethyl-phenoxy)-2-fluor-4-methyl-phenyl]-acetylamino)-benzoesäure;
2-[4-Chlor-3-(3-chlor-5-cyano-phenoxy)-2-fluor-phenyl]-N-(2-chlor-phenyl)-N-methyl-acetamid;
N-(2-Chlor-4-methansulfonyl-phenyl)-2-[3-(3-cyano-5-difluormethyl-phenoxy)-2-fluor-4-methyl-phenyl]-acetamid;
2-[3-(2-Chlor-5-cyano-phenoxy)-5-methoxy-phenyl]-N-(2-chlor-4-sulfamoyl-phenyl)-acetamid;
2-[4,5-Dibrom-3-(3-cyano-5-difluormethyl-phenoxy)-2-fluor-phenyl]-N-(4-sulfamoyl-phenyl)-acetamid;
2-[3-(3-Chlor-5-cyano-phenoxy)-4-methyl-phenyl]-N-[2-methyl-4-(morpholin-4-sulfonyl)-phenyl]-acetamid;
2-[3-(3-Chlor-5-cyano-phenoxy)-4-methyl-phenyl]-N-[2-methyl-4-(4-methyl-piperazin-1-sulfonyl)-phenyl]-acetamid;
2-[3-(3-Chlor-5-cyano-phenoxy)-4-methyl-phenyl]-N-[2-methyl-4-(thiomorpholin-4-sulfonyl)-phenyl]-acetamid;
2-[3-(3-Chlor-5-cyano-phenoxy)-4-methyl-phenyl]-N-[2-methyl-4-(2-morpholin-4-ylethyl-sulfamoyl)-phenyl]-acetamid;
2-[3-(3-Chlor-5-cyano-phenoxy)-4-methyl-phenyl]-N-[4-(3-hydroxy-propylsulfamoyl)-2-methyl-phenyl]-acetamid;
2-[4-Chlor-3-(3-cyano-5-trifluormethyl-phenoxy)-2-fluor-phenyl]-N-(2-methyl-4-sulfamoyl-phenyl)-acetamid;
2-[3-(3-Chlor-5-cyano-phenoxy)-4-methyl-phenyl]-N-[4-(4-hydroxy-cyclohexylsulfamoyl)-2-methyl-phenyl]-acetamid;
2-[3-(3-Chlor-5-cyano-phenoxy)-4-methyl-phenyl]-N-(2-methyl-4-sulfamoyl-phenyl)acetamid;
2-[3-(3-Chlor-5-cyano-phenoxy)-4-methyl-phenyl]-N-(2-chlor-4-sulfamoyl-phenyl)acetamid;
3-(4-{2-[3-(3-Chlor-5-cyano-phenoxy)-4-methyl-pheny]-acetylamino}-3-methyl-benzolsulfonyl)-propionsäure;
2-[4-Chlor-3-(3-chlor-5-cyano-phenoxy)-2-fluor-phenyl]-N-(2-chlor-4-propionylsulfamoy-phenyl)-acetamid; Natriumsalz;
N-[4-((S)-2-Amino-3-methyl-butyrylsulfamoyl)-2-chlor-phenyl]-2-[4-chlor-3-(3-chlor-5-cyano-phenoxy)-2-fluor-phenyl]-acetamid; Natriumsalz;
2-[3-(3-Chlor-5-cyano-phenoxy)-4-methyl-phenyl]-N-[2-methyl-4-(tetrahydro-pyran-4-yl-sulfamoyl)-phenyl]-acetamid;
2-[3-(3-Chlor-5-cyano-phenoxy)-4-methyl-phenyl]-N-[4-(2-hydroxy-propylsulfanloyl)-2-methyl-phenyl]-acetamid;
2-[3-(3-Chlor-5-cyano-phenoxy)-4-methyl-phenyl]-N-[2-methyl-4-(2-methylsulfanyl-ethyl-sulfamoyl)-phenyl]-acetamid;
2-[3-(3-Chlor-5-cyano-phenoxy)-4-methyl-phenyl]-N-[2-methyl-4-(3-methylsulfanyl-propyl-sulfamoyl)-phenyl]-acetamid;
2-[3-(3-Chlor-5-cyano-phenoxy)-5-methoxy-phenyl]-N-(4-sulfamoyl-phenyl)-acetamid;
2-[3-(3-Chlor-5-cyano-phenoxy)-5-methoxy-phenyl]-N-(2-methyl-4-sulfamoyl-phenyl)-acetamid;
N-(2-Chlor-4-sulfamoyl-phenyl)-2-[4,5-dibrom-3-(3-cyano-5-difluormethyl-phenoxy)-2-fluor-phenyl]-acetamid;
2-[4-Chlor-3-(4-cyano-2,6-dimethyl-phenoxy)-phenyl]-N-(2-chlor-4-sulfamoyl-phenyl)-acetamid;
2-[3-(3-Chlor-5-cyano-phenoxy)-5-methoxy-phenyl]-N-(2-chlor-4-sulfamoyl-phenyl)-acetamid;
2-[3-(3-Chlor-5-cyano-phenoxy)-4-methyl-phenyl]-N-[4-(2-cyano-ethansulfonyl)-2-methyl-phenyl]-acetamid;
N-(4-Butyrylsulfamoyl-2-chlor-phenyl)-2-[4-chlor-3-(3-chlor-5-cyano-phenoxy)-2-fluorphenyl]-acetamid; Natriumsalz;
N-[4-(Butyryl-methyl-sulfamoyl)-2-chlor-phenyl]-2-[4-chlor-3-(3-chlor-5-cyano-phenoxy)-2-fluor-phenyl]-acetamid;
N-(2-Chlor-4-sulfamoyl-phenyl)-2-[3-(3-cyano-5-difluormethyl-phenoxy)-4-ethyl-phenyl]-acetamid;
2-[3-(3-Chlor-5-cyano-phenoxy)-5-methyl-phenyl]-N-(4-sulfamoyl-phenyl)-acetamid;
2-[3-(3-Chlor-5-cyano-phenoxy)-5-methyl-phenyl]-N-(2-methyl-4-sulfamoyl-phenyl)-acetamid;
2-[3-(3-Chlor-5-cyano-phenoxy)-5-methyl-phenyl]-N-(2-chlor-4-sulfamoyl-phenyl)-acetamid;
2-[7-(3-Cyano-phenoxy)-benzofuran-5-yl]-N-(4-sulfanloyl-phenyl)-acetamid;
2-[7-(3-Cyano-phenoxy)-benzofuran-5-yl]-N-(2-methyl-4-sulfamoyl-phenyl)-acetamid;
2-[3-(2-Chlor-5-cyano-phenoxy)-5-methyl-phenyl]-N-(4-sulfamoyl-phenyl)-acetanlid;
2-[3-(2-Chlor-5-cyano-phenoxy)-5-methyl-phenyl]-N-(2-methyl-4-sulfamoyl-phenyl)-acetamid;
2-[3-(2-Chlor-5-cyano-phenoxy)-5-methyl-phenyl]-N-(2-chlor-4-sulfanloyl-phenyl)-acetamid;
N-(2-Chlor-4-sulfamoyl-phenyl)-2-[7-(3,5-dichlor-phenoxy)-benzofuran-5-yl]-acetamid;
2-[3-(3-Chlor-5-cyano-phenoxy)-4-methyl-phenyl]-N-[4-((S)-2,3-dihydroxypropoxy)-2-methyl-phenyl]-acetamid;
2-[3-(3-Chlor-5-cyano-phenoxy)-4-methyl-phenyl]-N-[4-((R)-3,4-dihydroxy-butoxy)-2-methyl-phenyl]-acetamid;
(4-{2-[3-(3-Chlor-5-cyano-phenoxy)-4-methyl-phenyl]-acetylamino}-3-methyl-benzolsulfonyl)-essigsäure;
N-[4-(3-Amino-propionylsulfamoyl)-2-chlor-phenyl]-2-[4-chlor-3-(3-chlor-5-cyano-phenoxy)-2-fluor-phenyl]-acetamid; Verbindung mit Salzsäure;
N-[4-(2-Amino-ethansulfonyl)-2-methyl-phenyl]-2-[3-(3-chlor-5-cyano-phenoxy)-4-methyl-phenyl]-acetamid;
2-[4-Chlor-3-(3-chlor-5-cyano-phenoxy)-2-fluor-phenyl]-N-[2-chlor-4-(2-methoxy-acetyl-sulfamoyl)-phenyl]-acetamid; Natriumsalz;
2-[4-Chlor-3-(3-chlor-5-cyano-phenoxy)-2-fluor-phenyl]-N-[2-chlor-4-(2-hydroxy-acetyl-sulfamoyl)-phenyl]-acetamid; Natriumsalz;
2-[3-(3-Chlor-5-cyano-phenoxy)-4-methyl-phenyl]-N-[4-(3-hydroxy-propan-1-sulfonyl)-2-methyl-phenyl]-acetamid;
2-[3-(3-Chlor-5-cyano-phenoxy)-4-methyl-phenyl]-N-[4-(1,1-dioxo-1λ6-[1,2]thiazinan-2-yl)-2-methyl-phenyl]-acetamid;
2-[3-(3-Chlor-5-cyano-phenoxy)-4-methyl-phenyl]-N-{2-methyl-4-[2-(4-methyl-piperazin-1-yl)-acetyl]-phenyl}-acetamid;
2-[3-(3-Chlor-5-cyano-phenoxy)-4-methyl-phenyl]-N-[2-methyl-4-(1-oxo-1λ4-thiomorpholin-4-yl)-phenyl]-acetamid;
2-[3-(3-Chlor-5-cyano-phenoxy)-4-methyl-phenyl]-N-[4-(1,1-dioxo-1λ6-thiomorpholin-4-yl)-2-methyl-phenyl]-acetamid;
2-[4-Chlor-3-(3-chlor-5-cyano-phenoxy)-2-fluor-phenyl]-N-[2-chlor-4-(methyl-propionyl-sulfamoyl)-phenyl]-acetamid;
2-[3-(3-Chlor-5-cyano-phenoxy)-4-methyl-phenyl]-N-[2-methyl-4-(2-morpholin-4-ylacetyl)-phenyl]-acetamid; Verbindung mit Trifluoressigsäure;
2-[4-Chlor-3-(3-chlor-5-cyano-phenoxy)-2-fluor-phenyl]-N-[2-chlor-4-(3-methoxy-propionylsulfamoyl)-phenyl]-acetamid; Natriumsalz;
2-[4-Brom-3-(3-cyano-5-difluormethyl-phenoxy)-2-fluor-phenyl]-N-(2-chlor-4-sulfamoyl-phenyl)-acetamid;
2-[4-Chlor-3-(3-cyano-5-difluormethoxy-phenoxy)-2-fluor-phenyl]-N-(4-sulfamoyl-phenyl)-acetamid;
2-[4-Chlor-3-(3-cyano-5-difluormethoxy-phenoxy)-2-fluor-phenyl]-N-(2-chlor-4-sulfamoyl-phenyl)-acetamid,
2-[4-Chlor-3-(3-chlor-5-cyano-phenoxy)-2-fluor-phenyl]-N-(2-chlor-4-methylsulfamoyl-phenyl)-acetamid;
2-[4-Brom-3-(3-cyano-5-difluormethyl-phenoxy)-2-fluor-phenyl]-N-(2-methyl-4-sulfamoyl-phenyl)-acetamid;
2-[3-(3-Chlor-5-cyano-phenoxy)-4-methyl-phenyl]-N-[4-(1,1-dioxo-1λ6-isothiazolidin-2-yl)-2-methyl-phenyl]-acetamid;
2-[4-Chlor-3-(3-chlor-5-cyano-phenoxy)-2-fluor-phenyl]-N- {2-chlor-4-[(2-methoxy-acetyl)-methyl-sulfamoyl]-phenyl}-acetamid;
2-[4-Chlor-3-(3-chlor-5-cyano-phenoxy)-2-fluor-phenyl]-N-{2-chlor-4-[(3-methoxy-propionyl)-methyl-sulfamoyl]-phenyl}-acetamid;
2-[4-Chlor-3-(3-chlor-5-cyano-phenoxy)-2-fluor-phenyl]-N-(2-chlor-4-pentanoylsulfamoyl-phenyl)-acetamid; Natriumsalz;
2-[4-Chlor-3-(3-chlor-5-cyano-phenoxy)-2-fluor-phenyl]-N-[2-chlor-4-(3-methyl-butyrylsulfamoyl)-phenyl]-acetamid; Natriumsalz;
2-[4-Chlor-3-(3-chlor-5-cyano-phenoxy)-2-fluor-phenyl]-N-[2-chlor-4-(2-diethylamino-acetylsulfamoyl)-phenyl]-acetamid; Verbindung mit Salzsäure: 2-[4-Chlor-3-(3-chlor-5-cyano-phenoxy)-2-fluor-phenyl]-N-[2-chlor-4-(4-methyl-pentanoyl-sulfamoyl)-phenyl]-acetamid; Natriumsalz;
2-[4-Chlor-3-(3-chlor-5-cyano-phenoxy)-2-fluor-phenyl]-N-[2-chlor-4-(2-morpholin-4-yl-acetylsulfamoyl)-phenyl]-acetamid; Verbindung mit Salzsäure;
N-(4-{2-[3-(3-Chlor-5-cyano-phenoxy)-4-methyl-phenyl]-acetylamino}-3-methyl-phenyl)-carbamoylameisensäure-ethylester;
N-(2-Chlor-4-sulfamoyl-phenyl)-2-[3-(3-cyano-5-difluormethyl-phenoxy)-4-ethyl-2-fluorphenyl]-acetamid;
2-[3-(3-Cyano-5-difluormethyl-phenoxy)-4-ethyl-2-fluor-phenyl]-N-(2-methyl-4-sulfamoyl-phenyl)-acetamid;
N-{4-[(3-Amino-propionyl)-methyl-sulfamoyl]-2-chlor-phenyl}-2-[4-chlor-3-(3-chlor-5-cyano-phenoxy)-2-fluor-phenyl]-acetamid; Verbindung mit Salzsäure;
2-[3-(3-Chlor-5-cyano-phenoxy)-4,5-dimethyl-phenyl]-N-(4-sulfanloyl-phenyl)-acetamid;
2-[3-(3-Chlor-5-cyano-phenoxy)-4,5-dimethyl-phenyl]-N-(2-methyl-4-sulfamoyl-phenyl)-acetamid;
2-[3-(3-Chlor-5-cyano-phenoxy)-4,5-dimethyl-phenyl]-N-(2-chlor-4-sulfamoyl-phenyl)-acetamid;
2-[3-(3-Brom-5-cyano-phenoxy)-4-chlor-2-fluor-phenyl]-N-(2-methyl-4-sulfamoyl-phenyl)-acetamid;
2-{4-Chlor-3-[3-cyano-5-(3-hydroxy-prop-1-inyl)-phenoxy]-2-fluor-phenyl}-N-(2-methyl-4-sulfamoyl-phenyl)-acetamid;
2-{4-Chlor-3-[3-cyano-5-(3-dimethylamino-prop-1-inyl)-phenoxy]-2-fluor-phenyl}-N-(2-methyl-4-sulfamoyl-phenyl)-acetamid;
4-(3-Chlor-4-{2-[4-chlor-3-(3-chlor-5-cyano-phenoxy)-2-fluor-phenyl]acetylamino}-benzolsulfonylamino)-4-oxo-buttersäure;
2-[4-Brom-3-(3-chlor-5-cyano-phenoxy)-2-fluor-phenyl]-N-(2-methyl-4-sulfamoyl-phenyl)-acetamid;
2-[3-(3-Chlor-5-cyano-phenoxy)-2-fluor-4-methoxy-phenyl]-N-(2-chlor-4-sulfamoyl-phenyl)-acetamid;
2-[3-(3-Chlor-5-cyano-phenoxy)-2-fluor-4-methoxy-phenyl]-N-(2-methyl-4-sulfamoyl-phenyl)-acetamid;
2-[4-Chlor-3-(3-chlor-phenoxy)-phenyl]-N-phenyl-acetamid;
2-[3-(2-Brom-5-chlor-phenoxy)-4-chlor-phenyl]-N-(4-sulfamoyl-phenyl)-acetamid;
2-[4-Chlor-3-(3,5-dicyano-phenoxy)-phenyl]-N-(2-pyrrol-1-yl-phenyl)-acetamid;
2-[4-Chlor-3-(3,5-dicyano-phenoxy)-phenyl]-N-(2-fluor-phenyl)-acetamid;
2-[4-Chlor-3-(3,5-dicyano-phenoxy)-phenyl]-N-(2-methoxy-phenyl)-acetamid:
2-[4-Chlor-3-(3,5-dicyano-phenoxy)-phenyl]-N-(2-isopropyl-phenyl)-acetamid;
2-[4-Chlor-3-(3,5-dicyano-phenoxy)-phenyl]-N-o-tolyl-acetamid;
2-[4-Chlor-3-(3,5-dicyano-phenoxy)-phenyl]-N-(2-ethyl-phenyl)-acetamid;
2-[4-Chlor-3-(3,5-dicyano-phenoxy)-phenyl]-N-(2,3-dimethyl-phenyl)-acetamid;
2-[4-Chlor-3-(3,5-dicyano-phenoxy)-phenyl]-N-(4-methoxy-2-methyl-phenyl)acetamid;
2-[4-Chlor-3-(3,5-dicyano-phenoxy)-phenyl]-N-(4-cydohexyl-phenyl)-acetanlid;
2-[4-Chlor-3-(3,5-dicyano-phenoxy)-phenyl]-N-(2-cyano-phenyl)-acetamid;
2-[4-Chlor-3-(3,5-dicyano-phenoxy)-phenyl]-N-(2-trifluormethyl-phenyl)-acetamid;
2-{2-[4-Chlor-3-(3,5-dicyano-phenoxy)-phenyl]-acetylamino}-4,5-dimethoxy-benzoesäure-methylester;
2-[3-(3-Chlor-5-cyano-phenoxy)-4-ethyl-phenyl]-N-phenyl-acetamid;
2-[3-(2-Chlor-3,5-dicyano-phenoxy)-4-ethyl-phenyl]-N-phenyl-acetamid;
2-[4-Chlor-3-(2,5-dichlor-benzyl)-phenyl]-N-(2-chlor-4-sulfamoyl-phenyl)acetamid;
2-[3-(3-Chlor-5-cyano-phenoxy)-4-methoxy-phenyl]-N-phenyl-acetamid;
2-[4-Chlor-3-(3,5-dicyano-phenoxy)-phenyl]-N-phenyl-acetamid;
2-[4-Chlor-3-(3,5-dicyano-phenoxy)-phenyl]-N-(4-methyl-3-sulfamoyl-phenyl)-acetamid;
2-[4-Chlor-3-(3,5-dicyano-phenoxy)-phenyl]-N-(3-methansulfonyl-phenyl)-acetamid;
(4-{2-[4-Chlor-3-(3,5-dicyano-phenoxy)-phenyl]-acetylamino}-phenoxy)-essigsäure-methylester;
3-(4-{2-[4-Chlor-3-(3,5-dicyano-phenoxy)-phenyl]-acetylamino}-phenyl)-propionsäure-methylester;
(4-{2-[4-Chlor-3-(3,5-dicyano-phenoxy)-phenyl]-acetylamino}-phenylsulfanyl)-essigsäure-methylester;
4-(4-{2-[4-Chlor-3-(3,5-dicyano-phenoxy)-phenyl]-acetylamino}-phenyl)-buttersäuremethylester;
2-[4-Chlor-3-(3,5-dicyano-phenoxy)-phenyl]-N-(2-fluor-5-methansulfonyl-phenyl)-acetamid;
(4-{2-[4-Chlor-3-(3,5-dicyano-phenoxy)-phenyl]-acetylamino}-phenyl)-essigsäure-methylester;
2-[4-Chlor-3-(3,5-dicyano-phenoxy)-phenyl]-N-(4-cyano-phenyl)-acetamid;
4-{2-[4-Chlor-3-(3,5-dicyano-phenoxy)-phenyl]-acetylamino}-benzoesäure-methylester
2-[4-Chlor-3-(3,5-dicyano-phenoxy)-phenyl]-N-(4-hydroxy-phenyl)-acetamid;
2-[3-(3-Chlor-5-cyano-phenoxy)-4-methyl-phenyl]-N-(4-methyl-pyridin-3-yl)-acetamid;
2-[3-(3-Chlor-5-cyano-phenoxy)-4-methyl-phenyl]-N-(3-methyl-pyridin-2-yl)-acetamid;
2-[3-(3-Chlor-5-cyano-phenoxy)-4-methyl-phenyl]-N-(2,5-dimethyl-2H-pyrazol-3-yl)-acetamid;
2-[3-(3-Chlor-5-cyano-phenoxy)-4-methyl-phenyl]-N-(2-methyl-2H-pyrazol-3-yl)-acetamid;
N-(6-Acetylamino-4-methyl-pyridin-3-yl)-2-[3-(3-chlor-5-cyano-phenoxy)-4-methyl-phenyl]-acetamid;
2-[3-(3-Chlor-5-cyano-phenoxy)-4-methyl-phenyl]-N-(6-methoxy-2-methyl-pyridin-3-yl)-acetamid;
2-[3-(3-Chlor-5-cyano-phenoxy)-4-methyl-phenyl]-N-(2-methyl-5-methylsulfanyl-2H-[1,2,4]triazol-3-yl)-acetamid;
N-(2-Chlor-phenyl)-2-[3-(3,5-dichlor-benzoyl)-5-methyl-phenyl]-acetamid;
2-[3-(3,5-Dichlor-benzoyl)-5-methyl-phenyl]-N-(2-methyl-4-sulfamoyl-phenyl)acetamid;
(4-{2-[3-(3-Chlor-5-cyano-phenoxy)-4-methyl-phenyl]-acetylamino}-3-methyl-phenoxy)-essigsäure;
(3-Chlor-4-{2-[3-(3-chlor-5-cyano-phenoxy)-4-methyl-phenyl]-acetylamino}-phenyl)-essigsäure-methylester;
(3-Chlor-4-{2-[3-(3-chlor-5-cyano-phenoxy)-4-methyl-phenyl]-acetylamino}-phenyl)-essigsäure;
2-[3-(3-Chlor-5-cyano-phenoxy)-4-methyl-phenyl]-N-(4-methansulfonylamino-2-methyl-phenyl)-acetamid;
2-[3-(3-Chlor-5-cyano-phenoxy)-4-methyl-phenyl]-N-[4-(methansulfonyl-methyl-amino)-2-methyl-phenyl]-acetamid;
4- {2-[4-Chlor-3-(3-clor-5-cyano-phenoxy)-2-fluor-phenyl]-acetylamino}-3-methyl-benzamid;
2-[7-(4-Chlor-benzoyl)-2,3-dihydro-benzofuran-5-yl]-N-(2-chlor-phenyl)-acetamid;
2-[7-(4-Chlor-benzoyl)-2,3-dihydro-benzofuran-5-yl]-N-(4-sulfamoyl-phenyl)-acetamid;
2-[4-Chlor-3-(3,5-dicyano-phenylsulfanyl)-phenyl]-N-(4-sulfamoyl-phenyl)-acetamid;
2-[3-(2-Brom-5-chlor-phenylsulfanyl)-4-chlor-phenyl]-N-(4-sulfamoyl-phenyl)-acetamid;
2-[3-(2-Brom-5-chlor-phenylsulfanyl)-4-chlor-phenyl]-N-(2-chlor-phenyl)-acetamid;
2-[4-Chlor-3-(3,5-dichlor-benzoyl)-phenyl]-N-(2-chlor-phenyl)-acetamid;
2-[4-Chlor-3-(2,5-dichlor-benzoyl)-phenyl]-N-(2-chlor-phenyl)-acetamid;
2-[4-Chlor-3-(2;5-dichlor-benzoyl)-phenyl]-N-(2-methyl-4-sulfamoyl-phenyl)-acetamid
2-[4-Chlor-3-(2,5-dichlor-benzoyl)-phenyl]-N-(4-sulfamoyl-phenyl)-acetamid:
3-[4-Chlor-3-(2,5-dichlor-benzoyl)-phenyl]-N-phenyl-acetamid;
2-[4-Chlor-3-(3,5-dichlor-benzoyl)-phenyl]-N-(2-methyl-4-sulfamoyl-phenyl)-acetamid;
2-[4-Chlor-3-(2,5-dichlor-benzoyl)-phenyl]-N-(2-chlor-4-sulfamoyl-phenyl)-acetamid;
2-[3-(4-Chlor-benzoyl)-5-methyl-phenyl]-N-(4-sulfamoyl-phenyl)-acetamid;
2-[3-(4-Chlor-benzoyl)-5-methyl-phenyl]-N-(2-methyl-4-sulfamoyl-phenyl)-acetamid;
2-[3-(2-Chlor-benzoyl)-5-methyl-phenyl]-N-(2-methyl-4-sulfamoyl-phenyl)-acetamid;
2-[3-(4-Chlor-benzoyl)-5-methyl-phenyl]-N-(2-chlor-4-sulfamoyl-phenyl)-acetamid;
2-[3-(4-Chlor-benzoyl)-5-methyl-phenyl]-N-(2-chlor-phenyl)-acetamid;
2-[3-(2-Chlor-benzoyl)-5-methyl-phenyl]-N-(2-chlor-phenyl)-acetamid;
2-[3-(3-Chlor-benzoyl)-5-methyl-phenyl]-N-(2-methyl-4-sulfamoyl-phenyl)-acetamid;
2-[3-(3-Chlor-benzoyl)-5-methyl-phenyl]-N-(4-sulfamoyl-phenyl)-acetamid;
2-[3-(3-Chlor-benzoyl)-5-methyl-phenyl]-N-(2-chlor-phenyl)=acetamid;
2-[3-(2-Chlor-benzoyl)-5-methyl-phenyl]-N-(4-sulfamoyl-phenyl)-acetamid;
2-[3-(5-Brom-2-chlor-benzoyl)-4-methoxy-phenyl]-N-(2-methyl-4-sulfamoyl-phenyl)-acetamid;
2-[3-(5-Cyano-2-methyl-benzoyl)-4-methyl-phenyl]-N-(2-methyl-4-sulfamoyl-phenyl)-acetamid;
2-[3-(5-Cyano-2-methyl-benzoyl)-4-methyl-phenyl]-N-(4-sulfamoyl-phenyl)-acetamid;
N-(2-Chlor-phenyl)-2-[3-(5-cyano-2-methyl-benzoyl)-4-methyl-phenyl]-acetamid;
2-[3-(5-Cyano-2-ethyl-benzoyl)-4-ethyl-phenyl]-N-(2-methyl-4-sulfamoyl-phenyl)acetamid;
2-[3-(3,5-Dichlor-benzoyl)-5-methyl-phenyl]-N-(4-sulfamoyl-phenyl)-acetamid oder
2-[3-(3-Chlor-5-cyano-phenoxy)-2-fluor-4-methyl-phenyl]-N-(2-chlor-4-propionylsulfamoyl-phenyl)-acetamid, Natriumsalz
ist.

6. Verwendung einer Verbindung der Formel I nach Anspruch 5, die
2-[4-Chlor-3-(3-chlor-5-cyano-phenoxy)-2-fluor-phenyl]-N-(2-chlor-4-sulfamoyl-phenyl)-acetamid;
2-[4-Chlor-3-(3-chlor-5-cyano-phenoxy)-2-fluor-phenyl]-N-(2-chlor-4-propionylsulfamoyl-phenyl)-acetamid; Natriumsalz;
2-[3-(3-Chlor-5-cyano-phenoxy)-2-fluor-4-methyl-phenyl]-N-(2-chlor-4-sulfamoyl-phenyl)-acetamid;
2-[4-Brom-3-(3-chlor-5-cyano-phenoxy)-2-fluor-phenyl]-N-(2-chlor-4-sulfamoyl-phenyl)-acetamid;
2-[4-Brom-3-(3-chlor-5-cyano-phenoxy)-2-fluor-phenyl]-N-(2-chlor-4-propionylsulfamoyl-phenyl)-acetamid; Natriumsalz;
N-(4-Butyrylsulfamoyl-2-chlor-phenyl)-2-[4-chlor-3-(3-chlor-5-cyano-phenoxy)-2-fluorphenyl]-acetamid; Natriumsalz;
N-[4-((S)-2-Amino-3-methyl-butyrylsulfamoyl)-2-chlor-phenyl]-2-[4-chlor-3-(3-chlor-5-cyano-phenoxy)-2-fluor-phenyl]-acetamid; Natriumsalz;
N-(2-Chlor-4-sulfamoyl-phenyl)-2-[3-(3-cyano-5-difluormethyl-phenoxy)-4-ethyl-2-fluorphenyl]-acetamid;
2-[3-(3-Cyano-5-difluormethyl-phenoxy)-4-ethyl-2-fluor-phenyl]-N-(2-methyl-4-sulfamoyl-phenyl)-acetamid;
2-[4-Brom-3-(3-chlor-5-cyano-phenoxy)-2-fluor-phenyl]-N-(2-methyl-4-sulfamoyl-phenyl)-acetamid;
2-[4-Brom-3-(3-cyano-5-difluormethyl-phenoxy)-2-fluor-phenyl]-N-(2-methyl-4-sulfamoyl-phenyl)-acetamid;
2-[4-Brom-3-(3-cyano-S-difluormethyl-phenoxy)-2-fluor-phenyl]-N-(2-chlor-4-sulfamoyl-phenyl)-acetamid;
2-[4-Chlor-3-(3-cyano-5-difluormethyl-phenoxy)-2-fluor-phenyl]-*N*-(2-methyl-4-sulfamoyl-phenyl)-acetamid;
2-[3-(3-Chlor-5-cyano-phenoxy)-2-fluor-4-methoxy-phenyl]-N-(2-chlor-4-sulfamoyl-phenyl)-acetamid;
2-[3-(3-Chlor-5-cyano-phenoxy)-2-fluor-4-methoxy-phenyl]-N-(2-methyl-4-sulfamoyl-phenyl)-acetamid oder
2-[3-(3-Chlor-5-cyano-phenoxy)-2-fluor-4-methyl-phenyl]-N-(2-chlor-4-propionylsulfamoyl-phenyl)-acetamid; Natriumsalz ist.

7. Verbindung gemäß Formel Ia 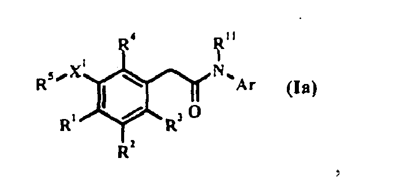 worin
X¹ -O- ist;
R¹ und R² jeweils unabhängig voneinander ausgewählt sind aus der Gruppe, bestehend aus Wasserstoff, C₁₋₆-Alkyl, C₁₋₆-Halogenalkyl, C₃₋₈-Cycloalkyl, C₁₋₆-Alkoxy, C₁₋₆-Alkylthio, C₁₋₆-Alkylsulfinyl, C₁₋₆-Sulfonyl, C₁₋₆-Halogenalkoxy, C₁₋₆-Halogenalkylthio, Halogen, Amino, Alkylamino, Dialkylamino, Aminoacyl, Nitro und Cyano; oder (ii) R¹ und R² zusammen -O-CH=CH- oder -O-CH₂CH₂- sind, mit der Maßgabe, dass R¹ nicht Wasserstoff ist;
R³ und R⁴ unabhängig voneinander ausgewählt sind aus der Gruppe, bestehend aus Wasserstoff, C₁₋₆-Alkyl, C₁₋₆-Halogenalkyl, C₁₋₆-Alkoxy, C₁₋₆-Halogenalkoxy, C₁₋₆-Alkylthio, C₁₋₆-Halogenalkylthio, Halogen, Amino, Nitro und Cyano;
R⁵ Aryl, substituiert mit ein bis drei Substituenten, unabhängig voneinander ausgewählt aus der Gruppe, bestehend aus C₁₋₆-Alkyl, C₂₋₆-Alkenyl, C₂₋₆-Alkinyl, -C≡CCH_{2O}H, -C≡CCH₂NMe₂, C₁₋₆-Halogenalkyl, C₃₋₈-Cycloalkyl, C₁₋₆-Alkoxy, C₁₋₆-Alkylthio, C₁₋₆-Alkylsulfinyl, C₁₋₆-Sulfonyl, C₁₋₆-Halogenalkoxy, C₁₋₆-Halogenalkylthio, Hydroxy, Halogen, Nitro und Cyano, ist, wobei Alkyl und Cycloalkyl gegebenenfalls mit ein oder zwei Substituenten, unabhängig voneinander ausgewählt aus der Gruppe, bestehend aus Alkyl, Hydroxy, Alkoxy, Thiol, Alkylthio, Halogen, Amino, Alkylamino, Aminoalkyl, Alkylaminoalkyl und Dialkylamino, substituiert sind;
Ar ein substituierter Phenylring nach Formel IIa ist, mit der Maßgabe, dass R^{7a} und R^{7c}, nicht beide Wasserstoff sind oder, wenn R^{7c}, Wasserstoff ist, dann R^{7a} Chlor ist: 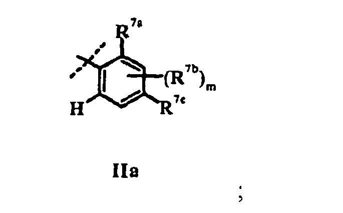
R^{7a} ausgewählt ist aus der Gruppe, bestehend aus Wasserstoff, C₁₋₆-Alkyl, C₁₋₃-Alkyl Halogenalkyl, C₁₋₆₋Alkoxy, C₁₋₆-Halogenalkoxy, Halogen und Cyano;
R^{7b} unabhängig voneinander ausgewählt ist aus der Gruppe, bestehend aus C₁₋₆-Halogenalkyl, C₁₋₆-Alkoxy, C₁₋₆-Alkylthio, C₁₋₆-Alkylsulfinyl, C₁₋₆-Sulfonyl, Amino-C₁₋₆-alkylsulfonyl, SO₂NR^{11a}R^{11b}, C₁₋₆-Halogenalkoxy, C₁₋₆-Halogenalkylthio, Hydroxy, Amino, C₁₋₆-Alkylamino, C₁₋₆-Dialkylamino, Aminoacyl, Acyl, CONR⁸R⁹, Nitro, Cyano, C₁₋₆-Heteroalkoxy, -X²(CH₂)ₚS(O)₂NR⁸R⁹, -X²(CH₂)ₚNHC(O)NHR⁸R⁹, -X²(CH₂)ₚNHS(O)₂NR⁸R⁹ und -X²(CH₂)ₚNHCOOR¹⁰;
R^{7c} ausgewählt ist aus der Gruppe, bestehend aus Wasserstoff, C₁₋₆-Heteroalkoxy, S(O)₂NR⁸R⁹, -X²CH₂(CH₂)ₚS(O)₂NR⁸R⁹; -X²(CH₂)ₚNHC(O)NHR⁸R⁹, X⁶(CH₂)ᵥCONR⁸R⁹, -SO₂R¹³, -NR⁸R⁹, X²(CH₂)ₚNR¹¹S(O)₂NR⁸R⁹, -X2 (CH₂)pNHCOOR¹⁰, -X⁶C(CH₂)ᵥCOOR¹⁰, -X⁶(CH₂)ᵥCN, -OR¹⁵ und C(=O)CH₂N[(CH₂)₂]₂X⁴;
R⁸ R⁹ und voneinander unabhängig sind, einer von R⁸ und R⁹ Wasserstoff oder C₁₋₆-Alkyl ist und der andere von R⁸ und R⁹ ausgewählt ist aus der Gruppe, bestehend aus Wasserstoff, -C(=O)R¹⁴, -C(=O)CR¹²NH₂, -(CH₂)₂N[(CH₂)₂]O, COCO₂Me, C₃₋₈-Cycloalkyl, wobei Cycloalkyl gegebenenfalls substituiert ist mit einem oder zwei Hydroxylsubstituenten, Pyranyl, C₁₋₆-Alkyl und Aryl, wobei die Alkyl- und Arylgruppen gegebenenfalls substituiert sind mit einem oder zwei Substituenten, unabhängig voneinander ausgewählt aus der Gruppe, bestehend aus Hydroxy, C₁₋₆-Alkoxy, Thiol, C₁₋₆-Alkylthio, C₁₋₆-Alkylsulfinyl, C₁₋₆-Sulfonyl und Halogen; oder zusammen (CH₂)₂-X⁵-(CH₂)₂, -(CH₂)ₒ- oder (CH₂)ᵣS(O)ₙ, gegebenenfalls substituiert mit einem oder zwei Substituenten, ausgewählt aus der Gruppe, bestehend aus Halogen, Hydroxyl und NR^{11a}R^{11b}, sind;
R¹⁰ C₁₋₆-Alkyl ist;
R¹¹ Wasserstoff oder C₁₋₆-Alkyl ist;
R^{11a} und R^{11b} unabhängig voneinander R¹¹ sind;
R¹² die Seitenkette einer natürlich vorkommenden α-Aminosäure ist;
R¹³ C₁₋₆-Alkyl, -(CH₂)ₛCO₂R¹¹, -(CH₂)₂CN, -(CH₂)₂NH₂, -(CH₂)ᵤOH ist;
R¹⁴ C₁₋₁₀-Alkyl, -(CH₂)ₛNHR^{11a}R^{11b}, -(CH₂)ₛOR¹¹ -CH₂CH(OH)CH₃, -CH₂N[(CH₂)₂]₂O, -(CH₂)₂CO₂R¹¹ gegebenenfalls substituiertes Phenyl oder Pyridinyl ist;
R¹⁵ C₁₋₆-Alkyl, substituiert mit ein bis drei Hydroxylgruppen, ist;
X² -O- oder eine Bindung ist;
X⁴ -O- oder -NMe- ist;
X⁵ -O-, -S(O)ₙ- oder NR¹¹ ist;
X⁶ -O- oder -S(O)ₙ- ist;
m eine ganze Zahl von 0 bis 2 ist;
n eine ganze Zahl von 0 bis2 ist;
o eine ganze Zahl von 4 bis 6 ist;
p eine ganze Zahl von 0 bis 6 ist;
r eine ganze Zahl von 3 bis 4 ist;
s eine ganze Zahl von 1 bis 2 ist;
u eine ganze Zahl von 2 bis 3 ist;
v eine ganze Zahl von 2 bis 6 ist; und
Hydrate, Solvate und Säureadditionssalze oder Salze der konjugierten Base davon.

8. Verbindung der Formel Ia nach Anspruch 7, worin
X¹ -O- ist;
R¹ und R² jeweils unabhängig voneinander ausgewählt sind aus der Gruppe, bestehend aus Wasserstoff, C₁₋₆-Alkyl, C₁₋₆-Alkoxy und Halogen, oder R¹ und R² zusammen -O-CH=CH- sind, mit der Maßgabe, dass R¹ nicht Wasserstoff ist;
R³ und R⁴ unabhängig voneinander ausgewählt sind aus der Gruppe, bestehend aus Wasserstoff und Halogen;
R⁵ Aryl, substituiert mit ein bis drei Substituenten, unabhängig voneinander ausgewählt aus der Gruppe, bestehend aus C₁₋₆-Alkyl, -C≡ CCH₂OH, -C≡ CCH₂NMe₂, C₁₋₆-Halogenalkyl, C₁₋₆-Halogenalkoxy, Cyano und Halogen, ist;
Ar ein substituierter Phenylring nach Formel IIa ist, mit der Maßgabe, dass R^{7a} und R^{7c} nicht beide Wasserstoff sind oder, wenn R^{7c} Wasserstoff ist, dann R^{7a} Chlor ist: 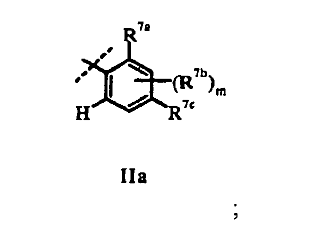
R^{7a} ausgewählt ist aus der Gruppe, bestehend aus Wasserstoff, C₁₋₆-Alkyl, C₁₋₃-Halogenalkyl, C₁₋₆-Alkoxy, C₁₋₆-Halogenalkoxy, Halogen und Cyano;
R^{7b} unabhängig voneinander ausgewählt ist aus der Gruppe, bestehend aus C₁₋₆-Halogenalkyl, C₁₋₆-Alkoxy, C₁₋₆-Alkylthio, C₁₋₆-Alkylsulfinyl, C₁₋₆-Sulfonyl, Amino-C₁₋₆-alkylsulfonyl, SO₂NR^{11a}R^{11b}, C₁₋₆-Halogenalkoxy, C₁₋₆-Halogenalkylthio,Hydroxy, Amino, C₁₋₆-Alkylamino, C₁₋₆-Dialkylamino, Aminoacyl, Acyl, CONR⁸R⁹, Nitro, Cyano, C₁₋₆-Heteroalkoxy, -X²(CH₂)ₚS(O)₂NR⁸R⁹, -X²(CH₂)ₚNHC(O)NHR⁸R⁹, -X2 (CH₂)ₚNHS(O)₂NR⁸R⁹ und -X²(CH₂)ₚNHCOOR¹⁰;
R^{7c} ausgewählt ist aus der Gruppe, bestehend aus Wasserstoff, C₁₋₆-Heteroalkoxy, -S(O)₂NR⁸R⁹, -X²CH₂(CH₂)ₚS(O)₂NR⁸R⁹; -X²(CH₂)ₚNHC(O)NHR⁸R⁹, X⁶(CH₂)ᵥCONR⁸R⁹, -SO₂R¹³, -NR⁸R⁹, X²(CH₂)ₚNR¹¹ S(O)₂NR⁸R⁹, -X²(CH₂)ₚNHCOOR¹⁰, -X⁶(CH₂)ᵥCOOR¹⁰, -X²(CH₂)ᵥCN, -OR¹⁵ und C(=O)CH₂N[(CH₂)₂]₂X⁴,
R⁸ und R⁹ voneinander unabhängig sind, einer von R⁸ und R⁹ Wasserstoff oder C₁₋₆-Alkyl ist und der andere von R⁸ und R⁹ ausgewählt ist aus der Gruppe, bestehend aus Wasserstoff, -C(=O)R¹⁴, -C(=O)CR¹²NH₂, -(CH₂)₂N[(CH₂)₂]₂O, COCO₂Me, C₃₋₈-Cycloalkyl, wobei Cycloalkyl gegebenenfalls substituiert ist mit einem oder zwei Hydroxylsubstituenten, Pyranyl, C₁₋₆-Alkyl und Aryl, wobei die Alkyl- und Arylgruppen gegebenenfalls substituiert sind mit einem oder zwei Substituenten, unabhängig voneinander ausgewählt aus der Gruppe, bestehend aus Hydroxy, C₁₋₆-Alkoxy, Thiol, C₁₋₆Alkylthio, C₁₋₆-Alkylsulfinyl, C₁₋₆-Sulfonyl und Halogen; oder
zusammen (CH₂)₂-X⁵-(CH₂)₂, -(CH₂)ₒ- oder (CH₂)ᵣS(O)ₙ, gegebenenfalls substituiert mit einem oder zwei Substituenten, ausgewählt aus der Gruppe, bestehend aus Halogen, Hydroxyl und NR^{11a}R^{11b}, sind;
R¹⁰ C₁₋₆-Alkyl ist;
R¹¹ Wasserstoff oder C₁₋₆-Alkyl ist;
R^{11a} und R¹¹ unabhängig voneinander R¹¹ sind;
R¹² die Seitenkette einer natürlich vorkommenden α-Aminosäure ist;
R¹³ C₁₋₆-Alkyl, -(CH₂)ₛCO₂R¹¹, -(CH₂)₂CN, -(CH₂)₂NH₂, -(CH₂)ᵤOH ist;
R¹⁴ C₁₋₁₀-Alkyl, -(CH₂)ₛNHR^{11a} R^{11b}, -(CH₂)ₛOR¹¹, -CH₂CH(OH)CH₃, -CH₂N[(CH₂)₂]₂O, -(CH₂)₂CO₂R¹¹, gegebenenfalls substituiertes Phenyl oder Pyridinyl ist;
R¹⁵ C₁₋₆-Alkyl, substituiert mit ein bis drei Hydroxylgruppen, ist;
X² -O- oder eine Bindung ist;
X⁴ -O- oder -NMe- ist;
X⁵ -O-, -S(O)ₙ- oder NR¹¹ ist;
X⁶ -O- oder -S(O)ₙ- ist;
m eine ganze Zahl von 0 bis 2 ist;
n eine ganze Zahl von 0 bis 2 ist;
o eine ganze Zahl von 4 bis 6 ist;
p eine ganze Zahl von 0 bis 6 ist;
r eine ganze Zahl von 3 bis 4 ist;
s eine ganze Zahl von 1 bis 2 ist;
u eine ganze Zahl von 2 bis 3 ist;
v eine ganze Zahl von 2 bis 6 ist; und
Hydrate, Solvate und Säureadditionssalze oder Salze der konjugierten Base davon.

9. Verbindung der Formel Ia nach Anspruch 8, worin
X¹ -O- ist;
R¹ und R² unabhängig voneinander ausgewählt sind aus der Gruppe, bestehend aus Wasserstoff, Methyl, Ethyl, i-Propyl, Methoxy, Chlor und Brom; oder R¹ und R² zusammen -O-CH=CH- sind;
R³ und R⁴ unabhängig voneinander ausgewählt sind aus der Gruppe, bestehend aus Wasserstoff und Fluor;
R⁵ Phenyl ist, wobei das Phenyl unsubstituiert oder mit ein bis drei Substituenten, unabhängig voneinander ausgewählt aus der Gruppe, bestehend aus Methyl, Ethyl, CHF₂, CF₃, -CF₂CH₃, -OCHF₂, Chlor, Brom, Fluor, -C≡CCH₂OH, -C≡CCH₂N(CH₃)₂ und Cyano, substituiert ist;
Ar Phenyl ist, wobei das Phenyl unsubstituiert oder mit 1 bis 3 Substituenten, ausgewählt aus der Gruppe, bestehend aus Methyl, Halogen, 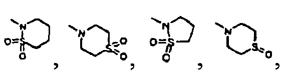 -SO₂NH₂, -SO₂NHCH₃, -SO₂NCH₃CH₂CH₂OH, -SO₂NHCH₂CHCH₃OH, -SO₂NHCH₂CH₂ 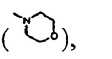 -SO₂NHCH₂CH₂CH₂OH, -SO₂ 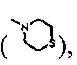 -SO₂ 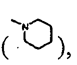 -SO₂ 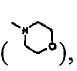 -SO₂ 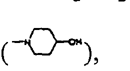 -SO₂NH 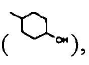 -SO₂ 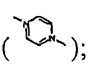 -SO₂NHCOCH₂CH₃, -SO₂NHCOCH₂CH₂CH₃, -SO₂NHCOCH(NH₂)CH(CH₃)₂, -SO₂NHCH₃COCH₂CH₂CH₃, - SO₂NHCOCH₂CH₂CH₂CH₃, -SO₂NHCOCH₂CH(CH₃)₂, -SO₂NHCOCH₂CH(CH₂CH₃)₂, -SO₂NHCOCH₂CH₂CH(CH₃)₂, -SO₂NHCOCH₂CH₂NH₂, -SO₂NCH₃COCH₂CH₂NH₂, -SO₂NHCOCH₂OCH₃, -SO₂NHCO 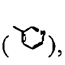 -SO₂NH 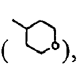 -SO₂NHCOCH(i-Propyl)NH₂, -SO₂NHCH₂CH₂SCH₃, -SO₂NCH₃COCH₂CH₃, -SO₂NCH₃COCH₂OCH₃, -SO₂NCH₃COCH₂CH₂OCH₃, -SO₂NHCOCH₂OH, -SO₂NHCOCH₂CH₂COOH, -SO₂CH₂COOH, -SO₂CH₂CH₂COOH, -SO₂CH₃, -SO₂CH₂CH₂NH₂, -SO₂CH₂CH₂CN, -SO₂CH₂CH₂CH₂OH, -O(CH₂)₃SO₂NH₂, -OCH₂CN, -OCH₂CHOHCH₂OH, -OCH₂CH₂CHOHCH₂OH, -COOCH₃, -COOH, -COCH₂ 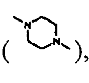 -COCH₂ 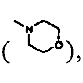 -N(CH₃)₂ und -NHCOCOOCH₂CH₃ substituiert ist;
R¹¹ Wasserstoff oder Methyl ist; und
Hydrate, Solvate und Salze davon.

10. Verbindung der Formel Ia nach Anspruch 7, die
2-[4-Chlor-3-(3-chlor-5-cyano-phenoxy)-phenyl]-N-(4-sulfamoyl-phenyl)-acetamid;
2-[4-Chlor-3-(3,5-dicyano-phenoxy)-phenyl]-N-(4-sulfamoyl-phenyl)-acetamid;
2-[3-(2-Brom-5-chlor-phenoxy)-4-chlor-phenyl]-N-[2-methyl-4-(3-sulfamoyl-propoxy)-phenyl]-acetamid;
2-[3-(2-Brom-5-chlor-phenoxy)-4-chlor-phenyl]-N-(4-sulfamoyl-phenyl)acetamid;
2-[4-Chlor-3-(3,5-dicyano-phenoxy)-phenyl]-N-(2-chlor-phenyl)-acetamid;
2-[4-Chlor-3-(3,5-dicyano-phenoxy)-phenyl]-N-(4-dimethylamino-phenyl)-acetamid;
2-[3-(2-Chlor-5-cyano-phenoxy)-4-ethyl-2-fluor-phenyl]-N-(4-sulfanloyl-phenyl)-acetamid;
2-[4-Chlor-3-(2,5-dicyano-phenoxy)-2-fluor-phenyl]-N-(4-sulfamoyl-phenyl)-acetamid;
2-[4-Chlor-3-(3,5-dicyano-phenoxy)-2-fluor-phenyl]-N-(4-sulfamoyl-phenyl)-acetamid;
2-[4-Chlor-2-fluor-3-(2,3,5-trichlor-phenoxy)-phenyl]-N-(4-sulfamoyl-phenyl)-acetamid;
2-[3-(3-Cyano-5-fluor-phenoxy)-4-methyl-phenyl]-N-(4-sulfamoyl-phenyl)-acetamid;
N-(2-Chlor-phenyl)-2-[3-(3-cyano-5-fluor-phenoxy)-4-methyl-phenyl]-acetamid;
2-[4-Chlor-3-(3-cyano-5-difluormethyl-phenoxy)-2-fluor-phenyl]-N-(4-sulfamoyl-phenyl)-acetamid;
2-[4-Chlor-3-(3-cyano-5-difluormethyl-phenoxy)-2-fluor-phenyl]-N-(2-chlor-phenyl)-acetamid;
2-[3-(2-Brom-5-chlor-phenoxy)-4-ethyl-phenyl]-N-(4-sulfamoyl-phenyl)-acetamid;
2-[³-(2-Brom-5-chlor-phenoxy)-4-ethyl-phenyl]-N-(2-chlor-phenyl)-acetamid;
2-[3-(5-Chlor-2-cyano-phenoxy)-4-ethyl-phenyl]-N-(4-sulfamoyl-phenyl)-acetamid;
2-[3-(5-Chlor-2-cyano-phenoxy)-4-ethyl-phenyl]-N-(2-chlor-phenyl)-acetamid;
2-[3-(2,5-Dichlor-phenoxy)-4-ethyl-phenyl]-N-(4-sulfamoyl-phenyl)-acetamid;
2-[3-(2,6-Dichlor-phenoxy)-4-ethyl-phenyl]-N-(4-sulfamoyl-phenyl)-acetamid;
N-(2-Chlor-phenyl)-2-[3-(2,5-dichlor-phenoxy)-4-ethyl-phenyl]-acetamid;
2-[3-(3-Brom-5-chlor-phenoxy)-4-ethyl-phenl]-N-(4-sulfamoyl-phenyl)-acetamid;
N-(2-Chlor-phenyl)-2-[3-(2,6-dichlor-phenoxy)-4-ethyl-phenyl]-acetamid;
2-[3-(3-Brom-5-chlor-phenoxy)-4-ethyl-phenyl]-N-(2-chlor-phenyl)-acetamid;
N-(2-Chlor-phenyl)-2-[3-(3,5-dicyano-phenoxy)-4-ethyl-phenyl]-acetamid;
2-[3-(2-Brom-5-chlor-phenoxy)-4-isopropyl-phenyl]-N-(4-sulfamoyl-phenyl)-acetamid;
2-[3-(2-Brom-5-chlor-phenoxy)-4-isopropyl-phenyl]-N-(2-chlor-phenyl)-acetamid;
2-[4-Chlor-3-(3,5-dicyano-phenoxy)-2-fluor-phenyl]-N-(2-chlor-phenyl)acetamid;
2-[4-Chlor-3-(3,5-dicyano-phenoxy)-phenyl]-N-[2-methyl-4-(3-sulfamoyl-propoxy)-phenyl]-acetamid;
2-[3-(2-Brom-5-chlor-phenoxy)-4-methyl-phenyl]-N-(4-sulfamoyl-phenyl)-acetamid;
2-[3-(2-Brom-5-chlor-phenoxy)-4-methyl-phenyl]-N-(2-methyl-4-sulfamoyl-phenyl)-acetamid;
2-[4-Chlor-3-(3,5-dicyano-phenoxy)-2-fluor-phenyl]-N-(2-methyl-4-sulfamoyl-phenyl)-acetamid;
2-[3-(2-Brom-5-chlor-phenoxy)-4-methyl-phenyl]-N-(2-chlor-phenyl)-acetamid;
N-(2-Chlor-phenyl)-2-[3-(3,5-dicyano-phenoxy)-4-methyl-phenyl]-acetamid;
2-[3-(3-Brom-2,5-dichlor-phenoxy)-4-ethyl-phenyl]-N-(4-sulfamoyl-phenyl)-acetamid;
2-[3-(3-Brom-2,5-dichlor-phenoxy)-4-ethyl-phenyl]-N-(2-methyl-4-sülfamoyl-phenyl)-acetamid;
2-[3-(3-Brom-2,5-dichlor-phenoxy)-4-ethyl-phenyl]-N-(2-chlor-phenyl)-acetamid;
N-(2-Chlor-phenyl)-2-[3-(3,5-dibrom-2-chlor-phenoxy)-4-ethyl-phenyl]-acetamid;
2-[3-(5-Brom-2-chlor-3-fluor-phenoxy)-4-ethyl-phenyl]-N-(4-sulfamoyl-phenyl)-acetamid;
2-[3-(5-Brom-2-chlor-3-fluor-phenoxy)-4-ethyl-phenyl]-N-(2-methyl-4-sulfamoyl-phenyl)-acetamid;
2-[3-(5-Brom-2-chlor-3-fluor-phenoxy)-4-ethyl-phenyl]-N-(2-chlor-phenyl)-acetamid;
2-[4-Chlor-3-(3-chlor-5-cyano-phenoxy)-2-fluor-phenyl]-N-(2-methyl-4-sulfamoyl-phenyl)-acetamid;
2-[3-(3,5-Dicyano-phenoxy)-4-methyl-phenyl]-N-(2-methyl-4-sulfamoyl-phenyl)-acetamid;
N-(2-Chlor-4-sulfamoyl-phenyl)-2-[3-(3,5-dicyano-phenoxy)-4-methyl-phenyl]-acetamid;
2-[3-(3-Chlor-5-cyano-phenoxy)-4-ethyl-phenyl]-N-(4-sulfamoyl-phenyl)-acetamid;
2-[3-(-5-Chlor-5-cyano-phenoxy)-4-ethyl-phenyl]-N-(2-methyl-4-sulfamoyl-phenyl)-acetamid;
2-[3-(3-Chlor-5-cyano-phenoxy)-4-ethyl-phenyl]-N-(2-chlor-4-sulfamoyl-phenyl)-acetamid;
2-[3-(3-Chlor-5-cyano-phenoxy)-4-ethyl-phenyl]-N-(4-methylsulfamoyl-phenyl)acetamid;
2-[3-(2-Chlor-5-cyano-3-fluor-phenoxy)-4-ethyl-phenyl]-N-(4-sulfamoyl-phenyl)-acetamid;
2-[3-(2-Chlor-5-cyano-3-fluor-phenoxy)-4-ethyl-phenyl]-N-(2-methyl-4-sulfamoyl-phenyl)-acetamid;
2-[4-Chlor-3-(3-chlor-5-cyano-phenoxy)-2-fluor-phenyl]-N-(2-methyl-4-propionylsulfamoylphenyl)-acetamid;
2-[3-(2-Chlor-3,5-dicyano-phenoxy)-4-ethyl-phenyl]-N-(4-sulfamoyl-phenyl)-acetamid;
2-[3-(2-Chlor-3,5-dicyano-phenoxy)-4-ethyl-phenyl]-N-(2-methyl-4-sulfamoylphenyl)-acetamid;
2-[3-(2-Chlor-3,5-dicyano-phenoxy)-4-ethyl-phenyl]-N-(2-chlor-4-sulfamoyl-phenyl)-acetamid;
2-[4-Chlor-3-(4-cyano-2,6-dimethyl-phenoxy)-phenyl]-N-(4-sulfamoyl-phenyl)-acetamid;
2-[4-Chlor-3-(4-cyano-2,6-dimethyl-phenoxy)-phenyl]-N-(2-methyl-4-sulfamoyl-phenyl)-acetamid;
2-[3-(3-Chlor-5-cyano-phenoxy)-4-methoxy-phenyl]-N-(4-sulfamoyl-phenyl)acetamid;
2-[3-(3-Chlor-5-cyano-phenoxy)-4-ethyl-phenyl]-N-(2-chlor-phenyl)-acetamid;
2-[3-(2-Chlor-5-cyano-3-fluor-phenoxy)-4-ethyl-phenyl]-N-(2-chlor-phenyl)-acetamid;
2-[3-(2-Chlor-3,5-dicyano-phenoxy)-4-ethyl-phenyl]-N-(2-chlor-phenyl)-acetamid;
2-[3-(3-Chlor-5-cyano-phenoxy)-4-methoxy-phenyl]-N-(2-chlor-phenyl)-acetamid;
2-[3-(2-Chlor-5-cyano-phenoxy)-4-methoxy-phenyl]-N-(2-chlor-phenyl)-acetamid;
2-[4-Brom-3-(3-chlor-5-cyano-phenoxy)-2-fluor-phenyl]-N-(2-chlor-4-propionylsulfamoyl-phenyl)-acetamid; Natriumsalz;
2-[3-(3-Chlor-5-cyano-phenoxy)-4-methoxy-phenyl]-N-(2-chlor-4-sulfamoyl-phenyl)-acetamid;
2-[3-(2-Chlor-5-cyano-phenoxy)-4-methoxy-phenyl]-N-(4-sulfamoyl-phenyl)-acetamid;
2-[3-(2-Chlor-5-cyano-phenoxy)-4-methoxy-phenyl]-N-(2-methyl-4-sulfamoyl-phenyl)-acetamid;
2-[3-(2-Chlor-5-cyano-phenoxy)-4-methoxy-phenyl]-N-(2-chlor-4-sulfamoyl-phenyl)-acetamid;
2-[4-Chlor-3-(4-cyano-2,6-dimethyl-phenoxy)-phenyl]-N-(2-chlor-phenyl)-acetamid;
2-[4-Chlor-3-(3,5-dicyano-phenoxy)-phenyl]-N-(4-methylsulfamoyl-phenyl)-acetamid;
2-[4-Chlor-3-(3,5-dicyano-phenoxy)-phenyl]-N-(4-dimethylsulfamoyl-phenyl)-acetamid;
2-[4-Chlor-3-(3,5-dicyano-phenoxy)-phenyl]-N-[4-(piperidin-1-sulfonyl)-phenyl]-acetamid;
2- [4-Chlor-3-(3,5-dicvano-phenoxy)-phenyl]-N-[4-(morpholin-4-sulfonyl)-phenyl]-acetamid.
2-[3-(3-Chlor-5-cyano-phenoxy)-4-methoxy-phenyl]-N-(2-methyl-4-sulfamoyl-phenyl)-acetamid;
2-[3-(3-Chlor-5-cyano-phenoxy)-2-fluor-4-methyl-phenyl]-N-(2-methyl-4-sulfalmoyl-phenyl)-acetamid;
2-[4-Chlor-3-(3-cyano-5-difluormethyl-phenoxy)-2-fluor-phenyl]-N-(2-methyl-4-sulfamoyl-phenyl)-acetamid;
2-[4-Chlor-3-(3,5-dicyano-phenoxy)-phenyl]-N-(2-chlor-4-methansulfonyl-phenyl)-acetamid;
N-[4-((S)-2-Amino-3-methyl-butyrylsulfamoyl)-2-methyl-phenyl]-2-[4-chlor-3-(3-chlor-5-cyano-phenoxy)-2-fluor-phenyl]-acetamid; Verbindung mit Salzsäure;
2-[4-Chlor-3-(3,5-dicyano-phenoxy)-phenyl]-N-(4-cyanomethoxy-phenyl)-acetamid;
2-[4-Chlor-3-(3,5-dicyano-phenoxy)-phenyl]-N-(4-methansulfonyl-phenyl)-acetamid;
3-Chlor-4- (2-[4-chlor-3-(3,5-dicyano-phenoxy)-phenyl]-acetylamino}-benzoesäure-methylester;
2-[4-Chlor-3-(3,5-dicyano-phenoxy)-phenyl]-N-{4-[(2-hydroxy-ethyl)-methyl-sulfamoyl]-phenyl}-acetamid;
2-[4-Chlor-3-(3,5-dicyano-phenoxy)-phenyl]-N-[4-(4-hydroxy-piperidin-1-sulfonyl)-phenyl]-acetamid;
2-[3-(3-Chlor-5-cyano-phenoxy)-2-fluor-4-methyl-phenyl]-N-(2-chlor-4-sulfamoyl-phenyl)-acetamid;
N-(2-Chlor-4-sulfalnoyl-phenyl)-2-[3-(3-cyano-5-difluormethyl-phenoxy)-2-fluor-4-methyl-phenyl]-acetamid;
2-[3-(3-Cyano-5-difluormethyl-phenoxy)-2-fluor-4-methyl-phenyl]-N-(2-methyl-4-sulfamoyl-phenyl)-acetamid;
N-(2-Chlor-phenyl)-2-[3-(3-cyano-5-difluormethyl-phenoxy)-2-fluor-4-methyl-phenyl]-acetamid;
2-[4-Chlor-3-(3-chlor-5-cyano-phenoxy)-2-fluor-phenyl]-N-{2-methyl-4-[(pyridin-3-carbonyl)-sulfamoyl]-phenyl}-acetamid; Verbindung mit Salzsäure;
2-[4-Chlor-3-(3-cyano-5-trifluormethyl-phenoxy)-phenyl]-N-(4-sulfamoyl-phenyl)acetamid;
2-[4-Chlor-3-(3-cyano-5-trifluormethyl-phenoxy)-phenyl]-N-(2-methyl-4-sulfamoylphenyl)-acetamid;
2-[4-Chlor-3-(3-cyano-5-trifluormethyl-phenoxy)-phenyl]-N-(2-chlor-4-sulfamoyl-phenyl)-acetamid;
2-[3-(3-Cyano-5-difluormethyl-phenoxy)-4-ethyl-phenyl]-N-(4-sulfamoyl-phenyl)-acetamid;
2-[3-(3-Cyano-5-difluormethyl-phenoxy)-4-methoxy-phenyl]-N-(4-sulfamoyl-phenyl)-acetamid;
2-[3-(3-Cyano-5-difluormethyl-phenoxy)-4-methoxy-phenyl]-N-(2-methyl-4-sulfamoyl-phenyl)-acetamid;
N-(2-Chlor-4-sulfamoyl-phenyl)-2-[3-(3-cyano-5-difluormethyl-phenoxy)-4-methoxy-phenyl]-acetamid;
2-{4-Chlor-3-[3-cyano-5-(1,1-difluor-ethyl)-phenoxy]-2-fluor-phenyl}-N-(4-sulfamoyl-phenyl)-acetamid;
2-{4-Chlor-3-[3-cyano-5-(1,1-difluor-ethyl)-phenoxy]-2-fluor-phenyl}-N-(2-methyl-4-sulfamoyl-phenyl)-acetamid;
2-{4-Chlor-3-[3-cyano-5-(1,1-difluor-ethyl)-phenoxy]-2-fluor-phenyl}-N-(2-chlor-4-sulfamoyl-phenyl)-acetamid;
2-[4-Chlor-3-(3-chlor-5-cyano-phenoxy)-2-fluor-phenyl]-N-(2-chlor-phenyl)-acetamid;
2-[4-Chlor-3-(3-chlor-5-cyano-phenoxy)-2-fluor-phenyl]-N-[2-methyl-4-(methyl-propionyl-sulfamoyl)-phenyl]-acetamid;
2-[4-Chlor-3-(3-chlor-5-cyano-phenoxy)-2-fluor-phenyl]-N-(2-chlor-4-sulfamoyl-phenyl)-acetamid;
2-[3-(3-Cyano-5-difluormethyl-phenoxy)-4-ethyl-phenyl]-N-(2-methyl-4-sulfamoyl-phenyl)-acetamid;
2-[3-(2-Chlor-5-cyano-phenoxy)-5-methoxy-phenyl]-N-(4-sulfamoyl-phenyl)-acetamid;
2-[3-(2-Chlor-5-cyano-phenoxy)-5-methoxy-phenyl]-N-(2-methyl-4-sulfamoyl-phenyl)-acetamid;
2-[4,5-Dibrom-3-(3-cyano-5-difluormethyl-phenoxy)-2-fluor-phenyl]-N-(2-methyl-4-sulfamoyl-phenyl)-acetamid;
2-[3-(2-Chlor-5-cyano-phenoxy)-5-ethyl-phenyl]-N-(4-sulfamoyl-phenyl)-acetamid;
2-[3-(2-Chlor-5-cyano-phenoxy)-5-ethyl-phenyl]-N-(2-methyl-4-sulfamoyl-phenyl)acetamid;
2-[3-(2-Chlor-5-cyano-phenoxy)-5-ethyl-phenyl]-N-(2-chlor-4-sulfamoyl-phenyl)-acetamid;
2-[4-Chlor-3-(3-chlor-5-cyano-phenoxy)-2-fluor-phenyl]-N-(2-methyl-4-methylsulfamoyl-phenyl)-acetamid;
2-[4-Brom-3-(3-chlor-5-cyano-phenoxy)-2-fluor-phenyl]-N-(2-chlor-4-sulfamoyl-phenyl)-acetamid;
3-Chlor-4-{2-[3-(3-cyano-5-difluormethyl-phenoxy)-2-fluor-4-methyl-phenyl]-acetylamino}-benzoesäure;
2-[4-Chlor-3-(3-chlor-5-cyano-phenoxy)-2-fluor-phenyl]-N-(2-chlor-phenyl)-N-methyl-acetamid;
N-(2-Chlor-4-methansulfonyl-phenyl)-2-[3-(3-cyano-5-difluormethyl-phenoxy)-2-fluor-4-methyl-phenyl]-acetamid;
2-[3-(2-Chlor-5-cyano-phenoxy)-5-methoxy-phenyl]-N-(2-chlor-4-sulfamoyl-phenyl)-acetamid;
2-[4,5-Dibrom-3-(3-cyano-5-difluormethyl-phenoxy)-2-fluor-phenyl]-N-(4-sulfamoyl-phenyl)-acetamid;
2-[3-(3-Chlor-5-cyano-phenoxy)-4-methyl-phenyl]-N-[2-methyl-4-(morpholin-4-sulfonyl)-phenyl]-acetamid;
2-[3-(3-Chlor-5-cyano-phenoxy)-4-methyl-phenyl]-N-[2-methyl-4-(4-methyl-piperazin-1-sulfonyl)-phenyl]-acetamid;
2-[3-(3-Chlor-5-cyano-phenoxy)-4-methyl-phenyl]-N-[2-methyl-4-(thiomorpholin-4-sulfonyl)-phenyl]-acetamid;
2-[3-(3-Chlor-5-cyano-phenoxy)-4-methyl-phenyl]-N-[2-methyl-4-(2-morpholin-4-ylethyl-sulfamoyl)-phenyl]-acetanlid;
2-[3-(3-Chlor-5-cyano-phenoxy)-4-methyl-phenyl]-N-[4-(3-hydroxy-propylsulfamoyl)-2-methyl-phenyl]-acetamid;
2-[4-Chlor-3-(3-cyano-5-trifluormethyl-phenoxy)-2-fluor-phenyl]-N-(2-methyl-4-sulfamoyl-phenyl)-acetamid;
2-[3-(3-Chlor-5-cyano-phenoxy)-4-methyl-phenyl]-N-[4-(4-hydroxy-cyclohexylsulfamoyl)-2-methyl-phenyl]-acetamid;
2-[3-(3-Chlor-5-cyano-phenoxy)-4-methyl-phenyl]-N-(2-methyl-4-sulfamoyl-phenyl)-acetamid;
2-[3-(3-Chlor-5-cyano-phenoxy)-4-methyl-phenyl]-N-(2-chlor-4-sulfamoyl-phenyl)-acetamid;
2-[4-Chlor-3-(4-cyano-6-methyl-pyridin-2-yloxy)-2-fluor-phenyl]-N-(2-methyl-4-sulfamoyl-phenyl)-acetamid;
3-(4-{2-[3-(3-Chlor-5-cyano-phenoxy)-4-methyl-phenyl]-acetylamino}-3-methyl-benzolsulfonyl)-propionsäure;
2-[4-Chlor-3-(3-chlor-5-cyano-phenoxy)-2-fluor-phenyl]-N-(2-chlor-4-propionylsulfamoyl-phenyl)-acetamid; Natriumsalz;
N-[4-((S)-2-Amino-3-methyl-butyrylsulfamoyl)-2-chlor-phenyl]-2-[4-chlor-3-(3-chlor-5-cyano-phenoxy)-2-fluor-phenyl]-acetamid; Natriumsalz;
2-[3-(3-Chlor-5-cyano-phenoxy)-4-methyl-phenyl]-N-[2-methyl-4-(tetrahydro-pyran-4-yl-sulfamoyl)-phenyl]-acetamid;
2-[3-(3-Chlor-5-cyano-phenoxy)-4-methyl-phenyl]-N-[4-(2-hydroxy-propylsulfamoyl)-2-methyl-phenyl]-acetamid;
2-[3-(3-Chlor-5-cyano-phenoxy)-4-methyl-phenyl]-N-[2-methyl-4-(2-methylsulfanylethyl-sulfamoyl)-phenyl]-acetamid;
2-[3-(3-Chlor-5-cyano-phenoxy)-4-methyl-phenyl]-N-[2-methyl-4-(3-methylsulfanyl-propyl-sulfamoyl)-phenyl]-acetamid;
2-[3-(3-Chlor-5-cyano-phenoxy)-5-methoxy-phenyl]-N-(4-sulfamoyl-phenyl)-acetamid;
2-[3-(3-Chlor-5-cyano-phenoxy)-5-methoxy-phenyl]-N-(2-methyl-4-sulfamoyl-phenyl)-acetamid;
N-(2-Chlor-4-sulfamoyl-phenyl)-2-[4,5-dibrom-3-(3-cyano-5-difluormethyl-phenoxy)-2-fluor-phenyl]-acetamid;
2-[4-Chlor-3-(4-cyano-2,6-dimethyl-phenoxy)-phenyl]-N-(2-chlor-4-sulfamoyl-phenyl)-acetamid;
2-[3-(3-Chlor-5-cyano-phenoxy)-5-methoxy-phenyl]-N-(2-chlor-4-sulfamoylphenyl)-acetamid;
2-[3-(3-Chlor-5-cyano-phenoxy)-4-methyl-phenyl]-N-[4-(2-cyano-ethansulfonyl)-2-methyl-phenyl]-acetamid;
N-(4-Butyrylsulfamoyl-2-chlor-phenyl)-2-[4-chlor-3-(3-chlor-5-cyano-phenoxy)-2-fluor-phenyl]-acetamid; Natriumsalz;
N-[4-(Butyryl-methyl-sulfamoyl)-2-chlor-phenyl]-2-[4-chlor-3-(3-chlor-5-cyano-phenoxy)-2-fluor-phenyl]-acetamid;
N-(2-Chlor-4-sulfamoyl-phenyl)-2-[3-(3-cyano-5-difluormethyl-phenoxy)-4-ethyl-phenyl]-acetamid;
2-[3-(3-Chlor-5-cyano-phenoxy)-5-methy.l-phenyl]-N-(4-sulfamoyl-phenyl)-acetamid;
2-[3-(3-Chlor-5-cyano-phenoxy)-5-methyl-phenyl]-N-(2-methyl-4-sulfamoyl-phenyl)-acetamid;
2-[3-(3-Chlor-5-cyano-phenoxy)-5-methyl-phenyl]-N-(2-chlor-4-sulfamoyl-phenyl)-acetamid;
2-[7-(3-Cyano-phenoxy)-benzofuran-5-yl]-N-(4-sulfamoyl-phenyl)-acetamid;
2-[7-(3-Cyano-phenoxy)-benzofuran-5-yl]-N-(2-methyl-4-sulfamoyl-phenyl)-acetamid;
2-[3-(2-Chlor-5-cyano-phenoxy)-5-methyl-phenyl]-N-(4-sulfamoyl-phenyl)-acetamid;
2-[3-(2-Chlor-5-cyano-phenoxy)-5-methyl-phenyl]-N-(2-methyl-4-sulfamoyl-phenyl)-acetamid;
2-[3-(2-Chlor-5-cyano-phenoxy)-5-methyl-phenyl]-N-(2-chlor-4-sulfamoyl-phenyl)-acetamid;
N-(2-Chlor-4-sulfamoyl-phenyl)-2-[7-(3,5-dichlor-phenoxy)-benzofuran-5-yl]-acetamid;
2-[3-(3-Chlor-5-cyano-phenoxy)-4-methyl-phenyl]-N-[4-((S)-2,3-dihydroxypropoxy)-2-methyl-phenyl]-acetamid;
2-[3-(3-Chlor-5-cyano-phenoxy)-4-methyl-phenyl]-N-[4-((R)-3,4-dihydroxy-butoxy)-2-methyl-phenyl]-acetamid;
(4-{2-[3-(3-Chlor-5-cyano-phenoxy)-4-methyl-phenyl]-acetylamino}-3-methylbenzolsulfonyl)-essigsäure;
N-[4-(3-Amino-propionylsulfamoyl)-2-chlor-phenyl]-2-[4-chlor-3-(3-chlor-5-cyano-phenoxy)-2-fluor-phenyl]-acetamid; Verbindung mit Salzsäure;
N-[4-(2-Amino-ethansulfonyl)-2-methyl-phenyl]-2-[3-(3-chlor-5-cyano-phenoxy)-4-methyl-phenyl]-acetamid;
2-[4-Chlor-3-(3-chlor-5-cyano-phenoxy)-2-fluor-phenyl]-N-[2-chlor-4-(2-methoxy-acetyl-sulfamoyl)-phenyl]-acetamid; Natriumsalz;
2-[4-Chlor-3-(3-chlor-5-cyano-phenoxy)-2-fluor-phenyl]-N-[2-chlor-4-(2-hydroxy-acetyl-sulfamoyl)-phenyl]-acetamid; Natriumsalz;
2-[3-(3-Chlor-5-cyano-phenoxy)-4-methyl-phenyl]-N-[4-(3-hydroxy-propan-1-sulfonyl)-2-methyl-phenyl]-acetamid;
2-[3-(3-Chlor-5-cyano-phenoxy)-4-methyl-phenyl]-N-[4-(1,1-dioxo-1λ6-[1,2]thiazinan-2-yl)-2-methyl-phenyl]-acetamid;
2-[3-(3-Chlor-5-cyano-phenoxy)-4-methyl-phenyl]-N-{2-methyl-4-[2-(4-methyl-piperazin-1-yl)-acetyl]-phenyl}-acetamid;
2-[3-(3-Chlor-5-cyano-phenoxy)-4-methyl-phenyl]-N-[2-methyl-4-(1-oxo-1λ4-thiomorpholin-4-yl)-phenyl]-acetamid;
2-[3-(3-Chlor-5-cyano-phenoxy)-4-methyl-phenyl]-N-[4-(1,1-dioxo-1λ6-thiomorpholin-4-yl)-2-methyl-phenyl]-acetamid;
2-[4-Chlor-3-(3-chlor-5-cyano-phenoxy)-2-fluor-phenyl]-N-[2-chlor-4-(methyl-propionyl-sulfamoyl)-phenyl]-acetamid;
2-[3-(3-Chlor-5-cyano-phenoxy)-4-methyl-phenyl]-N-[2-methyl-4(2-morpholin-4-yl-acetyl)-phenyl]-acetamid; Verbindung mit Trifluoressigsäure;
2-[4-Chlor-3-(3-chlor-5-cyano-phenoxy)-2-fluor-phenyl]-N-[2-chlor-4-(3-methoxy-propionylsulfamoyl)-phenyl]-acetamid; Natriumsalz;
2-[4-Brom-3-(3-cyano-5-difluormethyl-phenoxy)-2-fluor-phenyl]-N-(2-chlor-4-sulfamoyl-phenyl)-acetamid;
2-[4-Chlor-3-(3-cyano-5-difluormethoxy-phenoxy)-2-fluor-phenyl]-N-(4-sulfamoyl-phenyl)-acetamid;
2-[4-Chlor-3-(3-cyano-5-difluormethoxy-phenoxy)-2-fluor-phenyl]-N-(2-chlor-4-sulfamoyl-phenyl)-acetamid;
2-[4-Chlor-3-(3-chlor-5-cyano-phenoxy)-2-fluor-phenyl]-N-(2-chlor-4-methylsulfamoyl-phenyl)-acetamid;
2-[4-Brom-3-(3-cyano-5-difluormethyl-phenoxy)-2-fluor-phenyl]-N-(2-methyl-4-sulfamoyl-phenyl)-acetamid;
2-[3-(3-Chlor-5-cyano-phenoxy)-4-methyl-phenyl]-N-[4-(1,1-dioxo-1λ6-isothiazolidin-2-yl)-2-methyl-phenyl]-acetamid;
2-[4-Chlor-3-(3-chlor-5-cyano-phenoxy)-2-fluor-phenyl]-N-{2-chlor-4-[(2-methoxy-acetyl)-methyl-sulfamoyl]-phenyl}-acetamid;
2-[4-Chlor-3-(3-chlor-5-cyano-phenoxy)-2-fluor-phenyl]-N-{2-chlor-4-[(3-methoxy-propionyl)-methyl-sulfamoyl]-phenyl}-acetamide;
2-[4-Chlor-3-(3-chlor-5-cyano-phenoxy)-2-fluor-phenyl]-N-(2-chlor-4-pentanoylsulfamoyl-phenyl)-acetamid; Natriumsalz;
2-[4-Chlor-3-(3-chlor-5-cyano-phenoxy)-2-fluor-phenyl]-N-[2-chlor-4-(3-methyl-butyrylsulfamoyl)-phenyl]-acetamid; Natriumsalz;
2-[4-Chlor-3-(3-chlor-5-cyano-phenoxy)-2-fluor-phenyl]-N-[2-chlor-4-(2-diethylamino-acetylsulfamoyl)-phenyl)-acetamid; Verbindung mit Salzsäure;
2-[4-Chlor-3-(3-chlor-5-cyano-phenoxy)-2-fluor-phenyl]-N-[2-chlor-4-(4-methylpentanoyl-sulfamoyl)-phenyl]-acetamid; Natriumsalz;
2-[4-Chlor-3-(3-chlor-S-cyano-phenoxy)-2-fluor-phenyl]-N-[2-chlor-4-(2-morpholin-4-yl-acetylsulfamoyl)-phenyl]-acetamid; Verbindung mit Salzsäure;
N-(4-{2-[3-(3-Chlor-5-cyano-phenoxy)-4-methyl-phenyl]-acetylamino}-3-methyl-phenyl)-carbamoylameisensäure-ethylester;
N-(2-Chlor-4-sulfamoyl-phenyl)-2-[3-(3-cyano-5-difluormethyl-phenoxy)-4-ethyl-2-fluorphenyl]-acetamid;
2-[3-(3-Cyano-5-difluormethyl-phenoxy)-4-ethyl-2-fluor-phenyl]-N-(2-methyl-4-sulfamoyl-phenyl)-acetamid;
N-{4-[(3-Amino-propionyl)-methyl-sulfamoyl]-2-chlor-phenyl}-2-[4-chlor-3-(3-chlor-5-cyano-phenoxy)-2-fluor-phenyl]-acetamid; Verbindung mit Salzsäure;
2-[3-(3-Chlor-5-cyano-phenoxy)-4,5-dimethyl-phenyl]-N-(4-sulfamoyl-phenyl)-acetamid;
2-[3-(3-Chlor-5-cyano-phenoxy)-4,5-dimethyl-phenyl]-N-(2-methyl-4-sulfamoyl-phenyl)-acetamid;
2-[3-(3-Chlor-5-cyano-phenoxy)-4,5-dimethyl-phenyl]-N-(2-chlor-4-sulfamoyl-phenyl)-acetamid;
2-[3-(3-Brom-5-cyano-phenoxy)-4-chlor-2-fluor-phenyl]-N-(2-methyl-4-sulfamoyl-phenyl)-acetamid;
2-{4-Chlor-3-[3-cyano-5-(3-hydroxy-prop-1-inyl)-phenoxy]-2-fluor-phenyl}-N-(2-methyl-4-sulfamoyl-phenyl)-acetamid;
2-{4-Chlor-3-[3-cyano-5-(3-dimethylamino-prop-1-inyl)-phenoxy]-2-fluor-phenyl}-N-(2-methyl-4-sulfamoyl-phenyl)-acetamid;
4-(3-Chlor-4-{2-[4-chlor-3-(3-chlor-5-cyano-phenoxy)-2-fluor-phenyl]-acetylamino}-benzolsulfonylamino)-4-oxo-buttersäure;
2-[4-Brom-3-(3-chlor-5-cyano-phenoxy)-2-fluor-phenyl]-N-(2-methyl-4-sulfamoyl-phenyl)-acetamid;
2-[3-(3-Chlor-5-cyano-phenoxy)-2-fluor-4-methoxy-phenyl]-N-(2-chlor-4-sulfamoyl-phenyl)-acetamid;
2-[3-(3-Chlor-5-cyano-phenoxy)-2-fluor-4-methoxy-phenyl]-N-(2-methyl-4-sulfamoyl-phenyl)-acetamid oder
2-[4-Chlor-3-(3-chlor-phenoxy)-phenyl]-N-phenyl-acetamid
ist.

11. Verbindung der Formel Ia nach Anspruch 9, die
2-[4-Chlor-3-(3-chlor-5-cyano-phenoxy)-2-fluor-phenyl]-N-(2-chlor-4-sulfamoyl-phenyl)-acetamid;
2-[4-Chlor-3-(3-chlor-5-cyano-phenoxy)-2-fluor-phenyl]-N-(2-chlor-4-propionylsulfamoyl-phenyl)-acetamid; Natriumsalz;
2-[3-(3-Chlor-5-cyano-phenoxy)-2-fluor-4-methyl-phenyl]-N-(2-chlor-4-sulfamoyl-phenyl)-acetamid;
2-[4-Brom-3-(3-chlor-5-cyano-phenoxy)-2-fluor-phenyl]-N-(2-chlor-4-sulfamoyl-phenyl)-acetamid;
2-[4-Brom-3-(3-chlor-5-cyano-phenoxy)-2-fluor-phenyl]-N-(2-chlor-4-propionylsulfamoyl-phenyl)-acetamid; Natriumsalz;
N-(4-Butyrylsulfamoyl-2-chlor-phenyl)-2-[4-chior-3-(3-chlor-5-cyano-phenoxy)-2-fluorphenyl]-acetamid; Natriumsalz;
N-[4-((S)-2-Amino-3-methyl-butyrylsulfamoyl)-2-chlor-phenyl]-2-[4-chlor-3-(3-chlor-5-cyano-phenoxy)-2-fluor-phenyl]-acetamid; Natriumsalz;
N-(2-Chlor-4-sulfamoyl-phenyl)-2-[3-(3-cyano-5-difluormethyl-phenoxy)-4-ethyl-2-fluorphenyl]-acetamid;
2-[3-(3-Cyano-5-difluormethyl-phenoxy)-4-ethyl-2-fluor-phenyl]-N-(2-methyl-4-sulfamoyl-phenyl)-acetamid;
2-[4-Brom-3-(3-chlor-5-cyano-phenoxy)-2-fluor-phenyl]-N-(2-methyl-4-sulfamoyl-phenyl)-acetamid;
2-[4-Brom-3-(3-cyano-5-difluormethyl-phenoxy)-2-fluor-phenyl]-N-(2-methyl-4-sulfamoyl-phenyl)-acetamid;
2-[4-Brom-3-(3-cyano-5-difluormethyl-phenoxy)-2-fluor-phenyl]-N-(2-chlor-4-sulfamoyl-phenyl)-acetamid;
2-[4-Chlor-3-(3-cyano-5-difluormethyl-phenoxy)-2-fluor-phenyl]-*N*-(2-methyl-4-sulfamoyl-phenyl)-acetamid;
2-[3-(3-Chlor-5-cyano-phenoxy)-2-fluor-4-methoxy-phenyl]-N-(2-chlor-4-sulfamoyl-phenyl)-acetamid;
2-[3-(3-Chlor-5-cyano-phenoxy)-2-fluor-4-methoxy-phenyl]-N-(2-methyl-4-sulfamoyl-phenyl)-acetamid und
2-[3-(3-Chlor-5-cyano-phenoxy)-2-fluor-4-methyl-phenyl]-N-(2-chlor-4-propionylsulfamoyl-phenyl)-acetamid; Natriumsalz ist.

12. Verbindung der Formel Ia nach einem der Ansprüche 7 bis 11 zur Verwendung als Medikament.

13. Verwendung der Verbindung der Formel Ia nach einem der Ansprüche 7 bis 11 zur Herstellung eines Medikaments zur Behandlung von Krankheiten, vermittelt durch das menschliche Immunschwächevirus (HIV).

14. Pharmazeutische Zusammensetzung, umfassend eine pharmazeutisch wirksame Menge einer Verbindung oder eines pharmazeutisch akzeptablen Salzes davon wie in einem der Ansprüche 1 bis 11 definiert und einen pharmazeutisch inerten Träger.

15. Pharmazeutische Zusammensetzung nach Anspruch 14 zur Verwendung bei der Behandlung von Krankheiten, vermittelt durch das menschliche Immunschwächevirus (HIV).

## Revendications

1. Utilisation de composés de formule I 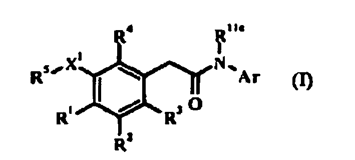 dans laquelle
X¹ est choisi dans le groupe constitué par -O-, -S-, -CH₂-, -C(O)-;
R¹ et R² sont choisis chacun indépendamment dans le groupe constitué par hydrogène, alkyle en C₁-C₆, halogénoalkyle en C₁-C₆, cycloalkyle en C₃-C₈, alcoxy en C₁-C₆, alkylthio en C₁-C₆, alkylsulfinyle en C₁-C₆, sulfonyle en C₁-C₆, halogénoalcoxy en C₁-C₆, halogénoalkylthio en C₁-C₆, halogène, amino, alkylamino, dialkylamino, acylamino, nitro et cyano; ou R¹ et R² ensemble sont -O-CH=CH- ou -O-CH₂CH₂-;
R³ et R⁴ sont choisis indépendamment dans le groupe constitué par hydrogène, alkyle en C₁-C₆, halogénoalkyle en C₁-C₆, alcoxy en C₁-C₆, halogénoalcoxy en C₁-C₆, alkylthio en C₁-C₆, halogénoalkylthio en C₁-C₆, halogène, amino, nitro et cyano;
R⁵ est choisi dans le groupe constitué par alkyle en C₁-C₆, halogénoalkyle en C₁-C₆, cycloalkyle en C₃-C₈, aryle ou hétéroaryle choisi dans le groupe constitué par pyridinyle, N-hydroxypyridine, pyrimidinyle, indole, pyrazinyle et pyrrolyle; ledit aryle et ledit hétéroaryle étant éventuellement substitués par 1 à 3 substituants choisis indépendamment dans le groupe constitué par alkyle en C₁-C₆, alcényle en C₂-C₆, alcynyle en C₂-C₆, halogénoalkyle en C₁-C₆, cycloalkyle en C₃-C₈, alcoxy en C₁-C₆, alkylthio en C₁-C₆, alkylsulfinyle en C₁-C₆, sulfonyle en C₁-C₆, halogénoalcoxy en C₁-C₆, halogénoalkylthio en C₁-C₆, hydroxy, halogène, amino, alkylamino en C₁-C₆, di(alkyl en C₁-C₆)amino, acylamino, acyle, (alcoxy en C₁-C₆)carbonyle, carbamoyle, N-(alkyl en C₁-C₆)carbamoyle, N,N-di(alkyl en C₁-C₆) carbamoyle, alcynol, -C≡CCH₂OH, -C≡CCH₂N(CH₃)₂, nitro et cyano, ledit alkyle et ledit cycloalkyle étant éventuellement substitués par 1 ou 2 substituants choisis indépendamment dans le groupe constitué par alkyle, hydroxy, alcoxy, thiol, alkylthio, halogène, amino alkylamino, aminoalkyle, alkylaminoalkyle et dialkylamino;
Ar est phényle éventuellement substitué par 1 à 3 substituants choisis indépendamment dans chaque cas dans le groupe constitué par alkyle en C₁-C₆, cycloalkyle en C₃-C₈, halogénoalkyle en C₁-C₃, alcoxy en C₁-C₆, halogénoalcoxy en C₁-C₆, halogène, cyano, alkylthio en C₁-C₆, alkylsulfinyle en C₁-C₆,
sulfonyle en C₁-C₆, aminoalkylsulfonyle en C₁-C₆, halogénoalcoxy en C₁-C₆, halogénoalkylthio en C₁-C₆, acyle en C₁-C₆, nitro, hétéroalkyle en C₁-C₆, hétéroalcoxy en C₁-C₆, hydroxy, -X²CCH₂)ₚS(O)ₙNR⁸R⁹; -(CH₂)ₚCOOR¹¹; -X²(CH₂)ₚNHC(O)NER⁸R⁹, -X²(CH₂)ₚCONR⁸R⁹, -SO₂R¹³, -NR^{8a}R^{9a}, -X² (CH₂)ₚNR¹¹S(O)₂NR⁸R⁹, -X²(CH₂)ₚNHCOOR¹⁰, -X⁶(CH₂)ₚCOOR¹⁰, -X²(CH₂)ₚCN, -OR¹⁵ et C(=O)CH₂N[(CH₂)₂]₂X⁴; ou
un cycle hétéroaryle choisi dans le groupe constitué par pyridinyle, pyridine-N-oxyde, pyridazinyle, pyrimidyle, pyrazyle, triazinyle, pyrrolyle, pyrazolyle, imidazolyle, (1,2,3)- et (1,2,4)-triazolyle, pyrazinyle, pyrimidinyle, tétrazolyle, furyle, thiényle, isoxazolyle, thiazolyle, thiényle, isoxazolyle, indole, indole-N-oxyde, quinoléine, quinoléine-N-oxyde et oxazolyle, ledit cycle hétéroaryle étant éventuellement substitué par 1 à 3 substituants choisis dans le groupe constitué par alkyle en C₁-C₆, alcoxy en C₁-C₆, alkylthio en C₁-C₆, halogène, aminoacyle en C₁-C₆ cyano et NR^{8b}R^{9b};
R⁸ et R⁹, pris indépendamment, l'un des R⁸ et R⁹ est hydrogène ou alkyle en C₁-C₆ et l'autre des R⁸ et R⁹ est choisi dans le groupe constitué par hydrogène, -C(=O)R¹⁴, -C(=O)CHR¹²NH₂, -(CH₂)₂N[(CH₂)₂]₂O, COCO₂Me, cycloalkyle en C₃-C₈, ledit cycloalkyle étant éventuellement substitué par 1 ou 2 substituants hydroxyle, pyranyle, alkyle en C₁-C₆ et aryle lesdits groupes alkyle et aryle étant éventuellement substitués par 1 ou 2 substituants choisis indépendamment dans le groupe constitué par hydroxy, alcoxy en C₁-C₆, thiol, alkylthio en C₁-C₆, alkylsulfinyle en C₁-C₆, sulfonyle en C₁-C₆ et halogène; ou (ii) R⁸ et R⁹ pris ensemble sont (CH₂)₂-X⁵-(CH₂)₂ ou -(CH₂)ₒ- éventuellement substitué par 1 ou 2 substituants choisis dans le groupe constitué par halogène, hydroxyle et NR^{11a}R^{11b};
R^{8a} et R^{9a}, pris indépendamment, sont choisis dans le groupe constitué par hydrogène, alkyle en C₁-C₆, C(=O)CO₂R¹¹ et SO₂R¹⁰, ou (ii), pris ensemble, sont (CH₂)ᵣSO₂, (CH₂)₂S(O)ₚ(CH₂)₂;
R¹⁰ est alkyle en C₁-C₆;
R¹¹ est hydrogène ou alkyle en C₁-C₆;
R^{11a}, R^{11b} et R^{11c} sont indépendamment R¹¹ ;
R¹² est la chaîne latérale d'un α-aminoacide naturel;
R¹³ est alkyle en C₁-C₆; -(CH₂)ₛCO₂R¹¹, -(CH₂)₂CN, -(CH₂)₂NH₂, -(CH₂)ᵤOH;
R¹⁴ est alkyle en C₁-C₆, -(CH₂)ₛNHR^{11a}R^{11b}, (CH₂)ₛOR¹¹, CH₂CH(OH)CH₃, -CH₂N[(CH₂)₂]O, -(CH₂)₂CO₂R¹¹, phényle ou pyridinyle éventuellement substitués;
R¹⁵ est alkyle en C₁-C₆ éventuellement substitué par 1 à 3 groupes hydroxyle;
X² est -O- ou une liaison;
X⁴ est -O- ou -NMe-;
X⁵ est -O-, -S(O)ₙ- ou NR¹¹;
X⁶ est -O- ou -S(O)ₙ-;
n est un entier de 0 à 2;
o est un entier de 4 à 6;
p est un entier de 0 à 6;
r est un entier de 3 à 4;
s est un entier de 1 à 2;
u est un entier de 2 à 3; et
des hydrates, des solvates et des sels de ceux-ci;
pour la fabrication d'un médicament destiné au traitement d'une infection par le VIH,
ou à la prévention d'une infection par le VIH, ou au traitement du sida ou du pré-sida.

2. Utilisation de composés de formule I selon la revendication 1, dans lesquels
X¹ est choisi dans le groupe constitué par -O-, -S-, -CH₂-, -C(O)-;
R¹ et R² sont choisis chacun indépendamment dans le groupe constitué par hydrogène, alkyle en C₁-C₆, halogénoalkyle en C₁-C₆, cycloalkyle en C₃-C₈, alcoxy en C₁-C₆, alkylthio en C₁-C₆, alkylsulfinyle en C₁-C₆, sulfonyle en C₁-C₆, halogénoalcoxy en C₁-C₆, halogénoalkylthio en C₁-C₆, halogène, amino, alkylamino, dialkylamino, acylamino, nitro et cyano; ou R¹ et R² ensemble sont -O-CH=CH- ou -O-CH₂CH₂-;
R³ et R⁴ sont choisis indépendamment dans le groupe constitué par hydrogène, alkyle en C₁-C₆, halogénoalkyle en C₁-C₆, alcoxy en C₁-C₆, halogénoalcoxy en C₁-C₆, alkylthio en C₁-C₆, halogénoalkylthio en C₁-C₆, halogène, amino, nitro et cyano;
R⁵ est choisi dans le groupe constitué par alkyle en C₁-C₆, halogénoalkyle en C₁-C₆, cycloalkyle en C₃-C₈, aryle ou hétéroaryle choisi dans le groupe constitué par pyridinyle, N-hydroxypyridine, pyrimidinyle, indole, pyrazinyle et pyrrolyle; ledit aryle et ledit hétéroaryle étant éventuellement substitués par 1 à 3 substituants choisis indépendamment dans le groupe constitué par alkyle en C₁-C₆, alcényle en C₂-C₆, alcynyle en C₂-C₆, halogénoalkyle en C₁-C₆, cycloalkyle en C₃-C₈, alcoxy en C₁-C₆, alkylthio en C₁-C₆, alkylsulfinyle en C₁-C₆, sulfonyle en C₁-C₆, halogénoalcoxy en C₁-C₆, halogénoalkylthio en C₁-C₆, hydroxy, halogène, amino, alkylamino en C₁-C₆, di(alkyl en C₁-C₆)amino, acylamino, acyle, (alcoxy en C₁-C₆)carbonyle, carbamoyle, N-(alkyl en C₁-C₆)carbamoyle, N,N-di(alkyl en C₁-C₆) carbamoyle, nitro et cyano, ledit alkyle et ledit cycloalkyle étant éventuellement substitués par 1 ou 2 substituants choisis indépendamment dans le groupe constitué par alkyle, hydroxy, alcoxy, thiol, alkylthio, halogène, amino, alkylamino, aminoalkyle, alkylaminoalkyle et dialkylamino;
Ar est phényle éventuellement substitué par 1 à 3 substituants choisis indépendamment dans chaque cas dans le groupe constitué par alkyle en C₁-C₆, cycloalkyle en C₃-C₈, halogénoalkyle en C₁-C₃, alcoxy en C₁-C₆, halogénoalcoxy en C₁-C₆, halogène, cyano, alkylthio en C₁-C₆, alkylsulfinyle en C₁-C₆,
sulfonyle en C₁-C₆, aminoalkylsulfonyle en C₁-C₆, halogénoalcoxy en C₁-C₆, halogénoalkylthio en C₁-C₆, acyle en C₁-C₆, nitro, hétéroalkyle en C₁-C₆, hétéroalcoxy en C₁-C₆, hydroxyle, -X²(CH₂)ₚS(O)ₙNR⁸R⁹; -(CH₂)ₚCOOR¹¹; -X²(CH₂)ₚNHC(O)NHR⁸R⁹, -X²(CH₂)ₚCONR⁸R⁹, -SO₂R¹³, -NR^{8a}R^{9a}, -X²(CH₂)ₚNR¹¹S(O)₂NR⁸R⁹, -X²(CH₂)ₚNHCOOR¹⁰, -X⁶(CH₂)ₚCOOR¹¹, -X²(CH₂)ₚCN, -OR¹⁵ et C(=O)CH₂N[(CH₂)₂]₂X⁴; ou
un cycle hétéroaryle choisi dans le groupe constitué par pyridinyle, pyridine-N-oxyde, pyridazinyle, pyrimidyle, pyrazyle, triazinyle, pyrrolyle, pyrazolyle, imidazolyle, (1,2,3)- et (1,2,4)-triazolyle, pyrazinyle, pyrimidinyle, tétrazolyle, furyle, thiényle, isoxazolyle, thiazolyle, thiényle, isoxazolyle, indole, indole-N-oxyde, quinoléine, quinoléine-N-oxyde et oxazolyle, ledit cycle hétéroaryle étant éventuellement substitué par 1 à 3 substituants choisis dans le groupe constitué par alkyle en C₁-C₆, alcoxy en C₁-C₆, alkylthio en C₁-C₆, halogène, aminoacyle en C₁-C₆ et NR^{8b}R^{9b};
R⁸ et R⁹, pris indépendamment, l'un des R⁸ et R⁹ est hydrogène ou alkyle en C₁-C₆ et l'autre des R⁸ et R⁹ est choisi dans le groupe constitué par hydrogène, -C(=O)R¹⁴, -C(=O)CHR¹²NH₂, -(CH₂)₂N[(CH₂)₂]₂O, COCO₂Me, cycloalkyle en C₃-C₈, ledit cycloalkyle étant éventuellement substitué par 1 ou 2 substituants hydroxyle, pyranyle, alkyle en C₁-C₆ et aryle lesdits groupes alkyle et aryle étant éventuellement substitués par 1 ou 2 substituants choisis indépendamment dans le groupe constitué par hydroxy, alcoxy en C₁-C₆, thiol, alkylthio en C₁-C₆, alkylsulfinyle en C₁-C₆, sulfonyle en C₁-C₆ et halogène; ou (ii) R⁸ et R⁹ pris ensemble sont (CH₂)₂-X⁵-(CH₂)₂ ou -(CH₂)ₒ- éventuellement substitué par 1 ou 2 substituants choisis dans le groupe constitué par halogène, hydroxyle et NR^{11a}R^{11b};
R^{8a} et R^{9a}, pris indépendamment, sont choisis dans le groupe constitué par hydrogène, alkyle en C₁-C₆, C(=O)CO₂R¹¹ et SO₂R¹⁰, ou (ii), pris ensemble, sont (CH₂),SO₂, (CH₂)₂S(O)ₚ(CH₂)₂;
R¹⁰ est alkyle en C₁-C₆;
R¹¹ est hydrogène ou alkyle en C₁-C₆
R^{11a}, R^{11b} et R^{11c} sont indépendamment R¹¹;
R¹² est la chaîne latérale d'un α-aminoacide naturel;
R¹³ est alkyle en C₁-C₆; -(CH₂)ₛCO₂R¹¹, -(CH₂)₂CN, -(CH₂)₂NH₂, -(CH₂)ᵤOH;
R¹⁴ est alkyle en C₁-C₆, -(CH₂)ₛNHR^{11a}NHR^{11a}R^{11b}, (CH₂)ₛOR¹¹, -CH₂CH(OH)CH₃, -CH₂N[(CH₂)₂]₂O, -(CH₂)₂CO₂R¹¹, phényle ou pyridinyle éventuellement substitués;
R¹⁵ est alkyle en C₁-C₆ éventuellement substitué par 1 à 3 groupes hydroxyle;
X² est -O- ou une liaison;
X⁴ est -O- ou -NMe-;
X⁵ est -O-, -S(O)ₙ- ou NR¹¹ ;
X⁶ est -O- ou -S(O)ₙ-;
n est un entier de 0 à 2;
O est un entier de 4 à 6;
p est un entier de 0 à 6;
r est un entier de 3 à 4;
s est un entier de 1 à 2;
u est un entier de 2 à 3; et
des hydrates, des solvates et des sels de ceux-ci;
pour la fabrication d'un médicament destiné au traitement d'une infection par le VIH,
ou à la prévention d'une infection par le VIH, ou au traitement du sida ou du pré-sida.

3. Utilisation de composés de formule I selon la revendication 1 ou 2, dans lesquels
X¹ est choisi dans le groupe constitué par -O-, -S-, -CH₂-, -C(O)-;
R¹ et R² sont choisis chacun indépendamment dans le groupe constitué par hydrogène, alkyle en C₁-C₆, alcoxy en C₁-C₆ et halogène; ou R¹ et R² ensemble sont -O-CH=CH- ou -O-CH₂CH₂-;
R³ et R⁴ sont choisis indépendamment dans le groupe constitué par hydrogène et halogène;
R⁵ est aryle ou hétéroaryle choisi dans le groupe constitué par pyridinyle, N-hydroxypyridine, pyrimidinyle, indole, pyrazinyle et pyrrolyle; ledit aryle et ledit hétéroaryle étant non substitués ou substitués par 1 à 3 substituants choisis indépendamment dans le groupe constitué par alkyle en C₁-C₆, halogénoalkyle en C₁-C₆, halogénoalcoxy en C₁-C₆, halogène, -C≡CCH₂OH, -C≡CCH₂N(CH₃)₂ et cyano;
Ar est phényle éventuellement substitué par 1 à 3 substituants choisis indépendamment dans le groupe constitué par alkyle en C₁-C₆, cycloalkyle en C₃-C₈, halogénoalkyle en C₁-C₃, alcoxy en C₁-C₆, halogène, cyano, alkylsulfinyle en C₁-C₆, sulfonyle en C₁-C₆, aminoalkylsulfonyle en C₁-C₆, halogénoalcoxy en C₁-C₆, halogénoalkylthio en C₁-C₆, acyle en C₁-C₆, nitro, hétéroalkyle en C₁-C₆, hétéroalcoxy en C₁-C₆, hydroxyle, -X²(CH₂)ₚS(O)ₙNR⁸R⁹; -(CH₂)ₚCOOR¹¹; -X²(CH₂)ₚNHC(O)NHR⁸R⁹, -X²(CH₂)ₚCONR⁸R⁹, -SO₂R¹³, -NR^{8a}R^{9a}, -X²(CH₂)ₚNR¹¹S(O)₂NR⁸R⁹ -X²(CH₂)ₚNHCOOR¹⁰, -X⁶(CH₂)ₚCOOR¹⁰, -X²(CH₂)ₚCN, -OR¹⁵ et C(=O)CH₂N[(CH₂)₂]₂X⁴; ou
un cycle hétéroaryle choisi dans le groupe constitué par pyridinyle, pyridine-N-oxyde, pyridazinyle, pyrimidyle, pyrazyle, triazinyle, pyrrolyle, pyrazolyle, imidazolyle, (1,2,3)- et (1,2,4)-triazolyle, pyrazinyle, pyrimidinyle, tétrazolyle, furyle, thiényle, isoxazolyle, thiazolyle, thiényle, isoxazolyle, indole, indole-N-oxyde, quinoléine, quinoléine-N-oxyde et oxazolyle, ledit cycle hétéroaryle étant éventuellement substitué par 1 à 3 substituants choisis dans le groupe constitué par alkyle en C₁-C₆, alcoxy en C₁-C₆, alkylthio en C₁-C₆, halogène, aminoacyle en C₁-C₆, cyano et NR^{8b}R^{9b};
R⁸ et R⁹, pris indépendamment, l'un des R⁸ et R⁹ est hydrogène ou alkyle en C₁-C₆ et l'autre des R⁸ et R⁹ est choisi dans le groupe constitué par hydrogène, -C(=O)R¹⁴, -C(=O)CHR¹²NH₂, -(CH₂)₂N[(CH₂)₂]₂O, COCO₂Me, cycloalkyle en C₃-C₈, ledit cycloalkyle étant éventuellement substitué par 1 ou 2 substituants hydroxyle, pyranyle, alkyle en C₁-C₆ et aryle lesdits groupes alkyle et aryle étant éventuellement substitués par 1 ou 2 substituants choisis indépendamment dans le groupe constitué par hydroxy, alcoxy en C₁-C₆, thiol, alkylthio en C₁-C₆, alkylsulfinyle en C₁-C₆, sulfonyle en C₁-C₆ et halogène; ou (ii) R⁸ et R⁹ pris ensemble sont (CH₂)₂-X⁵-(CH₂)₂ ou -(CH₂)ₒ- éventuellement substitué par 1 ou 2 substituants choisis dans le groupe constitué par halogène, hydroxyle et NR^{11a}R^{11b};
R^{8a} et R^{9a}, pris indépendamment, sont choisis dans le groupe constitué par hydrogène, alkyle en C₁-C₆, C(=O)CO₂R¹¹ et SO₂R¹⁰, ou (ii), pris ensemble, sont (CH₂)ᵣSO₂, (CH₂)₂S(O)ₚ(CH)₂;
R¹⁰ est alkyle en C₁-C₆;
R¹¹ est hydrogène ou alkyle en C₁-C₆;
R^{11a}, R^{11b} et R^{11c} sont indépendamment R¹¹;
R¹² est la chaîne latérale d'un α-aminoacide naturel;
R¹³ est alkyle en C₁-C₆; -(CH₂)ₛCO₂R¹¹, -(CH₂)₂CN, -(CH₂)₂NH₂, -(CH₂)ᵤOH;
R¹⁴ est alkyle en C₁-C₆, -(CH₂)ₛNHR^{11a}R^{11b}, (CH₂)ₛOR¹¹, -CH₂CH(OH)CH₃, -CH₂N[(CH₂)₂]₂O, -(CH₂)₂CO₂R¹¹, phényle ou pyridinyle éventuellement substitués;
R¹⁵ est alkyle en C₁-C₆ éventuellement substitué par 1 à 3 groupes hydroxyle;
X² est -O- ou une liaison;
X⁴ est -O- ou -NMe-;
X⁵ est -O-, -S(O)ₙ- ou NR¹¹;
X⁶ est -O- ou -S(O)ₙ-;
n est un entier de 0 à 2;
O est un entier de 4 à 6;
p est un entier de 0 à 6;
r est un entier de 3 à 4;
s est un entier de 1 à 2;
u est un entier de 2 à 3; et
des hydrates, des solvates et des sels de ceux-ci;
pour la fabrication d'un médicament destiné au traitement d'une infection par le VIH,
ou à la prévention d'une infection par le VIH, ou au traitement du sida ou du pré-sida.

4. Utilisation de composés de formule I 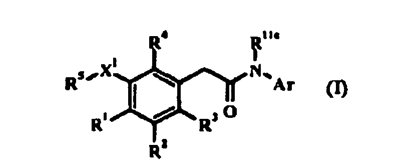 dans laquelle
X¹ est choisi dans le groupe constitué par -O-, -S-, -CH₂-, -C(O)-;
R¹ et R² sont choisis chacun indépendamment dans le groupe constitué par hydrogène, méthyle, éthyle, 1-propyle, méthoxy, chloro et bromo; ou R¹ et R² ensemble sont -O-CH=CH- ou -O-CH₂CH₂-:
R³ et R⁴ sont choisis indépendamment dans le groupe constitué par hydrogène et fluoro;
R⁵ est phényle; ledit phényle étant non substitué ou substitué par 1 à 3 substituants choisis indépendamment dans le groupe constitué par méthyle, éthyle, CHF₂, CF₃, -CF₂CH₃, -OCHF₂, chloro, bromo, fluoro, -C≡CCH₂OH, -C≡CCH₂N(CH₃)₂ et cyano;
ou pyridinyle, ledit pyridinyle étant non substitué ou substitué par 1 à 3 substituants choisis indépendamment dans le groupe constitué par méthyle et cyano;
Ar est phényle, ledit phényle étant non substitué ou substitué par 1 à 3 substituants choisis indépendamment dans le groupe constitué par méthyle, éthyle, i-propyle, 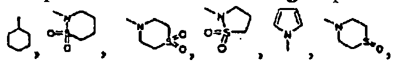 -CF₃, méthoxy, chloro, fluoro, cyano, hydroxyle, -SCH₃, -SO₃NH₂, -SO₂NHCH₃, -SO₂NCH₃CH₂CH₂OH, -SO₂NHCH₂CHCH₃OH, SO₂NHCH₂CH₂CH₂OH, -SO₂NHCH₂CH₂ 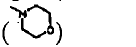 -SO₂ 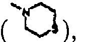 -SO₂N(CH₃)₂, -SO₂ 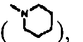 -SO₂ 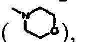 SO₂ 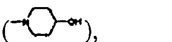 -SO₂NH 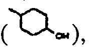 -SO₂ 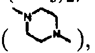 -SO₂ 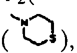 SO₂NHCOCH₂CH₃, -SO₂NHCOCH₂CH₂CH₃, -SO₂NHCOCH(NH₂)CH(CH₃)₂, -SO₂NHCOCH₂ 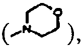 -SO₂NCH₃COCH₂CH₂CH₃, -SO₂NHCOCH₂CH₂CH₂CH₃, -SO₂NHCOCH₂CH(CH₃)₂, -SO₂NHCOCH₂CH(CH₂CH₃)₂, -SO₂NHCOCH₂CH₂CH(CH₃)₂, -SO₂NHCOCH₂CH₂NH₂, -SO₂NCH₃COCH₂CH₂NH₂, -SO₂NHCOCH₂OCH₃, -SO₂NHCO 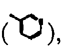 -SO₂NH 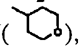 -SO₂NHCOCH(i-propyl)NH₂, -SO₂NHCH₂CH₂SCH₃, -SO₂NCH₃COCH₂CH₃, -SO₂NCH₃COCH₂OCH₃, -SO₂NCH₃COCH₂CH₂OCH₃, -SO₂NHCOCH₂OCH₃, -SO₂NHCOCH₂OH, -SO₂NHCOCH₂CH₂COOH, -SO₂CH₂CH₂NH₂, -SO₂CH₂COOH, -SO₂CH₂CH₂COOH, -SO₂CH₃, -SO₂CH₂CH₂CN, -SO₂CH₂CH₂CH₂OH, -O(CH₂)₃SO₂NH₂, -OCH₂COOH, -OCH₂COOCH₃, -OCH₂CN, -OCH₂CHOHCH₂OH, -OCH₂CH₂CHOHCH₂OH, -SCH₂COOCH₃, -COOCH₃, -COOH, -COCH₂ 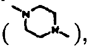 -COCH₂ 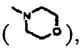 -CH₂COOCH₃, -CH₂COOH, -CH₂CH₂COOCH₃, CH₂CH₂CH₂COOCH₃, -N(CH₃)₂, -NHCOCOOCH₂CH₃, -NHSO₂CH₃ et -NCH₃HO₂CH₃; ou un cycle hétéroaryle choisi dans le groupe constitué par pyridinyle, pyrazolyle et triazolyle ledit hétéroaryle étant non substitué ou substitué par 1 ou 2 substituants choisis dans le groupe constitué par méthyle, méthoxy, -SCH₃ et -NHCOOCH₃;
R^{11c} est hydrogène ou méthyle; et
des hydrates, des solvates et des sels de ceux-ci.

5. Utilisation d'un composé de formule I selon la revendication 1 ou 4, ce composé étant:
le 2-[4-chloro-3-(3-chloro-5-cyanophénoxy)phényl]-N-(4-sulfamoylphényl)-acétamide;
le 2-[4-chloro-3-(3,5-dicyanophénoxy)phényl]-N-(4-sulfamoylphényl)acétamide;
le 2-[3-(2-bromo-5-chlorophénoxy)-4-chlorophényl]-N-[2-méthyl-4-(3-sulfamoylpropoxy)phényl]acétamide;
le 2-[3-(2-bromo-5-chlorophénoxy)-4-chlorophényl]-N-(4-sulfamoylphényl)-acétamide;
le 2-[4-chloro-3-(3,5-dicyanophénoxy)phényl],-N-(2-chlorophényl)acétamide;
le 2-[4-chloro-3-(3,5-dicyanophénoxy)phényl]-N-(4-diméthylaminophényl)-acétamide;
le 2-[3-(2-chloro-5-cyanophénoxy)-4-éthyl-2-fluorophényl]-N-(4-sulfamoylphényl)-acétamide;
le 2-[4-chloro-3-(2,5-dicyanophénoxy)-2-fluorophényl]-N-(4-sulfamoylphényl)-acétamide;
le 2-[4-chloro-3-(3,5-dicyanophénoxy)-2-fluorophényl]-N-(4-sulfamoylphényl)-acétamide;
le 2-[4-chloro-2-fluoro-3-(2,3,5-trichlorophénoxy)phényl]-N-(4-sulfamoylphényl)-acétamide;
le 2-[3-(3-cyano-5-fluorophénoxy)-4-méthylphényl]-N-(4-sulfamoylphényl)-acétamide;
le N-(2-chlorophényl)-2-[3-(3-cyano-5-fluorophénoxy)-4-méthylphényl]acétamide;
le 2-[4-chloro-3-(3-cyano-5-difluorométhylphénoxy)-2-fluorophényl]-N-(4-sulfamoylphényl)acétamide;
le 2-[4-chloro-3-(3-cyano-5-difluorométhylphénoxy)-2-fluorophényl]-N-(2-chlorophényl)acétamide;
le 2-[3-(2-bromo-5-chlorophénoxy)-4-éthylphényl]-N-(4-sulfamoylphényl)acétamide;
le 2-[3-(2-bromo-5-chlorophénoxy)-4-éthylphényl]-N-(2-chlorophényl)acétamide;
le 2-[3-(5-chloro-2-cyanophénoxy)-4-éthylphényl]-N-(4-sulfamoylphényl)acétamide;
le 2-[3-(5-chloro-2-cyanophénoxy)-4-éthylphényl]-N-(2-chlorophényl)acétamide;
le 2-[3-(2,5-dichlorophénoxy)-4-éthylphényl]-N-(4-sulfamoylphényl)acétamide;
le 2-[3-(2,6-dichlorophénoxy)-4-éthylphényl]-N-(4-sulfamoylphényl)acétamide;
le N-(2-chlorophényl)-2-[3-(2,5-dichlorophénoxy)-4-éthylphényl]acétamide;
le 2-[3-(3-bromo-5-chlorophénoxy)-4-éthylphényl]-N-(4-sulfamoylphényl)acétamide:
le N-(2-chlorophényl)-2-[3-(2>6-dichlorophénoxy)-4-éthylphényl]acétamide;
le 2-[3-(3-bromo-5-chlorophénoxy)-4-éthylphényl]-N-(2-chlorophényl)acétamide;
le N-(2-chlorophényl)-2-[3-(3,5-dicyanophénoxy)-4-éthylphényl]acétamide;
le 2-[3-(2-bromo-5-chlorophénoxy)-4-isopropylphényl]-N-(4-sulfamoylphényl)-acétamide;
le 2-[3-(2-bromo-5-chlorophénoxy)-4-isopropylphényl]-N-(2-chlorophényl)-acétamide;
le 2-[4-chloro-3-(3,5-dicyanophénoxy)-2-fluorophényl]-N-(2-chlorophényl)-acétamide;
le 2-[4-chloro-3-(3,5-dicyanophénoxy)phényl]-N-[2-méthyl-4-(3-sulfamoylpropoxy)-phényl]acétamide;
le 2-[3-(2-bromo-5-chlorophénoxy)-4-méthylphényl]-N-(4-sulfamoylphényl)-acétamide;
le 2-[3-(2-bromo-5-chlorophénoxy)-4-méthylphényl]-N-(2-méthyl-4-sulfamoylphényl)acétamide;
le 2-[4-chloro-3-(3,5-dicyanophénoxy)-2-fluorophényl]-N-(2-méthyl-4-sulfamoylphényl)acétamide;
le 2-[3-(2-bromo-5-chlorophénoxy)-4-méthylphényl]-N-(2-chlorophényl)acétamide;
le N-(2-chlorophényl)-2-[3-(3,5-dicyanophénoxy)-4-méthylphényl]acétamide;
le 2-[3-(3-bromo-2,5-dichlorophénoxy)-4-éthylphényl]-N-(4-sulfamoylphényl)-acétamide;
le 2-[3-(3-bromo-2,5-dichlorophénoxy)-4-éthylphényl]-N-(2-méthyl-4-sulfamoylphényl)acétamide;
le 2-[3-(3-bromo-2,5-dichlorophénoxy)-4-éthylphényl]-N-(2-chlorophényl)-acétamide;
le N-(2-chlorophényl)-2-[3-(3,5-dibromo-2-chlorophénoxy)-4-éthylphényl]-acétamide;
le 2-[3-(5-bromo-2-chloro-3-fluorophénoxy)-4-éthylphényl]-N-(4-sulfamoylphényl)-acétamide;
le 2-[3-(5-bromo-2-chloro-3-fluorophénoxy)-4-éthylphényl]-N-(2-méthyl-4-sulfamoylphényl)acétamide;
le 2-[3-(5-bromo-2-chloro-3-fluorophénoxy)-4-éthylphényl]-N-(2-chlorophényl)-acétamide;
le 2-[4-chloro-3-(3-chloro-5-cyanophénoxy)-2-fluorophényl]-N-(2-méthyl-4-sulfamoylphényl)acétamide;
le 2-[3-(3,5-dicyanophénoxy)-4-méthylphényl]-N-(2-méthyl-4-sulfamoylphényl)-acétamide;
le N-(2-chloro-4-sulfamoylphényl)-2-[3-(3,5-dicyanophénoxy)-4-méthylphényl]-acétamide;
le 2-[3-(3-chloro-5-cyanophénoxy)-4-éthylphényl]-N-(4-sulfamoylphényl)acétamide;
le 2-[3-(3-chloro-5-cyanophénoxy)-4-éthylphényl]-N-(2-méthyl-4-sulfamoylphényl)-acétamide;
le 2-[3-(3-chloro-5-cyanophénoxy)-4-éthylphényl]-N-(2-chloro-4-sulfamoylphényl)-acétamide;
le 2-[3-(3-chloro-5-cyanophénoxy)-4-éthylphényl]-N-(4-méthylsulfamoylphényl)-acétamide;
le 2-[3-(2-chloro-5-cyano-3-fluorophénoxy)-4-éthylphényl]-N-(4-sulfamoylphényl)-acétamide;
le 2-[3-(2-chloro-5-cyano-3-fluorophénoxy)-4-éthylphényl]-N-(2-méthyl-4-sulfamoylphényl)acétamide;
le 2-[4-chloro-3-(3-chloro-5-cyanophénoxy)-2-fluorophényl]-N-(2-méthyl-4-propionylsulfamoylphényl)acétamide;
le 2-[3-(2-chloro-3,5-dicyanophénoxy)-4-éthylphényl]-N-(4-sulfamoylphényl)-acétamide;
le 2-[3-(2-chloro-3,5-dicyanophénoxy)-4-éthylphényl]-N-(2-méthyl-4-sulfamoylphényl)acétamide;
le 2-[3-(2-chloro-3,5-dicyanophénoxy)-4-éthylphényl]-N-(2-chloro-4-sulfamoylphényl)acétamide;
le 2-[4-chloro-3-(4-cyano-2,6-diméthylphénoxy)phényl]-N-(4-sulfamoylphényl)-acétamide;
le 2-[4-chloro-3-(4-cyano-2,6-diméthylphénoxy)phényl]-N-(2-méthyl-4-sulfamoylphényl)acétamide;
le 2-[3-(3-chloro-5-cyanophénoxy)-4-méthoxyphényl]-N-(4-sulfamoylphényl)-acétamide;
le 2-[3-(3-chloro-5-cyanophénoxy)-4-éthylphényl]-N-(2-chlorophényl)acétamide;
le 2-[3-(2-chloro-5-cyano-3-fluorophénoxy)-4-éthylphényl]-N-(2-chlorophényl)-acétamide;
le 2-[3-(2-chloro-3,5-dicyanophénoxy)-4-éthylphényl]-N-(2-chlorophényl)acétamide;
le 2-[3-(3-chloro-5-cyanophénoxy)-4-méthoxyphényl]-N-(2-chlorophényl)acétamide;
le 2-[3-(2-chloro-5-cyanophénoxy)-4-méthoxyphényl]-N-(2-chloi-ophényl)acétamide;
le 2-[4-bromo-3-(3-chloro-5-cyanophénoxy)-2-fluorophényl]-N-(2-chloro-4-propionylsulfamoylphényl)acétamide; sel de sodium;
le 2-[3-(3-chloro-5-cyanophénoxy)-4-méthoxyphényl]-N-(2-chloro-4-sulfamoylphényl)acétamide;
le 2-[3-(2-chloro-5-cyanophénoxy)-4-méthoxyphényl]-N-(4-sulfamoylphényl)-acétamide;
le 2-[3-(2-chloro-5-cyanophénoxy)-4-méthoxyphényl]-N-(2-méthyl-4-sulfamoylphényl)acétamide;
le 2-[3-(2-chloro-5-cyanophénoxy)-4-méthoxyphényl]-N-(2-chloro-4-sulfamoylphényl)acétamide;
le 2-[4-chloro-3-(4-cyano-2,6-diméthylphénoxy)phényl]-N-(2-chlorophényl)-acétamide;
le 2-[4-chloro-3-(3,5-dicyanophénoxy)phényl]-N-(4-méthylsulfamoylphényl)-acétamide;
le 2-[4-chloro-3-(3,5-dicyanophénoxy)phényl]-N-(4-diméthylsulfamoylphényl)-acétamide;
le 2-[4-chloro-3-(3,5-dicyanophénoxy)phényl]-N-[4-(pipéridine-1-sulfonyl)phényl]-acétamide;
le 2-[4-chloro-3-(3,5-dicyanophénoxy)phényl]-N-[4-(morpholine-4-sulfonyl)phényl]-acétamide;
le 2-[3-(3-chloro-5-cyanophénoxy)-4-méthoxyphényl]-N-(2-méthyl-4-sulfamoylphényl)acétamide;
le 2-[3-(3-chloro-5-cyanophénoxy)-2-fluoro-4-méthylphényl]-N-(2-méthyl-4-sulfamoylphényl)acétamide;
le 2-[4-chloro-3-(3-cyano-5-difluorométhylphénoxy)-2-fluorophényl]-N-(2-méthyl-4-sulfamoylphényl)acétamide;
le 2-[4-chloro-3-(3,5-dicyanophénoxy)phényl]-N-(2-chloro-4-méthanesulfonylphényl)acétamide;
le N-[4-((S)-2-amino-3-méthylbutyrylsulfamoyl)-2-méthylphényl]-2-[4-chloro-3-(3-chloro-5-cyanophénoxy)-2-fluorophényl]acétamide; composé avec l'acide chlorhydrique;
le 2-[4-chloro-3-(3,5-dicyanophénoxy)phényl]-N-(4-cyanométhoxyphényl)acétamide;
le 2-[4-chloro-3-(3,5-dicyanophénoxy)phényl]-N-(4-méthanesulfonylphényl)-acétamide;
le 3-chloro-4-{2-[4-chloro-3-(3,5-dicyanophénoxy)phényl]acétylamino}benzoate de méthyle;
le 2-[4-chloro-3-(3,5-dicyanophénoxy)phényl]-N-{4-[(2-hydroxyéthyl)méthylsulfamoyl]phényl }acétamide;
le 2-[4-chloro-3-(3,5-dicyanophénoxy)phényl]-N-[4-(4-hydroxypipéridine-1-sulfonyl)phényl]acétamide;
le 2-[3-(3-chloro-5-cyanophénoxy)-2-fluoro-4-méthylphényl]-N-(2-chloro-4-sulfamoylphényl)acétamide;
le N-(2-chloro-4-sulfamoylphényl)-2-[3-(3-cyano-5-difluorométhylphénoxy)-2-fluoro-4-méthylphényl]acétamide;
le 2-[3-(3-cyano-5-difluorométhylphénoxy)-2-fluoro-4-méthylphényl]-N-(2-méthyl-4-sulfamoylphényl)acétamide;
le N-(2-chlorophényl)-2-[3-(3-cyano-5-difluorométhylphénoxy)-2-fluoro-4-méthylphényl]acétamide;
le 2-[4-chloro-3-(3-chloro-5-cyanophénoxy)-2-fluorophényl]-N-{2-méthyl-4-[(pyridine-3-carbonyl)sulfamoyl]phényl}acétamide; composé avec l'acide chlorhydrique;
le 2-[4-chloro-3-(3-cyano-5-trifluorométhylphénoxy)phényl]-N-(4-sulfamoylphényl)-acétamide;
le 2-[4-chloro-3-(3-cyano-5-trifluorométhylphénoxy)phényl]-N-(2-méthyl-4-sulfamoylphényl)acétamide;
le 2-[4-chloro-3-(3-cyano-5-trifluorométhylphénoxy)phényl]-N-(2-chloro-4-sulfamoylphényl)acétamide;
le 2-[3-(3-cyano-5-difluorométhylphénoxy)-4-éthylphényl]-N-(4-sulfamoylphényl)-acétamide;
le 2-[3-(3-cyano-5-difluorométhylphénoxy)-4-méthoxyphényl]-N-(4-sulfamoylphényl)acétamide;
le 2-[3-(3-cyano-5-difluorométhylphénoxy)-4-méthoxyphényl]-N-(2-méthyl-4-sulfamoylphényl)acétamide;
le N-(2-chloro-4-sulfamoylphényl)-2-[3-(3-cyano-5-difluorométhylphénoxy)-4-méthoxyphényl]acétamide;
le 2-{4-chloro-3-[3-cyano-5-(1,1-difluoroéthyl)phénoxy]-2-fluorophényl}-N-(4-sulfamoylphényl)acétamide;
le 2-{4-chloro-3-[3-cyano-5-(1,1-ditluoroéthyl)phénoxy]-2-fluorophényl}-N-(2-méthyl-4-sulfamoylphényl)acétamide;
le 2-{4-chloro-3-[3-cyano-5-(l,l-difluoroéthyl)phénoxy]-2-fluorophényl}-N-(2-chloro-4-sulfamoylphényl)acétamide;
le 2-[4-chloro-3-(3-chloro-5-cyanophénoxy)-2-fluorophényl]-N-(2-chlorophényl)-acétamide;
le 2-[-4-chloro-3-(3-chloro-5-cyanophénoxy)-2-fluorophényl]-N-[2-méthyl-4-méthylpropionylsulfamoyl)phényl]acétamide;
le 2-[4-chloro-3-(3-chloro-5-cyanophénoxy)-2-fluorophényl]-N-(2-chloro-4-sulfamoylphényl)acétamide;
le 2-[3-(3-cyano-5-difluorométhylphénoxy)-4-éthylphényl]-N-(2-méthyl-4-sulfamoylphényl)acétamide;
le 2-[3-(2-chloro-5-cyanophénoxy)-5-méthoxyphényl]-N-(4-sulfamoylphényl)-acétamide;
le 2-[3-(2-chloro-5-cyanophénoxy)-5-méthoxyphényl]-N-(2-méthyl-4-sulfamoylphényl)acétamide;
le 2-[4,5-dibromo-3-(3-cyano-5-difluorométhylphénoxy)-2-fluorophényl]-N-(2-méthyl-4-sulfamoylphényl)acétamide;
le 2-[3-(2-chloro-5-cyanophénoxy)-5-éthylphényl]-N-(4-sulfamoylphényl)acétamide;
le 2-[3-(2-chlbro-5-cyanophénoxy)-5-éthylphényl]-N-(2-méthyl-4-sulfamoylphényl)-acétamide;
le 2-[3-(2-chloro-5-cyanophénoxy)-5-éthylphényl]-N-(2-chloro-4-sulfamoylphényl)-acétamide;
le 2-[4-chloro-3-(3-chloro-5-cyanophénoxy)-2-fluorophényl]-N-(2-méthyl-4-méthylsulfamoylphényl)acétamide;
le 2-[4-bromo-3-(3-chloro-5-cyanophénoxy)-2-fluorophényl]-N-(2-chloro-4-sulfamoylphényl)acétamide;
l'acide 3-chloro-4-{2-[3-(3-cyano-5-difluorométhylphénoxy)-2-fluoro-4-méthylphényl]acétylamino}benzoïque;
le 2-[4-chloro-3-(3-chloro-5-cyanophénoxy)-2-fluorophényl]-N-(2-chlorophényl)-N-méthylacétamide;
le N-(2-chloro-4-méthanesulfonylphényl)-2-[3-(3-cyano-5-difluorométhylphénoxy)-2-fluoro-4-méthylphényl]acétamide;
le 2-[3-(2-chloro-5-cyanophénoxy)-5-méthoxyphényl]-N-(2-chloro-4-sulfamoylphényl)acétamide;
le 2-[4,5-dibromo-3-(3-cyano-5-difluorométhylphénoxy)-2-fluorophényl]-N-(4-sulfamoylphényl)acétamide;
le 2-[3-(3-chloro-5-cyanophénoxy)-4-méthylphényl]-N-[2-méthyl-4-(morpholine-4-sulfonyl)phényl]acétamide;
le 2-[3-(3-chloro-5-cyanophénoxy)-4-méthylphényl]-N-[2-méthyl-4-(4-méthylpipérazine-1-sulfonyl)phényl]acétamide;
le 2-[3-(3-chloro-5-cyanophénoxy)-4-méthylphényl]-N-[2-méthyl-4-(thiomorpholine-4-sulfonyl)phényl]acétamide;
le 2-[3-(3-chloro-5-cyanophénoxy)-4-méthylphényl]-N-[2-méthyl-4-(2-morpholin-4-yléthylsulfamoyl)phényl]acétamide;
le 2-[3-(3-chloro-5-cyanophénoxy)-4-méthylphényl]-N-[4-(3-hydroxypropyl-sulfamoyl)-2-méthylphényl]acétamide;
le 2-[4-chloro-3-(3-cyano-5-trifluorométhylphénoxy)-2-fluorophényl]-N-(2-méthyl-4-sulfamoylphényl)acétamide;
le 2-[3-(3-chloro-5-cyanophénoxy)-4-méthylphényl]-N-[4-(4-hydroxycyclohexyl-sulfamoyl)-2-méthylphényl]acétamide;
le 2-[3-(3-chloro-5-cyanophénoxy)-4-méthylphényl]-N-(2-méthyl-4-sulfamoylphényl)acétamide;
le 2-[3-(3-chloro-5-cyanophénoxy)-4-méthylphényl]-N-(2-chloro-4-sulfamoylphényl)acétamide;
l'acide 3-(4-{2-[3-(3-chloro-5-cyanophénoxy)-4-méthylphényl]acétylamino}-3-méthylbenzènesulfonyl)propionique;
le 2-[4-chloro-3-(3-chloro-5-cyanophénoxy)-2-fluorophényl]-N-(2-chloro-4-propionylsulfamoylphényl)acétamide; sel de sodium;
le N-[4-((S)-2-amino-3-méthylbutyrylsulfamoyl)-2-chlorophényl]-2-[4-chloro-3-(3-chloro-5-cyanophénoxy)-2-fluorophényl]acétamide; sel de sodium;
le 2-[3-(3-chloro-5-cyanophénoxy)-4-méthylphényl]-N-[2-méthyl-4-(tétrahydropyran-4-ylsulfamoyl)phényl]acétamide;
le 2-[3-(3-chloro-5-cyanophénoxy)-4-méthylphényl]-N-[4-(2-hydroxypropylsulfamoyl)-2-méthylphényl]acétamide;
le 2-[3-(3-chloro-5-cyanophénoxy)-4-méthylphényl]-N-[2-méthyl-4-(2-méthylsulfanyléthylsulfamoyl)phényl]acétamide;
le 2-[3-(3-chloro-5-cyanophénoxy)-4-méthylphényl]-N-[2-méthyl-4-(3-méthylsulfanylpropylsulfamoyl)phényl]acétamide;
le 2-[3-(3-chloro-5-cyanophénoxy)-5-méthoxyphényl]-N-(4-sulfamoylphényl)-acétamide;
le 2-[3-(3-chloro-5-cyanophénoxy)-5-méthoxyphényl]-N-(2-méthyl-4-sulfamoylphényl)acétamide;
le N-(2-chloro-4-sulfamoylphényl)-2-[4,5-dibromo-3-(3-cyano-5-difluorométhylphénoxy)-2-fluorophényl]acétamide;
le 2-[4-chloro-3-(4-cyano-2,6-diméthylphénoxy)phényl]-N-(2-chloro-4-sulfamoylphényl)acétamide;
le 2-[3-(3-chloro-5-cyanophénoxy)-5-méthoxyphényl]-N-(2-chloro-4-sulfamoylphényl)acétamide:
le 2-[3-(3-chloro-5-cyanophénoxy)-4-méthylphényl]-N-[4-(2-cyanoéthanesulfonyl)-2-méthylphényl]acétamide;
le N-(4-butyrylsulfamoyl-2-chlorophényl)-2-[4-chloro-3-(3-chloro-5-cyanophénoxy)-2-fluorophényl]acétamide; sel de sodium;
le N-[4-(butyrylméthylsulfamoyl)-2-chlorophényl]-2-[4-chloro-3-(3-chloro-5-cyanophénoxy)-2-fluorophényl]acétamide;
le N-(2-chloro-4-sulfamoylphényl)-2-[3-(3-cyano-5-difluorométhylphénoxy)-4-éthylphényl]acétamide;
le 2-[3-(3-chloro-5-cyanophénoxy)-5-méthylphényl]-N-(4-sulfamoylphényl)-acétamide;
le 2-[3-(3-chloro-5-cyanophénoxy)-5-méthylphényl]-N-(2-méthyl-4-sulfamoylphényl)acétamide;
le 2-[3-(3-chloro-5-cyanophénoxy)-5-méthylphényl]-N-(2-chloro-4-sulfamoylphényl)acétamide;
le 2-[7-(3-cyanophénoxy)benzofuran-5-yl]-N-(4-sulfamoylphényl)acétamide;
le 2-[7-(3-cyanophénoxy)benzofuran-5-yl]-N-(2-méthyl-4-sulfamoylphényl)-acétamide;
le 2-[3-(2-chloro-5-cyanophénoxy)-5-méthylphényl]-N-(4-sulfamoylphényl)-acétamide;
le 2-[3-(2-chloro-5-cyanophénoxy)-5-méthylphényl]-N-(2-méthyl-4-sulfamoylphényl)acétamide;
le 2-[3-(2-chloro-5-cyanophénoxy)-5-méthylphényl]-N-(2-chloro-4-sulfamoylphényl)acétamide;
le N-(2-chloro-4-sulfamoylphényl)-2-[7-(3,5-dichlorophénoxy)benzofuran-5-yl]-acétamide;
le 2-[3-(3-chloro-5-cyanophénoxy)-4-méthylphényl]-N-[4-((S)-2,3-dihydroxy-propoxy)-2-méthylphényl]acétamide;
le 2-[3-(3-chloro-5-cyanophénoxy)-4-méthylphényl]-N-[4-((R)-3,4-dihydroxy-butoxy)-2-méthylphényl]acétamide;
l'acide (4-{2-[3-(3-chloro-5-cyanophénoxy)-4-méthylphényl]acétylamino}-3-méthyl-benzènesulfonyl)acétique;
le N-[4-(3-aminopropionylsulfamoyl)-2-chlorophényl]-2-[4-chloro-3-(3-chloro-5-cyanophénoxy)-2-fluorophényl]acétamide; composé avec l'acide chlorhydrique;
le N-[4-(2-aminoéthanesulfonyl)-2-méthylphényl]-2-[3-(3-chloro-5-cyanophénoxy)-4-méthylphényl]acétamide;
le 2-[4-chloro-3-(3-chloro-5-cyanophénoxy)-2-fluorophényl]-N-[2-chioro-4-(2-méthoxyacétylsulfamoyl)phényl]acétamide; sel de sodium;
le 2-[4-chloro-3-(3-chloro-5-cyanophénoxy)-2-fluorophényl]-N-[2-chloro-4-(2-hydroxyacétylsulfamoyl)phényl]acétamide; sel de sodium;
le 2-[3-(3-chloro-5-cyanophénoxy)-4-méthylphényl]-N-[4-(3-hydroxypropane-1-sulfonyl)-2-méthylphényl]acétamide;
le 2-[3-(3-chloro-5-cyanophénoxy)-4-méthylphényl]-N-[4-(1,1-dioxo-1λ6-[1,2]thiazinan-2-yl)-2-méthylphényl]acétamide;
le 2-[3-(3-chloro-5-cyanophénoxy)-4-méthylphényl]-N-{2-méthyl-4-[2-(4-méthylpipérazin-1-yl)acétyl]phényl}acétamide;
le 2-[3-(3-chloro-5-cyanophénoxy)-4-méthylphényl]-N-[2-méthyl-4-(1-oxo-1λ4-thiomorpholin-4-yl)phényl]acétamide;
le 2-[3-(3-chloro-5-cyanophénoxy)-4-méthylphényl]-N-[4-(1,1-dioxo-1λ6-thiomorpholin-4-yl)-2-méthylphényl]acétamide;
le 2-[4-chloro-3-(3-chloro-5-cyanophénoxy)-2-fluorophényl]-N-[2-chloro-4-(méthylpropionylsulfamoyl)phényl]acétamide;
le 2-[3-(3-chloro-5-cyanophénoxy)-4-méthylphényl]-N-[2-méthyl-4-(2-morpholin-4-ylacétyl)phényl]acétamide; composé avec l'acide trifluoroacétique;
le 2-[4-chloro-3-(3-chloro-5-cyanophénoxy)-2-fluorophényl]-N-[2-chloro-4-(3-méthoxypropionylsulfamoyl)phényl]acétamide; sel de sodium;
le 2-[4-bromo-3-(3-cyano-5-difluorométhylphénoxy)-2-fluorophényl]-N-(2-chloro-4-sulfamoylphényl)acétamide;
le 2-[4-chloro-3-(3-cyano-5-difluorométhoxyphénoxy)-2-fluorophényl]-N-(4-sulfamoylphényl)acétamide;
le 2-[4-chloro-3-(3-cyano-5-difluorométhoxyphénoxy)-2-fluorophényl]-N-(2-chloro-4-sulfamoylphényl)acétamide;
le 2-[4-chloro-3-(3-chloro-5-cyanophénoxy)-2-fluorophényl]-N-(2-chloro-4-méthylsulfamoylphényl)acétamide;
le 2-[4-bromo-3-(3-cyano-5-difluorométhylphénoxy)-2-fluorophényl]-N-(2-méthyl-4-sulfamoylphényl)acétamide;
le 2-[3-(3-chloro-5-cyanophénoxy)-4-méthylphényl]-N-[4-(1,1-dioxo-1λ6-isothiazolidin-2-yl)-2-méthylphényl]acétamide;
le 2-[4-chloro-3-(3-chloro-5-cyanophénoxy)-2-fluorophényl]-N- 2-chloro-4-[(2-méthoxyacétyl)méthylsulfamoyl]phényl}acétamide;
le 2-[4-chloro-3-(3-ch loro-5-cyanophénoxy)-2-tluorophényl]-N-{2-chloro-4-[(3-méthoxypropionyl)méthylsulfumoyl]phényl}acétamide;
le 2-[4-chloro-3-(3-chloro-5-cyanophénoxy)-2-fiuorophényl]-N-(2-chloro-4-pentanoylsulfamoylphényl)acétamide; sel de sodium;
le 2-[4-chloro-3-(3-chloro-5-cyanophénoxy)-2-fluorophényl]-N-[2-chloro-4-(3-méthylbutyrylsulfamoyl)phényllacétamide; sel de sodium;
le 2-[4-chloro-3-(3-chloro-5-cyanophénoxy)-2-fluorophényl]-N-[2-chloro-4-(2-diéthylaminoacétylsulfamoyl)phényl]acétamide; composé avec l'acide chlorhydrique;
le 2-[4-chloro-3-(3-chloro-5-cyanophénoxy)-2-fluorophényl]-N-[2-chloro-4-(4-méthylpentanoylsulfamoyl)phényl]acétamide; sel de sodium;
le 2-[4-chloro-3-(3-chloro-5-cyanophénoxy)-2-fluorophényl]-N-[2-chloro-4-(2-morpholin-4-ylacétylsulfamoyl)phényl]acétamide; composé avec l'acide chlorhydrique;
le N-(4-{2-[3-(3-chloro-5-cyanophénoxy)-4-méthylphényl]acétylamino}-3-méthylphényl)oxalamate d'éthyle;
le N-(2-chloro-4-sulfamoylphényl)-2-[3-(3-cyano-5-difluorométhylphénoxy)-4-éthyl-2-fluorophényl]acétamide;
le 2-[3-(3-cyano-5-difluorométhylphénoxy)-4-éthyl-2-fluorophényl]-N-(2-méthyl-4-sulfamoylphényl)acétamide;
le N-{4-[(3-aminopropionyl)méthylsulfamoyl]-2-chlorophényl}-2-[4-chloro-3-(3-chloro-5-cyanophénoxy)-2-fluorophényl]acétamide; composé avec l'acide chlorhydrique;
le 2-[3-(3-chloro-5-cyanophénoxy)-4,5-diméthylphényl]-N-(4-sulfamoylphényl)-acétamide;
le 2-[3-(3-chloro-5-cyanophénoxy)-4,5-diméthylphényl]-N-(2-méthyl-4-sulfamoylphényl)acétamide;
le 2-[3-(3-chloro-5-cyanophénoxy)-4,5-diméthylphényl]-N-(2-chloro-4-sulfamoylphényl)acétamide;
le 2-[3-(3-bromo-5-cyanophénoxy)-4-chloro-2-fluorophényl]-N-(2-méthyl-4-sulfamoylphényl)acétamide;
le 2-{4-chloro-3-[3-cyano-5-(3-hydroxyprop-1-ynyl)phénoxy]-2-fluorophényl}-N-(2-méthyl-4-sulfamoylphényl)acétamide;
le 2-{4-chloro-3-[3-cyano-5-(3-diméthylaminoprop-1-ynyl)phénoxy]-2-fluorophényl }-N-(2-méthyl-4-sulfamoylphényl)acétamide;
l'acide 4-(3-chloro-4-{2-[4-chloro-3-(3-chloro-5-cyanophénoxy)-2-fluorophényl]-acétylamino}benzènesulfonylamino)-4-oxobutyrique;
le 2-[4-bromo-3-(3-chloro-5-cyanophénoxy)-2-fluorophényl]-N-(2-méthyl-4-sulfamoylphényl)acétamide;
le 2-[3-(3-chloro-5-cyanophénoxy)-2-fluoro-4-méthoxyphényl]-N-(2-chloro-4-sulfamoylphényl)acétamide;
le 2-[3-(3-chloro-5-cyanophénoxy)-2-fluoro-4-méthoxyphényl]-N-(2-méthyl-4-sulfamoylphényl)acétamide;
le 2-[4-chloro-3-(3-chlorophénoxy)phényl]-N-phénylacétamide;
le 2-[3-(2-bromo-5-chlorophénoxy)-4-chlorophényl]-N-(4-sulfamoylphényl)-acétamide;
le 2-[4-chloro-3-(3,5-dicyanophénoxy)phényl]-N-(2-pyrrol-1-ylphényl)acétamide;
le 2-[4-chloro-3-(3,5-dicyanophénoxy)phényl]-N-(2-fluorophényl)acétamide;
le 2-[4-chloro-3-(3,5-dicyanophénoxy)phényl]-N-(2-méthoxyphényl)acétamide;
le 2-[4-chloro-3-(3,5-dicyanophénoxy)phényl]-N-(2-isopropylphényl)acétamide;
le 2-[4-chloro-3-(3,5-dicyanophénoxy)phényl]-N-o-tolylacétamide;
le 2-[4-chloro-3-(3,5-dicyanophénoxy)phényl]-N-(2-éthylphényl)acétamide;
le 2-[4-chloro-3-(3,5-dicyanophénoxy)phényl]-N-(2,3-diméthylphényl)acétamide;
le 2-[4-chloro-3-(3,5-dicyanophénoxy)phényl]-N-(4-méthoxy-2-méthylphényl)-acétamide;
le 2-[4-chloro-3-(3,5-dicyanophénoxy)phényl]-N-(4-cyclohexylphényl)acétamide;
le 2-[4-chloro-3-(3,5-dicyanophénoxy)phényl]-N-(2-cyanophényl)acétamide;
le 2-[4-chloro-3-(3,5-dicyanophénoxy)phényl]-N-(2-trifluorométhylphényl)-acétamide;
le 2-{2-[4-chloro-3-(3,5-dicyanophénoxy)phényl]acétylamino}-4,5-diméthoxy-benzoate de méthyle;
le 2-[3-(3-chloro-5-cyanophénoxy)-4-éthylphényl]-N-phénylacétamide;
le 2-[3-(2-chloro-3,5-dicyanophénoxy)-4-éthylphényl]-N-phénylacétamide;
le 2-[4-chloro-3-(2,5-dichlorobenzyl)phényl]-N-(2-chloro-4-sulfamoylphényl)-acétamide;
le 2-[3-(3-chloro-5-cyanophénoxy)-4-méthoxyphényl]-N-phénylacétamide;
le 2-[4-chloro-3-(3,5-dicyanophénoxy)phényl]-N-phénylacétamide;
le 2-[4-chloro-3-(3,5-dicyanophénoxy)phényl]-N-(4-méthyl-3-sulfamoylphényl)-acétamide;
le 2-[4-chloro-3-(3,5-dicyanophénoxy)phényl]-N-(3-méthanesulfonylphényl)-acétamide;
le (4-{2-[4-chloro-3-(3,5-dicyanophénoxy)phényl]acétylamino}phénoxy)acétate de méthyle;
le 3-(4-{2-[4-chloro-3-(3,5-dicyanophénoxy)phényl]acétylamino}phényl)propionute de méthyle;
le (4-{2-[4-chloro-3-(3.5-dicyanophénoxy)phényl]acétylamino}phénylsulfanyl)-acétate de méthyle;
le 4-(4-{2-[4-chloro-3-(3,5-dicyanophénoxy)phényl]acétylamino}phényl)butyrate de méthyle;
le 2-[4-chloro-3-(3,5-dicyanophénoxy)phényl]-N-(2-fluoro-5-méthanesulfonylphényl)acétamide;
le (4-{2-[4-chloro-3-(3,5-dicyanophénoxy)phényl]acétylamino}phényl)acétate de méthyle;
le 2-[4-chloro-3-(3,5-dicyanophénoxy)phényl]-N-(4-cyanophényl)acétamide;
le 4-{2-[4-chloro-3-(3,5-dicyanophénoxy)phényl]acétylamino}benzoate de méthyle;
le 2-[4-chloro-3-(3,5-dicyanophénoxy)phényl]-N-(4-hydroxyphényl)acétamide;
le 2-[3-(3-chloro-5-cyanophénoxy)-4-méthylphényl]-N-(4-méthylpyridin-3-yl)acétamide;
le 2-[3-(3-chloro-5-cyanophénoxy)-4-méthylphényl]-N-(3-méthylpyridin-2-yl)acétamide;
le 2-[3-(3-chloro-5-cyanophénoxy)-4-méthylphényl]-N-(2,5-diméthyl-2H-pyrazol-3-yl)acétamide;
le 2-[3-(3-chloro-5-cyanophénoxy)-4-méthylphényl]-N-(2-méthyl-2H-pyrazol-3-yl)acétamide;
le N-(6-acétylamino-4-méthylpyridin-3-yl)-2-[3-(3-chloro-5-cyanophénoxy)-4-méthylphényl]acétamide;
le 2-[3-(3-chloro-5-cyanophénoxy)-4-méthylphényl]-N-(6-méthoxy-2-méthylpyridin-3-yl)acétamide;
le 2-[3-(3-chloro-5-cyanophénoxy)-4-méthylphényl]-N-(2-méthyl-5-méthylsulfanyl-2H-[1,2,4] triazol-3-yl)acétamide;
le N-(2-chlorophényl)-2-[3-(3,5-dichlorobenzoyl)-5-méthylphényl]acétamide;
le 2-[3-(3,5-dichlorobenzoyl)-5-méthylphényl]-N-(2-méthyl-4-sulfamoylphényl)-acétamide;
l'acide (4-{2-[3-(3-chloro-5-cyanophénoxy)-4-méthylphényl]acétylamino}-3-méthylphénoxy)acétique;
le (3-chloro-4-{2-[3-(3-chloro-5-cyanophénoxy)-4-méthylphényl]acétylamino}-phényl)acétate de méthyle;
l'acide (3-chloro-4-{2-[3-(3-chloro-5-cyanophénoxy)-4-méthylphényl]acétylamino}-phényl)acétique;
le 2-[3-(3-chloro-5-cyanophénoxy)-4-méthylphényl]-N-(4-méthanesulfonylamino-2-méthylphényl)acétamide;
le 2-[3-(3-chloro-5-cyanophénoxy)-4-méthylphényl]-N-[4-(méthanesulfonylméthylamino)-2-méthylphényl]acétamide;
le 4-{2-[4-chloro-3-(3-chloro-5-cyanophénoxy)-2-fluorophényl]acétylamino }-3-méthylbenzamide;
le 2-[7-(4-chlorobenzoyl)-2,3-dihydrobenzofuran-5-yl]-N-(2-chlorophényl)-acétamide;
le 2-[7-(4-chlorobenzoyl)-2,3-dihydrobenzofuran-5-yl]-N-(4-sulfamoylphényl)-acétamide;
le 2-[4-chloro-3-(3,5-dicyanophénylsulfanyl)phényl]-N-(4-sulfamoylphényl)-acétamide;
le 2-[3-(2-bromo-5-chlorophénylsulfanyl)-4-chlorophényl]-N-(4-sulfamoylphényl)acétamide;
le 2-[3-(2-bromo-5-chlorophénylsulfanyl)-4-chlorophényl]-N-(2-chlorophényl)-acétamide;
le 2-[4-chloro-3-(3,5-dichlorobenzoyl)phényl]-N-(2-chlorophényl)acétamide;
le 2-[4-chloro-3-(2,5-dichlorobenzoyl)phényl]-N-(2-chlorophényl)acétamide;
le 2-[4-chloro-3-(2,5-dichlorobenzoyl)phényl]-N-(2-méthyl-4-sulfamoylphényl)-acétamide;
le 2-[4-chloro-3-(2,5-dichlorobenzoyl)phényl]-N-(4-sulfamoylphényl)acétamide;
le 2-[4-chloro-3-(2,5-dichlorobenzoyl)phényl]-N-phénylacétamide;
le 2-[4-chloro-3-(3,5-dichlorobenzoyl)phényl]-N-(2-méthyl-4-sulfamoylphényl)-acétamide;
le 2-[4-chloro-3-(2,5-dichlorobenzoyl)phényl]-N-(2-chloro-4-sulfamoylphényl)-acétamide;
le 2-[3-(4-chlorobenzoyl)-5-méthylphényl]-N-(4-sulfamoylphényl)acétamide;
le 2-[3-(4-chlorobenzoyl)-5-méthylphényl]-N-(2-méthyl-4-sulfamoylphényl)-acétamide;
le 2-[3-(2-chlorobenzoyl)-5-méthylphényl]-N-(2-méthyl-4-sulfamoylphényl)-acétamide;
le 2-[3-(4-chlorobenzoyl)-5-méthylphényl]-N-(2-chloro-4-sulfamoylphényl)-acétamide;
le 2-[3-(4-chlorobenzoyl)-5-méthylphényl]-N-(2-chlorophényl)acétamide;
le 2-[3-(2-chlorobenzoyl)-5-méthylphényl]-N-(2-chlorophényl)acétamide;
le 2-[3-(3-chlorobenzoyl)-5-méthylphényl]-N-(2-méthyl-4-sulfamoylphényl)-acétamide;
le 2-[3-(3-chlorobenzoyl)-5-méthylphényl]-N-(4-sulfamoylphényl)ucétumide;
le 2-[3-(3-chlorobenzoyl)-5-méthylphényl]-N-(2-chlorophényl)acétamide;
le 2-[3-(2-chlorobenzoyl)-5-méthylphényl]-N-(4-sulfamoylphényl)acétamide;
le 2-[3-(5-bromo-2-chlorobenzoyl)-4-méthoxyphényl]-N-(2-méthyl-4-sulfamoylphényl)acétamide;
le 2-[3-(5-cyano-2-méthylbenzoyl)-4-méthylphényl]-N-(2-méthyl-4-sulfamoylphényl)acétamide;
le 2-[3-(5-cyano-2-méthylbenzoyl)-4-méthylphényl]-N-(4-sulfamoylphényl)-acétamide;
le N-(2-chlorophényl)-2-[3-(5-cyano-2-méthylbenzoyl)-4-méthylphényl]acétamide;
le 2-[3-(5-cyano-2-éthylbenzoyl)-4-éthylphényl]-N-(2-méthyl-4-sulfamoylphényl)-acétamide;
le 2-[3-(3,5-dichlorobenzoyl)-5-méthylphényl]-N-(4-sulfamoylphényl)acétamide; ou
le 2-[3-(3-chloro-5-cyanophénoxy)-2-fluoro-4-méthylphényl]-N-(2-chloro-4-propionylsulfamoylphényl)acétamide; sel de sodium.

6. Utilisation d'un composé de formule I selon la revendication 5, qui est
le 2-[4-chloro-3-(3-chloro-5-cyanophénoxy)-2-fluorophényl]-N-(2-chloro-4-sulfamoylphényl)acétamide;
le 2-[4-chloro-3-(3-chloro-5-cyanophénoxy)-2-fluorophényl]-N-(2-chloro-4-propionylsulfamoylphényl)acétamide; sel de sodium;
le 2-[3-(3-chloro-5-cyanophénoxy)-2-fluoro-4-méthylphényl]-N-(2-chloro-4-sulfamoylphényl)acétamide;
le 2-[4-bromo-3-(3-chloro-5-cyariophénoxy)-2-fluorophényl]-N-(2-chloro-4-sulfamoylphényl)acétamide;
le 2-[4-bromo-3-(3-chloro-5-cyanophénoxy)-2-fluorophényl]-N-(2-chloro-4-propionylsulfamoylphényl)acétamide; sel de sodium;
le N-(4-butyrylsulfamoyl-2-chlorophényl)-2-[4-chloro-3-(3-chloro-5-cyanophénoxy)-2-fluorophényl]acétanùde; sel de sodium;
le N-[4-((S)-2-amino-3-méthylbutyrylsulfamoyl)-2-chlorophényl]-2-[4-chloro-3-(3-chloro-5-cyanophénoxy)-2-fluorophényllacétamide; sel de sodium
le N-(2-chloro-4-sulfamoylphényl)-2-[3-(3-cyano-5-difluorométhylphénoxy)-4-éthyl-2-fluorophényl]acétamide;
le 2-[3-(3-cyano-5-difluorométhylphénoxy)-4-éthyl-2-fluorophényl]-N-(2-méthyl-4-sulfamoylphényl)acétamide;
le 2-[4-bromo-3-(3-chloro-5-cyanophénoxy)-2-fluorophényl]-N-(2-méthyl-4-sulfamoylphényl)acétamide;
le 2-[4-bromo-3-(3-cyano-5-difluorométhylphénoxy)-2-fluorophényl]-N-(2-méthyl-4-sulfamoylphényl)acétamide;
le 2-[4-bromo-3-(3-cyano-5-difluorométhylphénoxy)-2-tluorophényl]-N-(2-chloro-4-sulfamoylphényl)acétamide;
le 2-[4-chloro-3-(3-cyano-5-difluorométhylphénoxy)-2-fluorophényl]-N (2-méthyl-4-sulfamoylphényl)acétamide;
le 2-[3-(3-chloro-5-cyanophénoxy)-2-fluoro-4-méthoxyphényl]-N-(2-chloro-4-sulfamoylphényl)acétamide;
le 2-[3-(3-chloro-5-cyanophénoxy)-2-fluoro-4-méthoxyphényl]-N-(2-méthyl-4-sulfamoylphényl)acétamide; ou
le 2-[3-(3-chloro-5-cyanophénoxy)-2-fluoro-4-méthylphényl]-N-(2-chloro-4-propionylsulfamoylphényl)acétamide; sel de sodium.

7. Composé de formule la 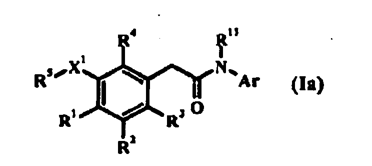 dans laquelle
X¹ est-O-;
R¹ et R² sont choisis chacun indépendamment dans le groupe constitué par hydrogène, alkyle en C₁-C₆, halogénoalkyle en C₁-C₆, cycloalkyle en C₃-C₈, alcoxy en C₁-C₆, alkylthio en C₁-C₆, alkylsulfinyle en C₁-C₆, sulfonyle en C₁-C₆, halogénoalcoxy en C₁-C₆, halogénoalkylthio en C₁-C₆, halogène, amino, alkylamino, dialkylamino, aminoacyle, nitro et cyano; ou (ii) R¹ et R² ensemble sont -O-CH=CH- ou -O-CH₂CH₂- à condition que R¹ ne soit pas l'hydrogène.
R³ et R⁴ sont choisis indépendamment dans le groupe constitué par hydrogène, alkyle en C₁-C₆, halogénoalkyle en C₁-C₆, alcoxy en C₁-C₆, halogénoalcoxy en C₁-C₆, alkylthio en C₁-C₆, halogénoalkylthio en C₁-C₆, halogène, amino, nitro et cyano;
R⁵ est aryle substitué par 1 à 3 substituants choisis indépendamment dans le groupe constitué par alkyle en C₁-C₆, alcényle en C₂-C₆, alcynyle en C₂-C₆, -C≡CCH₂OH, -C≡CCH₂NMe₂, halogénoalkyle en C₁-C₆, cycloalkyle en C₃-C₈, alcoxy en C₁-C₆, alkylthio en C₁-C_{6,} alkylsulfinyle en C₁-C₆, sulfonyle en C₁-C₆, halogénoalcoxy en C₁-C₆, halogénoalkylthio en C₁-C₆, hydroxy, halogène, nitro et cyano, ledit alkyle et ledit cycloalkyle étant éventuellement substitués par 1 ou 2 substituants choisis indépendamment dans le groupe constitué par alkyle, hydroxy, alcoxy, thiol, alkylthio, halogène, amino, alkylamino, aminoalkyle, alkylaminoalkyle et dialkylamino;
Ar est un cycle phényle substitué de formule IIa, à condition que R^{7a} et R^{7c} ne soient pas tous les deux un hydrogène ou que, si R^{7c} est un hydrogène, R^{7a} soit un chlore: 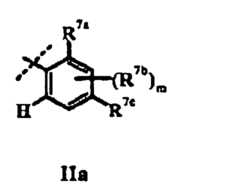
R^{7a} est choisi dans le groupe constitué par hydrogène, alkyle en C₁-C₆, halogénoalkyle en C₁-C₃, alcoxy en C₁-C₆, halogénoalcoxy en C₁-C₆, halogène et cyano;
R^{7b} est choisi indépendamment dans le groupe constitué par halogénoalkyle en C₁C₆, alcoxy en C₁-C₆, alkylthio en C₁-C₆, alkylsulfinyle en C₁-C₆, sulfonyle en C₁-C₆, aminoalkylsulfonyle en C₁-C₆, SO₂NR^{11a}NR^{11b}, halogénoalcoxy en C₁C₆, halogénoalkylthio en C₁-C₆, hydroxy, amino, alkylamino en C₁-C₆, di(alkyl en C₁-C₆)amino, aminoacyle, acyle, CONR⁸R⁹, nitro, cyano, hétéroalcoxy en C₁-C₆, -X²(CH₂)ₚS(O)₂NR⁸R⁹; -X²(CH₂)ₚNHC(O)NHR⁸R⁹, -X²(CH₂)ₚNHS(O)₂NR⁸R⁹ et -X²(CH₂)ₚNHCOOR¹⁰;
R^{7c} est choisi dans le groupe constitué par hydrogène, hétéroalcoxy en C₁-C₆, -S(O)₂NR⁸R⁹, -X²CH₂(CH₂)ₚS(O)ₚNR⁸R⁹; -X²(CH₂)ₚNHC(O)NHR⁸R⁹, X⁶(CH₂)ᵥCONR⁸R⁹, -SO₂R¹³, -NR⁸R⁹, X²(CH₂)pNR¹¹S(O)₂NR⁸R⁹, -X²(CH₂)ₚNBCOOR¹⁰, -X⁶(CH₂)ᵥCOOR¹⁰, -X⁶(CH₂)ᵥCN, -OR¹⁵ et C(=O)CH₂N[(CH₂)₂]₂X⁴;
R⁸ et R⁹, pris indépendamment: l'un des R⁸ et R⁹ est hydrogène ou alkyle en C₁-C₆ et l'autre des R⁸ et R⁹ est choisi dans le groupe constitué par hydrogène, -C(=O)R¹⁴, -C(=O)CR¹²NH₂, -(CH₂)₂N[(CH₂)₂]₂O, COCO₂Me, cycloalkyle en C₃-C₈, ledit cycloalkyle étant éventuellement substitué par 1 ou 2 substituants hydroxyle, pyranyle, alkyle en C₁-C₆ et aryle, lesdits groupes alkyle et aryle étant éventuellement substitués par 1 ou 2 substituants choisis indépendamment dans le groupe constitué par hydroxy, alcoxy en C₁-C₆, thiol, alkylthio en C₁-C₆, alkylsulfinyle en C₁-C₆, sulfonyle en C₁-C₆ et halogène; ou, pris ensemble, sont (CH₂)₂-X⁵-(CH₂)₂ -(CH₂)ₒ- ou (CH₂)ᵣS(O)ₙ éventuellement substitués par 1 ou 2 substituants choisis dans le groupe constitué par halogène, hydroxyle et NR^{11a}R^{11b}_{;}
R¹⁰ est alkyle en C₁-C₆;
R¹¹ est hydrogène ou alkyle en C₁-C₆;
R^{11a} et R^{11b} sont indépendamment R¹¹;
R¹² est la chaîne latérale d'un α-aminoacide naturel;
R¹³ est alkyle en C₁-C₆; -(CH₂)ₛCO₂R¹¹, -(CH₂)₂CN, -(CH₂)₂NH₂, -(CH₂)ᵤOH;
R¹⁴ est alkyle en C₁-C₁₀, -(CH₂)ₛNHR^{11a}R^{11b}, (CH₂)ₛOR¹¹, -CH₂CH(OH)CH₃, -CH₂N[(CH₂)₂]₂O, -(CH₂)₂CO₂R¹¹, phényle ou pyridinyle éventuellement substitués;
R¹⁵ est alkyle en C₁-C₆ éventuellement substitué par 1 à 3 groupes hydroxyle;
X² est -O- ou une liaison;
X⁴ est -O- ou -NMe-;
X⁵ est -O-, -S(O)ₙ- ou NR¹¹;
X⁶ est -O- ou -S(O)ₙ-;
m est un entier de 0 à 2;
n est un entier de 0 à 2;
o est un entier de 4 à 6;
p est un entier de 0 à 6;
r est un entier de 3 à 4;
s est un entier de 1 à 2;
u est un entier de 2 à 3;
v est un entier de 2 à 6; et
hydrates, solvates et sels d'addition d'acides ou sels de la base conjuguée de ceux-ci.

8. Composé de formule la selon la revendication 7, dans lequel
X¹ est -O-;
R¹ et R² sont choisis chacun indépendamment dans le groupe constitué par hydrogène, alkyle en C₁-C₆, alcoxy en C₁-C₆ et halogène; ou R¹ et R² ensemble sont -O-CH=CH- , à condition que R¹ ne soit pas l'hydrogène.
R³ et R⁴ sont choisis indépendamment dans le groupe constitué par hydrogène et halogène;
R⁵ est aryle substitué par 1 à 3 substituants choisis indépendamment dans le groupe constitué par alkyle en C₁-C₆, -C≡CCH₂OH, -C≡CCH₂NMe₂, halogénoalkyle en C₁-C₆, halogénoalcoxy en C₁-C₆. cyano et halogène;
Ar est un cycle phényle substitué de formule IIa, à condition que R^{7a} et R^{7e} ne soient pas tous les deux un hydrogène ou que, si R^{7c} est un hydrogène, R^{7a} soit un chlore: 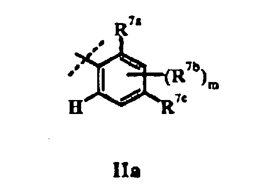
R^{7a} est choisi dans le groupe constitué par hydrogène, alkyle en C₁-C₆, halogénoalkyle en C₁-C₃, alcoxy en C₁-C₆, halogénoalcoxy en C₁-C₆, halogène et cyano;
R^{7b} est choisi indépendamment dans le groupe constitué par halogénoalkyle en C₁-C₆, alcoxy en C₁₋C₆, alkylthio en C₁-C₆, alkylsulfinyle en C₁-C₆, sulfonyle en C₁-C₆, aminoalkylsulfonyle en C₁-C₆, SO₂NR^{11a}NR^{11b}, halogénoalcoxy en C₁-C₆, halogénoalkylthio en C₁-C₆, hydroxy, amino, alkylamino en C₁-C₆, di(alkyl en C₁-C₆)amino, aminoacyle, acyle, CONR⁸R⁹, nitro, cyano, hétéroalcoxy en C₁-C₆, -X²(CH₂)ₚS(O)₂NR⁸R⁹; -X²(CH₂)pNHC(O)NHR^{g}R⁹, -X²(CH₂)ₚNHS(O)₂NR⁸R⁹ et -X²(CH₂)ₚNHCOOR¹⁰;
R^{7c} est choisi dans le groupe constitué par hydrogène, hétéroalcoxy en C₁-C₆, -S(O)₂NR⁸R⁹, -X₂CH₂(CH₂)ₚS(O)₂NR⁸R⁹; -X²(CH₂)ₚNHC(O)NHR⁸R⁹, X⁶(CH₂)ᵥCONR⁸R⁹, -SO₂R¹³, -NR⁸R⁹, X²(CH₂)ₚNR¹¹S(O)₂NR⁸R⁹, -X₂(CH₂)ₚNHCOOR¹⁰, -X⁶(CH₂)ᵥCOOR¹⁰, -X⁶(CH₂)ᵥCN, -OR¹⁵ et C(=O)CH₂N[(CH₂)₂]₂X⁴;
R⁸ et R⁹, pris indépendamment: l'un des R⁸ et R⁹ est hydrogène ou alkyle en C₁-C₆ et l'autre des R⁸ et R⁹ est choisi dans le groupe constitué par hydrogène, -C(=O)R¹⁴, -C(=O)CR¹²NH₂, -(CH₂)₂N[(CH₂)₂]₂O, COCO₂Me, cycloalkyle en C₃-C₈, ledit cycloalkyle étant éventuellement substitué par 1 ou 2 substituants hydroxyle, pyranyle, alkyle en C₁-C₆ et aryle, lesdits groupes alkyle et aryle étant éventuellement substitués par 1 ou 2 substituants choisis indépendamment dans le groupe constitué par hydroxy, alcoxy en C₁-C₆, thiol, alkylthio en C₁-C₆, alkylsulfinyle en C₁-C₆, sulfonyle en C₁-C₆ et halogène; ou, pris ensemble, sont (CH₂)₂-X⁵-(CH₂)₂ ou -(CH₂)ₒ- ou (CH₂)ᵣS(O)ₙ éventuellement substitué par 1 ou 2 substituants choisis dans le groupe constitué par halogène, hydroxyle et NR^{11a}R^{11b};
R¹⁰ est alkyle en C₁-C₆;
R¹¹ est hydrogène ou alkyle en C₁-C₆;
R^{11a} et R^{11b} sont indépendamment R¹¹;
R¹² est la chaîne latérale d'un α-aminoacide naturel;
R¹³ est alkyle en C₁-C₆; -(CH₂)SCO₂R¹¹, -(CH)₂CN, -(CH₂)NH₂, -(CH₂)ᵤOH:
R¹⁴ est alkyle en C₁-C₁₀, -(CH₂)ₛNHR^{11a}R^{11b} (CH₂)ₛOR¹¹, -CH₂CH(OH)CH₃, -CH₂N[(CH₂)₂]₂O, -(CH₂)₂CO₂R¹¹, phényle ou pyridinyle éventuellement substitués;
R¹⁵ est alkyle en C₁-C₆ éventuellement substitué par 1 à 3 groupes hydroxyle;
X² est -O- ou une liaison;
X⁴ est -O- ou -NMe-;
X⁵ est -O-, -S(O)ₙ- ou NR¹¹;
X⁶ est -O- ou -S(O)ₙ-;
m est un entier de 0 à 2;
n est un entier de 0 à 2;
o est un entier de 4 à 6;
p est un entier de 0 à 6;
r est un entier de 3 à 4;
s est un entier de 1 à 2;
u est un entier de 2 à 3;
v est un entier de 2 à 6; et
hydrates, solvates et sels d'addition d'acides ou sels de la base conjuguée de ceux-ci.

9. Composé de formule Ia selon la revendication 8, dans lequel:
I¹ est -O-;
R¹ et R² sont choisis chacun indépendamment dans le groupe constitué par hydrogène, méthyle, éthyle, i-propyle, méthoxy, chloro et bromo; ou R¹ et R² ensemble sont -O-CH=CH- ;
R³ et R⁴ sont choisis indépendamment dans le groupe constitué par hydrogène et fluoro;
R⁵ est phényle, ledit phényle étant substitué par 1 à 3 substituants choisis indépendamment dans le groupe constitué par méthyle, éthyle, -CHF₂, -CF₃, -CF₂CH₃, -OCHF₂, chloro, bromo, fluoro, -C≡CCH₂OH, -C≡CCH₂N(CH₃)₂ et cyano;
Ar est phényle, ledit phényle étant substitué par 1 à 3 substituants choisis indépendamment dans le groupe constitué par méthyle, halogène. 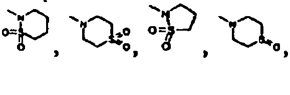 -SO₂NH₂, -SO₂NHCH₃, -SO₂NCH₃CH₂CH₂OH, -SO₂NHCH₂CHCH₃OH. -SO₂NHCH₂CH₂ 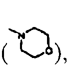 -SO₂NHCH₂CH₂CH₂OH, -SO₂ 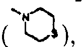 -SO₃ 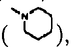 -SO₂ 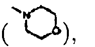 SO₂ 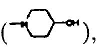 -SO₂NH 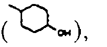 -SO₂ 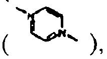 SO₂NHCOCH₂CH₃, -SO₂NHCOCH₂CH₂CH₃, -SO₂NHCOCH(NH₂)CH(CH₃)₂, -SO₂NCH₃COCH₂CH₂CH₃, -SO₂NHCOCH₂CH₂CH₂CH₃, -SO₂NHCOCH₂CH(CH₃)₂. -SO₂NHCOCH₂CH(CH₂CH₃)₂, -SO₂NHCOCH₂CH₂CH(CH₃)₂, -SO₂NHCOCH₂CH₂NH₂, -SO₂NCH₃COCH₂CH₂NH₂, -SO₂NHCOCH₂OCH₃, -SO₂NHCO 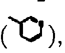 -SO₂NH 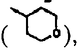 -SO₂NHCOCH(i-propyl)NH₂, -SO₂NHCH₂CH₂SCH₃, -SO₂NCH₃COCH₂CH₃, -SO₂NCH₃COCH₂OCH₃, -SO₂NCH₃COCH₂CH₂OCH₃, -SO₂NHCOCH₂OH, -SO₂NHCOCH₂CH₂COOH, -SO₂CH₂COOH, -SO₂CH₂CH₂COOH, -SO₂CH₃, ₋SO₂CH₂CH₂NH₂, ₋SO₂CH₂CH₂CN, -SO₂CH₂CH₂CH₂OH, -O(CH₂)₃SO₂NH₂, -OCH₂CN, -OCH₂CHOHCH₂OH, -OCH₂CH₂CHOHCH₂OH, -COOCH₃, -COOH, -COCH₂ 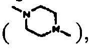 -COCH₂ 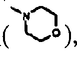 -N(CH₃)₂ et -NHCOCOOCH₂CH₃;
R¹¹ est hydrogène ou méthyle;
hydrates, solvates et sels de ceux-ci.

10. Composé de formule Ia selon la revendication 7, qui est
le 2-[4-chloro-3-(3-chloro-5-cyanophénoxy)phényl]-N-(4-sulfamoylphényl)-acétamide;
le 2-[4-chloro-3-(3,5-dicyanophénoxy)phényl]-N-(4-sulfamoylphényl)acétamide;
le 2-[3-(2-bromo-5-chlorophénoxy)-4-chlorophényl]-N-[2-méthyl-4-(3-sulfamoylpropoxy)phényl]acétamide;
le 2-[3-(2-bromo-5-chlorophénoxy)-4-chlorophényl]-N-(4-sulfamoylphényl)-acétamide;
le 2-[4-chloro-3-(3,5-dicyanophénoxy)phényl]-N-(2-chlorophényl)acématide;
le 2-[4-chloro-3-(3,5-dicyanophénoxy)phényl]-N-(4-diméthylaminophényl)-acétamide;
le 2-[3-(2-chloro-5-cyanophényl)-4-éthyl-2-fluorophényl]-N-(4-sulfamoylphényl)-acétamide;
le 2-[4-chloro-3-(2,5-dicyanophénoxy)-2-fluorophényl]-N-(4-sulfamoylphényl)-acétamide;
le 2-[4-chloro-3-(3,5-dicyanophénoxy)-2-fluorophényl]-N-(4-sulfamoylphényl)-acétamide;
le 2-[4-chloro-2-fluoro-3-(2,3,5-trichlorophénoxy)phényl]-N-(4-sulfamoylphényl)-acétamide;
le 2-[3-(3-cyano-5-fluorophénoxy)-4-méthylphényl]-N-(4-sulfamoylphényl)-acétamide;
le N-(2-chlorophényl)-2-[3-(3-cyano-5-fluorophénoxy)-4-méthylphényl]acétamide;
le 2-[4-chloro-3-(3-cyano-5-difluorométhylphénoxy)-2-fluorophényl]-N-(4-sulfamoylphényl)acétamide;
le 2-[4-chloro-3-(3-cyano-5-difluorométhylphénoxy)-2-fluorophényl]-N-(2-chlorophényl)acétamide;
le 2-[3-(2-bromo-5-chlorophénoxy)-4-éthylphényl]-N-(4-sulfamoylphényl)acétamide;
le 2-[3-(2-bromo-5-chlorophénoxy)-4-éthylphényl]-N-(2-chlorophényl)acétamide;
le 2-[3-(5-chloro-2-cyanophénoxy)-4-éthylphényl]-N-(4-sulfamoylphényl)acétamide;
le 2-[3-(5-chloro-2-cyanophénoxy)-4-éthylphényl]-N-(2-chlorophényl)acétamide;
le 2-[3-(2,5-dichlorophénoxy)-4-éthylphényl]-N-(4-sulfamoylphényl)acétamide;
le 2-[3-(2,6-dichlorophénoxy)-4-éthylphényl]-N-(4-sulfamoylphényl)acétamide;
le N-(2-chlorophényl)-2-[3-(2,5-dichlorophénoxy)-4-éthylphényl]acétamide;
le 2-[3-(3-bromo-5-chlorophénoxy)-4-éthylphényl]-N-(4-sulfamoylphényl)acétamide;
le N-(2-chlorophényl)-2-[3-(2,6-dichlorophénoxy)-4-éthylphényl]acétamide;
le 2-[3-(3-bromo-5-chlorophénoxy)-4-éthylphényl]-N-(2-chlorophényl)acétamide;
le N-(2-chlorophényl)-2-[3-(3,5-dicyanophénoxy)-4-éthylphényl]acétamide;
le 2-[3-(2-bromo-5-chlorophénoxy)-4-isopropylphényl]-N-(4-sulfamoylphényl)-acétamide;
le 2-[3-(2-bromo-5-chlorophénoxy)-4-isopropylphényl]-N-(2-chlorophényl)-acétamide;
le 2-[4-chloro-3-(3,5-dicyanophénoxy)-2-fluorophényl]-N-(2-chlorophényl)-acétamide;
le 2-[4-chloro-3-(3,5-dicyanophénoxy)phényl]-N-[2-méthyl-4-(3-sulfamoylpropoxy)-phényl]acétamide;
le 2-[3-(2-bromo-5-chlorophénoxy)-4-méthylphényl]-N-(4-sulfamoylphényl)-acétamide;
le 2-[3-(2-bromo-5-chlorophénoxy)-4-méthylphényl]-N-(2-méthyl-4-sulfamoylphényl)acétamide;
le 2-[4-chloro-3-(3,5-dicyanophénoxy)-2-fluorophényl]-N-(2-méthyl-4-sulfamoylphényl)acétamide;
le 2-[3-(2-bromo-5-chlorophénoxy)-4-méthylphényl]-N-(2-chlorophényl)acétamide;
le N-(2-chlorophényl)-2-[3-(3,5-dicyanophénoxy)-4-méthylphényl]acétamide;
le 2-[3-(3-bromo-2,5-dichlorophénoxy)-4-éthylphényl]-N-(4-sulfamoylphényl)-acétamide;
le 2-[3-(3-bromo-2,5-dichlorophénoxy)-4-éthylphényl]-N-(2-méthyl-4-sulfamoylphényl)acétamide;
le 2-[3-(3-bromo-2,5-dichlorophénoxy)-4-éthylphényl]-N-(2-chlorophényl)-acétamide;
le N-(2-chlorophényl)-2-[3-(3,5-dibromo-2-chlorophénoxy)-4-éthylphényl]-acétamide;
le 2-[3-(5-bromo-2-chloro-3-fluorophénoxy)-4-éthylphényl]-N-(4-sulfamoylphényl)-acétamide;
le 2-[3-(5-bromo-2-chloro-3-fluorophénoxy)-4-éthylphényl]-N-(2-méthyl-4-sulfamoylphényl)acétamide;
le 2-[3-(5-bromo-2-chloro-3-fluorophénoxy)-4-éthylphényl]-N-(2-chlorophényl)-acétamide;
le 2-[4-chloro-3-(3-chloro-5-cyanophénoxy)-2-fluorophényl]-N-(2-méthyl-4-sulfamoylphényl)acétamide;
le 2-[3-(3,5-dicyanophénoxy)-4-méthylphényl]-N-(2-méthyl-4-sulfamoylphényl)-acétamide;
le N-(2-chloro-4-sulfamoylphényl)-2-[3-(3,5-dicyanophénoxy)-4-méthylphényl]-acétamide;
le 2-[3-(3-chloro-5-cyanophénoxy)-4-éthylphényl]-N-(4-sulfamoylphényl)acétamide;
le 2-[3-(3-chloro-5-cyanophénoxy)-4-éthylphényl]-N-(2-méthyl-4-sulfamoylphényl)-acétamide;
le 2-[3-(3-chloro-5-cyanophénoxy)-4-éthylphényl]-N-(2-chloro-4-sulfamoylphényl)-acétamide;
le 2-[3-(3-chloro-5-cyanophénoxy)-4-éthylphényl)-N-(4-méthylsulfamoylphényl)-acétamide;
le 2-[3-(2-chloro-5-cyano-3-fluorophénoxy)-4-éthylphényl]-N-(4-sulfamoylphényl)-acétamide;
le 2-[3-(2-chloro-5-cyano-3-fluorophénoxy)-4-éthylphényl]-N-(2-méthyl-4-sulfamoylphényl)acétamide;
le 2-[4-chloro-3-(3-chloro-5-cyanophénoxy)-2-fluorophényl]-N-(2-méthyl-4-propionylsulfamoylphényl)acétamide;
le 2-[3-(2-chloro-3,5-dicyanophénoxy)-4-éthylphényl]-N-(4-sulfamoylphényl)-acétamide;
le 2-[3-(2-chloro-3,5-dicyanophénoxy)-4-éthylphényl]-N-(2-méthyl-4-sulfamoylphényl)acétamide;
le 2-[3-(2-chloro-3,5-dicyanophénoxy)-4-éthylphényl]-N-(2-chloro-4-sulfamoylphényl)acétamide;
le 2-(4-chloro-3-(4-cyano-2,6-diméthylphénoxy)phényl]-N-(4-sulfamoylphényl)-acétamide;
le 2-[4-chloro-3-(4-cyano-2,6-diméthylphénoxy)phényl]-N-(2-méthyl-4-sulfamoylphényl)acétamide;
le 2-[3-(3-chloro-5-cyanophénoxy)-4-méthoxyphényl]-N-(4-sulfamoylphényl)-acétamide;
le 2-[3-(3-chloro-5-cyanophénoxy)-4-éthylphényl]-N-(2-chlorophényl)acétamide;
le 2-[3-(2-chloro-5-cyano-3-fluorophénoxy)-4-éthylphényl]-N-(2-chlorophényl)-acétamide;
le 2-[3-(2-chloro-3,5-dicyanophénoxy)-4-éthylphényl]-N-(2-chlorophényl)acétamide;
le 2-[3-(3-chloro-5-cyanophénoxy)-4-méthoxyphényl]-N-(2-chlorophényl)acétamide;
le 2-[3-(2-chloro-5-cyanophénoxy)-4-méthoxyphényl]-N-(2-chlorophényl)acétamide;
le 2-[4-bromo-3-(3-chloro-5-cyanophénoxy)-2-fluorophényl]-N-(2-chloro-4-propionylsulfamoylphényl)acétamide; sel de sodium;
le 2-[3-(3-chloro-5-cyanophénoxy)-4-méthoxyphényl]-N-(2-chloro-4-sulfamoylphényl)acétamide;
le 2-[3-(2-chloro-5-cyanophénoxy)-4-méthoxyphényl]-N-(4-sulfamoylphényl)-acétamide;
le 2-[3-(2-chloro-5-cyanophénoxy)-4-méthoxyphényl]-N-(2-méthyl-4-sulfamoylphényl)acétamide;
le 2-[3-(2-chloro-5-cyanophénoxy)-4-méthoxyphényl]-N-(2-chloro-4-sulfamoylphényl)acétamide;
le 2-[4-chloro-3-(4-cyano-2,6-diméthylphénoxy)phényl]-N-(2-chlorophényl)-acétamide;
le 2-[4-chloro-3-(3,5-dicyanophénoxy)phényl]-N-(4-méthylsulfamoylphényl)-acétamide;
le 2-[4-chloro-3-(3,5-dicyanophénoxy)phényl]-N-(4-diméthylsulfamoylphényl)-acétamide;
le 2-[4-chloro-3-(3,5-dicyanophénoxy)phényl]-N-[4-(pipéridine-1-sulfonyl)phényl]-acétamide;
le 2-[4-chloro-3-(3,5-dicyanophénoxy)phényl]-N-[4-(morpholine-4-sulfonyl)phényl]-acétamide;
le 2-[3-(3-chloro-5-cyanophénoxy)-4-méthoxyphényl]-N-(2-méthyl-4-sulfamoylphényl)acétamide;
le 2-[3-(3-chloro-5-cyanophénoxy)-2-fluoro-4-méthylphényl]-N-(2-méthyl-4-sulfamoylphényl)acétamide:
le 2-[4-chloro-3-(3-cyano-5-difluorométhylphénoxy)-2-fluorophényl]-N-(2-méthyl-4-sulfamoylphényl)acétamide;
le 2-[4-chloro-3-(3,5-dicyanophénoxy)phényl]-N-(2-chloro-4-méthanesulfonylphényl)acétamide;
le N-[4-((S)-2-amino-3-méthylbutyrylsulfamoyl)-2-méthylphényl]-2-[4-chloro-3-(3-chloro-5-cyanophénoxy)-2-fluorophényl]acétamide; composé avec l'acide chlorhydrique;
le 2-[4-chloro-3-(3,5-dicyanophénoxy)phényl]-N-(4-cyanométhoxyphényl)acétamide;
le 2-[4-chloro-3-(3,5-dicyanophénoxy)phényl]-N-(4-méthanesulfonylphényl)-acétamide;
le 3-chloro-4-{2-[4-chloro-3-(3,5-dicyanophénoxy)phényl]acétylamino}benzoate de méthyle;
le 2-[4-chloro-3-(3,5-dicyanophénoxy)phényl]-N-{4-[(2-hydroxyéthyl)méthylsulfamoyl]phényl}acétamide;
le 2-[4-chloro-3-(3,5-dicyanophénoxy)phényl]-N-[4-(4-hydroxypipéridine-1-sulfonyl)phényl]acétamide;
le 2-[3-(3-chloro-5-cyanophénoxy)-2-fluoro-4-méthylphényl]-N-(2-chloro-4-sulfamoylphényl)acétamide;
le N-(2-chloro-4-sulfamoylphényl)-2-[3-(3-cyano-5-difluorométhylphénoxy)-2-fluoro-4-méthylphényl]acétamide;
le 2-[3-(3-cyano-5-difluorométhylphénoxy)-2-fluoro-4-méthylphényl]-N-(2-méthyl-4-sulfamoylphényl)acétamide;
le N-(2-chlorophényl)-2-[3-(3-cyano-5-difluorométhylphénoxy)-2-fluoro-4-méthylphényl]acétamide;
le 2-[4-chloro-3-(3-chloro-5-cyanophénoxy)-2-fluorophényl]-N-{2-méthyl-4-[(pyridine-3-carbonyl)sulfamoyl]phényl}acétamide; composé avec l'acide chlorhydrique;
le 2-[4-chloro-3-(3-cyano-5-trifluorométhylphénoxy)phényl]-N-(4-sulfamoylphénylacétamide;
le 2-[4-chloro-3-(3-cyano-5-trifluorométhylphénoxy)phényl]-N-(2-méthyl-4-sulfamoylphényl)acétamide;
le 2-[4-chloro-3-(3-cyano-5-trifluorométhylphénoxy)phényl]-N-(2-chloro-4-sulfamoylphényl)acétamide;
le 2-[3-(3-cyano-5-difluorométhylphénoxy)-4-éthylphényl]-N-(4-sulfamoylphényl)-acétamide;
le 2-[3-(3-cyano-5-difluorométhylphénoxy)-4-méthoxyphényl]-N-(4-sulfamoylphényl)acétamide;
le 2-[3-(3-cyano-5-difluorométhylphénoxy)-4-méthoxyphényl]-N-(2-méthyl-4-sulfamoylphényl)acétamide;
le N-(2-chloro-4-sulfamoylphényl)-2-[3-(3-cyano-5-difluorométhylphénoxy)-4-méthoxyphényl]acétamide;
le 2-{4-chloro-3-[3-cyano-5-(1,1-difluoroéthyl)phénoxy]-2-fluorophényl}-N-(4-sulfamoylphényl)acétamide;
le 2-{4-chloro-3-[3-cyano-5-(1,1-difluoroéthyl)phénoxy]-2-fluorophényl}1-N-(2-méthyl-4-sulfamoylphényl)acétamide;
le 2-{4-chloro-3-[3-cyano-5-(1,1-difluoroéthyl)phénoxy]-2-fluorophényl}-N-(2-chloro-4-sulfamoylphényl)acétamide;
le 2-[4-chloro-3-(3-chloro-5-cyanophénoxy)-2-fluorophényl]-N-(2-chlorophényl)-acétamide;
le 2-[4-chloro-3-(3-chloro-5-cyanophénoxy)-2-fluorophényl]-N-[2-méthyl-4-(méthylpropionylsulfamoyl)phényl]acétamide;
le 2-[4-chloro-3-(3-chloro-5-cyanophénoxy)-2-fluorophényl]-N-(2-chloro-4-sulfamoylphényl)acétamide;
le 2-[3-(3-cyano-5-difluorométhylphénoxy)-4-éthylphényl]-N-(2-méthyl-4-sulfamoylphényl)acétamide;
le 2-[3-(2-chloro-5-cyanophénoxy)-5-méthoxyphényl]-N-(4-sulfamoylphényl)-acétamide;
le 2-[3-(2-chloro-5-cyanophénoxy)-5-méthoxyphényl]-N-(2-méthyl-4-sulfamoylphényl)acétamide;
le 2-[4,5-dibromo-3-(3-cyano-5-difluorométhylphénoxy)-2-fluorophényl]-N-(2-méthyl-4-sulfamoylphényl)acétamide;
le 2-[3-(2-chloro-5-cyanophénoxy)-5-éthylphényl]-N-(4-sulfamoylphényl)acétamide;
le 2-[3-(2-chloro-5-cyanophénoxy)-5-éthylphényl]-N-(2-méthyl-4-sulfamoylphényl)-acétamide;
le 2-[3-(2-chloro-5-cyanophénoxy)-5-éthylphényl]-N-(2-chloro-4-sulfamoylphényl)-acétamide;
le 2-[4-chloro-3-(3-chloro-5-cyanophénoxy)-2-tluorophényl]-N-(2-méthyl-4-méthylsulfamoylphényl)acétamide;
le 2-[4-bromo-3-(3-chloro-5-cyanophénoxy)-2-fluorophényl]-N-(2-chloro-4-sulfamoylphényl)acétamide;
l'acide 3-chloro-4-{2-[3-(3-cyano-5-difluorométhylphénoxy)-2-fluoro-4-méthylphényl]acétylamino}benzoïque.
le 2-[4-chloro-3-(3-chloro-5-cyanophénoxy)-2-fluorophényl]-N-(2-chlorophényl)-N-méthylacétamide;
le N-(2-chloro-4-méthanesulfonylphényl)-2-[3-(3-cyano-5-difluorométhylphénoxy)-2-fluoro-4-méthylphényl] acétamide;
le 2-[3-(2-chloro-5-cyanophénoxy)-5-méthoxyphényl]-N-(2-chloro-4-sulfamoylphényl)acétamide;
le 2-[4,5-dibromo-3-(3-cyano-5-difluorométhylphénoxy)-2-fluorophényl]-N-(4-sulfamoylphényl)acétamide;
le 2-[3-(3-chloro-5-cyanophénoxy)-4-méthylphényl]-N-[2-méthyl-4-(morpholine-4-sulfonyl)phényl]acétamide;
le 2-[3-(3-chloro-5-cyanophénoxy)-4-méthylphényl]-N-[2-méthyl-4-(4-méthylpipérazine-1-sulfonyl)phényl] acétamide;
le 2-[3-(3-chloro-5-cyanophénoxy)-4-méthylphényl]-N-[2-méthyl-4-(thiomorpholine-4-sulfonyl)phényl] acétamide;
le 2-[3-(3-chloro-5-cyanophénoxy)-4-méthylphényl]-N-[2-méthyl-4-(2-morpholin-4-yléthylsulfamoyl)phényl]acétamide;
le 2-[3-(3-chloro-5-cyanophénoxy)-4-méthylphényl]-N-[4-(3-hydroxypropyl-sulfamoyl)-2-méthylphényl]acétamide;
le 2-[4-chloro-3-(3-cyano-5-trifluorométhylphénoxy)-2-fluorophényl]-N-(2-méthyl-4-sulfamoylphényl)acétamide;
le 2-[3-(3-chloro-5-cyanophénoxy)-4-méthylphényl]-N-[4-(4-hydroxycyclohexyl-sulfamoyl)-2-méthylphényl] acétamide;
le 2-[3-(3-chloro-5-cyanophénoxy)-4-méthylphényl]-N-(2-méthyl-4-sulfamoyl-phényl)acétamide;
le 2-[3-(3-chloro-5-cyanophénoxy)-4-méthylphényl]-N-(2-chloro-4-sulfamoyl-phényl)acétamide;
le 2-[4-chloro-3-(4-cyano-6-méthylpyridin-2-yloxy)-2-fluorophényl]-N-(2-méthyl-4-sulfamoylphényl)acétamide;
l'acide 3-(4-{2-[3-(3-chloro-5-cyanophénoxy)-4-méthylphényl]acétylamino}-3-méthylbenzènesulfonyl)propionique;
le 2-[4-chloro-3-(3-chloro-5-cyanophénoxy)-2-fluorophényl]-N-(2-chloro-4-propionylsulfamoylphényl)acétamide; sel de sodium;
le N-[4-((S)-2-amino-3-méthylbutyrylsulfamoyl)-2-chlorophényl]-2-[4-chloro-3-(3-chloro-5-cyanophénoxy)-2-fluorophényl]acétamide; sel de sodium;
le 2-[3-(3-chloro-5-cyanophénoxy)-4-méthylphényl]-N-[2-méthyl-4-(tétrahydro-pyran-4-ylsulfamoyl)phényl]acétamide;
le 2-[3-(3-chloro-5-cyanophénoxy)-4-méthylphényl]-N-[4-(2-hydroxypropylsulfamoyl)-2-méthylphényl]acétamide;
le 2-[3-(3-chloro-5-cyanophénoxy)-4-méthylphényl]-N-[2-méthyl-4-(2-méthylsulfanyléthylsulfamoyl)phényl]acétamide;
le 2-[3-(3-chloro-5-cyanophénoxy)-4-méthylphényl]-N-[2-méthyl-4-(3-méthylsulfanylpropylsulfamoyl)phényl]acétamide;
le 2-[3-(3-chloro-5-cyanophénoxy)-5-méthoxyphényl]-N-(4-sulfamoylphényl)-acétamide
le 2-[3-(3-chloro-5-cyanophénoxy)-5-méthoxyphényl]-N-(2-méthyl-4-sulfamoylphényl)acétamide;
le N-(2-chloro-4-sulfamoylphényl)-2-[4,5-dibromo-3-(3-cyano-5-difluorométhylphénoxy)-2-fluorophényl]acétamide;
le 2-[4-chloro-3-(4-cyano-2,6-diméthylphénoxy)phényl]-N-(2-chloro-4-sulfamoylphényl)acétamide
le 2-[3-(3-chloro-5-cyanophénoxy)-5-méthoxyphényl]-N-(2-chloro-4-sulfamoylphényl)acétamide;
le 2-[3-(3-chloro-5-cyanophénoxy)-4-méthylphényl]-N-[4-(2-cyanoéthanesulfonyl)-2-méthylphényl]acétamide;
le N-(4-butyrylsulfamoyl-2-chlorophényl)-2-[4-chloro-3-(3-chloro-5-cyanophénoxy)-2-fluorophényl]acétamide; sel de sodium;
le N-[4-(butyrylméthylsulfamoyl)-2-chlorophényl]-2-[4-chloro-3-(3-chloro-5-cyanophénoxy)-2-fluorophényl]acétamide;
le N-(2-chloro-4-sulfamoylphényl)-2-[3-(3-cyano-5-difluorométhylphénoxy)-4-éthylphényl]acétamide;
le 2-[3-(3-chloro-5-cyanophénoxy)-5-méthylphényl]-N-(4-sulfamoylphényl)-acétamide;
le 2-[3-(3-chloro-5-cyanophénoxy)-5-méthylphényl]-N-(2-méthyl-4-sulfamoylphényl)acétamide;
le 2-[3-(3-chloro-5-cyanophénoxy)-5-méthylphényl]-N-(2-chloro-4-sulfamoylphényl)acétamide;
le 2-[7-(3-cyanophénoxy)benzofuran-5-yl]-N-(4-sulfamoylphényl)acétamide;
le 2-[7-(3-cyanophénoxy)benzofuran-5-yl]-N-(2-méthyl-4-sulfamoylphényl)-acétamide;
le 2-[3-(2-chloro-5-cyanophénoxy)-5-méthylphényl]-N-(4-sulfamoylphényl)-acétamide;
le 2-[3-(2-chloro-5-cyanophénoxy)-5-méthylphényl]-N-(2-méthyl-4-sulfamoylphényl)acétamide;
le 2-[3-(2-chloro-5-cyanophénoxy)-5-méthylphényl]-N-(2-chloro-4-sulfamoylphényl)acétamide;
le N-(2-chloro-4-sulfamoylphényl)-2-[7-(3,5-dichlorophénoxy)benzofuran-5-yl]acétamide;
le 2-[3-(3-chloro-5-cyanophénoxy)-4-méthylphényl]-N-[4-((S)-2.3-dihydroxypropoxy)-2-méthylphényl]acétamide;
le 2-[3-(3-chloro-5-cyanophénoxy)-4-méthylphényl]-N-[4-((R)-3,4-dihydroxybutoxy)-2-méthylphényl]acétamide;
l'acide (4-{2-[3-(3-chloro-5-cyanophénoxy)-4-méthylphényl]acétylamino}-3-méthylbenzènesulfonyl)acétique;
le N-[4-(3-aminopropionylsulfamoyl)-2-chlorophényl]-2-[4-chloro-3-(3-chloro-5-cyanophénoxy)-2-fluorophényl]acétamide; composé avec l'acide chlorhydrique;
le N-[4-(2-aminoéthanesulfonyl)-2-méthylphényl]-2-[3-(3-chloro-5-cyanophénoxy)-4-méthylphényl]acétamide;
le 2-[4-chloro-3-(3-chloro-5-cyanophénoxy)-2-fluorophényl]-N-[2-chloro-4-(2-méthoxyacétylsulfamoyl)phényl]acétamide; sel de sodium;
le 2-[4-chloro-3-(3-chloro-5-cyanophénoxy)-2-fluorophényl]-N-[2-chloro-4-(2-hydroxyacétylsulfamoyl)phényl]acétamide; sel de sodium;
le 2-[3-(3-chloro-5-cyanophénoxy)-4-méthylphényl]-N-[4-(3-hydroxypropane-1-sulfonyl)-2-méthylphényl]acétamide;
le 2-[3-(3-chloro-5-cyanophénoxy)-4-méthylphényl]-N-[4-(1,1-dioxo-1λ6-[1,2]thiazinan-2-yl)-2-méthylphényl]acétamide;
le 2-[3-(3-chloro-5-cyanophénoxy)-4-méthylphényl]-N- {2-méthyl-4-[2-(4-méthylpipérazin-1-yl)acétyl]phényl} acétamide;
le 2-[3-(3-chloro-5-cyanophénoxy)-4-méthylphényl]--N-[2-méthyl-4-(1-oxo-1λ4 thiomorpholin-4-yl)phényl]acétamide;
le 2-[3-(3-chloro-5-cyanophénoxy)-4-méthylphényl]-N-[4-(1,1-dioxo-1λ6-thiomorpholin-4-yl)-2-méthylphényl]acétamide;
le 2-[4-chloro-3-(3-chloro-5-cyanophénoxy)-2-fluorophényl]-N-[2-chloro-4-(méthylpropionylsulfamoyl)phényl]acétamide;
le 2-[3-(3-chloro-5-cyanophénoxy)-4-méthylphényl]-N-[2-méthyl-4-(2-morpholin-4-ylacétyl)phényl]acétamide; composé avec l'acide triflùoroacétique;
le 2-[4-chloro-3-(3-chloro-5-cyanophénoxy)-2-fluorophényl]-N-[2-chloro-4-(3-méthoxypropionylsulfamoyl)phényl]acétamide; sel de sodium;
le 2-[4-bromo-3-(3-cyano-5-difluorométhylphénoxy)-2-fluorophényl]-N-(2-chloro-4-sulfamoylphényl)acétamide;
le 2-[4-chloro-3-(3-cyano-5-difluorométhoxyphénoxy)-2-tluorophényl]-N-(4-sulfamoylphényl)acétamide;
le 2-[4-chloro-3-(3-cyano-5-difluorométhoxyphénoxy)-2-fluorophényl]-N-(2-chloro-4-sulfamoylphényl)acétamide;
le 2-[4-chloro-3-(3-chloro-5-cyanophénoxy)-2-fluorophényl]-N-(2-chloro-4-méthylsulfamoylphényl)acétamide;
le 2-[4-bromo-3-(3-cyano-5-difluorométhylphénoxy)-2-fluorophényl]-N-(2-méthyl-4-sulfamoylphényl)acétamide;
le 2-[3-(3-chloro-5-cyanophénoxy)-4-méthylphényl]-N-[4-(1,1-dioxo-1λ6-isothiazolidin-2-yl)-2-méthylphényl]acétamide;
le 2-[4-chloro-3-(3-chloro-5-cyanophénoxy)-2-fluorophényl]-N-{2-chloro-4-[(2-méthoxyacétyl)méthylsulfamoyl]phényl}acétamide;
le 2-[4-chloro-3-(3-chloro-5-cyanophénoxy)-2-fluorophényl]-N-{2-chloro-4-[(3-méthoxypropionyl)méthylsulfamoyl]phényl}acétamide;
le 2-[4-chloro-3-(3-chloro-5-cyanophénoxy)-2-fluorophényl]-N-(2-chloro-4-pentanoylsulfamoylphényl)acétamide; sel de sodium;
le 2-[4-chloro-3-(3-chloro-5-cyanophénoxy)-2-fluorophényl]-N-[2-chloro-4-(3-méthylbutyrylsulfamoyl)phényl]acétamide; sel de sodium;
le 2-[4-chloro-3-(3-chloro-5-cyanophénoxy)-2-fluorophényl]-N-[2-chloro-4-(2-diéthylaminoacétylsulfamoyl)phényl]acétamide; composé avec l'acide chlorhydrique:
le 2-[4-chloro-3-(3-chloro-5-cyanophénoxy)-2-fluorophényl]-N-[2-chloro-4-(4-méthylpentanoylsulfamoyl)phényl]acétamide; sel de sodium;
le 2-[4-chloro-3-(3-chloro-5-cyanophénoxy)-2-fluorophényl]-N-[2-chloro-4-(2-morpholin-4-ylacétylsulfamoyl)phényl]acétamide; composé avec l'acide chlorhydrique;
le N-(4-(2-[3-(3-chloro-5-cyanophénoxy)-4-méthylphényl]acétylamino)-3-méthylphényl)oxalamate d'éthyle;
le N-(2-chloro-4-sulfamoylphényl)-2-[3-(3-cyano-5-difluorométhylphénoxy)-4-éthyl-2-fluorophényl]acétamide;
le 2-[3-(3-cyano-5-difluorométhylphénoxy)-4-éthyl-2-fluorophényl]-N-(2-méthyl-4-sulfamoylphényl)acétamide;
le N-(4-[(3-aminopropionyl)méthylsulfamoyl]-2-chlorophényl)-2-[4-chloro-3-(3-chloro-5-cyanophénoxy)-2-fluorophényl]acétamide; composé avec l'acide chlorhydrique;
le 2-[3-(3-chloro-5-cyanophénoxy)-4,5-diméthylphényl]-N-(4-sulfamoylphényl)-acétamide;
le 2-[3-(3-chloro-5-cyanophénoxy)-4,5-diméthylphényl]-N-(2-méthyl-4-sulfamoylphényl)acétamide;
le 2-[3-(3-chloro-5-cyanophénoxy)-4,5-diméthylphényl]-N-(2-chloro-4-sulfamoylphényl)acétamide;
le 2-[3-(3-bromo-5-cyanophénoxy)-4-chloro-2-fluorophényl]-N-(2-méthyl-4-sulfamoylphényl)acétamide;
le 2-{4-chloro-3-[3-cyano-5-(3-hydroxyprop-1-ynyl)phénoxy]-2-fluorophényl}-N-(2-méthyl-4-sulfamoylphényl)acétamide;
le 2-{4-chloro-3-[3-cyano-5-(3-diméthylaminoprop-1-ynyl)phénoxy]-2-fluorophényl }-N-(2-méthyl-4-sulfamoylphényl)acétamide;
l'acide 4-(3-chloro-4-{2-[4-chloro-3-(3-chloro-5-cyanophénoxy)-2-fluorophényl]-acétylamino}benzènesulfonylamino)-4-oxobutyrique;
le 2-[4-bromo-3-(3-chloro-5-cyanophénoxy)-2-fluorophényl]-N-(2-méthyl-4-sulfamoylphényl)acétamide;
le 2-[3-(3-chloro-5-cyanophénoxy)-2-fluoro-4-méthoxyphényl]-N-(2-chloro-4-sulfamoylphényl)acétamide;
le 2-[3-(3-chloro-5-cyanophénoxy)-2-fluoro-4-méthoxyphényl]-N-(2-méthyl-4-sulfamoylphényl)acétamide; ou
le 2-[4-chloro-3-(3-chlorophénoxy)phényl]-N-phénylacétamide.

11. Composé de formule Ia selon la revendication 9, qui est
le 2-[4-chloro-3-(3-chloro-5-cyanophénoxy)-2-fluorophényl]-N-(2-chloro-4-sulfamoylphényl)acétamide;
le 2-[4-chloro-3-(3-chloro-5-cyanophénoxy)-2-fluorophényl]-N-(2-chloro-4-propionylsulfamoylphényl)acétamide; sel de sodium;
le 2-[3-(3-chloro-5-cyanophénoxy)-2-fluoro-4-méthylphényl]-N-(2-chloro-4-sulfamoylphényl)acétamide;
le 2-[4-bromo-3-(3-chloro-5-cyanophénoxy)-2-fluorophényl]-N-(2-chloro-4-sulfamoylphényl)acétamide;
le 2-[4-bromo-3-(3-chloro-5-cyanophénoxy)-2-fluorophényl]-N-(2-chloro-4-propionylsulfamoylphényl)acétamide; sel de sodium;
le N-(4-butyrylsulfamoyl-2-chlorophényl)-2-[4-chloro-3-(3-chloro-5-cyanophénoxyj-2-fluorophényl]acétamide; sel de sodium;
le N-[4-((S)-2-amino-3-méthylbutyrylsulfamoyl)-2-chlorophényl]-2-[4-chloro-3-(3-chloro-5-cyanophénoxy)-2-fluorophényl]acétamide; sel de sodium;
le N-(2-chloro-4-sulfamoylphényl)-2-[3-(3-cyano-5-difluorométhylphénoxy)-4-éthyl-2-fluorophényl]acétamide;
le 2-[3-(3-cyano-5-difluorométhylphénoxy)-4-éthyl-2-fluorophényl]-N-(2-méthyl-4-sulfamoylphényl)acétamide;
le 2-[4-bromo-3-(3-chloro-5-cyanophénoxy)-2-fluorophényl]-N-(2-méthyl-4-sulfamoylphényl)acétamide;
le 2-[4-bromo-3-(3-cyano-5-difluorométhylphénoxy)-2-fluorophényl]-N-(2-méthyl-4-sulfamoylphényl)acétamide;
le 2-[4-bromo-3-(3-cyano-5-difluorométhylphénoxy)-2-fluorophényl]-N-(2-chloro-4-sulfàmoylphényl)acétamide;
le 2-[4-chloro-3-(3-cyano-5-difluorométhylphénoxy)-2-fluorophényl]-N-(2-méthyl-4-sulfamoylphényl)acétamide;
le 2-[3-(3-chloro-5-cyanophénoxy)-2-fluoro-4-méthoxyphényl]-N-(2-chloro-4-sulfamoylphényl)acétamide;
le 2-[3-(3-chloro-5-cyanophénoxy)-2-fluoro-4-méthoxyphényl]-N-(2-méthyl-4-sulfamoylphényl)acétamide; et
le 2-[3-(3-chloro-5-cyanophénoxy)-2-fluoro-4-méthylphényl]-N-(2-chloro-4-propionylsulfamoylphényl)acétamide; sel de sodium.

12. Composé de formule la selon l'une quelconque des revendications 7 à 11, destiné à une utilisation comme médicament.

13. Utilisation du composé de formule Ia selon l'une quelconque des revendications 7 à 11 pour la fabrication d'un médicament destiné au traitement de maladies médiées par le virus de l'immunodéficience humaine (VIH).

14. Composition pharmaceutique comprenant une quantité pharmaceutiquement efficace d'un composé ou d'un de ses sels pharmaceutiquement acceptables tels que définis dans l'une quelconque des revendications 1 à 11 et un support pharmaceutique inerte.

15. Composition pharmaceutique selon la revendication 14, destiné à une utilisation dans le traitement de maladies médiées par le virus de l'immunodéficience humaine (VIH).
